(19) Europäisches Patentamt / European Patent Office / Office européen des brevets

(11) **EP 4 136 457 B1**

(12) **EUROPEAN PATENT SPECIFICATION**

(45) Date of publication and mention
of the grant of the patent:
**19.02.2025  Bulletin 2025/08**

(21) Application number: **21727275.6**

(22) Date of filing: **19.04.2021**

(51) International Patent Classification (IPC):
*G01N 33/569* (2006.01)    *A61K 39/205* (2006.01)
*A61K 39/215* (2006.01)    *C12N 15/86* (2006.01)

(52) Cooperative Patent Classification (CPC):
**G01N 33/56983; A61K 39/12; C07K 14/005;
C12N 15/86;** A61K 2039/5256; C12N 2760/20222;
C12N 2760/20243; C12N 2770/20022;
C12N 2770/20034; G01N 2333/145;
G01N 2333/165; G01N 2469/20

(86) International application number:
**PCT/US2021/027909**

(87) International publication number:
**WO 2021/212096 (21.10.2021 Gazette 2021/42)**

(54) **DETECTION ASSAYS FOR CORONAVIRUS NEUTRALIZING ANTIBODIES**

NACHWEISTESTS FÜR CORONAVIRUSNEUTRALISIERENDE ANTIKÖRPER

DOSAGES DE DÉTECTION POUR DES ANTICORPS NEUTRALISANT LE CORONAVIRUS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.04.2020   US 202063012074 P
17.04.2020   US 202063012066 P
05.05.2020   US 202063020445 P
22.05.2020   US 202063029267 P
14.09.2020   US 202063078214 P
19.02.2021   US 202163151623 P**

(43) Date of publication of application:
**22.02.2023   Bulletin 2023/08**

(73) Proprietors:
• **Regeneron Pharmaceuticals, Inc.
Tarrytown, NY 10591 (US)**
• **Vyriad, Inc.
Rochester, Minnesota 55901 (US)**

(72) Inventors:
• **RUSSELL, Stephen J.
Rochester, Minnesota 55901 (US)**
• **PENG, Kah Whye
Rochester, Minnesota 55901 (US)**
• **LECH, Patrycja
Rochester, Minnesota 55901 (US)**
• **VANDERGAAST, Rianna
Rochester, Minnesota 55901 (US)**
• **CAREY, Timothy
Rochester, Minnesota 55901 (US)**

(74) Representative: **Elkington and Fife LLP
Prospect House
8 Pembroke Road
Sevenoaks, Kent TN13 1XR (GB)**

(56) References cited:
**WO-A1-2021/168318       WO-A1-2021/211704**

• **RENQIANG LIU ET AL: "A recombinant VSV-
vectored MERS-CoVvaccine induces
neutralizing antibody and T cell responses in
rhesus monkeys after single dose
immunization", ANTIVIRAL RESEARCH, vol. 150,
12 December 2017 (2017-12-12), NL, pages 30 -
38, XP055681795, ISSN: 0166-3542, DOI: 10.1016/
j.antiviral.2017.12.007**

- LI HONGYUE ET AL: "Establishment of replication-competent vesicular stomatitis virus-based recombinant viruses suitable for SARS-CoV-2 entry and neutralization assays", EMERGING MICROBES & INFECTIONS, vol. 9, no. 1, 1 October 2020 (2020-10-01), pages 2269 - 2277, XP055841933, DOI: 10.1080/22221751.2020.1830715
- NIE JIANHUI ET AL: "Establishment and validation of a pseudovirus neutralization assay for SARS-CoV-2", EMERGING MICROBES & INFECTIONS, vol. 9, no. 1, 24 March 2020 (2020-03-24), pages 680 - 686, XP055818011, Retrieved from the Internet <URL:https://www.tandfonline.com/doi/pdf/10.1080/22221751.2020.1743767?needAccess=true> DOI: 10.1080/22221751.2020.1743767
- XIONG HUA-LONG ET AL: "Robust neutralization assay based on SARS-CoV-2 S-bearing vesicular stomatitis virus (VSV) pseudovirus and ACE2-overexpressed BHK21 cells", BIORXIV, 9 April 2020 (2020-04-09), XP055818007, Retrieved from the Internet <URL:https://www.biorxiv.org/content/10.1101/2020.04.08.026948v1.full.pdf> [retrieved on 20210625], DOI: 10.1101/2020.04.08.026948
- HOFFMANN MARKUS ET AL: "SARS-CoV-2 Cell Entry Depends on ACE2 and TMPRSS2 and Is Blocked by a Clinically Proven Protease Inhibitor", CELL, ELSEVIER, AMSTERDAM NL, vol. 181, no. 2, 5 March 2020 (2020-03-05), pages 271, XP086136225, ISSN: 0092-8674, [retrieved on 20200305], DOI: 10.1016/J.CELL.2020.02.052
- LESTER SANDRA ET AL: "Middle East respiratory coronavirus (MERS-CoV) spike (S) protein vesicular stomatitis virus pseudoparticle neutralization assays offer a reliable alternative to the conventional neutralization assay in human seroepidemiological studies", ACCESS MICROBIOLOGY, vol. 1, no. 9, 1 November 2019 (2019-11-01), XP055818130, Retrieved from the Internet <URL:https://www.microbiologyresearch.org/docserver/fulltext/acmi/1/9/acmi000057.pdf?expires=1624618030&id=id&accname=guest&checksum=990E4F090A9ECB5F84F772D7DB315DD3> DOI: 10.1099/acmi.0.000057
- FUKUSHI SHUETSU ET AL: "Evaluation of a novel vesicular stomatitis virus pseudotype-based assay for detection of neutralizing antibody responses to SARS-CoV", JOURNAL OF MEDICAL VIROLOGY, vol. 78, no. 12, 1 December 2006 (2006-12-01), US, pages 1509 - 1512, XP055818133, ISSN: 0146-6615, Retrieved from the Internet <URL:https://www.ncbi.nlm.nih.gov/pmc/articles/PMC7166816/pdf/JMV-78-1509.pdf> DOI: 10.1002/jmv.20732
- MIZUKI YAMAMOTO ET AL: "Identification of Nafamostat as a Potent Inhibitor of Middle East Respiratory Syndrome Coronavirus S Protein-Mediated Membrane Fusion Using the Split-Protein-Based Cell - Cell Fusion Assay", ANTIMICROBIAL AGENTS AND CHEMOTHERAPY, vol. 60, no. 11, 1 November 2016 (2016-11-01), US, pages 6532 - 6539, XP055536983, ISSN: 0066-4804, DOI: 10.1128/AAC.01043-16
- AVILOV S. V. ET AL: "Replication-Competent Influenza A Virus That Encodes a Split-Green Fluorescent Protein-Tagged PB2 Polymerase Subunit Allows Live-Cell Imaging of the Virus Life Cycle", JOURNAL OF VIROLOGY, vol. 86, no. 3, 23 November 2011 (2011-11-23), US, pages 1433 - 1448, XP055818035, ISSN: 0022-538X, DOI: 10.1128/JVI.05820-11
- VANDERGAAST RIANNA ET AL: "Development and validation of IMMUNO-COVTM: a highthroughput clinical assay for detecting antibodies that neutralize SARS-CoV-2", BIORXIV, 27 May 2020 (2020-05-27), XP055818020, Retrieved from the Internet <URL:https://www.biorxiv.org/content/10.1101/2020.05.26.117549v1.full.pdf> [retrieved on 20210625], DOI: 10.1101/2020.05.26.117549

Remarks:

The complete document including Reference Table(s) and the Sequence Listing(s) can be downloaded from the EPO website

**Description**

**TECHNICAL FIELD**

Described herein are methods for determining the presence of coronavirus neutralizing antibodies in a sample as well as associated compositions and kits. The methods use replication competent recombinant rhabdovirus particles, e.g., vesicular stomatitis virus (VSV) particles, wherein (i) the rhabdovirus glycoprotein (G), e.g., the VSV glycoprotein (G), is replaced by a Severe Acute Respiratory Syndrome coronavirus 2 (SARS-CoV-2) spike (S) glycoprotein or a fragment of the SARS-CoV-2 S glycoprotein comprising the receptor binding domain thereof, wherein said SARS-CoV-2 S glycoprotein or fragment is capable of mediating infection of a target cell, and wherein (ii) the rhabdovirus particle comprises a nucleic acid molecule comprising a nucleic acid sequence encoding a reporter protein, wherein the nucleic acid sequence encoding the reporter protein is inserted between a nucleic acid sequence encoding the SARS-CoV-2 S glycoprotein or fragment thereof, and a nucleic acid sequence encoding rhabdovirus large (L) protein . The methods can be used for determining the presence of SARS-CoV-2 neutralizing antibodies.

**BACKGROUND**

Three coronaviruses are known to cause severe pneumonia in humans: Severe Acute Respiratory Syndrome coronavirus (SARS-CoV or SARS-CoV-1), Middle East Respiratory Syndrome coronavirus (MERS-CoV), and Severe Acute Respiratory Syndrome coronavirus 2 (SARS-CoV-2). SARS-CoV-1 emerged in China in 2002 and spread to five continents infecting over 8,000 people and causing 774 deaths. MERS-CoV emerged in 2012 in the Arabian Peninsula infecting almost 2,500 people and causing 858 deaths in 27 countries. In December 2019, a new coronavirus emerged in Wuhan, China and caused an acute respiratory disease now known as coronavirus disease 2019 (COVID-19) (Zhou et al., Nature, published online February 3, 2020; available at doi.org/10.1038/s41586-020-2012-7; Zhu et al., New Engl J Med, 2020, 382:727-733). COVID-19 symptoms include fever, cough, shortness of breath, pneumonia, acute respiratory distress syndrome (ARDS), acute lung syndrome, loss of sense of smell, loss of sense of taste, sore throat, nasal discharge, gastro-intestinal symptoms (e.g., diarrhea), organ failure (e.g., kidney failure and renal dysfunction), septic shock and death in severe cases. The virus causing COVID-19 was identified to be related to SARS-CoV-1 and thus was named SARS-CoV-2 (also sometimes referenced as nCov-2019, Wuhan coronavirus, or SARS nCoV19). While some individuals are asymptomatic or experience only mild illness, many experience severe symptoms and require hospitalization. SARS-CoV-2 spread quickly around the globe and was declared a global pandemic on March 11, 2020 by the World Health Organization. As of May 20, 2020, the virus has infected almost 5 million people worldwide causing more than 328,000 deaths. Many of the patients who develop COVID-19 have mild upper respiratory symptoms, but some (especially older people and people with underlying medical conditions such as chronic lung disease, asthma, heart conditions, diabetes, immunocompromised patients, etc.) develop atypical pneumonia and its associated complications (Wölfel et al., Nature, published online on April 1, 2020, available at doi.org/10.1038/s41586-020-2196-x). SARS-CoV-2 is highly contagious and can be spread by asymptomatic carriers.

PCR assays that detect active SARS-CoV-2 infection are playing an important role in tracking disease spread, while serological tests that detect antibodies against SARS-CoV-2 are being used to detect and measure previous infections, identify individuals who are likely immune to SARS-CoV-2 and evaluate the efficacy of vaccines and therapies. How well the available serological tests accurately reflect antibody-mediated immunity is still poorly understood.

Generation of virus-neutralizing antibodies is essential for blocking subsequent viral infections, and the presence of neutralizing antibodies correlates with protective immunity following vaccination (Koff 2013). Yet typically, only a small subset of virus-specific antibodies are neutralizing. It is not currently understood how total antibody levels relate to neutralizing antibody levels for SARS-CoV-2, and improved testing of SARS-CoV-2 neutralizing antibody responses is urgently needed. Early data from convalescent plasma therapy trials have shown promising results (Casadevall 2020), but screening of plasma donors is inadequate if acceptance criteria are based on total anti-SARS2-CoV-2 antibody levels and not specifically on neutralizing antibody levels. Likewise, efficacy studies of new SARS-CoV-2 vaccines must consider not only total antibody responses, but neutralizing antibody responses.

Thus, there is a great need in the art for a potent and efficient seropositivity assay for SARS-CoV-2 and other coronaviruses, especially for assays assessing virus neutralizing antibodies.

Nie et al. (Emerg Microbes Infect. 2020; 9(1): 680-686) discloses a VSV pseudovirus-based neutralization assay for detecting the presence of SARS-CoV-2 neutralizing antibodies in convalescing patients.

**SUMMARY**

As specified in the Background section, above, there is a great need for development of effective seropositivity assays for SARS-CoV-2 and other coronaviruses, especially for assays assessing virus neutralizing antibodies. The present disclosure addresses these and other needs. A replication competent recombinant rhabdovirus particle is provided, wherein (i) the rhabdovirus glycoprotein (G) is replaced by a Severe Acute Respiratory Syndrome coronavirus 2 (SARS-CoV-2) spike (S) glycoprotein or a fragment of the SARS-CoV-2 S glycoprotein comprising the receptor binding domain thereof, wherein said SARS-CoV-2 S glycoprotein or fragment is capable of mediating infection of a target cell, and wherein (ii) the rhabdovirus particle comprises a nucleic acid molecule comprising a nucleic acid sequence encoding a reporter protein, wherein the nucleic acid sequence encoding the reporter protein is inserted between a nucleic acid sequence encoding the SARS-CoV-2 S glycoprotein or fragment thereof, and a nucleic acid sequence encoding rhabdovirus large (L) protein. The present disclosure also provides assays for determining the presence of coronavirus neutralizing antibodies in a sample as well as associated compositions and kits. The seropositivity assays of the disclosure use replication competent recombinant VSV particles as described above. The methods are used for determining the presence of SARS-CoV-2 neutralizing antibodies.

In one aspect, provided herein a method for determining the presence of a SARS-CoV-2 neutralizing antibody in a sample, the method comprising:

a) contacting the sample with a replication competent recombinant rhabdovirus particle wherein (i) the rhabdovirus glycoprotein (G) is replaced by a Severe Acute Respiratory Syndrome coronavirus 2 (SARS-CoV-2) spike (S) glycoprotein or a fragment of the SARS-CoV-2 S glycoprotein comprising the receptor binding domain thereof, wherein said SARS-CoV-2 S glycoprotein or fragment is capable of mediating infection of a target cell and wherein (ii) the rhabdovirus particle comprises a nucleic acid molecule comprising a nucleic acid sequence encoding a reporter protein, wherein the nucleic acid sequence encoding the reporter protein is inserted between a nucleic acid sequence encoding the SARS-CoV-2 S glycoprotein or fragment thereof, and a nucleic acid sequence encoding rhabdovirus large (L) protein;
b) after step (a), contacting the replication competent recombinant rhabdovirus particle with the target cell;
c) measuring the reporter signal in the cell after step (b), and
d) comparing the reporter signal measured in step (c) with a control.

In one embodiment, the target cell is a Vero cell (including Vero-$\alpha$His cell), Vero-Ace-2 cell, Vero-TRMPSS2 cell, or Vero-E6 cell.

In one embodiment, the reporter protein comprises a luciferase. Non-limiting examples of useful luciferase include, e.g., *Renilla* luciferase, RLuc8 mutant *Renilla* luciferase, (dCpG)Luciferase, NanoLuc reporter, firefly luciferase, *Gaussia* luciferase (gLuc), MetLuc, *Vibrio fischeri* lumazine protein, *Vibrio harveyi* luminaze protein, inoflagellate luciferase, firefly luciferase YY5 mutant, firefly luciferase LGR mutant, firefly luciferase mutant E, and fragments or derivatives thereof.

In one embodiment, the reporter protein comprises a fluorescent protein. Non-limiting examples of useful fluorescent proteins include, e.g., green fluorescent protein (GFP), GFP-like fluorescent proteins, (GFP-like), enhanced green fluorescent protein (EGFP), yellow fluorescent protein (YFP), enhanced yellow fluorescent protein (EYFP), blue fluorescent protein (BFP), enhanced blue fluorescent protein (EBFP), cyan fluorescent protein (CFP), enhanced cyan fluorescent protein (ECFP); red fluorescent protein, superfolder GFP, superfolder YFP, orange fluorescent protein, red fluorescent protein, small ultrared fluorescent protein, FMN-binding fluorescent protein, dsRed, qFP611, Dronpa, TagRFP, KFP, EosFP, IrisFP, Dendra, Kaede, KikGr1, emerald fluorescent protein, Azami Green, mWasabi, TagGFP, TurboGFP, AcGFP, ZsGreen, T-Sapphire, and fragments or derivatives thereof.

In one embodiment, the method comprises adding a reporter protein substrate for obtaining the reporter signal. Non-limiting examples of useful reporter protein substrates for luciferases include, e.g., Luciferin (e.g., d-luciferin), EnduRen, and coelenterazine luciferase substrates.

In one embodiment, the SARS-CoV-2 S glycoprotein is a full-length SARS-CoV-2 S glycoprotein. In one specific embodiment, the SARS-CoV-2 S glycoprotein comprises the amino acid sequence of SEQ ID NO: 1. In one specific embodiment, the SARS-CoV-2 S glycoprotein consists of the amino acid sequence of SEQ ID NO: 1. In another embodiment, the SARS-CoV-2 S glycoprotein fragment is a SARS-CoV-2 S glycoprotein fragment lacking 19 C-terminal amino acids. In one specific embodiment, the SARS-CoV-2 S glycoprotein fragment comprises the amino acid sequence of SEQ ID NO: 3. In one specific embodiment, the SARS-CoV-2 S glycoprotein fragment consists of the amino acid sequence of SEQ ID NO: 3. In one embodiment, the SARS-CoV-2 S glycoprotein fragment has at least 77% amino acid sequence identity to the amino acid sequence of SEQ ID NO: 1. In one embodiment, the SARS-CoV-2 S glycoprotein fragment has at least 64% amino acid sequence identity to S1 subunit of the amino acid sequence of SEQ ID NO: 1. In one embodiment, the SARS-CoV-2 S glycoprotein fragment has at least 64% amino acid sequence identity to amino acids 14-684 of the amino acid sequence of SEQ ID NO: 1. In one embodiment, the SARS-CoV-2 S glycoprotein fragment has at least 74% amino acid sequence identity to RBD domain of the amino acid sequence of SEQ ID NO: 1. In one embodiment, the SARS-CoV-2 S glycoprotein fragment has at least 74% amino acid sequence identity to amino acids 319-541 of the amino acid sequence of SEQ ID NO: 1. In one embodiment, the coronavirus S glycoprotein fragment results in a more fusogenic recombinant rhabdovirus particle as compared to a comparable recombinant rhabdovirus comprising the rhabdovirus genome expressing a full-length wild-type SARS-CoV-2 S glycoprotein inserted in the same location of the rhabdovirus genome. In certain embodiments, the SARS-CoV-2 S glycoprotein, or fragment thereof, may comprise or consist of an insertion, deletion, and/or substitution of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, or 39 residues of the SARS-CoV-2 S glycoprotein. Non-limiting examples of amino acids for potential deletion include, e.g., a tyrosine at position (145), an asparagine at position (679), a serine at position (680), proline at position (681), an arginine at position (682), an arginine at position (683), an alanine at position (684), and/or an arginine at position (685), positions as denoted in SEQ ID NO: 1, or the equivalent amino acid residue in a mutant SARS-CoV-2 S glycoprotein sequence. Non-limiting examples of amino acids for potential substitution include, e.g., a leucine changed to a phenylalanine at position (5) a tyrosine changed to an asparagine at position (28), a threonine changed to an isoleucine at position (29), a histidine changed to a tyrosine at position (49), a leucine changed to a phenylalanine at position (54), an asparagine changed to a lysine at position (74), a glutamic acid changed to an aspartic acid at position (96), an aspartic acid changed to an asparagine at position (111), a phenylalanine changed to a leucine at position (157), a glycine changed to a valine at position (181), a serine changed to a tryptophan at position (221), a serine changed to an arginine at position (247), an alanine changed to a threonine at position (348), an arginine changed to an isoleucine at position (408), a glycine changed to a serine at position (476), a valine changed to an alanine at position (483), a histidine changed to a glutamine at position (519), an alanine changed to a serine at position (520), an aspartic acid changed to an asparagine at position (614), an aspartic acid changed to a glycine at position (614), an asparagine changed to an isoleucine at position (679), a serine change to a leucine at position (680), an arginine changed to a glycine at position (682), an arginine changed to a serine at position (683), an arginine changed to a glutamine at position (685), an arginine changed to a serine at position (685), a phenylalanine changed to a cysteine at position (797), an alanine changed to a valine at position (930), an aspartic acid changed to a tyrosine at position (936), an alanine changed to a valine at position (1078), an aspartic acid changed to a histidine at position (1168), and/or an aspartic acid changed to a histidine at position (1259), positions as denoted in SEQ ID NO: 1, or the equivalent amino acid residue in a mutant SARS-CoV-2 S glycoprotein sequence. *See* Becerra-Flores and Cardozo, "SARS-CoV-2 viral spike G614 mutation exhibits higher case fatality rate," The International Journal of Clinical Practice, published online May 6, 2020; Eaaswarkhanth et al., "Could the D614G substitution in the SARS-CoV-2 spike (S) protein be associated with higher COVID-19 mortality?" International Journal of Infectious Diseases, 96: July 2020, Pages 459-460; Tang et al., "The SARS-CoV-2 Spike Protein D614G Mutation Shows Increasing Dominance and May Confer a Structural Advantage to the Furin Cleavage Domain," Preprints 2020, 2020050407 (doi: 10.20944/preprints202005.0407.v1); Hansen et. al., "Studies in humanized mice and convalescent humans yield a SARS-CoV-2 antibody cocktail" Science, published online June 15, 2020; Lokman et al., "Exploring the genomic and proteomic variations of SARS-CoV-2 spike glycoprotein: A computational biology approach", Infection, Genetics and Evolution : Journal of Molecular Epidemiology and Evolutionary Genetics in Infectious Diseases, 2020 Jun;84:104389. DOI: 10.1016/j.meegid.2020.104389. Additional non-limiting examples of amino acid residue positions for insertion, deletion, and/or substitution include those as listed in Tables 8 and 9 (amino acid residue positions are denoted using SEQ ID NO: 1 as a reference sequence, which can be used as a reference for identifying the equivalent amino acid residue in any SARS-CoV-2 S glycoprotein sequence (same as above)). Each residue modification listed in Table 8 can separately be used alone or in combination with others to generate variants of a recombinant vesicular stomatitis virus (VSV) particle. In certain embodiments, the SARS-CoV-2 S glycoprotein, or fragment thereof, differs in amino acid sequence from the reference peptide or polypeptide (e.g., wild-type SARS-CoV-2 spike protein) by changing a serine to an arginine at position (247), an aspartic acid to an asparagine at position (614), and/or an arginine to a glutamine at position (685), positions as denoted in SEQ ID NO: 1, or the equivalent amino acid residue in a mutant SARS-CoV-2 S glycoprotein sequence. In certain embodiments, the SARS-CoV-2 S glycoprotein, or fragment thereof, differs in amino acid sequence from the reference peptide or polypeptide by changing a serine to an arginine at position (247). In certain embodiments, the SARS-CoV-2 S glycoprotein, or fragment thereof, differs in amino acid sequence from the reference peptide or polypeptide by changing an aspartic acid to an asparagine at position (614). In certain embodiments, the SARS-CoV-2 S glycoprotein, or fragment thereof, differs in amino acid sequence from the reference peptide or polypeptide by changing an arginine to a glutamine at position (685). In certain embodiments, the SARS-CoV-2 S glycoprotein, or fragment thereof, differs in amino acid sequence from the reference peptide or polypeptide by changing a serine to an arginine at position (247) and an aspartic acid to an asparagine at position (614). In certain embodiments, the SARS-CoV-2 S glycoprotein, or fragment thereof, differs in amino acid sequence from the reference peptide or polypeptide by changing a serine to an arginine at position (247) and an arginine to a glutamine at position (685). In certain embodiments, the SARS-CoV-2 S glycoprotein, or fragment thereof, differs in amino acid sequence from the reference peptide or polypeptide by changing an aspartic acid to an asparagine at position (614) and an arginine to a glutamine at position (685). In certain embodiments, the SARS-CoV-2 S glycoprotein, or fragment thereof, differs in amino acid sequence from the reference peptide or polypeptide by changing a serine to an arginine at position (247), an aspartic acid to an asparagine at position (614), and an arginine to a glutamine at position (685). In certain embodiments, the SARS-CoV-2 S glycoprotein, or fragment thereof, result in a more lytic phenotype. In certain embodiments, the SARS-CoV-2 S glycoprotein fragment may comprise the amino acid sequence of SEQ ID NO: 42, or a sequence at least 50%, 60%, 70%, 80%, 81%, 82%, 83%, 84%, 85%, 86%, 87%, 88%, 89%, 90%, 91%, 92%, 93%, 94%, 95%, 96%, 97%, 98%, 99%, or 99.5% identical to the amino acid sequence of SEQ ID NO: 42. In certain embodiments, the SARS-CoV-2 S glycoprotein fragment may be encoded by a codon optimized nucleotide sequence. In various embodiments, SARS-CoV-2 S glycoprotein fragment may be encoded by the polynucleotide sequence of SEQ ID NO: 43 or a sequence that has at least 50%, 60%, 70%, 80%, 81%, 82%, 83%, 84%, 85%, 86%, 87%, 88%, 89%, 90%, 91%, 92%, 93%, 94%, 95%, 96%, 97%, 98%, 99%, or 99.5% polynucleotide sequence identity to SEQ ID NO: 43. In certain embodiments, the SARS-CoV-2 S glycoprotein fragment may comprise the amino acid sequence of SEQ ID NO: 44, or a sequence at least 50%, 60%, 70%, 80%, 81%, 82%, 83%, 84%, 85%, 86%, 87%, 88%, 89%, 90%, 91%, 92%, 93%, 94%, 95%, 96%, 97%, 98%, 99%, or 99.5% identical to the amino acid sequence of SEQ ID NO: 44. In certain embodiments, the SARS-CoV-2 S glycoprotein, or fragment thereof, differs in amino acid sequence from the reference peptide or polypeptide ( a wild-type SARS-CoV-2 spike protein) by changing an asparagine to a tyrosine at position (501), and/or a glutamic acid to a lysine at position (484), and/or an aspartic acid to a glycine at position (614), and/or deletion of residues 69-70, positions as denoted in SEQ ID NO: 1, or the equivalent amino acid residue in a mutant SARS-CoV-2 S glycoprotein sequence. In certain embodiments, the SARS-CoV-2 S glycoprotein, or fragment thereof, differs in amino acid sequence from the reference peptide or polypeptide by changing an asparagine to a tyrosine at position (501). In certain embodiments, the SARS-CoV-2 S glycoprotein, or fragment thereof, differs in amino acid sequence from the reference peptide or polypeptide by changing a glutamic acid to a lysine at position (484). In certain embodiments, the SARS-CoV-2 S glycoprotein, or fragment thereof, differs in amino acid sequence from the reference peptide or polypeptide by changing an aspartic acid to a glycine at position (614). In certain embodiments, the SARS-CoV-2 S glycoprotein, or fragment thereof, differs in amino acid sequence from the reference peptide or polypeptide by deletion of residues 69-70. In certain embodiments, the SARS-CoV-2 S glycoprotein, or fragment thereof, differs in amino acid sequence from the reference peptide or polypeptide by changing an asparagine to a tyrosine at position (501) and a glutamic acid to a lysine at position (484). In certain embodiments, the SARS-CoV-2 S glycoprotein, or fragment thereof, differs in amino acid sequence from the reference peptide or polypeptide by changing an asparagine to a tyrosine at position (501) and an aspartic acid to a glycine at position (614). In certain embodiments, the SARS-CoV-2 S glycoprotein, or fragment thereof, differs in amino acid sequence from the reference peptide or polypeptide by changing an asparagine to a tyrosine at position (501) and deletion of residues 69-70. In certain embodiments, the SARS-CoV-2 S glycoprotein, or fragment thereof, differs in amino acid sequence from the reference peptide or polypeptide by changing a glutamic acid to a lysine at position (484) and an aspartic acid to a glycine at position (614). In certain embodiments, the SARS-CoV-2 S glycoprotein, or fragment thereof, differs in amino acid sequence from the reference peptide or polypeptide by changing a glutamic acid to a lysine at position (484) and deletion of residues 69-70. In certain embodiments, the SARS-CoV-2 S glycoprotein, or fragment thereof, differs in amino acid sequence from the reference peptide or polypeptide by changing an aspartic acid to a glycine at position (614) and deletion of residues 69-70. In certain embodiments, the SARS-CoV-2 S glycoprotein, or fragment thereof, differs in amino acid sequence from the reference peptide or polypeptide by changing an asparagine to a tyrosine at position (501), a glutamic acid to a lysine at position (484), and an aspartic acid to a glycine at position (614). In certain embodiments, the SARS-CoV-2 S glycoprotein, or fragment thereof, differs in amino acid sequence from the reference peptide or polypeptide by changing an asparagine to a tyrosine at position (501), changing a glutamic acid to a lysine at position (484), and deletion of residues 69-70. In certain embodiments, the SARS-CoV-2 S glycoprotein, or fragment thereof, differs in amino acid sequence from the reference peptide or polypeptide by changing an asparagine to a tyrosine at position (501), changing an aspartic acid to a glycine at position (614), and deletion of residues 69-70. In certain embodiments, the SARS-CoV-2 S glycoprotein, or fragment thereof, differs in amino acid sequence from the reference peptide or polypeptide by changing a glutamic acid to a lysine at position (484), changing an aspartic acid to a glycine at position (614), and deletion of residues 69-70. In certain embodiments, the SARS-CoV-2 S glycoprotein, or fragment thereof, differs in amino acid sequence from the reference peptide or polypeptide by changing an asparagine to a tyrosine at position (501), changing a glutamic acid to a lysine at position (484), changing an aspartic acid to a glycine at position (614) and deletion of residues 69-70. In certain embodiments, the SARS-CoV-2 S glycoprotein, or fragment thereof, differs in amino acid sequence from the reference peptide or polypeptide (a wild-type SARS-CoV-2 spike protein) by inactivating the furin cleavage site within the spike protein. In certain embodiments, the SARS-CoV-2 S glycoprotein, or fragment thereof, differs in amino acid sequence from the reference peptide or polypeptide (a wild-type SARS-CoV-2 spike protein) by changing $Q^{677}$TNSPRRARSV$^{687}$ (SEQ ID NO: 65), as denoted in SEQ ID NO: 1, or the equivalent amino acid residue in a mutant SARS-CoV-2 S glycoprotein sequence, to QTII,RSV (SEQ ID NO: 66) or to QTNSPGSASSV (SEQ ID NO: 67). In certain embodiments, the SARS-CoV-2 S glycoprotein, or fragment thereof, result in a monobasic furin cleavage site in the S1/S2 interface (QTII,RSV (SEQ ID NO: 66)) or deletion of the furin cleavage site (QTNSPGSASSV (SEQ ID NO: 67)) phenotype. In certain embodiments, the alteration to the furin cleavage site can lead to a spike stabilized pseudoparticles. *See* Hansen et. al., "Studies in humanized mice and convalescent humans yield a SARS-CoV-2 antibody cocktail" Science, published online June 15, 2020.

In certain embodiments of any of the above methods, step (a) comprises contacting the sample (e.g., simultaneously or sequentially) with two or more different recombinant rhabdovirus particles, wherein said two or more different replication competent recombinant rhabdovirus particles comprise different SARS-CoV-2 S glycoproteins or fragments thereof comprising the receptor binding domain, wherein said SARS-CoV-2 S glycoproteins or fragments are capable of mediating infection of a target cell. In certain embodiments, at least one of the two or more different SARS-CoV-2 S glycoproteins, or fragments thereof comprises the amino acid sequence selected from SEQ ID NO: 1, SEQ ID NO: 3 and SEQ ID NO: 44, or comprises one or more amino acid insertions, deletions, and/or substitutions listed in Tables 8 and 9, wherein the positions of said insertions, deletions, and/or substitutions are specified in relation to SEQ ID NO: 1. In certain embodiments, the two or more different replication competent recombinant rhabdovirus particles comprise different nucleic acid sequences encoding different reporter proteins. In certain embodiments, the two or more different recombinant rhabdovirus particles comprise the same nucleic acid sequences encoding said reporter protein.

In one embodiment, provided herein is a method for determining the presence of a Severe Acute Respiratory Syndrome coronavirus 2 (SARS-CoV-2) neutralizing antibody in a sample, the method comprising:

a) contacting the sample with a replication competent recombinant vesicular stomatitis virus (VSV) particle as described above, wherein (i) the genome of the recombinant VSV particle lacks a functional VSV glycoprotein (G) gene and encodes the full-length SARS-CoV-2 spike (S) glycoprotein or a fragment thereof lacking 19 C-terminal amino acids and wherein (ii) the genome of the recombinant VSV particle further encodes a luciferase protein;
b) after step (a), contacting the recombinant VSV particle with a cell selected from Vero cell, Vero-Ace-2 cell and Vero-E6 cell;
c) measuring the luciferase signal in the cell after step (b), and
d) comparing the signal measured in step (c) with a control.

In one embodiment, the full-length SARS-CoV-2 S glycoprotein consists of the amino acid sequence of SEQ ID NO: 1. In one embodiment of the above two methods, the SARS-CoV-2 S glycoprotein fragment lacking 19 C-terminal amino acids consists of the amino acid sequence of SEQ ID NO: 3.

In certain embodiments, step (a) comprises contacting the sample (e.g., simultaneously or sequentially) with two or more different recombinant VSV particles, wherein said two or more different recombinant VSV particles comprise different full-length SARS-CoV-2 S glycoproteins or fragments thereof lacking 19 C-terminal amino acids. In certain embodiments, at least one of the two or more different full-length SARS-CoV-2 S glycoproteins or fragments thereof lacking 19 C-terminal amino acids comprises the amino acid sequence selected from SEQ ID NO: 1, SEQ ID NO: 3 and SEQ ID NO: 44, or comprises one or more amino acid insertions, deletions, and/or substitutions listed in Tables 8 and 9, wherein the positions of said insertions, deletions, and/or substitutions are specified in relation to SEQ ID NO: 1. In certain embodiments of the methods, the two or more different recombinant VSV particles comprise different nucleic acid sequences encoding different reporter proteins. In certain embodiments of the methods, the two or more different recombinant VSV particles comprise the same nucleic acid sequences encoding said reporter protein.

In one embodiment of any of the above methods, after step (b) and before step (c) cells are exposed to trypsin.

In one embodiment, the replication competent recombinant rhabdovirus particle comprises a mutant rhabdovirus matrix (M) protein. In one embodiment, the genome of the recombinant rhabdovirus particle encodes a mutant rhabdovirus M protein. In one embodiment, the recombinant rhabdovirus particle is a recombinant VSV particle comprising the mutant VSV M protein which comprises a mutation at methionine 51. In one specific embodiment, the mutation at methionine 51 is from methionine (M) to arginine (R). In one specific embodiment, the mutant VSV M protein comprises the amino acid sequence of SEQ ID NO: 7. In one specific embodiment, the mutant VSV M protein consists of the amino acid sequence of SEQ ID NO: 7.

In one embodiment, the recombinant rhabdovirus particle comprises a wild-type rhabdovirus matrix (M) protein. In one embodiment, the genome of the recombinant rhabdovirus particle encodes a wild-type rhabdovirus M protein. In one embodiment, the recombinant rhabdovirus particle is a recombinant VSV particle comprising the wild-type VSV M protein which comprises the amino acid sequence of SEQ ID NO: 9. In one specific embodiment, the wild-type VSV M protein consists of the amino acid sequence of SEQ ID NO: 9.

In one embodiment of any of the above methods, the sample is serum or plasma. In one embodiment of any of the above methods, the sample is saliva. In one embodiment of any of the above methods, the sample is a dried bloodspot. In one embodiment, the method further comprises diluting the sample by a factor of about 1:10 to about 1:320. For example, the sample may be diluted by a factor of about 1:10, about 1:16, about 1:20, about 1:32, about 1:64, about 1:80, about 1:100, about 1:128, or about 1:160. In one embodiment the method further comprises diluting the sample by a factor of about 1:100. In one embodiment the method further comprises diluting the sample by a factor of about 1:20. In some embodiments, the method further comprises diluting the recombinant rhabdovirus particle to about 200-800 pfu/well. For example, the recombinant rhabdovirus particle may be diluted to about 200, about 300, about 400, about 500, about 600, about 700, about 720, or about 800 pfu/well. In one embodiment, the sample is heat-inactivated. In another embodiment, the sample is not heat-inactivated. In one embodiment, the method further comprises treating the sample with an antibiotic and/or filtering the sample to prevent bacterial contamination.

In one embodiment of any of the above methods, the control is the reporter signal obtained with a control sample not comprising the coronavirus neutralizing antibodies, and the method comprises concluding that the tested sample comprises the coronavirus neutralizing antibodies when the reporter signal obtained in step (c) is reduced as compared to the control. In one embodiment, the method comprises concluding that the tested sample comprises SARS-CoV-2 neutralizing antibodies when the reporter signal obtained in step (c) is reduced by more than 50% as compared to the control. In one embodiment, the method further comprises comparing the reporter signal obtained in step (c) with the reporter signal obtained with a control sample comprising a SARS-CoV-2 neutralizing antibody, or a molecule that blocks interaction of the SARS-CoV-2 S glycoprotein with a protein with which it interacts on the target cell, or a molecule that blocks target cell fusion, or any combination thereof. In one embodiment, the method comprises determining the concentration of SARS-CoV-2 neutralizing antibodies in the tested sample by comparing the reporter signal to a calibration curve determined from a serial dilution of the control sample comprising a SARS-CoV-2 neutralizing antibody, or a molecule that blocks interaction of the SARS-CoV-2 S glycoprotein with a protein with which it interacts on the target cell, or any combination thereof. In one specific embodiment, the control sample comprises mAb10914. In one specific embodiment, the serial dilution of the control sample comprises about 0.01 μg/mL to about 3 μg/mL mAb10914. In one specific embodiment, the control sample comprises mAb10922. In one specific embodiment, the serial dilution of the control sample comprises about 0.01 μg/mL to about 3 μg/mL mAb10922.

In one embodiment of any of the above methods, the report signal has been corrected to remove background signal measured from a control sample not contacted with the recombinant rhabdovirus particle.

In one embodiment of any of the above methods, the reporter signal is measured between about 18 to 30 hours after step (b). In one embodiment of any of the above methods, the reporter signal is measured between about 24 to 30 hours after step (b).

In one embodiment of any of the above methods, in step (a) the sample is contacted with the recombinant rhabdovirus particle for about 30 minutes at room temperature.

In one embodiment of any of the above methods, the method is conducted in a high throuput format. In one specific embodiment, the method is conducted in a 96-well plate. In one specific embodiment, when the method is conducted in a 96-well plate, density of the first target cell and the second target cell is about $6 \times 10^4$ cells/well.

In one embodiment, the recombinant rhabdovirus particle is a recombinant vesiculovirus particle. In one embodiment, the recombinant vesiculovirus particle is a recombinant vesicular stomatitis virus (VSV) particle.

In another aspect, provided herein is a polynucleotide encoding the replication competent recombinant rhabdovirus particle described above, wherein the polynucleotide comprises a nucleic acid sequence encoding a rhabdovirus nucleoprotein (N), a nucleic acid sequence encoding a rhabdovirus phosphoprotein (P), and the nucleic acid sequence encoding the rhabdovirus large protein (L), encoding the SARS-CoV-2 S glycoprotein or fragment for expression on the viral envelope of a recombinant rhabdovirus particle, and the nucleic acid sequence encoding the reporter protein inserted as specified above.

In one embodiment, the polynucleotide further comprises a Kozak sequence 3' to the sequence encoding SARS-CoV-2 S glycoprotein or a fragment thereof. In one embodiment, the Kozak sequence is a wild-type Kozak sequence. In one specific embodiment, the wild-type Kozak sequence comprises SEQ ID NO: 11. In one embodiment, the Kozak sequence is an optimized Kozak sequence. In one specific embodiment, the optimized Kozak sequence comprises SEQ ID NO: 12.

In another aspect, provided herein is a kit for determining the presence of a coronavirus neutralizing antibody in a sample comprising:

> a) a replication competent recombinant rhabdovirus particle wherein (i) the rhabdovirus glycoprotein (G) is replaced by a Severe Acute Respiratory Syndrome coronavirus 2 (SARS-CoV-2) spike (S) glycoprotein or a fragment of the SARS-CoV-2 S glycoprotein comprising the receptor binding domain thereof, wherein said SARS-CoV-2 S glycoprotein or fragment is capable of mediating infection of a target cell and wherein (ii) the recombinant rhabdovirus particle comprises a nucleic acid molecule comprising a nucleic acid sequence encoding a reporter protein, wherein the nucleic acid sequence encoding the reporter protein is inserted between a nucleic acid sequence encoding the SARS-CoV-2 S glycoprotein or fragment thereof, and a nucleic acid sequence encoding rhabdovirus large (L) protein;
> b) a target cell;
> c) optionally, a control sample not comprising the coronavirus neutralizing antibodies;
> d) optionally, a control sample comprising a SARS-CoV-2 neutralizing antibody, or a molecule that blocks interaction of the SARS-CoV-2 S glycoprotein with a protein with which it interacts on the target cell, or any combination thereof;
> e) optionally, a substrate for the reporter protein, and
> f) optionally, instructions for use.

In a further aspect, provided herein is a kit for determining the presence of a coronavirus neutralizing antibody in a sample comprising:

a) a polynucleotide encoding the replication competent recombinant rhabdovirus particle as described above, wherein the polynucleotide comprises a nucleic acid sequence encoding a rhabdovirus nucleoprotein (N), a nucleic acid sequence encoding a rhabdovirus phosphoprotein (P), a the nucleic acid sequence encoding the rhabdovirus large protein (L), the nucleic acid sequence encoding the SARS-CoV-2 S glycoprotein or fragment thereof, for expression on the viral envelope of a recombinant rhabdovirus particle, and the nucleic acid sequence encoding the reporter protein inserted as specified above;

b) a target cell;

c) optionally, a control sample not comprising the coronavirus neutralizing antibodies;

d) optionally, a control sample comprising a SARS-CoV-2 neutralizing antibody, or a molecule that blocks interaction of the SARS-CoV-2 S glycoprotein with a protein with which it interacts on the target cell, or any combination thereof;

e) optionally, a substrate for the reporter protein, and

f) optionally, instructions for use.

In one embodiment, the target cell is a Vero cell (including Vero-αHis cell), Vero-Ace-2 cell, Vero-TRMPSS2 cell, or Vero-E6 cell.

In one specific embodiment of any of the above kits, the reporter protein comprises a luciferase and the substrate for the reporter protein comprises Luciferin (e.g., d-luciferin), coelenterazine, or EnduRen luciferase substrate.

In one specific embodiment of any of the above kits, the control sample comprising a coronavirus neutralizing antibody is a control sample comprising mAb10914. In one specific embodiment of any of the above kits, the control sample comprising a coronavirus neutralizing antibody is a control sample comprising mAb 10922.

These and other aspects described herein will be apparent to those of ordinary skill in the art in the following description and drawings.

## BRIEF DESCRIPTION OF THE DRAWINGS

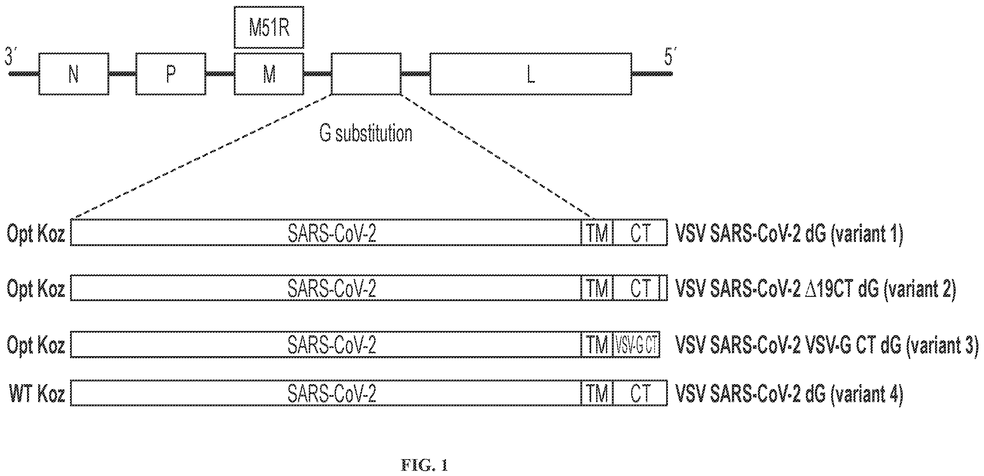

**Figure 1** depicts the VSV SARS-CoV-2 constructs tested in the Examples (variants 1-4). In these constructs, the VSV G glycoprotein was substituted by: (1) the full length SARS-CoV-2 spike (S) glycoprotein sequence (variant 1; VSV SARS-CoV-2 dG = VSV-SARS-CoV-2-S; amino acid sequence SEQ ID NO: 1; codon-optimized nucleotide sequence SEQ ID NO: 2), (2) SARS-CoV-2 S glycoprotein sequence with a deletion of 19 amino acids KFDEDDSEPVLKGVKLHYT (SEQ ID NO: 20) in the cytoplasmic tail (variant 2; VSV SARS-CoV-2 Δ19CT dG = VSV-SARS-CoV-2-S-Δ19CT; amino acid sequence SEQ ID NO: 3; codon-optimized nucleotide sequence SEQ ID NO: 4), (3) SARS-CoV-2 S glycoprotein sequence with a replacement of the S cytoplasmic tail with VSV G glycoprotein cytoplasmic tail sequence KLKHTKKRQIYTDIEMNRLGK (SEQ ID NO: 21) (variant 3; VSV SARS-CoV-2 VSV-G CT dG; amino acid sequence SEQ ID NO: 5; codon-optimized nucleotide sequence SEQ ID NO: 6), or (4) the full length SARS-CoV-2 S glycoprotein sequence but using the wild-type VSV Kozak sequence (cActATG; SEQ ID NO: 11) in place of the optimized Kozak sequence (caccATG; SEQ ID NO: 12) used in the other three constructs (variant 4; VSV-SARS-CoV-2-S; amino acid sequence SEQ ID NO: 1; codon-optimized nucleotide sequence SEQ ID NO: 2). One set of variant 1-4 constructs was prepared that encoded wild-type M protein (amino acid sequence SEQ ID NO: 9; nucleotide sequence SEQ ID NO: 10). A second set of variant 1-4 constructs was prepared that encoded M protein with the substitution M51R (amino acid sequence SEQ ID NO: 7; nucleotide sequence SEQ ID NO: 8) resulting in virus attenuation.

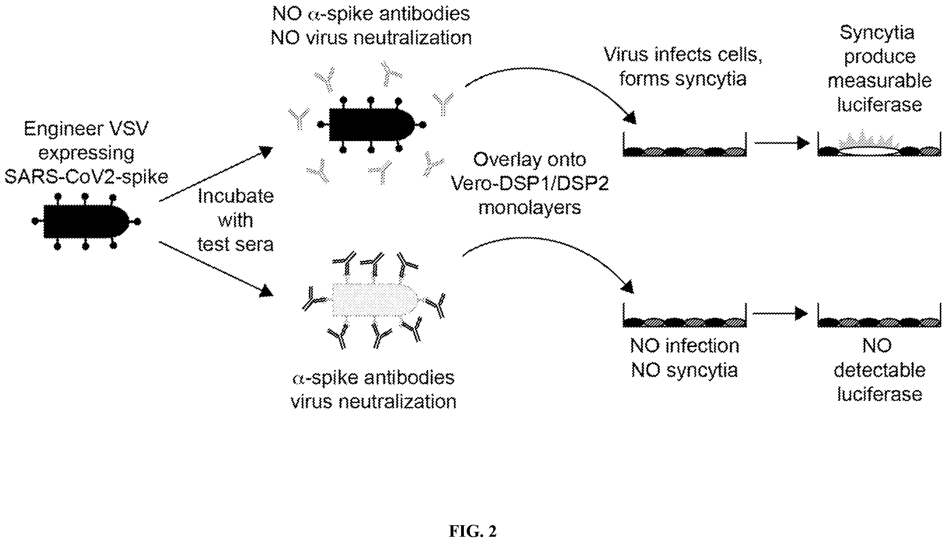

**Figure 2** represents an overview of a DSP Vero luciferase assay of the invention. A VSV expressing SARS-CoV-2 spike (e.g., VSV-SARS-CoV-2-S-Δ19CT or VSV-SARS-CoV-2-S) is incubated with test sera samples. In the absence of SARS-CoV-2 neutralizing antibodies (top) the virus retains infectivity and infects Vero-DSP1/Vero-DSP2 monolayers. If the test sample contains SARS-CoV-2 neutralizing antibodies (bottom), the antibodies bind to the spike protein causing virus neutralization by blocking cell entry. VSV-SARS-CoV-2-S-Δ19CT induces syncytia formation in Vero-DSP1/Vero-DSP2 monolayers, which reconstitutes a fully functional luciferase reporter that is used to quantitate virus-induced syncytia formation. High luciferase signal means the test sample did not neutralize the virus, while decreased luciferase indicates the presence of SARS-CoV-2-neutralizing antibodies in the test sample.

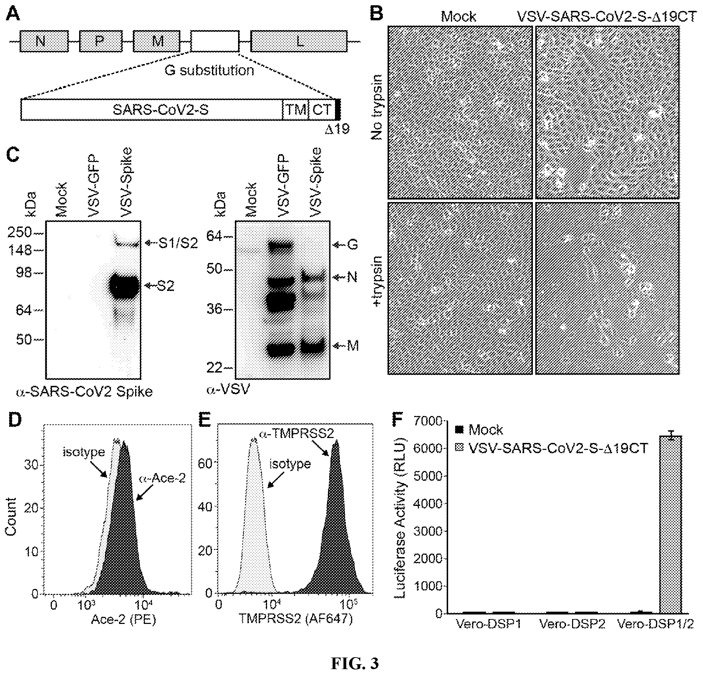

**Figures 3A-F. (A)** A schematic representation of the VSV-SARS-CoV-2-S-Δ19CT genome used in the assay. The location of the VSV N, P, M, and L genes are shown. VSV-G is replaced with a codon optimized SARS-CoV-2 spike gene with a 19 amino acid C-terminal (CT) deletion (Δ19CT). TM is transmembrane domain, CT is C-terminal domain. Not drawn to scale. **(B)** VSV-SARS-CoV-2-S-Δ19CT induces syncytia formation in Vero cell monolayers. Vero monolayers were infected with VSV-SARS-CoV-2-S-Δ19CT or mock-infected. After 4 hours, the inoculums were removed and replaced with fresh media ± 0.4 μg/mL trypsin. Images were taken 20 hours post infection at 100X magnification. **(C)** Immunoblot analysis. Viral supernatants prepared from Vero cells infected with VSV-GFP (encodes VSV-G) or VSV-SARS-CoV-2-S-Δ19CT or mock-infected were subjected to immunoblot analysis using anti-SARS-CoV-2-spike antibody (left) and rabbit anti-VSV antiserum (right). Arrows indicate the full-length S1/S2 variant and proteolytically cleaved S2 variant of SARS-CoV-2 spike protein and the VSV-G, VSV-N, and VSV-M proteins. **(D and E)** Flow cytometry. Monolayers of Vero-DSP1 cells were dislodged with versene, permeabilized (for TMPRSS2 staining only), stained with isotype control (grey), anti-ACE2 (**D**, black) or anti-TMPRSS2 (**E**, black) antibody, fixed, and subjected to flow cytometry analysis. **(F)** VSV-SARS-CoV-2-S-Δ19CT induced luciferase activity. Monolayers of Vero-DSP1, Vero-DSP2, or mixed Vero-DSP1/Vero-DSP2 cells were infected (10,000 TCID50 units/well) with VSV-SARS-CoV-2-S-Δ19CT or mock infected. Luciferase activity was measured as a marker of cell fusion at 24 hours after infection. Values represent the average (mean) RLU ± standard deviation from duplicate wells.

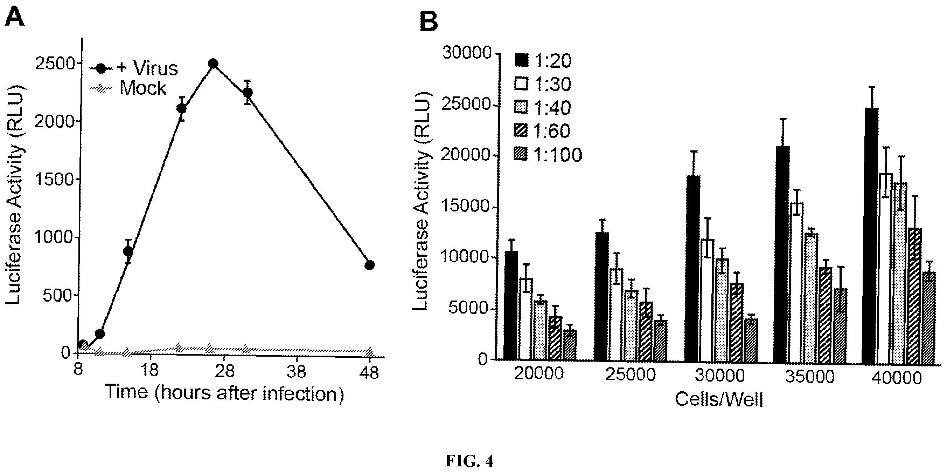

**Figures 4A-B.** Optimization of assay luciferase signal. **(A)** Kinetics of VSV-SARS-CoV-2-S-Δ19CT-induced luciferase activity. VSV-SARS-CoV-2-S-Δ19CT (2360 TCID50 units/well) or OptiMEM alone (mock) was overlaid onto monolayers of Vero-DSP1/Vero-DSP2 cells. Luciferase substrate EnduRen™ was added to wells and luciferase activity was measured at the indicated times (h) thereafter. Values represent the average (mean) RLU ± standard deviation from duplicate wells. **(B)** Optimization of Vero-DSP1/Vero-DSP2 cell density. The indicated numbers of Vero-DSP1/Vero-DSP2 cells were seeded in 96-well plates. The following day, VSV-SARS-CoV-2-S-Δ19CT was diluted in OptiMEM to the indicated dilutions and overlaid onto the cell monolayers. Luciferase substrate EnduRen™ was added to wells and luciferase activity was measured 28 hours after infection. Values represent the average (mean) RLU ± standard deviation from two separate experiments run in duplicate.

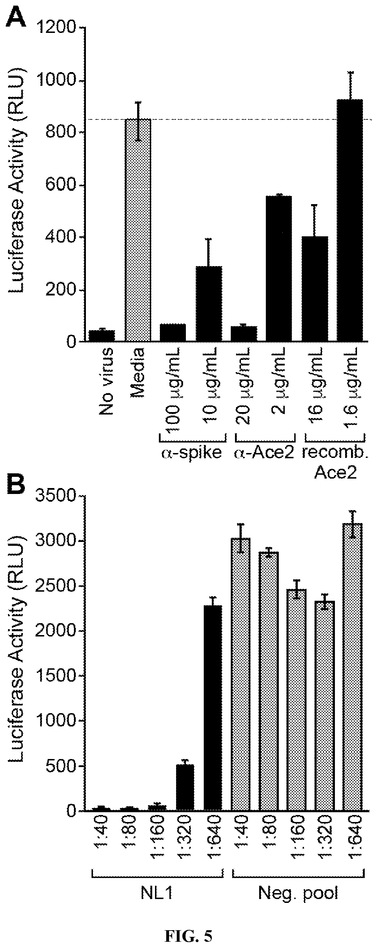

**Figures 5A-B.** Neutralization of VSV-SARS-CoV-2-S-Δ19CT-induced luciferase activity. **(A)** Inhibition by purified molecules. VSV-SARS-CoV-2-S-Δ19CT was incubated with media alone or the indicated concentrations of polyclonal anti-SARS-CoV-2-spike antibody, monoclonal anti-ACE2 antibody, or recombinant ACE2. After 1 hour at 37°C, the virus mixes were overlaid on Vero-DSP1/Vero-DSP2 monolayers. Control wells received media alone (no virus). Luciferase substrate EnduRen™ was added to wells and luciferase activity was measured 24 hours after infection. Values represent the average (mean) RLU ± standard deviation from duplicate wells. **(B)** Inhibition by COVID-19 convalescing plasma. VSV-SARS-CoV-2-S-Δ19CT was incubated with the indicated dilutions of plasma from a COVID-19 convalescing individual (NL1) or pooled plasma from three presumptive SARS-CoV-2 seronegative individuals. Plasma dilutions represent final dilutions after addition of virus. After 1 hour at 37°C, the virus mixes were overlaid on Vero-DSP1/Vero-DSP2 monolayers. Luciferase substrate EnduRen™ was added to wells and luciferase activity was measured 28 hours after infection. Values represent the average (mean) RLU ± standard deviation from duplicate wells.

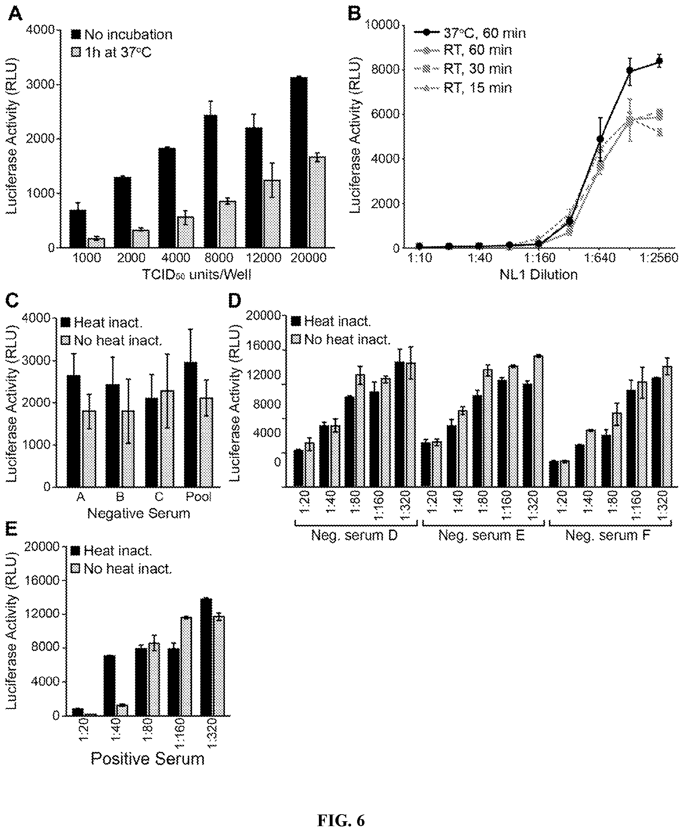

**Figures 6A-E.** Optimization of assay neutralization conditions. **(A)** Incubation of virus decreases syncytia formation. VSV-SARS-CoV-2-S-Δ19CT was thawed and diluted to the indicated TCID50 units/100 μL in OptiMEM. Duplicate samples were prepared; one set was immediately overlaid onto Vero-DSP1/Vero-DSP2 monolayers, while the other was incubated at 37°C for 1 hour before being overlaid onto Vero-DSP1/Vero-DSP2 monolayers. Luciferase substrate EnduRen™ was added to wells and luciferase activity was measured 27 hours after infection. Values represent the average (mean) RLU ± standard deviation from duplicate wells. **(B)** NL1 inhibition under different conditions. Dilutions of NL1 convalescing plasma were prepared and incubated with VSV-SARS-CoV-2-S-Δ19CT for the times and at the temperatures indicated, before being overlaid onto Vero-DSP1/Vero-DSP2 monolayers. Dilutions represent NL1 dilutions in the final virus/plasma mixes. Luciferase substrate EnduRen™ was added to wells and luciferase activity was measured 25 hours after infection. Values represent the average (mean) RLU ± standard deviation from duplicate wells. **(C and D)** Effect of heat inactivation on negative sera. Presumptive negative serum samples A, B, C, D, E, F, G, and a commercial SARS-CoV-2 seronegative pooled sera (Pool) were prepared as duplicate aliquots. One aliquot was stored on ice while the other was incubated for 30 minutes at 56°C. Each aliquot was then diluted in OptiMEM and mixed with VSV-SARS-CoV-2-S-Δ19CT to a final dilution of 1:100 **(C)** or the dilutions indicated **(D).** Following a 30-minute incubation at room temperature, the virus/sera mixes were overlaid onto Vero-DSP1/Vero-DSP2 monolayers. Luciferase substrate EnduRen™ was added to wells and luciferase activity was measured 23 hours after infection. Values represent the average (mean) RLU ± standard deviation from duplicate wells. (**E**) Effect of heat inactivation on COIVD-19 convalescing serum. A presumptive SARS-CoV-2 seropositive sample was thawed and assayed as described in Panel D.

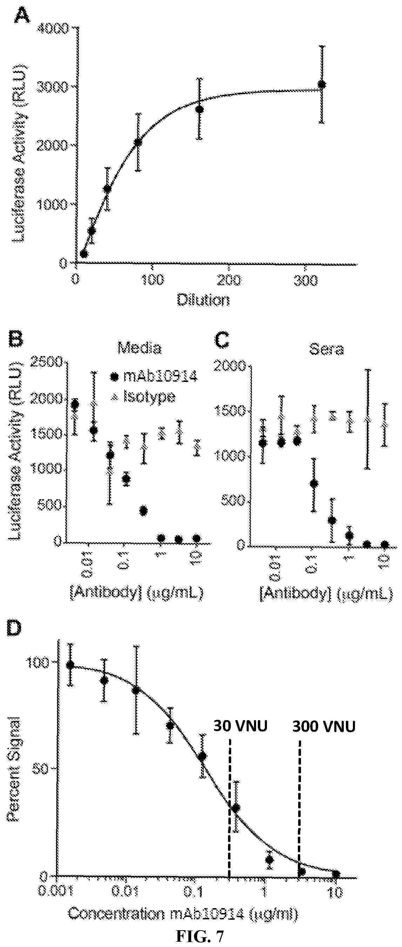

**Figures 7A-D.** Development of a contrived positive control. **(A)** Minimum recommended dilution. Thirty-nine sera from presumed SARS-CoV-2 seronegative individuals were serially diluted and mixed with VSV-SARS-CoV-2-S-Δ19CT in singlet. Dilutions represent serum dilution in the virus/serum mix. After 30 minutes at room temperature, the virus/serum mixes were overlaid on Vero-DSP1/Vero-DSP2 monolayers. Luciferase substrate EnduRen™ was added to wells and luciferase activity was measured 22 hours after infection. Values represent the average (mean) RLU ± standard deviation fit with a non-linear regression curve. **(B and C)** Neutralization of VSV-SARS-CoV-2-S-Δ19CT by mAb10914. mAb10914 or isotype control antibody were diluted in OptiMEM **(B)** or pooled SARS-CoV-2 seronegative sera **(C)** and incubated with VSV-SARS-CoV-2-S-Δ19CT. Concentrations represent the antibody concentration in the virus/antibody mixes; sera concentration was 1:100. After 30 minutes at room temperature, the virus mixes were overlaid onto Vero-DSP1/Vero-DSP2 monolayers. Luciferase substrate EnduRen™ was added to wells and luciferase activity was measured 24 hours after infection. Values represent the average (mean) RLU $\pm$ standard deviation from duplicate wells. **(D)** Establishment of contrived positive control values. mAb10914 was diluted in pooled SARS-CoV-2 seronegative sera as described in panel C and assayed as in panel C in triplicate runs performed by three different operators. Diluted pooled negative sera was used as a control (0 $\mu$g/mL mAb10914). Percent signal relative to the control was determined for each sample. Values represent the average (mean) percent signal $\pm$ standard deviation from three independent experiments performed in duplicate, fit with a non-linear regression curve. Percent luciferase signal: luciferase signal from test sample relative to that in a negative control (pooled negative sera): above 50% = negative sample; below 50% = positive or borderline. A virus neutralizing unit (VNU): an arbitrary unit equal to 100 times the mAb10914 concentration with equivalent percent luciferase signal. Samples must have a VNU of 30 to be positive.

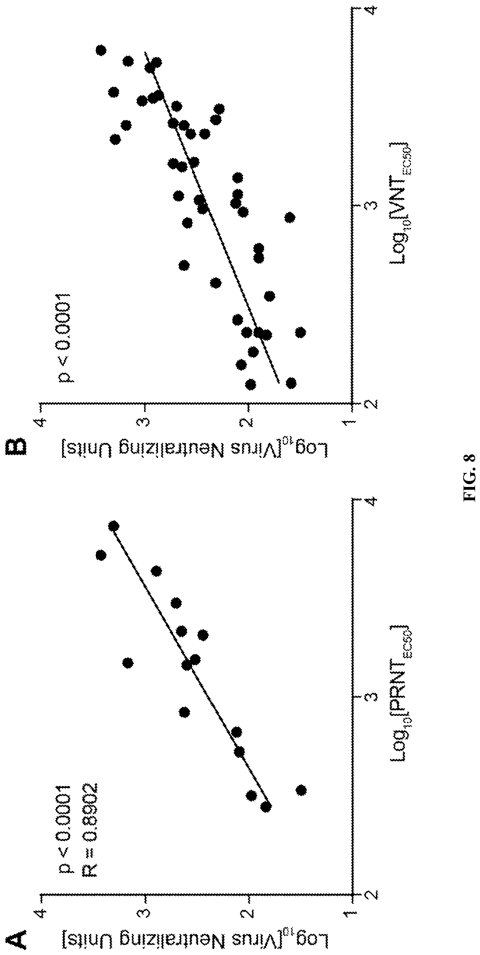

**Figures 8A-D.** Correlation of virus neutralizing units to $PRNT_{EC50}$ and $VNT_{EC50}$. SARS-CoV-2 seropositive sera samples were serially diluted in OptiMEM and mixed with VSV-SARS-CoV-2-S-Δ19CT. After 30 minutes at room temperature, the virus/serum mixes were overlaid onto Vero-DSP1/Vero-DSP2 monolayers. Luciferase substrate EnduRen™ was added to wells and luciferase activity was measured between 23 and 27 hours after infection. Each assay plate also included a calibration curve in which mAb10914 was spiked into pooled SARS-CoV-2 seronegative sera and mixed with virus. The concentrations of mAb 10914 in the virus mixes for the calibration curve were 3, 1, 0.33, 0.11, and 0.037 $\mu$g/mL. The percent signal for each test sample and calibration curve point were determined. A virus neutralizing unit (VNU) was then determined for each sample based on its percent signal relative to the calibration curve, where a VNU equals the concentration of mAb10914 for the given percent signal multiplied by 100. Samples must have a VNU of 30 to be positive. **(A)** Comparison with $PRNT_{EC50}$. Of the positive samples tested, 15 serum samples were subjected to a plaque reduction neutralization test (PRNT) using a clinical isolate of SARS-CoV-2. Two-fold serial dilutions of samples were tested in the PRNT50 assay from 1:20 through 1:40960. For each sample, the number of plaques at each dilution was plotted and used to determine the $PRNT_{EC50}$ value for each sample. Statistical comparison of VNU relative to $PRNT_{EC50}$ was performed. Data was transformed due to non-normal distribution (p < 0.0001). **(B)** Comparison with $VNT_{EC50}$. The percent signal for each sample dilution was plotted and used to determine the VNTEC50. Statistical comparison of VNU relative to $VNT_{EC50}$ was performed. Data was transformed due to non-normal distribution (p < 0.0001). (**C**) Representative distribution of VNUs in PCR-positive cohort. (**D**) Correlation between VNU values and symptom severity in 31 positive samples for which clinical symptoms were self-reported.

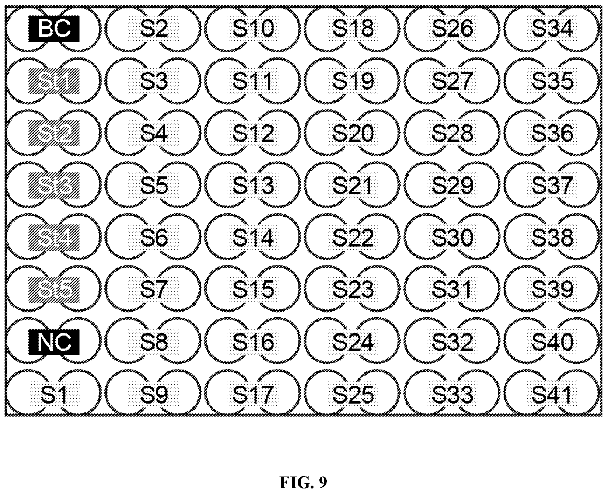

**Figure 9** shows an example of an assay plate layout for the DSP Vero luciferase assay. Example assay plate layout. All controls and samples are assayed in duplicate. A total of 41 samples (S1 to S41) can be run on a single plate using a single dilution for each sample. BC (background control) consists of pooled SARS-CoV-2 seronegative serum at 1:100 without virus. NC (negative control), consists of pooled SARS-CoV-2 seronegative serum at 1:100 with VSV-SARS-CoV-2-S-Δ19CT. NC is used to determine 100% luciferase signal in the assay. St1 through St5 are standards for the calibration curve. All standards are pooled SARS-CoV-2 seronegative serum at 1:100 with VSV-SARS-CoV-2-S-Δ19CT and mAb10914 spiked into each standard at different concentrations: Std1 is 3 $\mu$g/mL, Std2 is 1 $\mu$g/mL, Std3 is 0.33 $\mu$g/mL, Std4 is 0.11 $\mu$g/mL, and Std5 is 0.037 $\mu$g/mL.

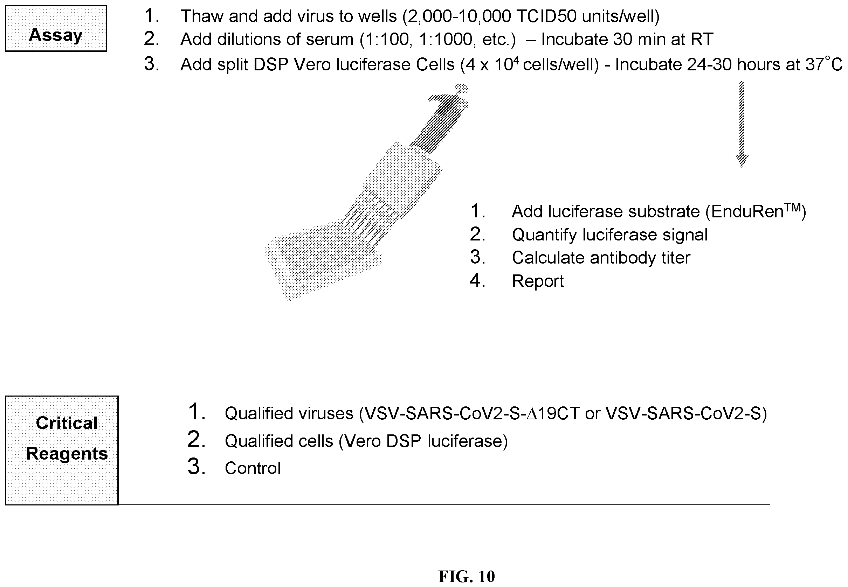

**Figure 10** is a schematic representation of DSP Vero luciferase assay workflow.

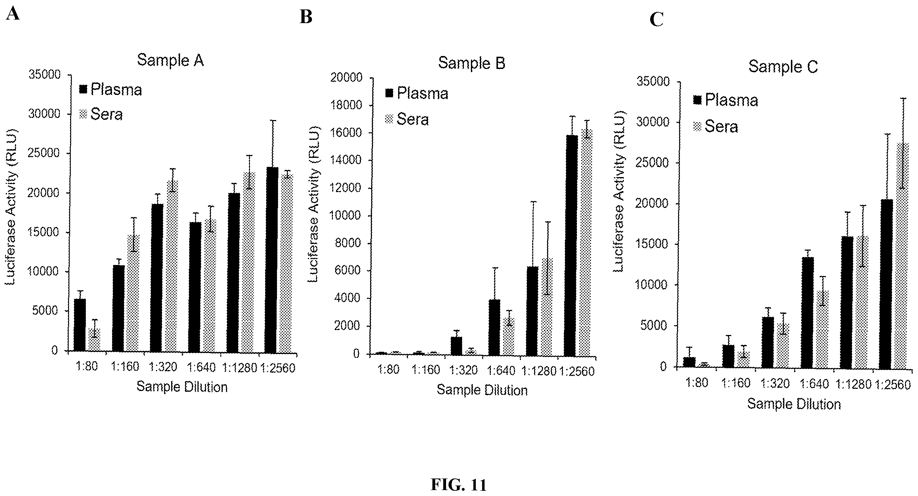

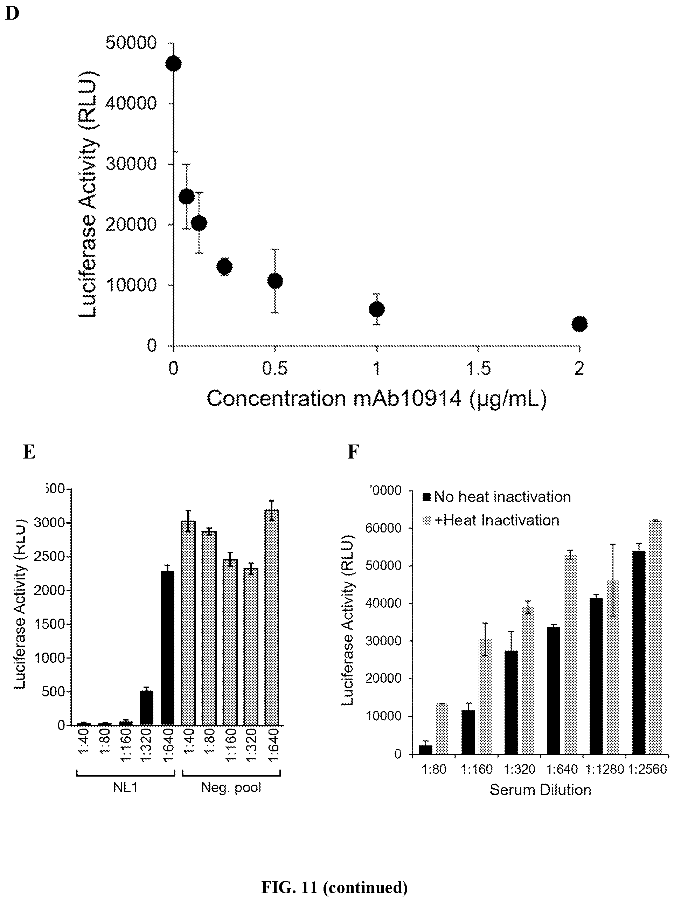

**Figures 11A-C** show luciferase activity in plasma and serum of three subjects at different dilutions. Plasma and serum from three COVID-19 convalescing individuals were heat inactivated for 30 min at 56°C. Serial dilutions were then prepared and incubated with VSV-SARS-CoV-2-S-Δ19CT for 30 minutes at room temperature before being overlaid onto Vero-DSP1/DPS2 monolayers. Luciferase activity was measured after approximately 24 hours. **Figure 11D** shows luciferase activity in pooled SARS-CoV-2 seronegative plasma containing stepped concentrations of anti-SARS-CoV-2 spike antibody mAb10914 mixed with VSV-SARS-CoV2 v1.0 (VSV-SARS-CoV-2-S-Δ19CT) virus. After a 30-minute incubation at room temperature, the plasma/virus mixes were overlaid onto Vero-DSP1/DSP2 monolayers. Luciferase activity was measured after approximately 24 hours. **Figure 11E** shows luciferase activity in dilutions of pooled SARS-CoV-2 seronegative plasma or plasma from a COVID-19 convalescing individual (NL1) mixed with VSV-SARS-CoV2 v.1.0 (VSV-SARS-CoV-2-S-Δ19CT) virus. After a 1 hour incubation at 37°C, the plasma/virus mixes were overlaid onto Vero-DSP1/DSP2 cell monolayers. Luciferase activity was measured approximately 24 hours later. **Figure 11F** shows the effect of heat inactivation on plasma samples. One aliquot was stored on ice while the other was incubated for 30 minutes at 56°C. Each aliquot was then diluted in OptiMEM and mixed with VSV-SARS-CoV-2-S-Δ19CT to a final dilution indicated in the graph. Following a 30-minute incubation at room temperature, the virus/plasma mixes were overlaid onto Vero-DSP1/Vero-DSP2 monolayers. Luciferase activity was measured after approximately 24 hours.

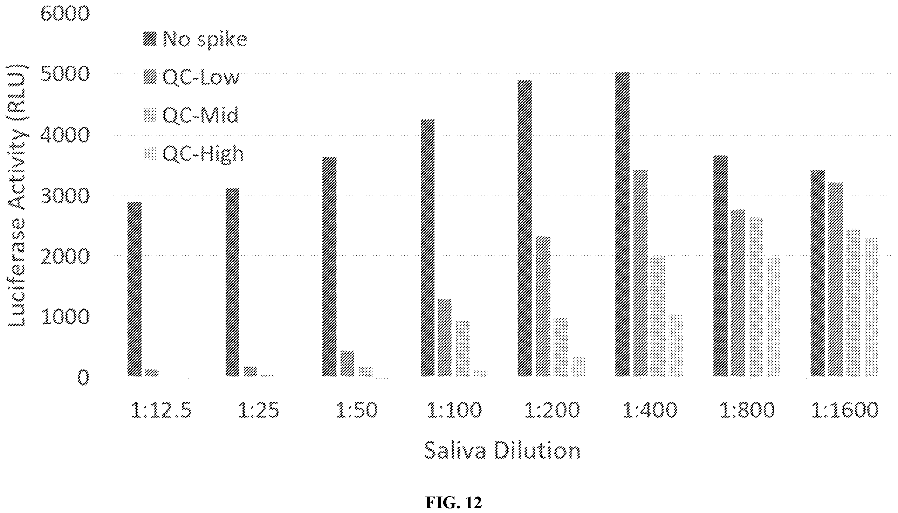

**Figure 12** shows luciferase activity in pooled saliva prepared from individuals prior to the COVID pandemic spiked with mAb10914 at stepped concentrations (2 $\mu$g/mL (QC-High), 0.6 $\mu$g/mL (QC-Mid), and 0.4 $\mu$g/mL (QC-Low)) mixed with VSV-SARS-CoV2 v.1.0 (VSV-SARS-CoV-2-S-Δ19CT) virus at 1:100 dilution. After a 30-minute incubation at room temperature the saliva/virus mixes were overlaid onto Vero-DSP1/Vero-DSP2 monolayers. Luciferase activity was measured approximately 24 hours later. Bars corresponding to data generated using No Spike, QC-Low, QC-Mid, QC-High are shown in order of appearance (from left to right) for each dilution tested.

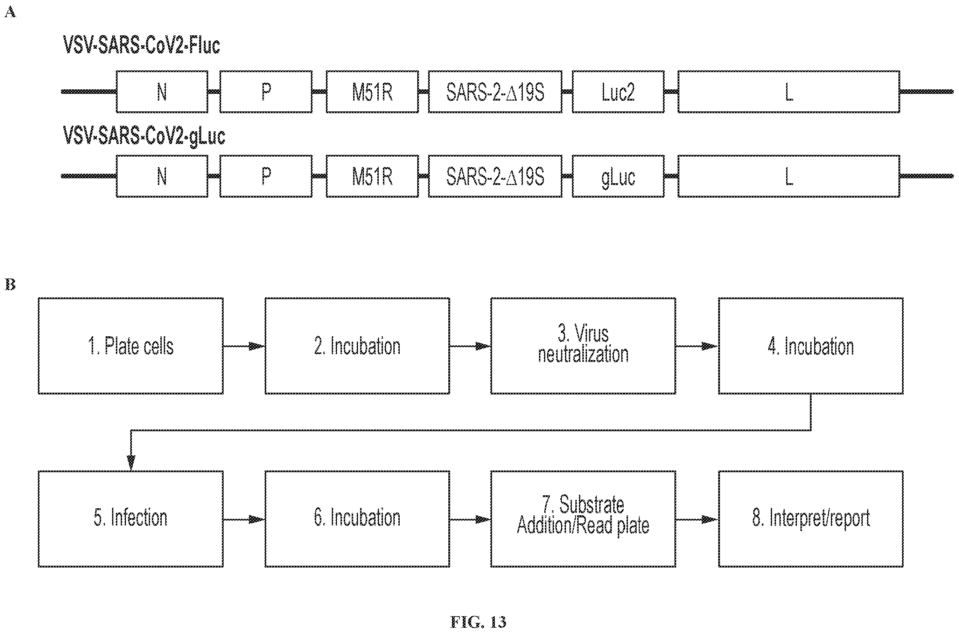

**Figure 13A** is a schematic representation of VSV-SARS-CoV-2-Fluc and VSV-SARS-CoV-2-S-Δ19CT-gLuc. VSV-SARS-CoV-2-Fluc encodes a mutant Luc2 variant of firefly luciferase (GenBank Accession No. AY738222). VSV-SARS-CoV-2-S-Δ19CT-gLuc encodes a secreted *Gaussia* luciferase (gLuc). **Figure 13B** shows the workflow of IMMUNO-COV assay version 2.

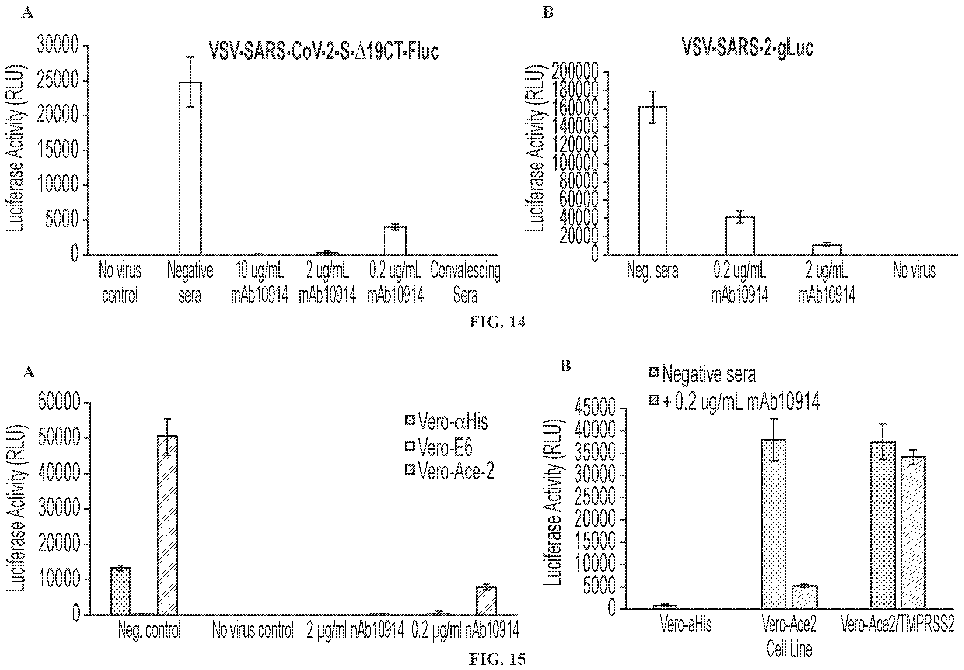

**Figure 14A** shows luciferase activity upon incubation of VSV-SARS-CoV-2-S-Δ19CT-Fluc encoding a mutant Luc2 variant of firefly luciferase with pooled negative sera (at 1:80 dilution), COVID-19 convalescing sera (at 1:80 dilution), or pooled negative sera (at 1:80 dilution) mixed with 10, 2, or 0.2 μg/mL mAb10914. After a 45-minute incubation at room temperature, the virus/sera mixes were overlaid onto Vero cell monolayers. After an additional 18 hours, d-luciferin substrate was added to wells and luciferase activity (RLUs) were measured using a luminometer. **Figure 14B** shows luciferase activity upon incubation of VSV-SARS-CoV-2-S-Δ19CT-gLuc encoding secreted *Gaussia* luciferase (1:48 dilution of virus stock) with pooled SARS-CoV-2 seronegative sera (at 1:80 dilution) alone or spiked with 0.2 or 2 μg/mL mAb10914. After 30-minute incubation at room temperature, the virus/sera mixes were overlaid onto Vero-Ace-2 monolayers. After an additional 24 hours, coelenterazine (20 μL of a 5 μM stock) was added and luciferase activity was measured.

**Figures 15A-B** demonstrate that Vero-Ace-2 cells are particularly effective in IMMUNO-COV assay v. 2. **Figure 15A** shows luciferase activity upon incubation of VSV-SARS-CoV-2-S-Δ19CT-Fluc or media alone (no virus control) with pooled SARS-CoV-2 seronegative sera (at a dilution of 1:80) mixed with 2 or 0.2 μg/mL of mAb10914. After a 30-minute room temperature incubation, the virus/sera mixes were overlaid onto monolayers of cells (Vero-αHis, Vero-E6 or Vero-Ace-2). After an additional 26 hours, d-luciferin substrate was added to the wells and luciferase activity (RLUs) was measured using a luminometer. **Figure 15B** shows luciferase activity upon incubation of pooled SARS-CoV-2 seronegative sera (at 1:80 dilution) with VSV-SARS-CoV-2-S-Δ19CT-gLuc (4800 TCID50/well) alone or spiked with 0.2 ug/mL mAb10914. After 30 minutes at room temperature, the virus/sera mixes were overlaid onto Vero-αHis, Vero-Ace-2, or Vero-Ace-2/TMPRSS2 cell monolayers seeded the day before in black, clear-bottomed 96-well plates at 1e4 cells/well. After an additional 24 hours, luciferase activity in the wells was measured after the addition of d-luciferin.

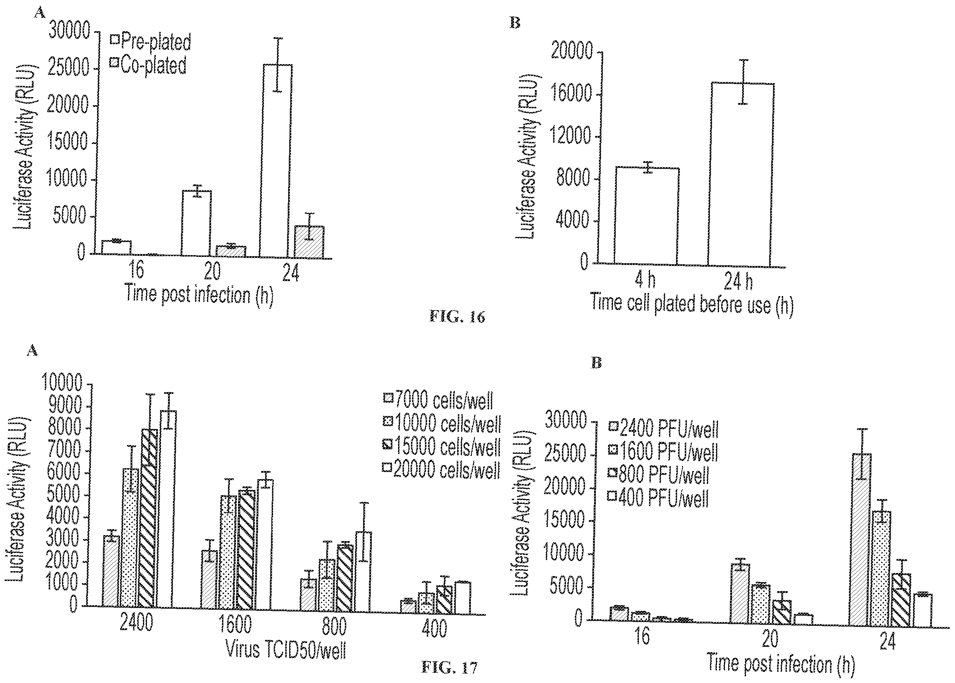

**Figures 16A-B** shows that luciferase signal is substantially lower when cells are co-plated with virus (as compared to when cells are pre-plated), but significantly recovers when given a 4 hour recovery time. (**A**) Virus was incubated with pooled SARS-CoV-2 seronegative sera (1:80) for 30 minutes at room temperature then overlaid onto Vero cell monolayers or mixed with Vero cells at a similar density. At the indicated times after cell overlay, luciferase activity was measured. (**B**) Virus was incubated with pooled SARS-CoV-2 seronegative sera (1:80) for 30 minutes at room temperature then overlaid onto Vero cell monolayers plated either 4 or 24 hours prior. After an additional 24 hours luciferase activity was measured.

**Figures 17A-B** show that luciferase activity increases with increased cell density (**A**) and virus quantity/well (**B**). Virus was diluted in OptiMEM to the indicated TCID50 per well and overlaid onto Vero cell monolayers plated at the indicated cells/well the day before. After an additional 16, 20, and 24 hours luciferase activity was measured. Data in panel **A** are from 24 hours. In panel A, bars corresponding to data generated using 7000 cells/well, 10000 cells/well, 15000 cells/well, and 20000 cells/well are shown in order of appearance (from left to right) for each virus TCID50 condition tested. In panel B, bars corresponding to data generated using 2400 PFU/well, 1600 PFU/well, 800 PFU/well, and 400 PFU/well are shown in order of appearance (from left to right) for each time point tested.

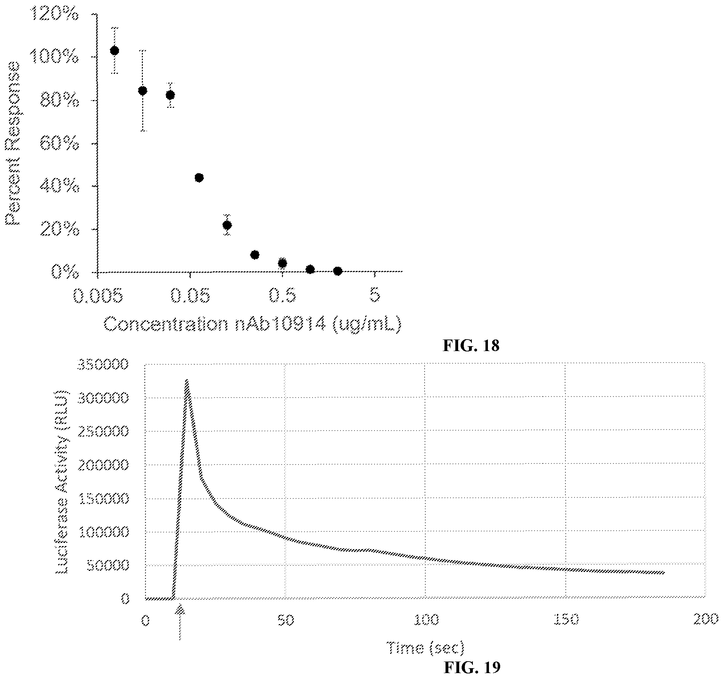

**Figure 18** shows that IMMUNO-COV assay v. 2 is highly sensitive and shows dose-dependent inhibition of VSV-SARS-CoV-2-S-Δ19CT-Fluc. Virus was incubated with pooled negative serum containing stepped concentrations of mAb10914 for 30 minutes at room temperature. The virus/serum mixes were then overlaid onto Vero cell monolayers. Luciferase activity was measured after an additional 24 hours.

**Figure 19** shows kinetic curve of Fluc activity in VSV-SARS-CoV-2-S-Δ19CT-Fluc-infected Vero-Ace-2 cells following d-luciferin addition. Vero-Ace-2 cell monolayers (seeded at 1e4 cells/well the day before infection) were infected with 4800 TCID50 units of VSV-SARS-CoV-2-S-Δ19CT-Fluc. After 24 hours the plate was loaded in the TECAN instrument (luminescence plate reader) that was fitted with an injector. The injector was programmed to inject the desired quantity of substrate (20 μL of a 3.75 mg/mL solution of d-luciferin) into each well, and then after 0.5 sec read luminescence (1000 ms integration time). Additional luciferase reads were performed every 5 seconds for 3 min to generate a kinetic curve of luciferase activity (two reads done, d-luciferin added (final concentration 0.44 mg/mL in well) at 9 sec (arrow), additional reads done through 3 min).

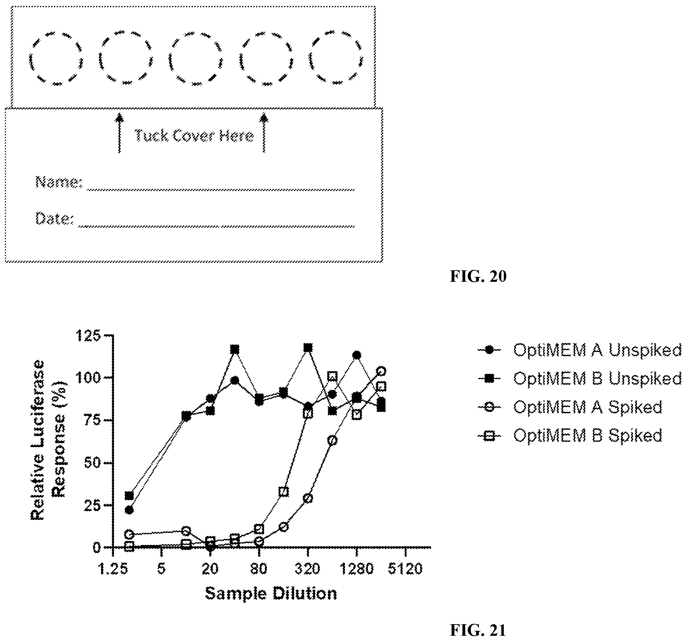

**Figure 20** shows a description of an exemplar Dried Bloodspot (DBS) collection card. Droplets of blood are allowed to fall onto the filter paper, which is printed with 5 circles.

**Figure 21** shows a graphical summary of exemplar percent relative luciferase response data generated by extracting the bloodspots using OptiMEM across various sample dilutions. OptiMEM A and B are independent samples of OptiMEM.

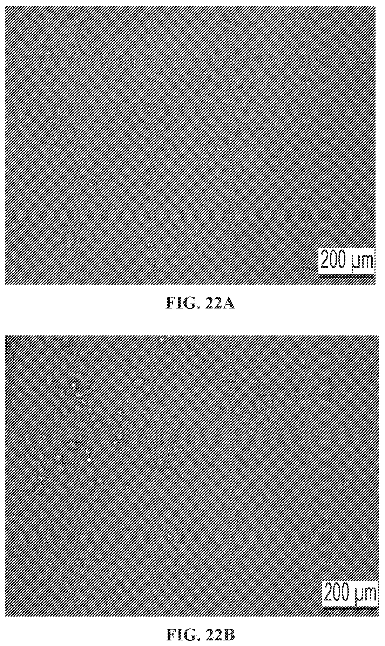

**Figures 22A-B** show exemplar photomicrographs of bloodspot matrixes. (A) B7 Opti-20x Positive Matrix A and (**B**) A7 Opti 20x Negative Matrix A. Bloodspot matrix was compatible at a 1:20 dilution of matrix. No issues were seen regarding either cell health or virus infectivity in the bloodspot samples that were tested.

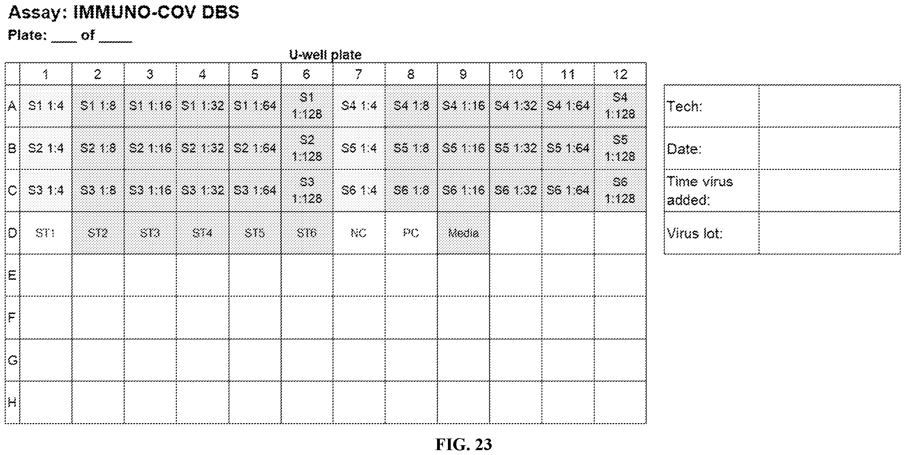

**Figure 23** depicts an exemplar layout map for a 96-well U-well (or U-bottom) Plate.

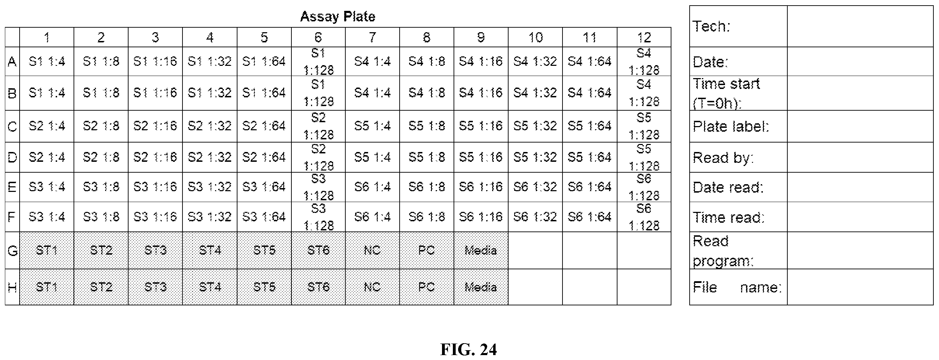

**Figure 24** depicts an exemplar layout map for a 96-well Assay Plate.

## DETAILED DESCRIPTION

Definitions

Unless defined otherwise, all technical and scientific terms used herein have the same meaning as commonly understood by one of ordinary skill in the art to which this disclosure belongs.

Singular forms "a", "an", and "the" include plural references unless the context clearly dictates otherwise. Thus, for example, a reference to "a method" includes one or more methods, and/or steps of the type described herein and/or which will become apparent to those persons skilled in the art upon reading this disclosure.

The term "about" or "approximately" includes being within a statistically meaningful range of a value. Such a range can be within an order of magnitude, preferably within 50%, more preferably within 20%, still more preferably within 10%, and even more preferably within 5% of a given value or range. The allowable variation encompassed by the term "about" or "approximately" depends on the particular system under study, and can be readily appreciated by one of ordinary skill in the art.

The terms "comprise(s)," "include(s)," "having," "has," and "contain(s)," are intended to be open-ended transitional phrases, terms, or words that do not preclude the possibility of additional acts or structures.

The terms "assay" or "test" as used herein may be used interchangeably with the term "method".

"Antibody" as used herein encompasses polyclonal and monoclonal antibodies and refers to immunoglobulin molecules of classes IgA (e.g., IgA1 or IgA2), IgD, IgE, IgG (e.g., IgG1, IgG2, IgG3 and IgG4) or IgM, or fragments, or derivatives thereof, including without limitation Fab, F(ab')2, Fd, single chain antibodies, diabodies, bispecific antibodies, bifunctional antibodies, humanized antibodies, and various derivatives thereof.

In the context of the present disclosure, the term "neutralizing antibody" refers to an antibody that binds to a pathogen (e.g., a virus) and interferes with its ability to infect a cell. Non-limiting examples of neutralizing antibodies include antibodies that bind to a viral particle and inhibit successful transduction, e.g., one or more steps selected from binding, entry, trafficking to the nucleus, and transcription of the viral genome. Some neutralizing antibodies may block a virus at the post-entry step.

The term "immune response" refers to a response of a cell of the immune system (e.g., a B-cell, T-cell, macrophage or polymorphonucleocyte) to a stimulus such as an antigen (e.g., a viral antigen). Active immune responses can involve differentiation and proliferation of immunocompetent cells, which leads to synthesis of antibodies or the development of cell-mediated reactivity, or both. An active immune response can be mounted by the host after exposure to an antigen (e.g., by infection or by vaccination). Active immune response can be contrasted with passive immunity, which can be acquired through the transfer of substances such as, e.g., an antibody, transfer factor, thymic graft, and/or cytokines from an actively immunized host to a non-immune host.

As used herein in connection with a viral infection and vaccination, the terms "protective immune response" or "protective immunity" refer to an immune response that that confers some benefit to the subject in that it prevents or reduces the infection or prevents or reduces the development of a disease associated with the infection. Without wishing to be bound by theory, the presence of SARS-CoV-2 neutralizing antibodies in a subject can indicate the presence of a protective immune response in the subject.

The terms "immunogenic composition", "vaccine composition", or "vaccine", which are used interchangeably, refer to a composition comprising at least one immunogenic and/or antigenic component that induces an immune response in a subject (e.g., humoral and/or cellular response). In certain embodiments, the immune response is a protective immune response. A vaccine may be administered for the prevention or treatment of a disease, such as an infectious disease. A vaccine composition may include, for example, live or killed infectious agents, recombinant infectious agents (e.g., recombinant viral particles, virus-like particles, nanoparticles, liposomes, or cells expressing immunogenic and/or antigenic components), antigenic proteins or peptides, nucleic acids, etc. Vaccines may be administered with an adjuvant to boost the immune response.

The term "operably linked" includes a linkage of nucleic acid elements in a functional relationship. A nucleic acid sequence is "operably linked" when it is placed into a functional relationship with another nucleic acid sequence. For instance, a promoter or enhancer, or a 5' regulatory region containing a promoter or enhancer, is operably linked to a coding sequence if it effects the transcription of the coding sequence.

As used herein, the term "promoter" means a genetic sequence generally in cis and located upstream of a gene, and which facilitates the transcription of the gene. Promoters can be regulated (developmental, tissue specific, or inducible (chemical, temperature)) or constitutively active. In certain embodiments, the promoter is a constitutive mammalian promoter, such as the ubiquitin C promoter (see Schorpp et al., Nucl. Acids Res. 24(9): 1787-1788, 1996); Byun et al., Biochem. Biophys. Res. Comm 332(2): 518-523, 2005) or the CMV-IE promoter (see Addison et al., J. Gen. Virol. 78(7): 1653-1661, 1997; Hunninghake et al., J. Virol. 63(7): 3026-3033, 1989), or the hCMV-IE promoter (human cytomegalovirus immediate early gene promoter) (see Stinski & Roehr, J. Virol. 55(2): 431-441, 1985; Hunninghake et al., J. Virol. 63(7): 3026-3033, 1989). A promoter can comprise one or more specific transcriptional regulatory sequences to further enhance expression and/or to alter the spatial expression and/or temporal expression of same. A promoter can also comprise distal enhancer or repressor elements, which can be located as much as several thousand base pairs from the start site of transcription. A promoter can be derived from sources including viral, bacterial, fungal, plants, insects, and animals. A promoter can regulate the expression of a gene component constitutively or differentially with respect to cell, the tissue or organ in which expression occurs or, with respect to the developmental stage at which expression occurs, or in response to external stimuli such as physiological stresses, pathogens, metal ions, or inducing agents. Representative non-limiting examples of promoters include the bacteriophage T7 promoter, bacteriophage T3 promoter, SP6 promoter, lac operator-promoter, tac promoter, SV40 late promoter, SV40 early promoter, RSV-LTR promoter, CMV IE promoter, SV40 early promoter or SV40 late promoter and the CMV IE promoter.

"Signal peptide" and "leader sequence" are used interchangeably herein and refer to an amino acid sequence that can direct localization of a polypeptide. A signal peptide may be capable of directing the polypeptide into a cell's secretory pathway. Signal peptides are often located at the N-terminus of the polypeptides and are often cleaved from the remainder of the polypeptide (often referred to as the "mature protein"), upon secretion from the cell.

The terms "derivative" and "variant" are used herein interchangeably to refer to an entity that has significant structural identity with a reference entity but differs structurally from the reference entity in the presence or level of one or more chemical moieties as compared with the reference entity. In many embodiments, a derivative also differs functionally from its reference entity. In general, whether a particular entity is properly considered to be a "derivative" of a reference entity is based on its degree of structural identity with the reference entity. As will be appreciated by those skilled in the art, any biological or chemical reference entity has certain characteristic structural elements. A derivative, by definition, is a distinct entity that shares one or more such characteristic structural elements. To give but a few examples, a small molecule may have a characteristic core structural element (e.g., a macrocycle core) and/or one or more characteristic pendent moieties so that a derivative of the small molecule is one that shares the core structural element and the characteristic pendent moieties but differs in other pendent moieties and/or in types of bonds present (single vs double, E vs Z, etc.) within the core. Derivatives which are nucleic acids and polypeptides/proteins encompass mutants. A derivative nucleic acid may have a characteristic sequence element comprised of a plurality of nucleotide residues having designated positions relative to one another in linear or three-dimensional space. In some embodiments, the nucleic acid sequence of a derivative may be 50%, 60%, 70%, 80%, 81%, 82%, 83%, 84%, 85%, 86%, 87%, 88%, 89%, 90%, 91%, 92%, 93%, 94%, 95%, 96%, 97%, 98%, 99%, 99.5% or more identical over the full length of the reference sequence or a fragment thereof. A derivative peptide or polypeptide may have a characteristic sequence element comprised of a plurality of amino acids having designated positions relative to one another in linear or three-dimensional space and/or contributing to a particular biological function. Derivative peptides and polypeptides include peptides and polypeptides that differ in amino acid sequence from the reference peptide or polypeptide by the insertion, deletion, and/or substitution of one or more amino acids, but retain at least one biological activity of such reference peptide or polypeptide (e.g., the ability to mediate cell infection by a virus, the ability to mediate membrane fusion, the ability to be bound by a specific antibody or to promote an immune response, etc.). In some non-limiting embodiments, a derivative peptide or polypeptide shows the sequence identity over the full length with the reference peptide or polypeptide (or a fragment thereof) that is at least 50%, 60%, 70%, 80%, 81%, 82%, 83%, 84%, 85%, 86%, 87%, 88%, 89%, 90%, 91%, 92%, 93%, 94%, 95%, 96%, 97%, 98%, 99%, 99.5% or more. Alternatively or in addition, a derivative peptide or polypeptide may differ from a reference peptide or polypeptide as a result of one or more and/or one or more differences in chemical moieties attached to the polypeptide backbone (e.g., in glycosylation, phosphorylation, acetylation, myristoylation, palmitoylation, oxidation, formylation, amidation, polyglutamylation, ADP-ribosylation, pegylation, biotinylation, etc.). In some embodiments, a derivative peptide or polypeptide lacks one or more of the biological activities of the reference polypeptide or has a reduced or increased level of one or more biological activities as compared with the reference polypeptide. Derivatives of a particular peptide or polypeptide may be found in nature or may be synthetically or recombinantly produced. As used herein, the term "derivative" or "variant" also encompassed various chimeric, fusion proteins and conjugates, including fusions or conjugates with detection tags (e.g., HA tag, histidine tag, biotin, fusions with fluorescent or luminescent domains, etc.), dimerization/multimerization sequences, Fc, signaling sequences, etc.

The term "coronavirus" as used herein refers to the subfamily *Coronavirinae* within the family *Coronaviridae,* within the order *Nidovirales.* Based on the phylogenetic relationships and genomic structures, this subfamily consists of four genera: *Alphacoronavirus, Betacoronavirus, Gammacoronavirus* and *Deltacoronavirus.* The alphacoronaviruses and betacoronaviruses infect only mammals. The gammacoronaviruses and deltacoronaviruses infect birds, but some of them can also infect mammals. Alphacoronaviruses and betacoronaviruses usually cause respiratory illness in humans and gastroenteritis in animals. The three highly pathogenic viruses, SARS-CoV-2, SARS-CoV-1 and MERS-CoV cause severe respiratory syndrome in humans. The other four human coronaviruses, HCoV-NL63, HCoV-229E, HCoV-OC43 and HKU1, induce only mild upper respiratory diseases in immunocompetent hosts, although some of them can cause severe infections in infants, young children and elderly individuals. Additional non-limiting examples of commercially important coronaviruses include transmissible gastroenteritis coronavirus (TGEV), porcine respiratory coronavirus, canine coronavirus, feline enteric coronavirus, feline infectious peritonitis virus, rabbit coronavirus, murine hepatitis virus, sialodacryoadenitis virus, porcine hemagglutinating encephalomyelitis virus, bovine coronavirus, avian infectious bronchitis virus, and turkey coronavirus. Reviewed in Cui et al., Nature Reviews Microbiology, 2019, 17:181-192; Fung et al., Annu. Rev. Microbiol., 2019, 73:529-557.

Coronaviruses form enveloped and spherical particles of 80-160 nm in diameter. They contain a positive-sense, non-segmented, single-stranded RNA (ssRNA) genome of 27-32 kb in size. The 5'-terminal two-thirds of the genome encodes polyproteins, pp1a and pp1ab. The 3' terminus encodes structural proteins, including envelope glycoproteins spike (S), envelope (E), membrane (M) and nucleocapsid (N). The genomic RNA is 5'-capped and 3'-polyadenylated and contains multiple open reading frames (ORFs). The invariant gene order is 5'-replicase-S-E-M-N-3', with numerous small ORFs (encoding accessory proteins) scattered among the structural genes. The coronavirus replicase is encoded by two large overlapping ORFs (ORF 1a and ORF1b) occupying about two-thirds of the genome and is directly translated from the genomic RNA (gRNA). The structural and accessory genes, however, are translated from subgenomic RNAs (sgRNAs) generated during genome transcription/replication. Infection starts with the attachment of the coronavirus to the cognate cellular receptor, which induces endocytosis. Membrane fusion typically occurs in the endosomes, releasing the viral nucleocapsid to the cytoplasm. The genomic RNA (gRNA) serves as the template for translation of polyproteins pp1a and pp 1 ab, which are cleaved to form nonstructural proteins (nsps). Nsps induce the rearrangement of cellular membrane to form double-membrane vesicles (DMVs), where the viral replication transcription complexes (RTCs) are anchored. Full-length gRNA is replicated via a negative-sense intermediate, and a nested set of subgenomic RNA (sgRNA) species are synthesized by discontinuous transcription. These sgRNAs encode viral structural and accessory proteins. Particle assembly occurs in the ER-Golgi intermediate complex (ERGIC), and mature virions are released in smooth-walled vesicles via the secretory pathway.

Coronavirus entry into host cells is mediated by the transmembrane spike (S) glycoprotein (interchangeably referred to as "spike glycoprotein", "S glycoprotein", "S protein" or "spike protein") which is the main target of anti-viral neutralizing antibodies and is the focus of therapeutic and vaccine design. S glycoprotein forms homotrimers protruding from the viral surface. S glycoprotein comprises two functional subunits responsible for binding to the host cell receptor (S1 subunit) and fusion of the viral and cellular membranes (S2 subunit). For many coronaviruses, including SARS-CoV-1 and SARS-CoV-2, S glycoprotein is cleaved at the boundary between the S1 and S2 subunits, which remain non-covalently bound in the prefusion conformation. The distal S1 subunit comprises the receptor-binding domain(s) (RBD) and contributes to stabilization of the prefusion state of the membrane-anchored S2 subunit that contains the fusion machinery. S is further cleaved by host proteases at the S2' site located immediately upstream of the fusion peptide. This cleavage has been proposed to activate the protein for membrane fusion via conformational changes. Walls et al., Cell, published online March 9, 2020; available at doi.org/10.1016/j.cell.2020.02.058.

SARS-CoV-1 and SARS-CoV-2 interact directly with angiotensin-converting enzyme 2 (ACE2) to enter target cells and may employ the cellular serine protease, transmembrane protease, serine 2 (TMPRSS2) for S protein priming (Hoffmann et al., Cell, 2020, 181:1-10; available at doi.org/10.1016/j.cell.2020.02.052). SARS-CoV-S and SARS-CoV-2-S share 76% amino acid identity. The receptor binding domain (RBD) in the S glycoprotein is involved in binding to the ACE2 receptor. Six RBD amino acids have been shown to be critical for binding to the ACE2 receptor and for determining the host range of SARS-CoV-like viruses. They are Y442, L472, N479, D480, T487 and Y4911 in SARS-CoV, which correspond to L455, F486, Q493, S494, N501 and Y505 in SARS-CoV-2 (Andersen et al., Nature Medicine, 2020; available at doi.org/10.1038/s41591-020-0820-9).

The term "rhabdovirus" as used herein refers to *Rhabdoviridae* family of viruses in the order *Mononegavirales* encompassing more than 150 viruses of vertebrates, invertebrates and plants. Examples of rhabdoviruses include rabies virus (RABV) from the *Lyssavirus* genus, vesiculoviruses from *Vesiculovirus* genus, the viral hemorrhagic septicemia virus (VHSV) and infectious hematopoietic necrosis virus, both from the *Novirhabdovirus* genus. Rhabdoviruses are bullet-shaped enveloped viruses with negative-sense single-stranded RNA genome 11-15 kb in length. The genome of rhabdoviruses comprises up to ten genes among which only five are common to all members of the family. These genes encode the nucleoprotein (N), the phosphoprotein (P), the matrix protein (M), the glycoprotein (G) and the large protein (L). The genome is associated with N, L and P to form the nucleocapsid, which is condensed by the M protein into a tightly coiled helical structure. The condensed nucleocapsid is surrounded by a lipid bilayer containing the viral glycoprotein G that constitutes the spikes that protrude from the viral surface. Rhabdoviruses enter the cell via the endocytic pathway and subsequently fuse with the cellular membrane within the acidic environment of the endosome. Both receptor recognition and membrane fusion are mediated by a single transmembrane viral glycoprotein (G). Fusion between the viral envelope and the endosomal membrane is triggered via a low-pH induced (in the endosome) structural rearrangement of the G

**EP 4 136 457 B1**

resulting in the release the viral genome and associated proteins into the cytoplasm of target cells.

As used herein, the term "vesiculovirus" refers to any virus in the *Vesiculovirus* genus. Non-limiting examples of vesiculoviruses include, e.g., Vesicular Stomatitis Virus (VSV) (e.g., VSV-New Jersey, VSV-Indiana), Alagoas vesiculovirus, Cocal vesiculovirus, Jurona vesiculovirus, Carajas vesiculovirus, Maraba vesiculovirus, Piry vesiculovirus, Calchaqui vesiculovirus, Yug Bogdanovac vesiculovirus, Isfahan vesiculovirus, Chandipura vesiculovirus, Perinct vesiculovirus, Porton-S vesiculovirus. Vesicular Stomatitis Virus (VSV), in the *Vesiculovirus* genus, is a prototypic rhabdovirus. While VSV is used as an example in the present disclosure, this disclosure can also be used for other vesiculoviruses and other rhabdoviruses. There are two major serotypes of VSV, New Jersey and Indiana, both of which can infect insects and mammals, causing economically important diseases in cattle, equines and swine. The VSV genome is composed of single-stranded, negative-sense RNA of 11-12 kb, which encodes five viral proteins: the nucleoprotein (N), the phosphoprotein (P), the matrix protein (M), the glycoprotein (G) and the large protein (L). G monomers associate to form trimeric spikes anchored in the viral membrane. Reviewed in, e.g., Sun et al., Future Virol., 2010, 5(1):85-96 and Aurélie et al., Viruses 2012, 4:117-139.

As used herein, the phrase "non-essential portion(s) of the recombinant VSV genome" refers to a region of the VSV genome that can be modified without affecting the development and/or growth of the virus *in vitro* and/or *in vivo* and without affecting the virus's functions required to act as an immunogenic composition or vaccine.

As used herein in connection with various recombinant enveloped viral particles, the term "pseudotyped" refers to viral particles comprising in their lipid envelope molecules, e.g., proteins, glycoproteins, etc, which are mutated and/or heterologous compared to molecules typically found on the surface of a virus from which the particles are derived (i.e., a "reference virus"), and which may affect, contribute to, direct, redirect and/or completely change the tropism of the viral particle in comparison to the reference virus. In some embodiments, a viral particle is pseudotyped such that it recognizes, binds and/or infects a target (ligand or cell) that is different to that of the reference virus. In some embodiments, a viral particle is pseudotyped such that it does not recognize, bind, and/or infect a target (ligand or cell) of the reference virus.

The term "fusogen" or "fusogenic molecule" is used herein to refer to any molecule that can trigger membrane fusion when present on the surface of a virus particle. A fusogen can be, for example, a protein (e.g., a viral glycoprotein) or a fragment or derivative thereof.

The term "replication-competent" is used herein to refer to viruses (including wild-type and recombinant viral particles) that are capable of infecting and propagating within a susceptible cell.

The term "encoding" as used herein can refer to encoding from either the (+) or (-) sense strand of the polynucleotide for expression in the virus particle.

The terms "specifically binds", "specifically adheres", "specifically targets", "selectively binds" or "binding specificity" refer to the ability of spike (S) glycoproteins, recombinant viral particles, vaccines, neutralizing antibodies, or other molecules described herein to bind to their target. In certain embodiments, specific binding refers to binding to a target with an affinity that is at least 10, 50, 100, 250, 500, or 1000 times greater than the affinity for a non-target. In certain embodiments, this affinity is determined by an affinity ELISA assay. In certain embodiments, affinity is determined by a BIAcore assay. In certain embodiments, affinity is determined by a kinetic method. In certain embodiments, affinity is determined by an equilibrium/solution method.

As used herein, the term "reporter protein" refers to any protein which produces a detectable quantifiable signal when present in a cell. Non-limiting examples of reporter proteins useful in the assays of the present disclosure include, e.g., luciferases (including but not limited to, *Renilla* luciferase, mutant *Renilla* luciferase RLuc 8, (dCpG)Luciferase, NanoLuc reporter, firefly luciferase, *Gaussia* luciferase (gLuc), MetLuc, *Vibrio fischeri* lumazine protein, *Vibrio harveyi* luminaze protein, inoflagellate luciferase, firefly luciferase YY5 mutant, firefly luciferase LGR mutant, firefly luciferase mutant E, and derivatives thereof) and fluorescent proteins (including but not limited to, green fluorescent protein (GFP) [e.g., *Aequoria victoria* GFP, *Renilla muelleri* GFP, *Renilla reniformis* GFP, *Renilla ptilosarcus* GFP], GFP-like fluorescent proteins, (GFP-like), enhanced green fluorescent protein (EGFP), yellow fluorescent protein (YFP) [e.g., Topaz, Venus, mCitrine, YPet, TagYFP, PhiYFP, ZsYellow1, mBanana], enhanced yellow fluorescent protein (EYFP), blue fluorescent protein (BFP) [e.g., EBFP2, Azurite, GFP2, GFP10, and mTagBFP], enhanced blue fluorescent protein (EBFP), cyan fluorescent protein (CFP) [e.g., mECFP, Cerulean, CyPet, AmCyan1, Midori-Ishi Cyan, TagCFP, mCFPmm, mTFP1 (Teal)], enhanced cyan fluorescent protein (ECFP), superfolder GFP, superfolder YFP, orange fluorescent protein [e.g., Kusabira Orange, Kusabira Orange2, mOrange, mOrange2, dTomato, dTomato-Tandem, TagRFP, TagRFP-T, DsRed, DsRed2, DsRed-Express (T1), DsRed-Monomer, mTangerine], red fluorescent protein [e.g., mRuby, mApple, mStrawberry, AsRed2, mRFP1, JRed, mCherry, HcRed1, mRaspberry, dKeima-Tandem, HcRed-Tandem, mPlum, tdTomato, AQ143], small ultrared fluorescent protein, FMN-binding fluorescent protein, dsRed, qFP611, Dronpa, TagRFP, KFP, EosFP, IrisFP, Dendra, Kaede, KikGr1, emerald fluorescent protein, Azami Green, mWasabi, TagGFP, TurboGFP, AcGFP, ZsGreen, T-Sapphire, and derivatives thereof), β-galactosidase, β-glucuronidase, β-geo, etc.

The term "effective" applied to dose or amount refers to that quantity of a compound (e.g., a recombinant virus) or composition (e.g., pharmaceutical, vaccine or immunogenic composition) that is sufficient to result in a desired activity upon administration to a subject in need thereof. Note that when a combination of active ingredients is administered, the effective amount of the combination may or may not include amounts of each ingredient that would have been effective if administered individually. The exact amount required will vary from subject to subject, depending on the species, age, and general condition of the subject, the severity of the condition being treated, the particular drug or drugs employed, the mode of administration, and the like.

In the context of the present disclosure insofar as it relates to any of the disease conditions recited herein, the terms "treat", "treatment", and the like mean to relieve or alleviate at least one symptom associated with such condition, or to slow or reverse the progression of such condition. Within the meaning of the present disclosure, the term "treat" also denotes to arrest, delay the onset (i.e., the period prior to clinical manifestation of a disease) and/or reduce the risk of developing or worsening a disease. The terms "treat", "treatment", and the like regarding a state, disorder or condition may also include (1) preventing or delaying the appearance of at least one clinical or sub-clinical symptom of the state, disorder or condition developing in a subject that may be afflicted with or predisposed to the state, disorder or condition but does not yet experience or display clinical or subclinical symptoms of the state, disorder or condition; or (2) inhibiting the state, disorder or condition, i.e., arresting, reducing or delaying the development of the disease or a relapse thereof (in case of maintenance treatment) or at least one clinical or sub-clinical symptom thereof; or (3) relieving the disease, i.e., causing regression of the state, disorder or condition or at least one of its clinical or sub-clinical symptoms. Non-limiting examples of the symptoms of the COVID-19 disease, include, without limitation, fever, cough, shortness of breath, pneumonia, acute respiratory distress syndrome (ARDS), acute lung syndrome, loss of sense of smell, loss of sense of taste, sore throat, nasal discharge, gastro-intestinal symptoms (e.g., diarrhea), organ failure (e.g., kidney failure and renal dysfunction), septic shock, and death. When used in connection with a disease caused by a viral infection (e.g., SARS-CoV-2 infection), the terms "prevent", "preventing" or "prevention" refer to prevention of spread of infection in a subject exposed to the virus, e.g., prevention of the virus from entering the subject's cells. The claimed invention though does not cover methods of treatment by therapy.

The terms "individual" or "subject" or "patient" or "animal" refers to humans, veterinary animals (e.g., cats, dogs, cows, horses, sheep, pigs, etc.), veterinary avian species, and experimental animal models of diseases (e.g., mice, rats, ferrets, monkeys, etc.). In a preferred embodiment, the subject is a human.

The terms "nucleic acid", "polynucleotide" and "nucleotide" are used interchangeably and encompass both DNA and RNA, including positive- and negative- stranded, single- and double-stranded, unless specified otherwise.

The phrase "pharmaceutically acceptable", as used in connection with compositions described herein, refers to molecular entities and other ingredients of such compositions that are physiologically tolerable and do not typically produce untoward reactions when administered to a subject (e.g., a human). Preferably, the term "pharmaceutically acceptable" means approved by a regulatory agency of the Federal or a state government or listed in the U.S. Pharmacopeia or other generally recognized pharmacopeia for use in mammals, and more particularly in humans.

The practice of the present disclosure employs, unless otherwise indicated, conventional techniques of statistical analysis, molecular biology (including recombinant techniques), virology, microbiology, cell biology, chemistry and biochemistry, which are within the skill of the art. Such tools and techniques are described in detail in e.g., Sambrook, Fritsch & Maniatis, Molecular Cloning: A Laboratory Manual, Second Edition. Cold Spring Harbor, NY: Cold Spring Harbor Laboratory Press, 1989 (herein "Sambrook et al., 1989"); DNA Cloning: A Practical Approach, Volumes I and II (D.N. Glover ed. 1985); Oligonucleotide Synthesis (M.J. Gait ed. 1984); Nucleic Acid Hybridization [B.D. Hames & S.J. Higgins eds. (1985)]; Transcription And Translation [B.D. Hames & S.J. Higgins, eds.

(1984)]; Animal Cell Culture [R.I. Freshney, ed. (1986)]; Immobilized Cells And Enzymes [IRL Press, (1986)]; B. Perbal, A Practical Guide To Molecular Cloning (1984); Ausubel, F.M. et al. (eds.). Current Protocols in Molecular Biology. John Wiley & Sons, Inc., 1994. These techniques include site directed mutagenesis as described in Kunkel, Proc. Natl. Acad. Sci. USA 82: 488- 492 (1985), U. S. Patent No. 5,071, 743, Fukuoka et al., Biochem. Biophys. Res. Commun. 263: 357-360 (1999); Kim and Maas, BioTech. 28: 196-198 (2000); Parikh and Guengerich, BioTech. 24: 4 28-431 (1998); Ray and Nickoloff, BioTech. 13: 342-346 (1992); Wang et al., BioTech. 19: 556-559 (1995); Wang and Malcolm, BioTech. 26: 680-682 (1999); Xu and Gong, BioTech. 26: 639-641 (1999), U.S. Patents Nos. 5,789, 166 and 5,932, 419, Hogrefe, Strategies l4. 3: 74-75 (2001), U. S. Patents Nos. 5,702,931, 5,780,270, and 6,242,222, Angag and Schutz, Biotech. 30: 486-488 (2001), Wang and Wilkinson, Biotech. 29: 976-978 (2000), Kang et al., Biotech. 20: 44-46 (1996), Ogel and McPherson, Protein Engineer. 5: 467-468 (1992), Kirsch and Joly, Nucl. Acids. Res. 26: 1848-1850 (1998), Rhem and Hancock, J. Bacteriol. 178: 3346-3349 (1996), Boles and Miogsa, Curr. Genet. 28: 197-198 (1995), Barrenttino et al., Nuc. Acids. Res. 22: 541-542 (1993), Tessier and Thomas, Meths. Molec. Biol. 57: 229-237, and Pons et al., Meth. Molec. Biol. 67: 209-218.

Viral Particles

One aspect of the disclosure provides replication competent recombinant vesicular stomatitis virus (VSV) particles. VSV is an attractive virus for production of recombinant viral particles, because it can be produced in high titers and does not cause serious pathology in humans. In the replication competent recombinant VSV particles of the

EP 4 136 457 B1

disclosure, the VSV glycoprotein (G) is replaced by a SARS-CoV-2 S glycoprotein or a fragment of the SARS-CoV-2 S glycoprotein comprising the receptor binding domain thereof, wherein said SARS-CoV-2 S glycoprotein or fragment is capable of mediating infection of a target cell.

Recombinant VSV particles of the disclosure can be produced using methods known in the art, e.g., by providing in an appropriate host cell: (a) DNA that can be transcribed to encode VSV antigenomic (+) RNA (complementary to the VSV genome), (b) a recombinant source of VSV nucleoprotein (N) protein, (c) a recombinant source of VSV phosphoprotein (P) protein, (d) a recombinant source of VSV large protein (L), and (e) foreign DNA; under conditions such that the DNA is transcribed to produce the antigenomic RNA, and a VSV is produced that contains genomic RNA complementary to the antigenomic RNA produced and foreign RNA, which is not naturally a part of the VSV genome, from the DNA. Methods and compositions useful for generating recombinant VSV particles may be found, for example, in U.S. Patent Nos. 7,153,510; 9,861,668; 8,012,489; 9,630,996; 8,287,878; 9,248,178 U.S. Patent Publication Nos. 2014/0271564; 2012/0121650; Fukishi et al., J. Gen. Virol., 2005, 86:2269-2274.

In addition to encoding the S glycoprotein from SARS-CoV-2 or the fragment thereof, the genome of the recombinant VSV encodes a reporter protein. Non-limiting examples of reporter proteins include, e.g., luciferases (including but not limited to, *Renilla* luciferase, RLuc8 mutant *Renilla* luciferase, (dCpG)Luciferase, NanoLuc reporter, firefly luciferase, *Gaussia* luciferase (gLuc), MetLuc, *Vibrio fischeri* lumazine protein, *Vibrio harveyi* luminaze protein, inoflagellate luciferase, firefly luciferase YY5 mutant, firefly luciferase LGR mutant, firefly luciferase mutant E, and derivatives thereof) and fluorescent proteins (including but not limited to, green fluorescent protein (GFP) [e.g., *Aequoria victoria* GFP, *Renilla muelleri* GFP, *Renilla reniformis* GFP, *Renilla ptilosarcus* GFP], GFP-like fluorescent proteins, (GFP-like), enhanced green fluorescent protein (EGFP), yellow fluorescent protein (YFP) [e.g., Topaz, Venus, mCitrine, YPet, TagYFP, PhiYFP, ZsYellow1, mBanana], enhanced yellow fluorescent protein (EYFP), blue fluorescent protein (BFP) [e.g., EBFP2, Azurite, GFP2, GFP10, and mTagBFP], enhanced blue fluorescent protein (EBFP), cyan fluorescent protein (CFP) [e.g., mECFP, Cerulean, CyPet, AmCyan1, Midori-Ishi Cyan, TagCFP, mCFPmm, mTFP1 (Teal)], enhanced cyan fluorescent protein (ECFP), superfolder GFP, superfolder YFP, orange fluorescent protein [e.g., Kusabira Orange, Kusabira Orange2, mOrange, mOrange2, dTomato, dTomato-Tandem, TagRFP, TagRFP-T, DsRed, DsRed2, DsRed-Express (T1), DsRed-Monomer, mTangerine], red fluorescent protein [e.g., mRuby, mApple, mStrawberry, AsRed2, mRFP1, JRed, mCherry, HcRed1, mRaspberry, dKeima-Tandem, HcRed-Tandem, mPlum, tdTomato, AQ143], small ultrared fluorescent protein, FMN-binding fluorescent protein, dsRed, qFP611, Dronpa, TagRFP, KFP, EosFP, IrisFP, Dendra, Kaede, KikGr1, emerald fluorescent protein, Azami Green, mWasabi, TagGFP, TurboGFP, AcGFP, ZsGreen, T-Sapphire, and derivatives thereof), β-galactosidase, β-glucuronidase, β-geo, etc.

The replication competent recombinant VSV particles can be used for seropositivity assays for determining the presence of a coronavirus neutralizing antibody (e.g., a SARS-CoV-2 neutralizing antibody) in a sample from a subject. Such seropositivity assays can be used, for example, for determining whether or not the subject was previously infected by said coronavirus and has produced neutralizing antibodies protecting such subject from the infection (and thus allowing such subject to return to work, etc.). Seropositivity assays of the disclosure can be also used, for example, for determining effectiveness of vaccination, for development of therapeutic antibodies, for selecting best donors for convalescent plasma therapy, for patient contact tracing, for identifying the viral reservoir hosts, for determining the burden of disease, for determining the rate of asymptomatic infections, for identifying the extent of virus spread in households, communities, and specific settings, etc.

In certain embodiments, the seropositivity assays of the disclosure use more than one type of replication competent recombinant VSV particles. In certain embodiments, such assays use a mixture of two or more replication competent recombinant VSV particles encoding different SARS-CoV-2 S glycoproteins originating from different viral strains, variants or mutants.

In certain aspects, the current disclosure provides cells for production of the replication competent recombinant VSV particles of the disclosure. Exemplary cells include, but are not limited to, any cell in which VSV grows, e.g., mammalian cells and some insect (e.g., Drosophila) cells. A vast number of primary cells and cell lines commonly known in the art can be used as host cells. By way of example, useful cell lines include but are not limited to BHK (baby hamster kidney) cells, CHO (Chinese hamster ovary) cells, HeLA (human) cells, mouse L cells, Vero (monkey) cells (including Vero-αHis cells), Vero-Ace-2 cells, Vero-TRMPSS2 cells, Vero-E6 cells, ESK-4, PK-15, EMSK cells, MDCK (Madin-Darby canine kidney) cells, MDBK (Madin-Darby bovine kidney) cells, 293 (human) cells, Hep-2 cells, primary chick embryo fibroblasts, primary chick embryo fibroblasts, quasi-primary continuous cell lines (e.g. AGMK-African green monkey kidney cells), human diploid primary cell lines (e.g. WI-38 and MRC5 cells), and Monkey Diploid Cell Line (e.g. FRhL-Fetal Rhesus Lung cells).

## VSV DNA for Transcription to Produce VSV Antigenomic (+) RNA

Vesicular Stomatitis Virus (VSV) is a rhabdovirus that comprises a single (non-segmented) negative-stranded genomic RNA that is generally transcribed by a virion polymerase into five mRNAs encoding five structural proteins. The five structural proteins include glycoprotein (G), large protein (L), phosphoprotein (P), matrix protein (M), and nucleoprotein (N). The nucleocapsid protein encapsidates the RNA genome. Two proteins that form a polymerase complex are bound to the nucleocapsid. A matrix (M) protein is associated with the nucleocapsid and the membrane. A single (transmembrane) envelope spike glycoprotein (G) extends from the viral envelope. The G protein functions to bind virus to a cellular receptor and to catalyze fusion of the viral membrane with cellular membranes to initiate the infectious cycle. The size of the VSV genome is about 11 kilobases.

VSV can be transmitted to a variety of mammalian hosts, generally cattle, horses, swine and rodents. VSV infection of humans is uncommon, and in general is either asymptomatic or characterized by mild flu-like symptoms that resolve in three to eight days without complications. VSV is not considered a human pathogen and pre-existing immunity to VSV is rare in the human population making VSV an attractive viral vector for vaccine and therapeutic applications. Other beneficial characteristics of VSV include, but are not limited to, (i) ability to replicate robustly in cell culture, (ii) inability to either integrate into host cell DNA or undergo genetic recombination, (iii) multiple serotypes can allow for prime-boost immunization strategies, and (iv) foreign genes of interest can be inserted into the VSV genome and expressed abundantly by the viral transcriptase.

Fusion of rhabdoviruses (e.g., VSV) to cells, and their subsequent uptake, is described in Belot, L. et al., "Structural and cellular biology of rhabdovirus entry", Adv. Virus Res., 2019, 104:147-183, and Albertini, A.A.V. et al., "Molecular and Cellular Aspects of Rhabdovirus Entry" Viruses, 2012, 4:117-139. Further description of endocytosis of VSV is found in Sun, X. et al., "Internalization and fusion mechanism of vesicular stomatitis virus and related rhabdoviruses" Future Virol., 2010, 5(1):85-96. For general information on virus-cell fusion, see Igonet, S. et al., "SnapShot: Viral and Eukaryotic Protein Fusogens" Cell, 2012, 151:1634e1.

Cell-cell fusion mediated by other viruses, such as HIV virus, has been described in Kondo, N. et al., "Conformational changes of the HIV-1 envelope protein during membrane fusion are inhibited by the replacement of its membrane-spanning domain" Journ. Biol. Chem., 2010, 285(19):14681-88.

Many vesiculoviruses are known in the art and can be made recombinant according to the methods disclosed herein. Non-limiting examples of such vesiculoviruses are listed below:

| Virus | Source of virus in nature |
|---|---|
| VSV-New Jersey | Mammals, mosquitoes, midges, blackflies, houseflies |
| VSV-Indiana | Mammals, mosquitoes, sandflies |
| Alagoas | Mammals, sandflies |
| Cocal | Mammals, mosquitoes, mites |
| Jurona | Mosquitoes |
| Carajas | Sandflies |
| Maraba | Sandflies |
| Piry | Mammals |
| Calchaqui | Mosquitoes |
| Yug Bogdanovac | Sandflies |
| Isfahan | Sandflies, ticks |
| Chandipura | Mammals, sandflies |
| Perinct | Mosquitoes, sandflies |
| Porton-S | Mosquitoes |

In certain embodiments, VSVs are the vesiculoviruses used to make the replication competent recombinant viruses of the disclosure. In certain embodiments, the recombinant VSV is a recombinant VSV-New Jersey or VSV-Indiana. In certain embodiments, the recombinant VSV is a recombinant VSV-New Jersey. VSV is used as an example in the disclosure below, and this disclosure can also be used for other vesiculoviruses.

Any DNA that can be transcribed to produce VSV antigenomic (+) RNA (complementary to the VSV genome) can be used for the construction of a recombinant DNA containing foreign DNA encoding a heterologous (foreign) protein or peptide, for use in producing the replication competent recombinant VSV particles of the disclosure.

The VSV vector can be genetically modified to include one or more mutations or "mutation classes" in the genome. "Mutation class", "mutation classes" or "classes of mutation" are used interchangeably, and refer to mutations known in the art, when used singly, to attenuate VSV. Exemplary mutation classes include, but are not limited to, a VSV temperature-sensitive N gene mutation (hereinafter, "N(ts)"), a temperature-sensitive L gene mutation (hereinafter, "L(ts)"), a point mutation, a G-stem mutation (hereinafter, "G(stem)"), a non-cytopathic M gene mutation (hereinafter, "M(ncp)"), a gene shuffling or rearrangement mutation, a truncated G gene mutation (hereinafter, "G(ct)"), an ambisense RNA mutation, a G gene insertion mutation, a gene deletion mutation and the like. Mutations can be insertions, deletions, substitutions, gene rearrangement or shuffling modifications.

Also provided is a VSV particle wherein (i) the VSV glycoprotein (G) is replaced by a Severe Acute Respiratory Syndrome coronavirus 2 (SARS-CoV-2) spike (S) glycoprotein or a fragment of the SARS-CoV-2 S glycoprotein comprising the receptor binding domain thereof, wherein said SARS-CoV-2 S glycoprotein or fragment is capable of mediating infection of a target cell and wherein (ii) the VSV particle comprises a nucleic acid molecule comprising a nucleic acid sequence encoding said reporter protein, wherein the nucleic acid sequence encoding the reporter protein is inserted between the nucleic acid sequence encoding the SARS-CoV-2 S glycoprotein and the nucleic acid sequence encoding VSV large (L) protein.

The mutations can attenuate the infectivity, virulence or pathogenic effects of VSV. The attenuation can be additive or synergistic. With synergistic attenuation, the level of VSV attenuation is greater than additive. Synergistic attenuation of VSV can arise from combining at least two classes of mutation in the same VSV genome, thereby resulting in a reduction of VSV pathogenicity much greater than an additive attenuation level observed for each VSV mutation class alone. A synergistic attenuation of VSV can provide for an LD50 at least greater than the additive attenuation level observed for each mutation class alone (i.e., the sum of the two mutation classes), where attenuation levels (i.e., the LD50) are determined in a small animal neurovirulence model.

The VSV M gene encodes the virus matrix (M) protein, and two smaller in-frame polypeptides (M2 and M3). The M2 and M3 polypeptides can be translated from the same open reading frame (ORF) as the M protein and lack the first 33 and 51 amino acids, respectively. A recombinant VSV vector comprising non-cytopathic M gene mutations (i.e., VSV vectors that also do not express M2 and M3 proteins) can be generated and can further comprise one or more additional mutation(s) thereby resulting in a VSV vector that was highly attenuated in cell culture and in animals.

In certain embodiments, the replication competent recombinant VSV particles of the disclosure comprise a non-cytopathic mutation in the M gene. The VSV (Indiana serotype) M gene encodes a 229 amino acid M (matrix) protein in which the first thirty amino acids of the NH2-terminus comprise a proline-rich PPPY (PY) motif (SEQ ID NO: 68). The PY motif of VSV M protein is located at amino acid positions 24-27 in both VSV Indiana (Genbank Accession Number X04452) and New Jersey (Genbank Accession Number M14553) serotypes. The VSV may comprise mutations in the PY motif (e.g., APPY (SEQ ID NO: 69), AAPY (SEQ ID NO: 70), PPAY (SEQ ID NO: 71), APPA (SEQ ID NO: 72), AAPA (SEQ ID NO: 73) and PPPA (SEQ ID NO: 74)). The VSV can comprise any of various amino acid mutations (e.g., deletions, substitutions, insertions, etc.) into the M protein PSAP (PS) motif (SEQ ID NO: 75). These and other mutations in the PY motif may be effective to reduce virus yield by blocking a late stage in virus budding.

The replication competent recombinant VSV particles of the disclosure may comprise one or more M gene mutations. Non-limiting examples of M protein mutations include, e.g., a glycine changed to a glutamic acid at position (21), a leucine changed to a phenylalanine at position (111), a methionine changed to an arginine at position (51), a glycine changed to a glutamic acid at position (22), a methionine changed to an arginine at position (48), a leucine changed to a phenylalanine at position (110), a methionine changed to an alanine at position (51), and a methionine changed to an alanine at position (33). See, e.g., U.S. Patent No. 9,630,996. In various embodiments of the methods described herein, the genome of the recombinant VSV encodes a mutant VSV matrix M protein comprising the M51R mutation. Mutation M51R eliminates M protein's ability to block cellular nucleo-cytoplasmic transport and thus substantially attenuates VSV infectivity.

In certain embodiments, the DNA that can be transcribed to encode VSV antigenomic (+) RNA comprises a gene that encodes a VSV M protein. In certain embodiments, the VSV M protein used in the methods and compositions described herein may comprise the amino acid sequence of SEQ ID NO: 9, or a sequence at least 50%, 60%, 70%, 80%, 81%, 82%, 83%, 84%, 85%, 86%, 87%, 88%, 89%, 90%, 91%, 92%, 93%, 94%, 95%, 96%, 97%, 98%, 99%, 99.5% or more identical to the amino acid sequence of SEQ ID NO: 9. In certain embodiments, the VSV M protein used in the vaccines or methods described herein may consist of the amino acid sequence of SEQ ID NO: 9, or a sequence at least 50%, 60%, 70%, 80%, 81%, 82%, 83%, 84%, 85%, 86%, 87%, 88%, 89%, 90%, 91%, 92%, 93%, 94%, 95%, 96%, 97%, 98%, 99%, 99.5% or more identical to the amino acid sequence of SEQ ID NO: 9. In certain embodiments, the mutated VSV M protein used in the vaccines or methods described herein may comprise the amino acid sequence of SEQ ID NO: 7, or a sequence at least 50%, 60%, 70%, 80%, 81%, 82%, 83%, 84%, 85%, 86%, 87%, 88%, 89%, 90%, 91%, 92%, 93%, 94%, 95%, 96%, 97%, 98%, 99%, 99.5% or more identical to the amino acid sequence of SEQ ID NO: 7. In certain embodiments, the mutant VSV M protein used in the vaccines or methods described herein may consist of the amino acid sequence of SEQ ID NO: 7, or a sequence at least 50%, 60%, 70%, 80%, 81%, 82%, 83%, 84%, 85%, 86%, 87%, 88%, 89%, 90%, 91%, 92%, 93%, 94%, 95%, 96%, 97%, 98%, 99%, 99.5% or more identical to the amino acid sequence of SEQ ID NO: 7.

DNA that can be transcribed to produce VSV (for example) antigenomic (+) RNA (such DNA being referred to herein as "VSV (-) DNA") is available in the art and/or can be obtained by standard methods. VSV (-) DNA for any serotype or strain known in the art, e.g., the New Jersey or Indiana serotypes of VSV, can be used. The complete nucleotide and deduced protein sequence of the VSV genome is known, and is available as Genbank VSVCG, Accession No. J02428; NCBI Seq ID 335873; and is published in Rose and Schubert, 1987, in The Viruses: The Rhabdoviruses, Plenum Press, NY, pp. 129-166. An example of the complete sequence of the VSV(-) DNA that is contained in plasmid pVSVFL(+)is shown, e.g., in U.S. Patent No. 7,153,510. Sequences of other vesiculovirus genomes have been published and are available in the art.

VSV (-) DNA, if not already available, can be prepared by standard methods, as follows: VSV genomic RNA can be purified from virus preparations, and reverse transcription with long distance polymerase chain reaction used to generate the v (-) DNA. Alternatively, after purification of genomic RNA, VSV mRNA can be synthesized *in vitro,* and cDNA prepared by standard methods, followed by insertion into cloning vectors (see, e.g., Rose and Gallione, 1981, J. Virol. 39(2):519-528). Individual cDNA clones of VSV RNA can be joined by use of small DNA fragments covering the gene junctions, generated by use of reverse transcription and polymerase chain reaction (RT-PCR) (Mullis and Faloona, 1987, Meth. Enzymol. 155:335-350) from VSV genomic RNA (see Section 6, infra). VSV and other vesiculoviruses are available in the art.

In certain embodiments, one or more, usually unique, restriction sites (e.g., in a polylinker) are introduced into the VSV (-) DNA, in intergenic regions, or 5' of the sequence complementary to the 3' end of the VSV genome, or 3' of the sequence complementary to the 5' end of the VSV genome, to facilitate insertion of the foreign DNA.

In certain embodiments, the VSV (-) DNA is constructed so as to have a promoter operatively linked thereto. The promoter should be capable of initiating transcription of the (-) DNA in an animal or insect cell in which it is desired to produce the recombinant VSV. Promoters which may be used include, but are not limited to, the SV40 early promoter region (Bernoist and Chambon, 1981, Nature 290:304-310), the promoter contained in the 3' long terminal repeat of Rous sarcoma virus (Yamamoto, et al., 1980, Cell 22:787-797), the herpes thymidine kinase promoter (Wagner et al., 1981, Proc. Natl. Acad. Sci. U.S.A. 78:1441-1445), the regulatory sequences of the metallothionein gene (Brinster et al., 1982, Nature 296:39-42); heat shock promoters (e.g., hsp70 for use in Drosophila S2 cells); the ADC (alcohol dehydrogenase) promoter, PGK (phosphoglycerol kinase) promoter, alkaline phosphatase promoter, and the following animal transcriptional control regions, which exhibit tissue specificity and have been utilized in transgenic animals: elastase I gene control region which is active in pancreatic acinar cells (Swift et al., 1984, Cell 38:639-646; Ornitz et al., 1986, Cold Spring Harbor Symp. Quant. Biol. 50:399-409; MacDonald, 1987, Hepatology 7:425-515); insulin gene control region which is active in pancreatic beta cells (Hanahan, 1985, Nature 315:115-122), immunoglobulin gene control region which is active in lymphoid cells (Grosschedl et al., 1984, Cell 38:647-658; Adames et al., 1985, Nature 318:533-538; Alexander et al., 1987, Mol. Cell. Biol. 7:1436-1444), mouse mammary tumor virus control region which is active in testicular, breast, lymphoid and mast cells (Leder et al., 1986, Cell 45:485-495), albumin gene control region which is active in liver (Pinkert et al., 1987, Genes and Devel. 1:268-276), alpha-fetoprotein gene control region which is active in liver (Krumlauf et al., 1985, Mol. Cell. Biol. 5:1639-1648; Hammer et al., 1987, Science 235:53-58; alpha 1-antitrypsin gene control region which is active in the liver (Kelsey et al., 1987, Genes and Devel. 1:161-171), beta-globin gene control region which is active in myeloid cells (Mogram et al., 1985, Nature 315:338-340; Kollias et al., 1986, Cell 46:89-94; myelin basic protein gene control region which is active in oligodendrocyte cells in the brain (Readhead et al., 1987, Cell 48:703-712); and myosin light chain-2 gene control region which is active in skeletal muscle (Sani, 1985, Nature 314:283-286). Preferably, the promoter is an RNA polymerase promoter, preferably a bacteriophage or viral or insect RNA polymerase promoter, including but not limited to the promoters for T7 RNA polymerase, SP6 RNA polymerase, and T3 RNA polymerase. If an RNA polymerase promoter is used in which the RNA polymerase is not endogenously produced by the host cell in which it is desired to produce the recombinant VSV, a recombinant source of the RNA polymerase must also be provided in the host cell.

The VSV (-) DNA can be operably linked to a promoter before or after insertion of foreign DNA. In certain embodiments, a transcriptional terminator is situated downstream of the VSV (-) DNA.

In another embodiment, a DNA sequence that can be transcribed to produce a ribozyme sequence is situated at the immediate 3' end of the VSV (-) DNA, prior to the transcriptional termination signal, so that upon transcription a self-cleaving ribozyme sequence is produced at the 3' end of the antigenomic RNA, which ribozyme sequence will autolytically cleave (after a U) this fusion transcript to release the exact 3' end of the VSV antigenomic (+) RNA. Any ribozyme sequence known in the art may be used, as long as the correct sequence is recognized and cleaved. In a preferred aspect, hepatitis delta virus (HDV) ribozyme is used (Perrotta and Been, 1991, Nature 350:434-436; Pattnaik et al., 1992, Cell 69:1011-1020).

An example of a VSV(-) DNA for use, for insertion of foreign DNA, can thus comprises (in 5' to 3' order) the following operably linked components: the T7 RNA polymerase promoter, VSV (-) DNA, a DNA sequence that is transcribed to produce an HDV ribozyme sequence (immediately downstream of the VSV (-) DNA), and a T7 RNA polymerase transcription termination site.

Examples of plasmids that can be used are, pVSVFL(+) or pVSVSS1.

The G gene of VSV in the VSV (-) DNA of plasmid pVSVFL(+) can be excised and replaced, by cleavage at the NheI and MluI sites flanking the G gene and insertion of the desired sequence encoding the SARS-CoV-2 S glycoprotein or the fragment thereof. The SARS-CoV-2 S protein forms a part of the VSV envelope and thus is surface-displayed in the VSV particle.

Coronavirus Spike (S) Glycoprotein

Coronavirus entry into host cells is mediated by the transmembrane spike (S) glycoprotein (interchangeably referred to as "spike glycoprotein", "S glycoprotein", "S protein" or "spike protein") which is the main target of anti-viral neutralizing antibodies and is the focus of therapeutic and vaccine design. S glycoprotein forms homotrimers protruding from the viral surface. S glycoprotein comprises two functional subunits responsible for binding to the host cell receptor (S1 subunit) and fusion of the viral and cellular membranes (S2 subunit). For many coronaviruses, including SARS-CoV-1 and SARS-CoV-2, S glycoprotein is cleaved at the boundary between the S1 and S2 subunits, which remain non-covalently bound in the prefusion conformation. The distal S1 subunit comprises the receptor-binding domain(s) (RBD) and contributes to stabilization of the prefusion state of the membrane-anchored S2 subunit that contains the fusion machinery. S is further cleaved by host proteases at the S2' site located immediately upstream of the fusion peptide. This cleavage has been proposed to activate the protein for membrane fusion via conformational changes. Walls et al., Cell, published online March 9, 2020; available at doi.org/10.1016/j.cell.2020.02.058.

SARS-CoV-1 and SARS-CoV-2 can interact directly with angiotensin-converting enzyme 2 (ACE2) to enter target cells and may also employ the cellular serine protease, transmembrane protease, serine 2 (TMPRSS2) for S protein priming (Hoffmann et al., Cell, 2020, 181:1-10; available at doi.org/10.1016/j.cell.2020.02.052). SARS-CoV-S und SARS-CoV-2-S share about 76% amino acid identity. The receptor binding domain (RBD) in the S glycoprotein is the most variable part of the coronavirus genome. Six RBD amino acids have been shown to be critical for binding to ACE2 receptors and for determining the host range of SARS-CoV-like viruses. They are Y442, L472, N479, D480, T487 and Y4911 in SARS-CoV, which correspond to L455, F486, Q493, S494, N501 and Y505 in SARS-CoV-2 (Andersen et al., Nature Medicine, 2020; available at doi.org/10.1038/s41591-020-0820-9).

In various embodiments, the SARS-CoV-2 S glycoprotein may be a full-length SARS-CoV-2 S glycoprotein (comprising or consisting of SEQ ID NO: 1) or a fragment thereof that has at least 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 99.5% or more amino acid sequence identity to SEQ ID NO: 1, wherein the SARS-CoV-2 S glycoprotein or the fragment comprises the receptor binding domain and is capable of mediating infection of a target cell.

The wild-type coronavirus S glycoprotein comprises an S1 subunit that facilitates binding of the coronavirus to cell surface proteins. Without wishing to be bound by theory, the S1 subunit of the wildtype S glycoprotein controls which cells are infected by the coronavirus. The wild-type S glycoprotein also comprises a S2 subunit, which is a transmembrane subunit that facilitates viral and cellular membrane fusion. In the various aspects and embodiments described herein, a fragment of SARS-CoV-2 S glycoprotein can comprise the S1 subunit of the SARS-CoV-2 S glycoprotein or a fragment that has at least 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 99.5% or more amino acid sequence identity to the S1 subunit of the SARS-CoV-2 S glycoprotein. In some embodiments described herein, a fragment of SARS-CoV-2 S glycoprotein can comprise a sequence that has at least 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 99.5% or more amino acid sequence identity to amino acids 14-684 of SEQ ID NO: 1. In the various aspects and embodiments described herein, a fragment of SARS-CoV-2 S glycoprotein comprises the S2 subunit of the SARS-CoV-2 S glycoprotein or a fragment that has at least 91%, 92%, 93%, 94%, 95%, 96%, 97%, 98%, 99%, 99.5% or more amino acid sequence identity to the S2 subunit of the SARS-CoV-2 S glycoprotein.

The wild-type coronavirus S glycoprotein comprises a receptor binding domain (RBD) that facilitates binding of the coronavirus to its receptor on the host cell. The RBD of the SARS-CoV-2 spike (S) glycoprotein is described, e.g., in Anderson et al., Nature Medicine, 2020 (available at doi.org/10.1038/s41591-020-0820-9). In the various aspects and embodiments described herein, a fragment of SARS-CoV-2 S glycoprotein comprises the RBD of the SARS-CoV-2 S glycoprotein that has at least 74%, 75%, 76%, 77%, 78%, 80%, 81%, 82%, 83%, 84%, 85%, 86%, 87%, 88%, 89%, 90%, 91%, 92%, 93%, 94%, 95%, 96%, 97%, 98%, 99%, 99.5% or more amino acid sequence identity to the RBD of the SARS-CoV-2 S glycoprotein, e.g., amino acids 319-541 of SEQ ID NO: 1.

In certain embodiments, the SARS-CoV-2 S glycoprotein, or fragment thereof, may comprise or consist of an insertion, deletion, and/or substitution of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, or 39 residues of the SARS-CoV-2 S glycoprotein. Non-limiting examples of amino acids for potential deletion include, e.g., a tyrosine at position (145), an asparagine at position (679), a serine at position (680), proline at position (681), an arginine at position (682), an arginine at position (683), an alanine at position (684), and/or an arginine at position (685), positions as denoted in SEQ ID NO: 1, or the equivalent amino acid residue in a mutant SARS-CoV-2 S glycoprotein sequence. Non-limiting examples of amino acids for potential substitution include, e.g., a leucine changed to a phenylalanine at position (5) a tyrosine changed to an asparagine at position (28), a threonine changed to an isoleucine at position (29), a histidine changed to a tyrosine at position (49), a leucine changed to a phenylalanine at position (54), an asparagine changed to a lysine at position (74), a glutamic acid changed to an aspartic acid at position (96), an aspartic acid changed to an asparagine at position (111), a phenylalanine changed to a leucine at position (157), a glycine changed to a valine at position (181), a serine changed to a tryptophan at position (221), a serine changed to an arginine at position (247), an alanine changed to a threonine at position (348), an arginine changed to an isoleucine at position (408), a glycine changed to a serine at position (476), a valine changed to an alanine at position (483), a histidine changed to a glutamine at position (519), an alanine changed to a serine at position (520), an aspartic acid changed to an asparagine at position (614), an aspartic acid changed to a glycine at position (614), an asparagine changed to an isoleucine at position (679), a serine change to a leucine at position (680), an arginine changed to a glycine at position (682), an arginine changed to a serine at position (683), an arginine changed to a glutamine at position (685), an arginine changed to a serine at position (685), a phenylalanine changed to a cysteine at position (797), an alanine changed to a valine at position (930), an aspartic acid changed to a tyrosine at position (936), an alanine changed to a valine at position (1078), an aspartic acid changed to a histidine at position (1168), and/or an aspartic acid changed to a histidine at position (1259), positions as denoted in SEQ ID NO: 1, or the equivalent amino acid residue in a mutant SARS-CoV-2 S glycoprotein sequence. *See* Becerra-Flores and Cardozo, "SARS-CoV-2 viral spike G614 mutation exhibits higher case fatality rate," The International Journal of Clinical Practice, published online May 6, 2020; Eaaswarkhanth et al., "Could the D614G substitution in the SARS-CoV-2 spike (S) protein be associated with higher COVID-19 mortality?" International Journal of Infectious Diseases, 96: July 2020, Pages 459-460; Tang et al., "The SARS-CoV-2 Spike Protein D614G Mutation Shows Increasing Dominance and May Confer a Structural Advantage to the Furin Cleavage Domain," Preprints 2020, 2020050407 (doi: 10.20944/preprints202005.0407.v1); Hansen et. al., "Studies in humanized mice and convalescent humans yield a SARS-CoV-2 antibody cocktail" Science, published online June 15, 2020; Lokman et al., "Exploring the genomic and proteomic variations of SARS-CoV-2 spike glycoprotein: A computational biology approach", Infection, Genetics and Evolution : Journal of Molecular Epidemiology and Evolutionary Genetics in Infectious Diseases, 2020 Jun;84:104389. DOI: 10.1016/j.meegid.2020.104389. Additional non-limiting examples of amino acid residue positions for insertion, deletion, and/or substitution include those as listed in Tables 8 and 9 (amino acid residue positions are denoted using SEQ ID NO: 1 as a reference sequence, which can be used as a reference for identifying the equivalent amino acid residue in any SARS-CoV-2 S glycoprotein sequence (same as above)). Each residue modification listed in Table 8 can separately be used alone or in combination with others to generate variants of a replication competent recombinant vesicular stomatitis virus (VSV) particle. In certain embodiments, the SARS-CoV-2 S glycoprotein, or fragment thereof, differs in amino acid sequence from the reference peptide or polypeptide (a wild-type SARS-CoV-2 spike protein) by changing a serine to an arginine at position (247), an aspartic acid to an asparagine at position (614), and/or an arginine to a glutamine at position (685), positions as denoted in SEQ ID NO: 1, or the equivalent amino acid residue in a mutant SARS-CoV-2 S glycoprotein sequence. In certain embodiments, the SARS-CoV-2 S glycoprotein, or fragment thereof, differs in amino acid sequence from the reference peptide or polypeptide by changing a serine to an arginine at position (247). In certain embodiments, the SARS-CoV-2 S glycoprotein, or fragment thereof, differs in amino acid sequence from the reference peptide or polypeptide by changing an aspartic acid to an asparagine at position (614). In certain embodiments, the SARS-CoV-2 S glycoprotein, or fragment thereof, differs in amino acid sequence from the reference peptide or polypeptide by changing an arginine to a glutamine at position (685). In certain embodiments, the SARS-CoV-2 S glycoprotein, or fragment thereof, differs in amino acid sequence from the reference peptide or polypeptide by changing a serine to an arginine at position (247) and an aspartic acid to an asparagine at position (614). In certain embodiments, the SARS-CoV-2 S glycoprotein, or fragment thereof, differs in amino acid sequence from the reference peptide or polypeptide by changing a serine to an arginine at position (247) and an arginine to a glutamine at position (685). In certain embodiments, the SARS-CoV-2 S glycoprotein, or fragments thereof, differs in amino acid sequence from the reference peptide or polypeptide by changing an aspartic acid to an asparagine at position (614) and an arginine to a glutamine at position (685). In certain embodiments, the SARS-CoV-2 S glycoprotein, or fragment thereof, differs in amino acid sequence from the reference peptide or polypeptide by changing a serine to an arginine at position (247), an aspartic acid to an asparagine at position (614), and an arginine to a glutamine at position (685). In certain embodiments, the SARS-CoV-2 S glycoprotein, or fragment thereof, result in a more lytic phenotype. In certain embodiments, the SARS-CoV-2 S glycoprotein fragment may comprise the amino acid sequence of SEQ ID NO: 42, or a sequence at least 50%, 60%, 70%, 80%, 81%, 82%, 83%, 84%, 85%, 86%, 87%, 88%, 89%, 90%, 91%, 92%, 93%, 94%, 95%, 96%, 97%, 98%, 99%, or 99.5% identical to the amino acid sequence of SEQ ID NO: 42. In certain embodiments, the SARS-CoV-2 S glycoprotein fragment may be encoded by a codon optimized nucleotide sequence. In various embodiments, the SARS-CoV-2 S glycoprotein fragment may be encoded by the polynucleotide sequence of SEQ ID NO: 43 or a sequence that has at least 50%, 60%, 70%, 80%, 81%, 82%, 83%, 84%, 85%, 86%, 87%, 88%, 89%, 90%, 91%, 92%, 93%, 94%, 95%, 96%, 97%, 98%, 99%, or 99.5% polynucleotide sequence identity to SEQ ID NO: 43. In certain embodiments, the SARS-CoV-2 S glycoprotein fragment may comprise the amino acid sequence of SEQ ID NO: 44, or a sequence at least 50%, 60%, 70%, 80%, 81%, 82%, 83%, 84%, 85%, 86%, 87%, 88%, 89%, 90%, 91%, 92%, 93%, 94%, 95%, 96%, 97%, 98%, 99%, or 99.5% identical to the amino acid sequence of SEQ ID NO: 44. In certain embodiments, the SARS-CoV-2 S glycoprotein, or fragments thereof, differs in amino acid sequence from the reference peptide or polypeptide (a wild-type SARS-CoV-2 spike protein) by changing an asparagine to a tyrosine at position (501), and/or a glutamic acid to a lysine at position (484), and/or an aspartic acid to a glycine at position (614), and/or deletion of residues 69-70, positions as denoted in SEQ ID NO: 1, or the equivalent amino acid residue in a mutant SARS-CoV-2 S glycoprotein sequence. In certain embodiments, the SARS-CoV-2 S glycoprotein, or fragment thereof, differs in amino acid sequence from the reference peptide or polypeptide by changing an asparagine to a tyrosine at position (501). In certain embodiments, the SARS-CoV-2 S glycoprotein, or fragment thereof, differs in amino acid sequence from the reference peptide or polypeptide by changing a glutamic acid to a lysine at position (484). In certain embodiments, the SARS-CoV-2 S glycoprotein, or fragment thereof, differs in amino acid sequence from the reference peptide or polypeptide by changing an aspartic acid to a glycine at position (614). In certain embodiments, the SARS-CoV-2 S glycoprotein, or fragment thereof, differs in amino acid sequence from the reference peptide or polypeptide by deletion of residues 69-70. In certain embodiments, the SARS-CoV-2 S glycoprotein, or fragment thereof, differs in amino acid sequence from the reference peptide or polypeptide by changing an asparagine to a tyrosine at position (501) and a glutamic acid to a lysine at position (484). In certain embodiments, the SARS-CoV-2 S glycoprotein, or fragment thereof, differs in amino acid sequence from the reference peptide or polypeptide by changing an asparagine to a tyrosine at position (501) and an aspartic acid to a glycine at position (614). In certain embodiments, the SARS-CoV-2 S glycoprotein, or fragment thereof, differs in amino acid sequence from the reference peptide or polypeptide by changing an asparagine to a tyrosine at position (501) and deletion of residues 69-70. In certain embodiments, the SARS-CoV-2 S glycoprotein, or fragment thereof, differs in amino acid sequence from the reference peptide or polypeptide by changing a glutamic acid to a lysine at position (484) and an aspartic acid to a glycine at position (614). In certain embodiments, the SARS-CoV-2 S glycoprotein, or fragment thereof, differs in amino acid sequence from the reference peptide or polypeptide by changing a glutamic acid to a lysine at position (484) and deletion of residues 69-70. In certain embodiments, the SARS-CoV-2 S glycoprotein, or fragment thereof, differs in amino acid sequence from the reference peptide or polypeptide by changing an aspartic acid to a glycine at position (614) and deletion of residues 69-70. In certain embodiments, the SARS-CoV-2 S glycoprotein, or fragment thereof, differs in amino acid sequence from the reference peptide or polypeptide by changing an asparagine to a tyrosine at position (501), a glutamic acid to a lysine at position (484), and an aspartic acid to a glycine at position (614). In certain embodiments, the SARS-CoV-2 S glycoprotein, or fragment thereof, differs in amino acid sequence from the reference peptide or polypeptide by changing an asparagine to a tyrosine at position (501), changing a glutamic acid to a lysine at position (484), and deletion of residues 69-70. In certain embodiments, the SARS-CoV-2 S glycoprotein, or fragment thereof, differs in amino acid sequence from the reference peptide or polypeptide by changing an asparagine to a tyrosine at position (501), changing an aspartic acid to a glycine at position (614), and deletion of residues 69-70. In certain embodiments, the SARS-CoV-2 S glycoprotein, or fragment thereof, differs in amino acid sequence from the reference peptide or polypeptide by changing a glutamic acid to a lysine at position (484), changing an aspartic acid to a glycine at position (614), and deletion of residues 69-70. In certain embodiments, the SARS-CoV-2 S glycoprotein, or fragment thereof, differs in amino acid sequence from the reference peptide or polypeptide by changing an asparagine to a tyrosine at position (501), changing a glutamic acid to a lysine at position (484), changing an aspartic acid to a glycine at position (614) and deletion of residues 69-70. In certain embodiments, the SARS-CoV-2 S glycoprotein, or fragment thereof, differs in amino acid sequence from the reference peptide or polypeptide (a wild-type SARS-CoV-2 spike protein) by inactivating the furin cleavage site within the spike protein. In certain embodiments, the SARS-CoV-2 S glycoprotein, or fragment thereof, differs in amino acid sequence from the reference peptide or polypeptide (a, wild-type SARS-CoV-2 spike protein) by changing $Q^{677}$TNSPRRARSV$^{687}$ (SEQ ID NO: 65), as denoted in SEQ ID NO: 1, or the equivalent amino acid residue in a mutant SARS-CoV-2 S glycoprotein sequence, to QTII,RSV (SEQ ID NO: 66) or to QTNSPGSASSV (SEQ ID NO: 67). In certain embodiments, the SARS-CoV-2 S glycoprotein, or fragment thereof, result in a monobasic furin cleavage site in the S1/S2 interface (QTII,RSV (SEQ ID NO: 66)) or deletion of the furin cleavage site (QTNSPGSASSV (SEQ ID NO: 67)) phenotype. In certain embodiments, the alteration to the furin cleavage site can lead to a spike stabilized pseudoparticles. See Hansen et. al., "Studies in humanized mice and convalescent humans yield a SARS-CoV-2 antibody cocktail" Science, published online June 15, 2020.

In certain embodiments, the SARS-CoV-2 S glycoprotein fragment lacks one or more C-terminal residues of the full-length SARS-CoV-2 S glycoprotein. For example, the SARS-CoV-2 S glycoprotein fragment may lack 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 of the C-terminal residues of the SARS-CoV-2 S glycoprotein. In certain embodiments, the SARS-CoV-2 S glycoprotein fragment lacks the 19 C-terminal residues of the SARS-CoV-2 S glycoprotein. The SARS-CoV-2 S glycoprotein fragment may consist of the amino acid sequence of SEQ ID NO: 3, or a sequence at least 78%, 79%, 80%, 81%, 82%, 83%, 84%, 85%, 86%, 87%, 88%, 89%, 90%, 91%, 92%, 93%, 94%, 95%, 96%, 97%, 98%, 99%, 99.5% or more identical to the amino acid sequence of SEQ ID NO: 3.

In certain embodiments, the SARS-CoV-2 S glycoprotein is a chimeric or fusion molecule which comprises fusogen sequences from viruses other than SARS-CoV-2. In certain embodiments, such chimeras comprise S1 or RBD sequences of SARS-CoV-2 S glycoprotein. In certain embodiments, the fusion protein is a fusion between the extracellular and transmembrane sequence of SARS-CoV-2 S glycoprotein, or a fragment thereof and a cytoplasmic domain of a non-SARS-CoV-2 fusogen or a fragment thereof. In certain embodiments, the fusion protein is a fusion between the extracellular sequence of SARS-CoV-2 S glycoprotein, or a fragment thereof and transmembrane and cytoplasmic domains of a non-SARS-CoV-2 fusogen or a fragment thereof. Non-limiting examples of non-SARS-CoV-2 fusogens which can be used in the above chimeric and fusion molecules include, for example, coronavirus fusogens (e.g., from SARS-CoV-1 or MERS-CoV), fusogens from VSV or other vesiculoviruses or other viruses from the *Rhabdoviridae* family, viruses from the *Retroviridae* family (e.g., human immunodeficiency virus (HIV), murine leukemia virus (MLV), Avian sarcoma leukosis virus (ASLV), Jaagsiekte sheep retrovirus (JSRV)), viruses from the *Paramyxoviridae* family (e.g., parainfluenza virus 5 (PIV5)), viruses from the *Herpesviridae* family (e.g., herpes simplex virus (HSV)), viruses from the *Togaviridae* family (e.g., Semliki Forest virus (SFV), Rubella virus), viruses from the *Flaviviridae* family (e.g., tick-borne encephalitis virus (TBE), Dengue virus), viruses from the *Orthomyxoviridae* family (e.g., influenza virus), viruses from the *Arenaviridae* family (e.g., lymphocytic choriomenengitis virus (LCMV), Lassa fever virus (LASV)), viruses from the *Bunyaviridae* family (e.g., Uukuniemi Virus (UUKV)), viruses from the *Filoviridae* family (e.g., Ebola virus (EBOV)), viruses from the *Poxviridae* family (e.g., Vaccinia virus (VV)), viruses from the *Asfaviridae* family (e.g., African swine fever virus (ASFV)), viruses from the *Arteriviridae* family (e.g., porcine reproductive and respiratory syndrome virus (PRRSV)), viruses from the *Bornaviridae* family (e.g., Borna disease virus (BDV)), viruses from the *Hepadnaviridae* family (e.g., Hepatitis B virus (HBV)), and viruses from *Hantaviridae* family (e.g., Andes virus).

In certain embodiments of the assays of the disclosure, to ensure detection of a broader spectrum of the SARS-CoV-2 neutralizing antibodies, the SARS-CoV-2 S glycoprotein or fragment thereof can comprise a consensus sequence derived from two or more different strains, mutants or variants of SARS-CoV-2. In other embodiments, to ensure detection of a broader spectrum of the SARS-CoV-2 neutralizing antibodies, the methods of the disclosure use a mixture of SARS-CoV-2 S glycoproteins (or fragments thereof) from two or more different strains, mutants or variants of SARS-CoV-2.

Polynucleotide molecules encoding SARS-CoV-2 S glycoprotein or a fragment thereof can comprise a consensus sequence and/or modification(s) for improved expression of the SARS-CoV-2 S glycoprotein or the fragment thereof. Modification can include codon optimization, the addition of a Kozak sequence or modified (e.g., optimized) Kozak sequence for increased translation initiation, and/or the addition of a signal peptide/leader sequence (e.g., an immunoglobulin signal peptide such as, e.g., IgE or IgG signal peptide). In certain embodiments, the Kozak sequence or modified (e.g., optimized) Kozak sequence is 3' to the foreign gene. In certain embodiments, the Kozak sequence or modified (e.g., optimized) Kozak sequence is immediately 3' to the foreign gene. In certain embodiments, the Kozak sequence or modified (e.g., optimized) Kozak sequence is 5' to the foreign gene. In certain embodiments, the Kozak sequence or modified (e.g., optimized) Kozak sequence is immediately 5' to the foreign gene.

In some embodiments, the SARS-CoV-2 S glycoprotein or fragment thereof comprises a fusions or conjugate with a detection tag (e.g., HA tag, histidine tag, biotin), , dimerization/multimerization sequences, Fc, signaling sequences, etc. Non-limiting examples of reporter proteins include, e.g., luciferases (including but not limited to, *Renilla* luciferase, RLuc8 mutant *Renilla* luciferase, (dCpG)Luciferase, NanoLuc reporter, firefly luciferase, *Gaussia* luciferase (gLuc), MetLuc, *Vibrio fischeri* lumazine protein, *Vibrio harveyi* luminaze protein, inoflagellate luciferase, firefly luciferase YY5 mutant, firefly luciferase LGR mutant, firefly luciferase mutant E, and derivatives thereof) and fluorescent proteins (including but not limited to, green fluorescent protein (GFP) [e.g., *Aequoria victoria* GFP, *Renilla muelleri* GFP, *Renilla reniformis* GFP, *Renilla ptilosarcus* GFP], GFP-like fluorescent proteins, (GFP-like), enhanced green fluorescent protein (EGFP), yellow fluorescent protein (YFP) [e.g., Topaz, Venus, mCitrine, YPet, TagYFP, PhiYFP, ZsYellow1, mBanana], enhanced yellow fluorescent protein (EYFP), blue fluorescent protein (BFP) [e.g., EBFP2, Azurite, GFP2, GFP10, and mTagBFP], enhanced blue fluorescent protein (EBFP), cyan fluorescent protein (CFP) [e.g., mECFP, Cerulean, CyPet, AmCyan1, Midori-Ishi Cyan, TagCFP, mCFPmm, mTFP1 (Teal)], enhanced cyan fluorescent protein (ECFP), superfolder GFP, superfolder YFP, orange fluorescent protein [e.g., Kusabira Orange, Kusabira Orange2, mOrange, mOrange2, dTomato, dTomato-Tandem, TagRFP, TagRFP-T, DsRed, DsRed2, DsRed-Express (T1), DsRed-Monomer, mTangerine], red fluorescent protein [e.g., mRuby, mApple, mStrawberry, AsRed2, mRFP1, JRed, mCherry, HcRed1, mRaspberry, dKeima-Tandem, HcRed-Tandem, mPlum, tdTomato, AQ143], small ultrared fluorescent protein, FMN-binding fluorescent protein, dsRed, qFP611, Dronpa, TagRFP, KFP, EosFP, IrisFP, Dendra, Kaede, KikGr1, emerald fluorescent protein, Azami Green, mWasabi, TagGFP, TurboGFP, AcGFP, ZsGreen, T-Sapphire, and derivatives thereof), β-galactosidase, β-glucuronidase, β-geo, and fragments thereof.

## Production of Recombinant VSV Particles

Replication competent recombinant VSV particles are used as an example in the disclosure below, but this disclosure can also be used for other vesiculoviruses.

The replication competent recombinant VSV particles of the disclosure are produced by providing in an appropriate host cell: VSV (-) DNA, in which regions non-essential for replication have been inserted into or replaced by a foreign DNA comprising a sequence encoding a coronavirus S glycoprotein or a fragment thereof and optionally other sequences discussed above, and recombinant sources of VSV N protein, P protein, L protein and any additional desired VSV protein (e.g., M protein and/or G glycoprotein). In certain embodiments, the production is preferably *in vitro* (e.g., in cell culture).

The host cell used for recombinant VSV production can be any cell in which VSVs grows. Non-limiting sources of host cells include, prokaryotic cells or a eukaryotic cells, vertebrate cells, mammalian cells, some insect (e.g., Drosophila) cells, primary cells (e.g., primary chick embryo fibroblasts), or cell lines (e.g., BHK (baby hamster kidney) cells, CHO (Chinese hamster ovary) cells, HeLA (human) cells, mouse L cells, Vero (monkey) cells (including Vero-$\alpha$His cells), Vero-Ace-2 cells, Vero-TRMPSS2 cells, Vero-E6 cells, ESK-4, PK-15, EMSK cells, MDCK (Madin-Darby canine kidney) cells, MDBK (Madin-Darby bovine kidney) cells, 293 (human) cells, Hep-2 cells, Human Diploid Primary Cell Lines (e.g. WI-38 and MRC5 cells), Monkey Diploid Cell Line (e.g. FRhL-Fetal Rhesus Lung cells), and Quasi-Primary Continues Cell Line (e.g. AGMK-African green monkey kidney cells), etc.).

The sources of N, P, and L proteins and any additional desired VSV protein (e.g., M protein and/or G glycoprotein) can be the same or can be different recombinant nucleic acid(s), encoding and capable of expressing these proteins in the host cell in which it is desired to produce recombinant VSVs. The nucleic acids encoding the N, P and L proteins and any additional desired VSV protein (e.g., M protein and/or G glycoprotein) can be obtained by any means available in the art. The VSV N, P, L, M and G-encoding nucleic acid sequences have been disclosed and can be used. For example, see Genbank accession no. J02428; Rose and Schubert, 1987, in The Viruses: The Rhabdoviruses, Plenum Press, NY, pp. 129-166. The sequences encoding the N, P and L genes can also be obtained, for example, from plasmid pVSVFL(+), deposited with the ATCC and assigned accession no. 97134, e.g., by PCR amplification of the desired gene (see also U.S. Pat. Nos. 4,683,202, 4,683,195 and 4,889,818; Gyllenstein et al., 1988, Proc. Natl. Acad. Sci. USA 85:7652-7656; Ochman et al., 1988, Genetics 120:621-623; Loh et al., 1989, Science 243:217-220). If a nucleic acid clone of any of the N, P, L, M or G genes is not already available, the clone can be obtained by use of standard recombinant DNA methodology. For example, the DNA may be obtained by standard procedures known in the art such as, e.g., by purification of RNA from VSV virions followed by reverse transcription and PCR (Mullis and Faloona, 1987, Methods in Enzymology 155:335-350). Alternatives include, but are not limited to, chemically synthesizing the gene sequence itself. Other methods are also possible.

Nucleic acids that encode fragments and derivatives of VSV N, P, L, and M genes, as well as fragments and derivatives of the VSV (-) DNA can also be used in the present disclosure, as long as such fragments and derivatives retain the ability to produce replication competent VSV particles which can be used in one or more methods of the disclosure. In particular, derivatives can be made by altering sequences by substitutions, additions, or deletions. Furthermore, due to the inherent degeneracy of the genetic code, other DNA sequences which encode substantially the same or a functionally equivalent amino acid sequence may be used in the practice of the methods of the disclosure. Amino acid substitutions may be made on the basis of similarity in polarity, charge, solubility, hydrophobicity, hydrophilicity and/or the amphipathic nature of the residues involved.

The desired N/P/L/M-encoding nucleic acid can be inserted into an appropriate expression vector, *i.e.,* a vector which contains the necessary elements for the transcription and translation of the inserted protein-coding sequence in the host cell in which it is desired to produce replication competent recombinant VSV particles, to create a vector that functions to direct the synthesis of the VSV proteins that will subsequently assemble with the VSV genomic RNA (e.g., produced in the host cell from antigenomic VSV (+) RNA produced, e.g., by transcription of the VSV (-) DNA).

A variety of vector systems may be utilized to express the N, P and L VSV proteins and any additional desired VSV protein (e.g., M), as well as to transcribe the VSV (-) DNA (e.g., comprising a foreign DNA), as long as the vector is functional in the host cell and compatible with any other vector present. The expression elements of vectors vary in their strengths and specificities. Any one of a number of suitable transcription and translation elements may be used, as long as they are functional in the host cell.

Standard recombinant DNA methods may be used to construct expression vectors containing DNA encoding the VSV proteins, and the VSV (-) DNA containing the foreign DNA, comprising appropriate transcriptional/translational control signals (*see, e.g.,* Sambrook et al., 1989, *supra,* and methods described hereinabove). Expression may be controlled by any promoter/enhancer element known in the art. Promoters which may be used to control expression can be constitutive or inducible. In a specific embodiment, the promoter is an RNA polymerase promoter.

Transcription termination signals (downstream of the gene), and selectable markers are preferably also included in the expression vector. In addition to promoter sequences, expression vectors for the N, P, L, and any additionally desired VSV proteins, as well as the SARS-CoV-2 S glycoprotein, may contain specific initiation signals for efficient translation of the inserted sequences, *e.g.,* a ribosome binding site.

Specific initiation signals maybe required for efficient translation of the protein coding sequences. These signals include the ATG initiation codon and adjacent sequences. In cases where the entire N, P, L, or other (e.g., M) VSV gene, including its own initiation codon and adjacent sequences, are inserted into the appropriate vectors, no additional translational control signals may be needed. However, in cases where only a portion of the gene sequence is inserted, exogenous translational control signals, including the ATG initiation codon, must be provided. The initiation codon must furthermore be in phase with the reading frame of the protein coding sequences to ensure translation of the entire insert. These exogenous translational control signals and initiation codons can be of a variety of origins, both natural and synthetic.

In a specific embodiment, a recombinant expression vector provided herein, encoding an N, P, L, and other (e.g., M) protein or functional derivative thereof, comprises the following operatively linked components: a promoter which controls the expression of the VSV N, P, L proteins and optionally the M protein, the SARS-CoV-2 spike glycoprotein or a fragment thereof, a translation initiation signal, a DNA sequence encoding the VSV proteins or functional fragment thereof, and a transcription termination signal, e.g., as shown in Fig. 1.

In certain embodiments, the genes encoding the N, P, L, and optional M VSV proteins are inserted downstream of the T7 RNA polymerase promoter from phage T7 gene 10, situated with an A in the -3 position. A T7 RNA polymerase terminator and a replicon can be also included in the expression vector. T7 RNA polymerase can be provided to transcribe the VSV protein sequence. The T7 RNA polymerase can be produced from a chromosomally integrated sequence or an episomal vector. In certain embodiments, T7 RNA polymerase can be provided by intracellular expression from a recombinant vaccinia virus vector encoding the T7 RNA polymerase. In certain embodiments, the N, P, L, and optional M VSV proteins are each encoded by a DNA sequence operably linked to a promoter in an expression plasmid, containing the necessary regulatory signals for transcription and translation of the encoded proteins. Such an expression plasmid preferably includes a promoter, the coding sequence, and a transcription termination/polyadenylation signal, and optionally, a selectable marker (e.g., β-galactosidase).

In certain embodiments, the N, P, L, and optional M proteins can be encoded by the same or different plasmids, or a combination thereof. In other embodiments, one or more of the N, P, L, and optional M VSV proteins can be expressed intrachromosomally.

The cloned sequences comprising the VSV (-) DNA containing the foreign DNA, and the cloned sequences comprising sequences encoding the VSV and foreign proteins can be introduced into the desired host cell by any method known in the art, e.g., transfection, electroporation, infection (when the sequences are contained in, e.g., a viral vector), microinjection, etc. In certain embodiments, a transfection facilitating reagent is added to increase DNA uptake by cells. Many of these reagents are known in the art (e.g., calcium phosphate; Lipofectace (Life Technologies, Gaithersburg, Md.), and Effectene (Qiagen, Valencia, Calif.) are non-limiting examples).

In certain embodiments, DNA comprising VSV (-) DNA containing foreign DNA encoding a SARS-CoV-2 S glycoprotein or a fragment thereof, operably linked to an RNA polymerase promoter (e.g., a bacteriophage RNA polymerase promoter); DNA encoding N, operably linked to the same RNA polymerase promoter; DNA encoding P, operably linked to the same polymerase promoter; and DNA encoding L, operably linked to the same polymerase promoter; are all introduced (e.g., by transfection) into the same host cell, in which host cell the RNA polymerase has been cytoplasmically provided. In certain embodiments, the RNA polymerase is cytoplasmically provided by expression from a recombinant virus vector that replicates in the cytoplasm and expresses the RNA polymerase, most preferably a vaccinia virus vector, that has been introduced (e.g., by infection) into the same host cell. Cytoplasmic provision of RNA polymerase can be used, as this will result in cytoplasmic transcription and processing, of the VSV (-) DNA comprising the foreign DNA and of the N, P, L, and other (e.g., M and/or G protein) VSV proteins, avoiding splicing machinery in the cell nucleus, and, thereby, maximizing proper processing and production of N, P, L, and other (e.g., M and/or G protein) VSV proteins, and resulting assembly of the recombinant VSVs. Vaccinia virus vectors also cytoplasmically provide enzymes for processing (capping and polyadenylation) of mRNA, facilitating proper translation. In a most preferred aspect, T7 RNA polymerase promoters are employed, and a cytoplasmic source of T7 RNA polymerase is provided by also introducing into the host cell a recombinant vaccinia virus vector encoding T7 RNA polymerase into the host cell. Such vaccinia virus vector can be obtained by well-known methods. In certain embodiments, a recombinant vaccinia virus vector such as vTF7-3 (Fuerst et al., 1986, Proc. Natl. Acad. Sci. U.S.A. 83:8122-8126) can be used.

In other embodiments, the RNA polymerase (e.g., T7 RNA polymerase) can be provided by use of a host cell that expresses T7 RNA polymerase from a chromosomally integrated sequence (*e.g.,* originally inserted into the chromosome by homologous recombination), optionally constitutively, or that expresses T7 RNA polymerase episomally, from a plasmid.

In other embodiments, the VSV (-) DNA encoding a SARS-CoV-2 S glycoprotein or a fragment thereof, operably linked to a promoter, can be transfected into a host cell that stably recombinantly expresses the N, P, L, and optional M VSV proteins from chromosomally integrated sequences.

The cells are cultured and replication competent recombinant VSV particles can be recovered, e.g., using standard methods. By way of example, and not limitation, after approximately 24 hours, cells and medium can be collected, freeze-thawed, and the lysates clarified to yield virus preparations. Alternatively, the cells and medium can be collected and simply cleared of cells and debris by low-speed centrifugation.

Confirmation that the appropriate foreign sequence is present in the genome of the recombinant VSV and directs the production of the desired protein(s) in an infected cell, can be performed. Standard procedures known in the art can be used for this purpose. By way of example, and not limitation, genomic RNA can be obtained from the VSV by SDS phenol extraction from virus preparations, and can be subjected to reverse transcription (and/or PCR), followed by e.g., sequencing, Southern hybridization using a probe specific to the foreign DNA, or restriction enzyme mapping, etc. The virus can be used to infect host cells, which can then be assayed for expression of the desired protein by standard immunoassay techniques using an antibody to the protein (e.g., Western blotting), or by assays based on functional activity of the protein. Other techniques are known in the art and can be used.

Large Scale Growth and Purification of the Recombinant Viruses

VSVs are used as an example in the disclosure below, and this disclosure can also be used for other vesiculoviruses.

A non-limiting example of a large-scale production of a recombinant VSV virus following plaque-purification is presented below. Virus from a single plaque (~$10^5$ pfu) is recovered and used to infect ~$10^7$ cells (e.g., BHK cells), to yield, generally, 10 ml at a titer of $10^9$-$10^{10}$ pfu/ml for a total of approximately $10^{11}$ pfu. Infection of ~$10^{12}$ cells can then be carried out (with a multiplicity of infection of e.g., 0.1), and the cells can be grown in suspension culture, large dishes, or roller bottles by standard methods known to those in the art.

Virus for vaccine preparations can then be collected from culture supernatants, and the supernatants clarified to remove cellular debris. If desired, one method of isolating and concentrating the virus that can be employed is by passage of the supernatant through a tangential flow membrane concentration. The harvest can be further reduced in volume by pelleting through a glycerol cushion and by concentration on a sucrose step gradient. An alternate method of concentration is affinity column purification (Daniel et al., 1988, Int. J. Cancer 41:601-608). However, other methods can also be used for purification (see, e.g., Arthur et al., 1986, J. Cell. Biochem. Suppl. 10A:226), and any possible modifications of the above procedure will be readily recognized by one skilled in the art. Purification should be as gentle as possible, so as to maintain the integrity of the virus particle.

Assays

In one aspect, the disclosure provides a method for determining the presence of a coronavirus (i.e. SARS-CoV-2) neutralizing antibody in a sample, wherein the sample is contacted with a replication competent recombinant vesicular stomatitis virus (VSV) particle where (i) the VSV glycoprotein (G) is replaced by a SARS-CoV-2 S glycoprotein or a fragment comprising the receptor binding domain thereof, wherein said S glycoprotein or fragment is capable of mediating infection of a target cell, and wherein (ii) the VSV particle comprises a nucleic acid molecule comprising a nucleic acid sequence encoding a reporter protein, wherein the nucleic acid sequence encoding the reporter protein is inserted between a nucleic acid sequence encoding the SARS-CoV-2 S glycoprotein or fragment thereof, and a nucleic acid sequence encoding rhabdovirus large (L) protein. The replication competent recombinant VSV particle is then contacted with the target cell. The reporter signal is then measured and compared with a control. The target cell may be a Vero cell (including Vero-αHis cell), Vero-Ace-2 cell, Vero-TRMPSS2 cell, Vero-E6 cell, or any other cell comprising an angiotensin-converting enzyme 2 (ACE2).

The sample used in the above method may be, e.g., serum or plasma (e.g., heat-inactivated serum or plasma).

The first step in which the sample is contacted with the replication competent recombinant VSV particle may be conducted for about 1 hour at about 37°C. The second step in which the replication competent recombinant VSV particle with the target cell may be conducted for 1-12, 1-3, 2-4, 3-5, 4-6, 5-7, 6-8, 7-9, or 8-10 hours at about 37°C.

In various embodiments of the above method, the method comprises adding the reporter protein substrate for obtaining the reporter signal. The reporter protein may be a luciferase and the reporter protein substrate may be Luciferin (e.g., d-luciferin), coelenterazine, or EnduRen luciferase substrate.

In certain embodiments, the SARS-CoV-2 S protein is a full-length SARS-CoV-2 S protein (e.g., a protein comprising or consisting of the amino acid sequence of SEQ ID NO: 1). In certain embodiments, the SARS-CoV-2 S protein is a SARS-CoV-2 S protein lacking 19 C-terminal amino acids (e.g., a protein comprising or consisting of the amino acid sequence of SEQ ID NO: 3). In certain embodiments, the SARS-CoV-2 S protein, fragment has at least 77% amino acid sequence identity to the amino acid sequence of SEQ ID NO: 1. In certain embodiments, the SARS-CoV-2 S protein, fragment has at least 64% amino acid sequence identity to the S1 subunit of the amino acid sequence of SEQ ID NO: 1. In certain embodiments, the SARS-CoV-2 S protein, fragment has at least 64% amino acid sequence identity to amino acids 14-684 of the amino acid sequence of SEQ ID NO: 1. In certain embodiments, the SARS-CoV-2 S protein, fragment has at least 74% amino acid sequence identity to the RBD domain of the amino acid sequence of SEQ ID NO: 1. In certain embodiments, the SARS-CoV-2 S protein, fragment has at least 74% amino acid sequence identity to amino acids 319-541 of the amino acid sequence of SEQ ID NO: 1. In certain embodiments, the recombinant VSV particle comprises a mutant VSV matrix (M) protein. In certain embodiments, the genome of the recombinant VSV encodes a mutant VSV matrix M protein. In certain embodiments, the mutant matrix M protein comprises a mutation at methionine (M) 51 (e.g., a change from methionine (M) to arginine (R)). In certain embodiments, the mutant VSV matrix M protein comprises or consists of the amino acid sequence of SEQ ID NO: 7.

In certain embodiments of the above methods of the disclosure, the control is the reporter signal obtained with a control sample not comprising coronavirus neutralizing antibodies and the method comprises concluding that the tested sample comprises SARS-CoV-2 neutralizing antibodies when the reporter signal obtained in the sample is reduced as compared to the control. In certain embodiments, the control sample comprises a VSV particle without the SARS-CoV-2 S glycoprotein or a VSV particle with a mutant SARS-CoV-2 S glycoprotein which does not mediate infection of target cells. In certain embodiments of the above methods, the method further comprises comparing the reporter signal obtained in the sample with the reporter signal obtained with a control sample comprising coronavirus neutralizing antibodies or a molecule that blocks interaction of the SARS-CoV-2 S glycoprotein with a protein with which it interacts on the target cell.

In certain embodiments of the above methods, the method is conducted in a high throughput format.

In another specific embodiment, provided herein is a method for determining the presence of a Severe Acute Respiratory Syndrome coronavirus 2 (SARS-CoV-2) neutralizing antibody in a sample, the method comprising:

a) contacting the sample with a replication competent recombinant vesicular stomatitis virus (VSV) particle as described above, wherein (i) the VSV glycoprotein (G) is replaced by the full-length SARS-CoV-2 spike (S) glycoprotein or a fragment thereof lacking 19 C-terminal amino acids and wherein (ii) the VSV particle comprises a nucleic acid molecule comprising a nucleic acid sequence encoding a luciferase protein, wherein the nucleic acid sequence encoding the luciferase protein is inserted between a nucleic acid sequence encoding the SARS-CoV-2 S glycoprotein or fragment thereof, and a nucleic acid sequence encoding rhabdovirus large (L) protein;
b) after step (a), contacting the replication competent recombinant VSV particle with a cell selected from Vero cell, Vero-Ace-2 cell and Vero-E6 cell;
c) measuring the luciferase signal in the cell after step (b), and
d) comparing the signal measured in step (c) with a control.

The cells used in any of the methods disclosed comprise angiotensin-converting enzyme 2 (ACE2). ACE2 is a functional receptor for coronaviruses, in particular SARS-CoV-1 and SARS-CoV-2. The cells used in any of the methods disclosed herein may further comprise transmembrane protease, serine 2 (TMPRSS2). The non-limiting examples of susceptible target cells which can be used in the methods of the disclosure include, e.g., Vero green monkey kidney epithelial cells (including Vero-αHis cells), Vero-Ace-2 cells, Vero-TRMPSS2 cells, Vero-E6 cells, avian erythrocytes, and Madin-Darby canine kidney epithelial cells.

In some embodiments, addition of trypsin to a virus and cell mixture may be used to increase virus-mediated cell fusion. Trypsin may be added before the reporter signal is measured in the cells. Trypsin may be added after a virus and cell mixture are incubated with one another, e.g., 2-6 hours after incubation starts, 3-5 hours after incubation starts, about 4 hours after incubation starts, or 4 hours after incubation starts. Trypsin does not need to be removed from the virus and cell mixture after addition. As shown in the data below, addition of trypsin can promote cell fusion. The concentration of trypsin used can range from 0.1 to 4.0 μL trypsin/mL media (e.g., OptiMEM), or from 0.1 to 0.6 μL trypsin/mL media, 0.2 to 0.7 μL trypsin/mL media, 0.3 to 1.2 μL trypsin/mL media, 0.5 to 2.0 μL trypsin/mL media, 1.0 to 2.5 μL trypsin/mL media, 1.5 to 3.0 μL trypsin/mL media, or from 2.0 to 4.0 μL trypsin/mL media. Trypsin addition to the media during the fusion assay may make the assay more sensitive. Additional trypsin may allow for cell fusion to occur in less time. Additional trypsin may allow for the assay readout (e.g., luciferase activity) to occur more quickly than with less or no trypsin.

In some embodiments, trypsin is not used in the assay. If trypsin is excluded, an additional wash step may not be required even if the serum/virus mixes, or plasma/virus mixes, are removed and replaced with fresh serum media, or media suitable for plasma.

Reporter Proteins

In the methods described herein, the reporter protein may be a luciferase. Various luciferase enzymes known to those of ordinary skill in the art may be used, with exemplary luciferases including but not limited to, Renilla luciferase, RLuc8 mutant *Renilla* luciferase, (dCpG)Luciferase, NanoLuc reporter, firefly luciferase, *Gaussia* luciferase (gLuc), MetLuc, Vibrio fischeri lumazine protein, Vibrio harveyi luminaze protein, inoflagellate luciferase, firefly luciferase mutant YY5 mutant, firefly luciferase LGR mutant, firefly luciferase mutant E, and derivatives thereof.

Various fluorescent proteins may be used in any of the methods and compositions described herein. In the methods disclosed herein, the reporter protein may comprise a fluorescent protein.

The fluorescent protein may be green fluorescent protein (GFP), GFP-like fluorescent proteins (GFP-like), enhanced green fluorescent protein (EGFP). In alternative embodiments, the fluorescent protein is yellow fluorescent protein (YFP), an enhanced yellow fluorescent protein (EYFP), a blue fluorescent protein (BFP), an enhanced blue fluorescent protein (EBFP), a cyan fluorescent protein (CFP), an enhanced cyan fluorescent protein (ECFP), a red fluorescent protein (dsRED) superfolder GFP, superfolder YFP, orange fluorescent protein, red fluorescent protein, small ultrared fluorescent protein, FMN-binding fluorescent protein, dsRed, qFP611, Dronpa, TagRFP, KFP, EosFP, IrisFP, Dendra, Kaede, KikGr1, and derivatives thereof, or any other natural or genetically engineered fluorescent protein of those listed above. In yet further embodiments, the reconstituted fluorescent proteins may comprise of a mixture of fragments from the same or a combination any of the above listed fluorescent proteins.

Green Fluorescent Protein (GFP) is a 238 amino acid long protein derived from the jellyfish *Aequorea Victoria.* The engineered GFP used in the split constructs of the present disclosure is 231 amino acids long and consists of SEQ ID NO: 18. Fluorescent proteins have also been isolated from other members of the *Coelenterata,* such as the red fluorescent protein from *Discosoma* sp. (Matz, M. V. et al. 1999, Nature Biotechnology 17: 969-973), GFP from *Renilla reniformis,* GFP from *Renilla Muelleri* or fluorescent proteins from other animals, fungi or plants. There are various modified forms of GFP. The blue fluorescent variant of GFP (BFP) is described by Heim et al., Proc. Natl. Acad. Sci. 91:26, 1994, pp 12501-12504), and is a Y66H variant of wild type GFP. The yellow fluorescent variant of GFP (YFP) comprises the S65G, S72A, and T203Y mutations, and is disclosed in International Patent Publication No. WO98/06737. The cyan fluorescent variant of GFP (CFP) comprises the Y66W color mutation and optionally the F64L, S65T, N1461, M153T, V163A folding/solubility mutations. CFP is described in Heim, R., Tsien, R. Y., Curr. Biol. 6, 1996, 178-182). The EGFP variant comprises the F64L and S65T mutations of GFP and insertion of one valine residue after the first methionine. EGFP is described in WO 97/11094 and WO96/23810. The F64L mutation is the amino acid in position 1 upstream from the chromophore. A GFP or GFP variant containing this folding mutation provides an increase in fluorescence intensity when the GFP or GFP variant is expressed in cells at a temperature above about 30° C. (WO 97/11094).

Non-limiting examples of GFPs include: *Aequoria victoria* GFP, *Renilla muelleri* GFP, *Renilla reniformis* GFP, *Renilla ptilosarcus* GFP, Emerald, Superfolder GFP, Azami Green, mWasabi, TagGFP, TurboGFP, AcGFP, ZsGreen, T-Sapphire.

Non-limiting examples of BFPs include: EBFP2, Azurite, GFP2, GFP10, and mTagBFP.

Non-limiting examples of CFPs include: mECFP, Cerulean, CyPet, AmCyan1, Midori-Ishi Cyan, TagCFP, mCFPmm, and mTFP1 (Teal).

Non-limiting examples of YFPs include: Topaz, Venus, mCitrine, YPet, TagYFP, PhiYFP, ZsYellow1, and mBanana.

Non-limiting examples of orange fluorescent proteins include: Kusabira Orange, Kusabira Orange2, mOrange, mOrange2, dTomato, dTomato-Tandem, TagRFP, TagRFP-T, DsRed, DsRed2, DsRed-Express (T1), DsRed-Monomer, and mTangerine

Non-limiting examples of red fluorescent proteins include: mRuby, mApple, mStrawberry, AsRed2, mRFP1, JRed, mCherry, HcRed1, mRaspberry, dKeima-Tandem, HcRed-Tandem, mPlum, tdTomato, and AQ143.

Also encompassed are fragments and derivatives of the above reporter proteins.

The fluorescent protein may detectable by one or more of: flow cytometry, fluorescence plate reader, fluorometer, microscopy, fluorescence resonance energy transfer (FRET), by the naked eye or by other methods known to persons skilled in the art. When using flow cytometry, fluorescence may be detected using a florescence activated cell sorter (FACS) or time lapse microscopy.

In various embodiments, fluorescence may be measured through the use of a flow cytometer or a bead array reader. For example, aBioPlex-100, a BioPlex-200, a Luminex-100, or a Luminex-200 bead array reader may be used.

In some embodiments, before adding the replication competent recombinant VSV particle to the cells, plating media is removed and replaced with serum media (e.g., 50 μL/well OptiMEM). In other embodiments, before adding the virus/serum mixes to the cells, plating media is not changed.

The replication competent recombinant VSV particle may be present in the serum as a virus/serum mixture. In other embodiments, before adding the virus/serum mixes to the cells, plating media is not changed. The replication competent recombinant VSV particle may be present in the plasma as a virus/plasma mixture. In other embodiments, before adding the virus/plasma mixes to the cells, plating media is not changed.

The reporter signal in the cells is measured, and then compared with a control.

The period of time for incubating the replication competent recombinant VSV particle with the virus/serum mixture, or virus/plasma mixture, may be set as a time period appropriate for such an incubation, such as from about 30 minutes to 3 days, or from about 30 minutes to 75 minutes, from 45 minutes to 90 minutes, from 60 minutes to 2 hours, or from 90 minutes to 3 hours. An exemplary period of time may be about 1 hour.

In some embodiments, after the replication competent recombinant VSV particle (e.g., in a mixture with serum or plasma) is added to target cells, the cells with virus are further incubated for 2-6 hours, for about 4 hours, or for 4 hours. If serum or plasma is present after the incubation, the serum or plasma may be removed so as to enhance cell fusion or luciferase activity.

The sample (e.g., serum or plasma) can be obtained from a subject, for example from humans exposed to SARS-CoV-2 infection or vaccinated with SARS-CoV-2 vaccine or another immunogenic composition. The sample may or may not contain some level of SARS-CoV-2 neutralizing antibodies. Antibody responses in COVID-19 patients are described, e.g., in Okba, N.M.A. et al., "SARS-CoV-2 specific antibody responses in COVID-19 patients" Emerg. Infect. Dis., 2020, 26(7) (available at doi.org/10.3201/eid2607.200841). It is desirable to quickly determine the level of neutralizing antibody response in a patient sample, and the methods described herein permit rapid evaluation of the presence and/or level of SARS-CoV-2 neutralizing antibodies.

The methods described in Montefiori, D.C., "Evaluating neutralizing antibodies against HIV, SIV, and SHIV in luciferase reporter gene assays." Curr. Protoc. Immunol., 2005, Chapter 12, Unit 12.11, may also be used as part of the methods described herein.

Kits

The disclosure also provides kits in conjunction with various methods disclosed herein. In certain embodiments, the kit comprises in one or more containers (e.g., separate containers): (a) a first recombinant DNA that can be transcribed in a suitable host cell to produce a VSV antigenomic (+) RNA in which a foreign RNA sequence has been inserted; (b) a second recombinant DNA comprising a sequence encoding a VSV N protein or functional fragment or derivative thereof; (c) a third recombinant DNA comprising a sequence encoding a VSV L protein or functional fragment or derivative thereof; and (d) a fourth recombinant DNA comprising a sequence encoding a VSV P protein or functional fragment or derivative thereof; and optionally (e) a fifth recombinant DNA comprising a sequence encoding a VSV M protein or functional fragment or derivative thereof. The second, third, fourth, and optionally fifth recombinant DNAs are part of the same DNA molecule. The sequences encoding the N, L, P, and other proteins are each operably linked to a promoter that controls expression of the N, L, P, and optional M proteins, respectively, in the suitable host cell. In various embodiments, the kit can contain the various nucleic acids, e.g., plasmid expression vectors, described herein above for use in production of replication competent recombinant VSV particles.

The disclosure further provides a kit for detecting coronavirus neutralizing antibodies in a sample (e.g., neutralizing antibodies that target the SARS-CoV-2 S glycoprotein). The kit may comprise:

a) a replication competent recombinant vesicular stomatitis virus (VSV) particle, where (i) the VSV glycoprotein (G) is replaced by a the SARS-CoV-2 S glycoprotein or a fragment comprising the receptor binding domain thereof, wherein the SARS-CoV-2 S glycoprotein or fragment is capable of mediating infection of target cells, and wherein (ii) the VSV particle comprises a nucleic acid molecule comprising a nucleic acid sequence encoding a reporter protein, wherein the nucleic acid sequence encoding the reporter protein is inserted between a nucleic acid sequence encoding the SARS-CoV-2 S glycoprotein or fragment thereof, and a nucleic acid sequence encoding rhabdovirus large (L) protein;
b) a target cell ;
c) optionally, a positive control comprising a SARS-CoV-2 neutralizing antibody, or a molecule that blocks interaction of the SARS-CoV-2 S glycoprotein with a protein with which it interacts on the target cell, or any combination thereof and/or a negative control not comprising coronavirus neutralizing antibodies;
d) optionally, a substrate for the reporter protein, and
e) optionally, instructions for use.

The reporter protein may be any of the enzymes (e.g., luciferase) or fluorescent proteins (e.g., GFP) described herein.

A non-limiting example of a neutralizing antibody against the SARS-CoV-2 S glycoprotein is mAb10914 or mAb10922 (the amino acid and nucleotide sequences of mAb10914 and mAb10922 are provided in the Sequences section, below). Known amounts of a neutralizing antibody can be added to serum or plasma that does not comprise the neutralizing antibody so as to generate a positive control. In some embodiments, the positive control may comprise a molecule that blocks the interaction between the SARS-CoV-2 spike (S) glycoprotein and a protein with which it interacts on the cell surface, e.g., ACE2 or TMPRSS2. Non-limiting examples of such molecules include, e.g., anti-ACE2 antibodies (e.g., CL4013, AC18F, 881CT16.4.4, MA5-31395, and OTI1D2 antibodies), soluble ACE2 proteins (e.g., as disclosed in Fukuski et al., Journal of General Virology, 2005, 86:2269-74), and anti-TMPRSS2 antibodies (e.g., H1H7017N antibody as described in International Patent Pub. No. WO2019/147831). In some embodiments, the positive control may comprise a molecule (e.g., an antibody) that blocks cell-cell fusion. Non-limiting examples of such molecules include, e.g., MC5 and DL11 antibodies described in Saw, W.T. et al., Methods, 2015, 90:68-75.

The negative control can comprise serum or plasma that does not comprise the neutralizing antibody but comprises other antibodies, such as, an antibody that specifically targets a VSV glycoprotein (G), an antibody that specifically targets a VSV matrix protein (M), an antibody that specifically targets another coronavirus spike (S) glycoprotein different from that being tested in the method (e.g., an antibody that specifically targets the SARS-CoV-1 spike (S) glycoprotein but does not bind to the SARS-CoV-2 spike (S) glycoprotein), or monoclonal antibodies specific for HIV antigens (e.g., IgG1b12, 2G12, X5, 2F5, 4E10 as described in Montefiori, D.C., Current Protocols in Immunology, 2004, Chapter 12: Unit 12.11.1), or any isotype-control antibodies.

A cell growth media and/or fixative may optionally be included in any of the kits.

**EXAMPLES**

The present disclosure is also described and demonstrated by way of the following examples. However, the use of this and other examples anywhere in the specification is illustrative only. The disclosure is to be limited only by the terms of the appended claims.

**Example 1: Preparation of VSV Expressing Spike** (S) **Glycoprotein Constructs**

Infectious clones of Indiana strain VSV were used to generate four recombinant VSV constructs, wherein the VSV (G) glycoprotein was deleted and replaced by codon optimized sequences suitable for expression in human cells and encoding: (1) the full length SARS-CoV-2 spike (S) glycoprotein sequence (NCBI Reference Sequence: NC_045512.2; Protein_ID: YP_009724390.1) (variant 1; VSV SARS-CoV-2 dG = VSV-SARS-CoV-2-S; amino acid sequence SEQ ID NO: 1; codon optimized coding nucleotide sequence SEQ ID NO: 2); (2) the SARS-CoV-2 S glycoprotein sequence with a deletion of 19 amino acids KFDEDDSEPVLKGVKLHYT (SEQ ID NO: 20) at the C terminus (variant 2; VSV SARS-CoV-2 Δ19CT dG = VSV-SARS-CoV-2-S-Δ19CT; amino acid sequence SEQ ID NO: 3; codon optimized coding nucleotide sequence SEQ ID NO: 4); (3) the SARS-CoV-2 S glycoprotein sequence with a replacement of the S glycoprotein cytoplasmic tail with VSV G cytoplasmic tail KLKHTKKRQIYTDIEMNRLGK (SEQ ID NO: 21) (variant 3; VSV SARS-CoV-2 VSV-G CT dG; amino acid sequence SEQ ID NO: 5; codon optimized coding nucleotide sequence SEQ ID NO: 6), or (4) variant 4 - the full length SARS-CoV-2 S glycoprotein sequence (VSV-SARS-CoV-2-S; amino acid sequence SEQ ID NO: 1; codon optimized coding nucleotide sequence SEQ ID NO: 2) with the wild-type VSV Kozak sequence (cActATG; SEQ ID NO: 11) in place of the optimized Kozak sequence (caccATG; SEQ ID NO: 12) used in the other three constructs. One set of variant 1-4 constructs (constructs 1-4) was prepared that encoded wild-type VSV M protein (amino acid sequence SEQ ID NO: 9; nucleotide sequence SEQ ID NO: 10). A second set of variant 1-4 constructs (constructs 5-8) was prepared that encoded M protein with the substitution M51R (amino acid sequence SEQ ID NO: 7; nucleotide sequence SEQ ID NO: 8) resulting in virus attenuation. See Figure 1.

The variant 1-4 recombinant viral particles were produced using standard published protocol using transfection with vaccinia-T7 virus (expressing T7 polymerase) followed by co-transfection with N, P and L expression plasmids (with respective genes under the control of T7 promoter) and the viral genome plasmid. A plasmid expressing VSV G was also transfected into the cells to facilitate rescue. The viruses were amplified and propagated in Vero cells. The amplified recombinant viruses do not have VSV (G) glycoprotein and depend on SARS-CoV-2 spike (S) glycoprotein for entry and infection.

Correct incorporation of VSV G, N and M proteins and SARS-CoV-2 S glycoprotein in the recombinant variant 2 VSV-SARS-CoV-2-S-Δ19CT construct 6 (VSV-M51R-nCoV19-S Δ19CT) virions was analyzed by Western blotting. The results are shown in Figure 3C. Viral supernatants prepared from Vero cells infected with VSV-GFP (encodes VSV-G) or VSV-SARS-CoV-2-S-Δ19CT or mock-infected were subjected to immunoblot analysis using anti-SARS-CoV-2-spike antibody (left) and rabbit anti-VSV antiserum (right). SARS-CoV-2-S-Δ19CT S glycoprotein produced two bands corresponding to the full-length S1/S2 variant (180 kDa) and the proteolytically cleaved S2 variant (75 kDa) glycoprotein. The Western blot shows the presence of VSV N, M and G proteins in the VSV-GFP virus and the presence of VSV N and M proteins (but not VSV G glycoprotein) in the variant 2 VSV-SARS-CoV-2-S-Δ19CT virus. The Western blot for variant 2 VSV-SARS-CoV-2-S-Δ19CT virus also shows efficient incorporation of VSV-SARS-CoV-2-S-Δ19CT S glycoprotein in place of the VSV G glycoprotein.

**Reference Example 2: Development of a high-throughput IMMUNO-COV v. 1 assay for detecting antibodies that neutralize SARS-CoV-2**

Introduction

Laboratory assays to detect SARS-CoV-2 neutralizing antibodies have been developed using clinical isolates of SARS-CoV-2, pseudotyped lentiviral vectors, and pseudotyped Vesicular stomatitis viruses (VSVs). Describe herein is a high-throughput, scalable clinical assay for the detection and quantitation of SARS-CoV-2-neutralizing antibodies using a recombinant VSV engineered to express the SARS-CoV-2 spike (S) glycoprotein in place of the VSV-G glycoprotein. The SARS-CoV-2 spike (S) glycoprotein binds to angiotensin converting enzyme-2 (ACE2) on the cell surface to initiate virus entry into the cell (Hoffman 2020, Walls 2020, Wan 2020). As the primary surface exposed viral protein, the coronavirus spike is a major target of the host immune system (Corman 2016, Liu 2006, Nie 2004, Zhao 2017). Blocking spike interaction with ACE2 prevents virus entry, making spike the primary target of neutralizing antibodies (Walls 2020, Wu 2020). Therefore, the present inventors hypothesized that the detection of antibodies capable of neutralizing VSV expressing spike (S) glycoprotein should directly reflect the level of SARS-CoV-2 neutralizing antibodies present in a sample. Importantly, using spike-expressing VSV in place of SARS-CoV-2 significantly increases assay safety and scalability.

The present assay (IMMUNO-COV v. 1) exploits the fusion phenotype of SARS-CoV-2 spike glycoprotein-expressing VSV by using a dual split protein (DSP) luciferase reporter system to quantitate virus-induced cell fusion and thereby the level of virus neutralization (Figure 2). The DSP system of the present embodiment uses a chimeric split engineered green fluorescent protein (GFP) and split *Renilla* luciferase mutant Rluc8. Fusion between two cell lines expressing complementary pieces of the split reporter facilitates reassociation of fully functional GFP and Rluc8 luciferase. Thus, luciferase activity can be used to measure virus-induced cell fusion in a high-throughput 96-well plate format. Described herein is the development of the assay, including optimization of assay conditions and validation of the final clinical assay. Excellent correlation was observed between the assay results, clinical symptoms, and results from other serological tests. In particular, quantitation of SARS-CoV-2 neutralizing antibody levels correlated closely with plaque reduction neutralization IC50 values from an assay using a clinical isolate of SARS-CoV-2. Thus, the present assay accurately quantitates SARS-CoV-2 neutralizing antibodies, making it a valuable tool for assessing plasma therapies and immune responses to new SARS-CoV-2 vaccines.

The IMMUNO-COV v. 1 assay exhibited 100% specificity in validation tests, and across all tests no false positives were detected. In blinded analyses, assay results demonstrated near-perfect correlation (196/197) with available clinical data and qRT-PCR or other serological testing results (commercially available ELISA). To quantitate the strength of the anti-viral response, a calibration curve was developed consisting of different concentrations of anti-SARS-CoV-2-spike monoclonal antibody spiked into pooled SARS-CoV-2 seronegative serum or plasma matrix. Using the calibration curve, neutralizing antibody levels were quantitated in the assay from a single 1:100 serum test dilution, determined to be the minimum recommended dilution. For samples with high levels of SARS-CoV-2-neutralizing antibodies, a second dilution facilitated more precise quantitation. The virus neutralization units (VNUs) calculated using this calibrator method correlated closely ($p < 0.0001$) with plaque reduction neutralization titer-EC50 ($PRNT_{EC50}$) values determined by plaque reduction neutralization test against a clinical isolate of SARS-CoV-2. Taken together, these results demonstrate that the present IMMUNO-COV v. 1 assay accurately quantitates SARS-CoV-2 neutralizing antibodies in samples (e.g., sera or plasma) in biosafety level 2 (BSL2), high-throughput format and can provide vital information for, e.g., evaluating donor eligibility for convalescent plasma therapy programs and participant eligibility for various clinical trials, as well assessing immune responses to candidate SARS-CoV-2 vaccines.

Results

*Measurement of VSV-SARS-CoV-2-S-Δ19CT fusion through luciferase assay*

Biosafety level 3 (BSL3) contaminant and practices are required to safely perform neutralization assays utilizing SARS-CoV-2, making widespread testing of patient samples using these assays impractical. The present inventors therefore sought to develop a safer, scalable assay that could be used to detect SARS-CoV-2 neutralizing antibodies in blood samples. The present inventors designed an assay in which syncytia formation in Vero cells, induced by infection with SARS-CoV-2 spike (S) glycoprotein-expressing VSV, is detected using a dual split protein (DSP) luciferase reporter (Figure 2). Using reverse genetics, a recombinant Vesicular stomatitis virus (VSV) was generated in which the VSV glycoprotein (G) was replaced by the SARS-CoV-2 spike (S) glycoprotein (Figures 1 and 3A). Deletion of the last 19 amino acids in the C-terminal (cytoplasmic) domain of SARS-CoV-2 spike (S) glycoprotein improved virus rescue and amplification. The resulting virus (variant 2 VSV-SARS-CoV-2-S-Δ19CT), induced syncytia formation in Vero cell monolayers, which was enhanced in the presence of 0.4 μg/mL trypsin (Figure 3B). Virion incorporation of SARS-CoV-2-Δ19CT spike glycoprotein was confirmed in viral supernatants by immunoblot analysis (Figure 3C). SARS-CoV-2-Δ19CT spike glycoprotein, but not VSV-G, was detected, confirming efficient replacement of VSV-G with SARS-CoV-2-S-Δ19CT in the recombinant virus.

Since Vero cells were infected by VSV-SARS-CoV-2-S-Δ19CT and express both ACE2 and TMPRSS2 (Figures 3D and E), they were chosen as the reporter cell line for the assay. Parental Vero cells were transduced with self-inactivating lentiviral vectors SFFV-DSP₁₋₇-P2A-Puro or SFFV-DSP₈₋₁₁-P2A-Puro expressing Rluc8 155-156DSP1-7 and Rluc8 155-156DSP8-11, respectively, under control of the spleen focus forming virus (SFFV) promoter and linked to the puromycin resistance gene via a P2A cleavage peptide. Vero-DSP1 cells express Rluc8 155-156DSP1-7 luciferase-GFP fusion protein (SEQ ID NO: 14) comprising RLuc8 mutant *Renilla* luciferase fragment amino acids 1-155 and engineered GFP fragment amino acids 1-156. Vero-DSP2 cells express Rluc8 155-156DSP8-11 luciferase-GFP fusion protein (SEQ ID NO: 16) comprising RLuc8 mutant *Renilla* luciferase fragment amino acids 157-311 and engineered GFP fragment amino acids 157-231. RLuc8 mutant *Renilla* luciferase contains the mutations A55T, C124A, S130A, K136R, A143M, M185V, M253L, and S287L (see SEQ ID NO: 19). The sequence of engineered GFP is provided in SEQ ID NO: 18.

When mixed monolayers of Vero-DSP1 and Vero-DSP2 cells seeded at a 1:1 ratio were infected with VSV-SARS-CoV-2-S-Δ19CT, syncytia formation was observed and luciferase activity was readily detected at 24 hours post infection (Figure 3F). In contrast, luciferase activity was not detected in infected monolayers of Vero-DSP 1 alone or Vero-DSP2 alone, or in mock-infected Vero-DSP1/Vero-DSP2 monolayers. Thus, luciferase activity was specific to virus-induced cell fusion of mixed Vero-DSP1/Vero-DSP2 monolayers.

*Optimization of VSV-SARS-CoV-2-S-Δ19CT fusion*

Syncytia formation is required to produce functional luciferase in the present assay. However, complete destruction of the cell monolayer following syncytia formation reduces luciferase activity due to loss of cell viability. The inventors therefore determined the optimal time after VSV-SARS-CoV-2-S-Δ19CT infection to measure luciferase. EnduRen™ Live Cell Substrate (EnduRen™) is an engineered "pro-substrate", which must be cleaved by cellular esterases to produce the luciferase substrate coelenterazine. Because the pro-substrate is highly stable, luciferase activity can be measured repeatedly at any time between 2 and 24 hours after EnduRen™ addition to cells. By 15 hours after infection, luciferase activity in infected Vero-DSP1/Vero-DSP2 monolayers was readily distinguished from background signal in mock-infected cells (Figure 4A). Luciferase activity continued to rise, reaching peak levels around 26 hours after infection. Thus, optimal luciferase readout in the assay was between 24 to 30 hours after infection.

To enhance cell fusion and luciferase activity, and thereby increase assay sensitivity, the optimal cell density and virus concentration were also determined. Vero-DSP1/Vero-DSP2 cells were seeded at increasing cell densities and after 24 hours the monolayers were infected with increasing concentrations of VSV-SARS-CoV-2-S-Δ19CT. At all virus dilutions, increasing cell density improved luciferase signal (Figure 4B). As expected, syncytia formation and luciferase activity also increased when more virus was used to inoculate the monolayers. It was concluded that $6 \times 10^4$ cells/well should be used for the assay. It was also noted that for the best results cells should be seeded from flasks that are "under-confluent" and that cells can be seeded directly from cryopreserved stocks if given 2 days to recover.

Not surprisingly, additional studies across many experiments indicated that different lots of VSV-SARS-CoV-2-S-Δ19CT exhibited different potencies for inducing fusion. An appropriate concentration of virus for assay was therefore determined for each lot of virus based on performance against a set of contrived controls (see below).

*Detection of VSV-SARS-CoV-2-S-Δ19CT neutralization using the Vero-DSP reporter assay*

To demonstrate that virus neutralization could be detected using the present assay, the capacity of several purified proteins to inhibit virus-induced fusion was first tested. Affinity-purified polyclonal anti-SARS-CoV-spike antibody and soluble recombinant Ace2 protein bind spike on the virus surface and block entry into cells. Similarly, monoclonal anti-Ace2 antibody blocks the cellular receptors and limits virus entry. All three inhibitors successfully blocked VSV-SARS-CoV-2-S-Δ19CT-induced fusion in a dose-dependent manner (Figure 5A), demonstrating that the present assay can detect inhibition of VSV-SARS-CoV-2-S-Δ19CT infection. Importantly, VSV-SARS-CoV-2-S-Δ19CT neutralization by plasma acquired from a SARS-CoV-2 convalescing individual (NL1) was also detected. Compared to pooled plasma from three presumed SARS-CoV-2 seronegative individuals, heat-inactivated NL1 plasma neutralized VSV-SARS-CoV-2-S-Δ19CT in a dose-dependent manner (Figure 5B). Thus, the present assay was able to detect VSV-SARS-CoV-2-S-Δ19CT-neutralizing antibodies in convalescing plasma.

*Optimization of VSV-SARS-CoV-2-S-Δ19CT neutralization time*

In an early experiment it was observed that even in the absence of any neutralizing serum or inhibitors, incubation of VSV-SARS-CoV-2-S-Δ19CT at 37°C for 1 hour resulted in a loss of luciferase activity (Figure 6A). This loss of activity was partially abrogated at higher concentrations of virus. Nevertheless, the present inventors explored whether a shorter incubation time at room temperature could facilitate efficient virus neutralization. Twofold serial dilutions of neutralizing NL1 plasma were prepared and incubated with VSV-SARS-CoV-2-S-Δ19CT for 60 minutes at 37°C or for 15, 30, or 60 minutes at room temperature. Relative to the other conditions, luciferase activity was slightly higher at the 1:160 dilution in the sample that had been incubated for 15 minutes at room temperature (Figure 6B), suggesting that this condition may not be sufficient for complete neutralization. However, NL1 neutralized VSV-SARS-CoV-2-S-Δ19CT as well, if not more efficiently, when incubated at room temperature for 30 or 60 minutes, relative to incubation at 37°C for 1 hour. It was concluded that a 30-minute incubation at room temperature is sufficient for virus neutralization.

*Heat inactivation is not necessary for assay compatibility*

To further streamline the assay workflow, it was determined whether heat inactivation of samples is necessary to achieve reliable results. Serum samples were used for testing, due to their more ready availability compared to plasma samples. The inventors initially tested three presumptive SARS-CoV-2 seronegative samples and a commercially available sera pool (generated before fall of 2019) at a 1:100 dilution. In the sera pool, and two of the three negative sera, heat-inactivation lead to a modest decrease in luciferase activity (Figure 6C), possibly due to low-levels of virus inactivation by complement. Yet overall, heat-inactivation had little effect on VSV-SARS-CoV-2-S-Δ19CT-induced cell fusion and luciferase activity. When three additional presumptive negative sera were tested at multiple dilutions, there was again little difference in luciferase activity between heat-inactivated and non-heat-inactivated samples (Figure 6D). In contrast, heat inactivation reduced the capacity of a SARS-CoV-2 convalescing serum to neutralize VSV-SARS-CoV-2-S-Δ19CT (Figure 6E). It was therefore concluded that using non-heat inactivated samples is preferred, as it streamlines assay workflow and increases assay sensitivity.

*Determination of the minimum recommended dilution*

Sample interference is common in serological assays at high sample concentrations. Therefore, the inventors defined the minimum recommended dilution for assay samples, in which false positives due to sample interference are nearly eliminated. To this end, 39 presumptive SARS-CoV-2 seronegative samples were assayed at 2-fold serial dilutions ranging from 1:10 through 1:320. As expected, interference with virus fusion and luciferase activity was observed at higher serum concentrations (Figure 7A) but disappeared as the samples were further diluted. Statistical analyses indicated that the minimum recommended dilution was approximately 1:100. At this dilution there is greater than 95% confidence that the luciferase signal from a negative serum sample will be ≥ 50%. In all future studies, therefore, 1:100 was used as the dilution for both test serum samples as well as pooled SARS-CoV-2 seronegative sera controls.

*Development of a contrived positive control*

Due to the limited availability of large quantities of qualified SARS-CoV-2 seropositive sera, the inventors sought to develop a contrived positive control for the assay. To this end, various concentrations of mAb10914, a monoclonal antibody that binds to the SARS-CoV-2 spike (S) glycoprotein, were spiked into pooled SARS-CoV-2 seronegative serum or media alone. As a control, isotype antibody was also used. As expected, mAb10914 caused dose-dependent neutralization of VSV-SARS-CoV-2-S-Δ19CT in both media and pooled negative sera (Figure 7B and C). In contrast, the isotype control antibody did not significantly alter virus-induced luciferase activity, indicating that neutralization by mAb 10914 was specific to spike binding. To establish appropriate concentrations to use for contrived controls in validation studies, a total of nine 3-fold serial dilutions of mAb 10914 (from 10 μg/mL through ~0.0015 μg/mL) were tested in duplicate on three separate runs performed by three separate analysts. Because of variability in raw RLU (relative light unit) values between plates, results were normalized as percent luciferase signal relative to the luciferase signal in control wells containing pooled SARS-CoV-2 seronegative sera. The average (mean) percent luciferase signal at each concentration was plotted and a non-linear regression curve was generated (Figure 7D). The linear range of the assay was between approximately 10% to 80% signal or 0.01 to 0.6 μg/mL mAb10914. 2 μg/mL mAb10914 was selected as a contrived positive control high (CPC High), which consistently reduces signal to < 20%. Based on a 95% confidence of the response curve, the concentration of mAb10914 that should elicit a signal below 50% was calculated as 0.18 μg/mL, which thus represents an approximate limit of detection of the assay.

*Examination of assay specificity and sensitivity*

CPC High value was used to define assay specificity and sensitivity. To measure specificity, 30 presumptive SARS-CoV-2 seronegative samples were tested alone or spiked with 2 μg/mL (CPC High) of mAb10914. This evaluation was conducted to verify that at the optimized 1:100 serum dilution no false positives were obtained and that all contrived positive controls showed virus neutralization. In two separate analytical runs performed by different analysts, no false positives or negatives were observed (Table 1). One negative sample was qualified as indeterminant in the first assay due to one replicate having signal < 50%. However, in the second assay the sample was correctly identified as negative. Thus, in this validation IMMUNO-COV v. 1 assay sensitivity and specificity was 100%. To measure assay specificity near the limit of detection, 30 presumptive SARS-CoV-2 seronegative samples (at 1:100 serum dilution) were tested alone; and 19 of the samples were also measured after spike with 0.35 μg/mL of mAb10914. 11 of the samples were spiked with 2 μg/mL mAb10914, which is not near the limit of detection. The experiment demonstrated 68% sensitivity near the limit of detection (Table 2).

To evaluate assay sensitivity, a similar, but single-blinded analysis was performed using the same 30 presumptive SARS-CoV-2 seronegative samples. Duplicate samples were assayed at 1:100, where one replicate sample was spiked with mAb 10914 at 2 μg/mL (CPC High) or 0.35 μg/mL (borderline inhibition), before being assigned blinded IDs. Of the 30 negative samples, 26 were identified as negative and 4 as indeterminant (Table 2). 10 of the 11 CPC High samples were detected, and sample preparation error was suspected in the single positive not identified. From the 19 borderline samples, 13 were identified as positive, 4 as negative, and 2 as indeterminant, concordant with the samples containing only a low-level of neutralizing antibody and 0.35 μg/mL being close to the previously estimated assay limit of detection.

To further evaluate assay specificity, samples from patients who had recovered from documented infections with four common coronaviruses (HCoV-NL63, HCoV-229E, HCoV-OC43 and HKU1) prior to December 2019 were tested. No cross-reactivity with the common coronaviruses was observed demonstrating strong IMMUNO-COV v. 1 assay specificity for SARS-CoV-2.

*Demonstration of Clinical Agreement*

Given that the present assay performed well with the contrived controls, the inventors proceeded with blinded studies to determine whether results correlated well with clinical symptoms. Serum samples were acquired from negative donors (symptomless, negative for SARS-CoV-2 by PCR, or SARS-CoV-2 seronegative by ELISA), positive donors (positive for SARS-CoV-2 by PCR or SARS-CoV-2 seropositive by ELISA), and contact positive donors (not tested, but in direct contact with someone positive for SARS-CoV-2 by PCR). A total of 230 samples were received and blinded to the assay analysts until after testing was complete. Of the 125 negative samples received, all 125 tested negative in the assay (Table 3), providing further evidence of the assay's excellent specificity. All 38 of the known SARS-CoV-2 seropositive samples also tested positive in the assay. From the 34 samples obtained from donors that had previously tested positive for SARS-CoV-2 by PCR, neutralizing antibodies were identified in 31 samples. Upon closer examination, one sample was expected to be negative because it was collected only 8 days after positive PCR test, which is not sufficient time for a strong antibody response to develop. From donors who had contact with someone positive for SARS-CoV-2, 13 of 33 samples (39.4%) were identified as positive for neutralizing antibodies. Taken together, the present data indicate that the assay has excellent clinical agreement.

*Correlation between VSV-SARS-CoV-2-S-Δ19CT and SARS-CoV-2 neutralization*

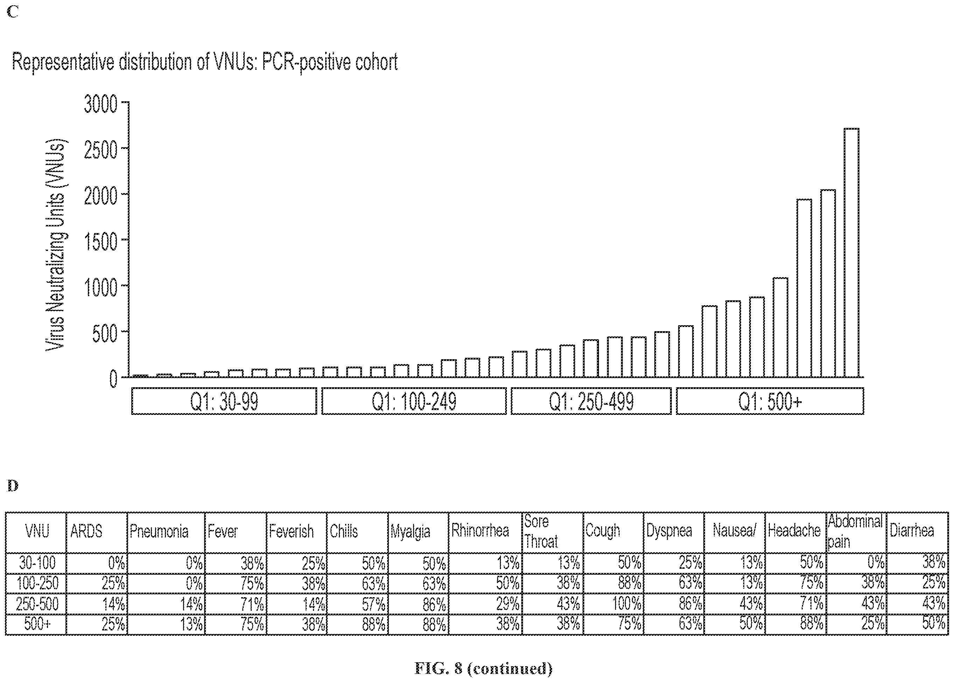

The strong clinical agreement observed for the assay supported the inventors' premise that neutralization of VSV-SARS-CoV-2-S-Δ19CT accurately reflects neutralization of SARS-CoV-2. In order to directly demonstrate this correlation, 20 of the serum samples from above were also tested against a clinical isolate of SARS-CoV-2 in a BSL-3 plaque reduction neutralization test (PRNT) (PRNT is described, e.g., in Wang et al., Nat Commun 11, 2251 (2020) doi.org/10.1038/s41467-020-16256-y; Okba et al., Emerg Infect Dis. 2020 Jul. doi.org/10.3201/eid2607.200841; Ranawaka et al., Euro Surveill. 2020;25(16):pii=2000421. doi.org/10.2807/1560-7917.ES.2020.25.16.2000421). Each of the 5 samples that tested negative in the present assay were also below the limit of detection in the PRNT assay (Table 4). Likewise, the 15 samples that tested positive in the present assay also were positive in the PRNT assay. Moreover, quantitative virus neutralizing units (VNUs) determined in the present assay correlated closely with PRNT50% and $PRNT_{EC50}$ values (Tables 4-5 and Figure 8A). To calculate VNUs, a calibration curve consisting of a five-point dilution series of mAb10914 in 1:100 pooled SARS-CoV-2 seronegative sera was included on each assay plate. Based on percent luciferase signal, the equivalent mAb10914 concentration for each sample was determined and multiplied by 100 to give the VNUs for that sample. Strong correlation (Pearson ρ = 0.8902, p < 0.0001) was observed between VNUs and $PRNT_{EC50}$ values. The relationship between VNU and virus neutralization titer (VNT) EC50 values was also examined. Five 2-fold serial dilutions of forty-four SARS-CoV-2 positive samples were prepared and tested in the assay. The percent luciferase signal for each dilution was plotted and the resulting curves were used to determine the $VNT_{EC50}$ values. Statistical comparison of the VNU and $VNT_{EC50}$ indicated excellent correlation (Spearman ρ = 0.8327, p < 0.0001) between the two values (Figure 8B), providing additional demonstration of the accuracy of the VNU values. It was concluded that neutralization of VSV-SARS-CoV-2-S-Δ19CT closely replicates neutralization of SARS-CoV-2 and that VNUs provide an accurate method to quantitate the strength of the anti-SARS-CoV-2 antibody response. Figure 8C depicts representative VNUs from PCR-positive samples showing that convalescent samples exhibit a range of VNUs.

Correlation between VNU values and symptom severity was investigated for 31 positive samples for which clinical symptoms were self-reported (Figure 8D). 3 patients reported being asymptomatic: two were in lowest VNU quadrant and one in the second-lowest VNU quadrant. One patient who experienced only chills generated over 1000 VNUs of neutralizing antibodies. It follows that individuals with severe disease are more likely to develop strong neutralizing antibody responses, but severe disease is not required for a strong neutralizing antibody response.

*Example of a 96-well plate layout for the assay*

A non-limiting example of a 96-well plate layout for the assay is shown in Figure 9. Each sample and control is prepared in singlet in u-well plates or tubes and then transferred to duplicate wells of the Vero-DSP1/DSP2 mixed cells in 96-well black-walled, clear-bottom plates. Samples S1 to S41 are shown. Each sample is assayed at 1:100 in OptiMEM. It is possible to change the plate location of the samples and controls. The important part is to ensure that all of the controls are included on each plate. The controls include BC, NC, and Standards (St) 1 to 5.

- BC is negative (matrix) pooled serum at a 1:100 dilution and NO virus
- NC is negative (matrix) pooled serum at a 1:100 dilution + VSV-SARS-CoV-2-S-Δ19CT
- Each Standard contains 3 components:

    Negative (matrix) pooled serum at 1:100 dilution

$$\text{VSV-SARS-C}_\text{o}\text{V-2-S-}\Delta19\text{CT}$$

    Positive control (e.g., mAb10914) antibody at a specified concentration

    The final concentration of mAb10914 for each standard is currently: St1 = 3 μg/mL, St2 = 1 μg/mL, St3 = 0.3333 μg/mL, St4 = 0.1111 μg/mL, St5 = 0.0370 μg/mL (An alternative standard curve can contain 6 standards and the range will be: St1 = 2 μg/mL, St2 = 1 μg/mL, St3 = 0.5 μg/mL, St4 = 0.25 μg/mL, St5 = 0.125 μg/mL, St6 = 0.0625 μg/mL)

To determine the result the following steps are performed:

1. Luciferase activity is measured (2000 ms integration/well, 200 ms settle time between wells) and the output unit is relative light unit (RLU).
2. The average (mean) RLU from the BC wells is determined and subtracted from each other to give background-corrected RLU values.
3. The mean corrected RLUs from the NC wells is determined.
4. Controls are examined for any obvious outliers, which are removed from mean values.
5. The percent luciferase signal is determined for each sample and standard (calculated for each replicate well individually). Percent luciferase signal is:

$$percent\ luciferase\ signal = \frac{corrected\ RLU\ of\ sample}{corrected\ mean\ RLU\ of\ NC} \times 100\%$$

6. To determine qualitative result the percent signal for each sample replicate is examined and resulted as follows:

- Negative: both sample replicates have percent luciferase signal ≥ 50%
- Positive both sample replicates have percent luciferase signal < 50%
- Indeterminant: one sample replicate has percent luciferase signal ≥50% and other <50%.

7. To calculate the quantitative virus neutralizing units (VNUs) for positive samples, the following steps are performed within the Magellan software automatically following reading of the luciferase signal:

    a. The standards are converted to arbitrary VNUs by multiplying their values by 100 (e.g. 3 μg/mL mAb10914 becomes 300 VNUs and 0.33 μg/mL become 33 VNUs).
    b. The VNUs are plotted relative to background-corrected RLU values and a 4-parameter fit curve from the standards is generated.
    c. Using the standard curve, the VNU value for each sample replicate and the mean of duplicate samples is determined.
    d. VNUs above 300 are above the limit of quantification. They can either be reported as >300 VNUs or the samples can be assayed again at a higher dilution to get the sample within the linear range of the standard curve. After assay, the VNU is then multiplied by the increased dilution factor. (For example, a sample assayed at a 1:1000 dilution that reads as 180 VNUs is multiplied by 10 to give 1800 VNUs because the 1:1000 dilution is 10 times more dilute than a 1:100 dilution). Informal parallelism testing has been conducted, and dilutions have been performed out to 1:3200 for use in the assay (a dilution factor of 32).

Discussion

High demand for serological tests to detect anti-SARS-CoV-2 antibodies is fueled by necessity to understand past rates of infection and evaluate the efficacy of vaccines and plasma therapies. Numerous ELISA and rapid diagnostic tests have been developed to detect anti-SARS-CoV-2 antibodies, and several are approved in various countries for diagnostic testing. Yet, an easily scalable serological assay for detecting antibodies capable of neutralizing SARS-CoV-2 has remained elusive. Described herein is IMMUNO-COV v. 1 assay for the detection of SARS-CoV-2 neutralizing antibodies that is high-throughput, performed under lower biosafety (BSL2) conditions, scalable, and quantitative. The present assay is useful, e.g., for screening donor samples for SARS-CoV-2 plasma therapy trials, evaluating immune responses to candidate vaccines, and better understanding how antibody responses relate to SARS-CoV-2 immunity.

The present assay exploits the fusion phenotype of recombinant VSV-SARS-CoV-2-S-Δ19CT (or other similar constructs such as VSV-SARS-CoV-2-S) to produce quantitative luciferase signal in a 96-well plate format (Figures 2, 3, 9, and 10). Virus neutralization by test serum samples is then detected as a reduction in luciferase activity. Importantly, results from the present assay correlate closely ($\rho = 0.8987$) with virus neutralization titers determined using a clinical isolate of SARS-CoV-2 (Figure 8). Interestingly, when positive control antibody mAb10914 was tested in the SARS-CoV-2 plaque reduction neutralization test, it had a PRNT50% titer of 40960, which corresponds to a concentration of 0.026 μg/mL. In the present assay, it was determined that the lowest concentration of mAb10914 that could inhibit > 50% of VSV-SARS-CoV-2-S-Δ19CT was approximately 0.18 μg/mL (Figure 8). The present data indicate that even though the present assay uses a different virus (VSV-SARS-CoV-2-S-Δ19CT), it accurately measures anti-SARS-CoV-2 neutralizing antibodies and is unlikely to detect positives that are not also able to inhibit SARS-CoV-2. The present assay also demonstrated perfect correlation with the EUROIMMUN assay.

During testing, three samples collected from donors that were positive for SARS-CoV-2 by PCR test were negative using the present assay (Table 4). One of these samples was deemed a true negative, as it was received only 8 days after the positive PCR test, earlier than a strong antibody immune response is expected, and the sample also tested negative in the SARS-CoV-2 PRNT50 assay. Whether the other two samples represent false or true negatives has not yet been determined. Possibly, the samples indeed lacked any significant level of neutralizing antibodies, which supports the hypothesis that a positive PCR test results alone do not guarantee a protective antibody response. Alternatively, the samples may have only possessed low levels of neutralizing antibodies that were missed by the present assay. Studies performed using the contrived control spiked into negative pooled sera at a low level, suggested that near the limit of detection assay sensitivity is 68% (Table 2). However, sensitivity was 90.9% when the contrived control was spiked in at a higher concentration (2 μg/mL). Over half of the clinical samples tested exhibited virus neutralization units (VNUs) equivalent to at least 2 μg/mL neutralizing antibody. It is not surprising, therefore, that when calculating sensitivity based on blinded tests, a much higher degree of sensitivity (97.2%) is observed. Signal below 50% is cutoff. Variability was within acceptable limits.

The RBD of spike is likely the primary target of neutralizing antibodies, yet additional studies are needed to determine whether the assay, which only measures disruption of RBD-ACE2 binding, will accurately quantitate total SARS-CoV-2 neutralizing antibodies. Notably, the RBD domain of coronaviruses constantly switches between a standing-up and lying-down position (Yuan 2017 and Gui 2017), suggesting that some neutralizing antibody targeting may be context dependent. As proteolytic activation of spike is also required for membrane fusion and virus entry into cells the S1/S2 cleavage boundary may also be a target for neutralizing antibodies. Several recently developed assays use viral vectors pseudotyped with SARS-CoV-2 spike glycoprotein. However, scalability of pseudotyped viruses is more challenging than with live-replicating viruses (as used in the present assay), offering the present assay an advantage for widespread diagnostic testing.

Another advantage of the present assay is that the level of SARS-CoV-2 neutralizing antibodies is quantitated without the need for a full dilution series of the test samples. A minimum recommended dilution of 1:100 was determined for the assay. At this dilution, about 16.9% of samples fell above the linear range of the calibration curve. For samples containing high levels of SARS-CoV-2 neutralizing antibodies, a second dilution (e.g., 1: 1000) can facilitate a more precise quantitation of antibody levels.

Additional testing has indicated that the Vero-DSP1 and Vero-DSP2 cell lines can be passaged for at least three weeks without impact on assay performance and that the SARS-CoV-2 neutralizing antibodies detected using this assay are stable under a number of storage conditions, including refrigeration for up to one week and multiple freeze-thaw cycles. Experiments with NL1 and a small pool of presumptive SARS-CoV-2 seronegative plasma (Figures 5B and 6B) suggest the assay is also compatible with plasma samples.

Studies with MERS and SARS-CoV have demonstrated antibody dependent enhancement of infection *in vitro* (Yip, Wang, Wan, and Jaume). Studies in NHP also suggest possible disease augmentation with increasing antibody levels for SARS-CoV (Liu et al. 2019). Some evidence supports antibody dependent enhancement in clinical cases of SARS (Ho 2005, Peiris 2003). Yet early studies using COVID-19 convalescing plasma to treat severe cases of COVID-19 have shown great promise. As researchers work to further refine convalescing plasma therapies, develop monoclonal antibody therapies, and engineer effective vaccines that produce strong anti-SARS-CoV-2 immune responses, there is clearly still much to be learned about the interaction between SARS-CoV-2 and the immune system. How total anti-SARS-CoV-2 antibody levels relate to neutralizing antibody levels is a key piece of this puzzle. A piece that the present assay can help to answer.

Materials and Methods

*Cells.* African green monkey Vero cells, Vero-αHis, and baby hamster kidney BHK-21 cells were maintained in high-glucose DMEM supplemented with 5% fetal bovine serum and 1X penicillin/streptomycin (complete media) at 37°C/5% $CO_2$.

Vero-DSP1 (Vero-DSP-1-Puro; CLR-73) and Vero-DSP2 (Vero-DSP-2-Puro; CLR-74) cells were generated by transducing Vero cells (African green monkey-derived kidney epithelial cells) with self-inactivating lentiviral vectors SFFV-DSP$_{1-7}$-P2A-Puro or SFFV-DSP$_{8-11}$-P2A-Puro expressing Rluc8 155-156DSP1-7 and Rluc8 155-156DSP8-11, respectively, under control of the spleen focus forming virus (SFFV) promoter and linked to the puromycin resistance gene via a P2A cleavage peptide. Transduced cells were selected using 10 μg/mL puromycin. Following selection Vero-DSP cells were maintained in complete media supplemented with 5 μg/mL puromycin. Puromycin was excluded removed when cells were seeded for assays.

Vero-DSP1 cells express Rluc8 155-156DSP1-7 luciferase-GFP fusion protein (SEQ ID NO: 14) comprising RLuc8 mutant *Renilla* luciferase fragment amino acids 1-155 and engineered GFP fragment amino acids 1-156. Vero-DSP2 cells express Rluc8 155-156DSP8-11 luciferase-GFP fusion protein (SEQ ID NO: 16) comprising RLuc8 mutant *Renilla* luciferase fragment amino acids 157-311 and engineered GFP fragment amino acids 157-231. RLuc8 mutant *Renilla* luciferase contains the mutations A55T, C124A, S130A, K136R, A143M, M185V, M253L, and S287L (see SEQ ID NO: 19). The sequence of engineered GFP is provided in SEQ ID NO: 18.

*Generation of VSV-SARS-CoV-2-S-Δ19CT.* As discussed in Example 1, above, the full length SARS-CoV-2 spike (S) glycoprotein sequence (NCBI Reference Sequence: NC_045512.2; Protein_ID: YP_009724390.1) (variant 1; VSV SARS-CoV-2 dG = VSV-SARS-CoV-2-S; amino acid sequence SEQ ID NO: 1; codon optimized coding nucleotide sequence SEQ ID NO: 2); (2) the SARS-CoV-2 S glycoprotein sequence with a deletion of 19 amino acids KFDEDDSEPVLKGVKLHYT (SEQ ID NO: 20) at the C terminus (variant 2; VSV SARS-CoV-2 Δ19CT dG = VSV-SARS-CoV-2-S-Δ19CT; amino acid sequence SEQ ID NO: 3; codon optimized coding nucleotide sequence SEQ ID NO: 4). Full-length human codon-optimized SARS-CoV-2 spike (S) glycoprotein (NC_045512.2) was cloned in pUC57. The plasmid was used as a PCR template to generate a cDNA encoding SARS-CoV-2-S with a deletion in the nucleotides encoding the C-terminal 19 amino acids (S-Δ19CT) and 5' MluI and 3' NheI restriction sites. To generate the viral genome, the amplified PCR product containing S-Δ19CT was cloned into pVSV-M5 1R in place of VSV-G using the MluI and NheI restriction sites (Figures 1 and 3A). Plasmid was sequence verified and used for infectious virus rescue on BHK-21 cells as previously described. VSV-G was co-transfected into the BHK-21 cells to facilitate rescue but was not present in subsequent passages of the virus. The virus was propagated in Vero-αHis cells by inoculating 80% confluent monolayers in 10-cm plates with 1 mL of virus. Viruses were harvested 48 hours after inoculation, aliquoted, and stored at -80°C until use. Aliquots were used for tissue culture infectious dose 50 ($TCID_{50}$) assay on Vero-αHis cells.

*Infections.* Vero monolayers in 6-well plates were inoculated with 2 mL of passage 3 VSV-SARS-CoV-2-S-Δ19CT in OptiMEM or OptiMEM alone (mock). After 4 hours at 37°C/5% $CO_2$, the inoculums were removed and the cells were rinsed once with OptiMEM. Fresh OptiMEM alone or OptiMEM containing 4 μg/mL trypsin were added to wells. Cells were returned to the 37°C/5% $CO_2$ incubator until 20 hours after infection, when they were photographed at 100x magnification using an inverted microscope.

*Reagents.* EnduRen™ live cell substrate was prepared according to manufacturer's instructions (Promega #E6482). For inhibition studies, mouse anti-SARS-CoV-2 spike antibody (GeneTex #GTX632604), affinity-purified, polyclonal anti-human-ACE2 antibody (R&D Systems #AF933), and recombinant human ACE2 (R&D Systems #933-ZN-010) were diluted in OptiMEM to the desired concentrations.

*Luciferase Assays.* Assays were performed in 96-well black-walled plates with clear bottoms. EnduRen™ substrate was added to wells (final concentration of 7.5 μM) at various times after infection, but at least 2 hours before reading bioluminescence on a Tecan Infinity or Tecan M Plex instrument (2000 ms integration, 200 ms settle time). For kinetic experiments, repeated bioluminescence reads were performed without the addition of more EnduRen™ for up to 24 hours after initial EnduRen™ addition; at later time points additional EnduRen™ was added to wells at least 2 hours prior to reading bioluminescence.

*Collection of plasma and sera samples.* A clinical protocol to collect blood samples for assay validation was reviewed and approved by Western IRB (study ID: VYR-COV-001). Serum and plasma samples were collected from patients who had previously tested positive for SARS-CoV-2 infection by a PCR test, from patients who had known exposure to individuals infected with SARS-CoV-2 and symptoms of COVID-19, as well as a cohort of patients of were presumed negative for antibodies, i.e. individuals who never had symptoms of COVID-19 disease and were likely never exposed. A total of 150 adults were enrolled at BioTrial based in Newark, New Jersey and Olmsted Medical Center in Rochester, Minnesota. Eighty additional sera aliquots from samples that had been tested using the EUROIMMUN assay.

*Blinded sample testing.* Sera samples were assigned a random ID prior to being given to analysts for assay testing. Samples were assayed in batches, with an unknown number of expected positive and negative samples in each batch. Each sample was assayed at a 1:100 dilution, with all positive and some negative samples being further assayed in a dilution series including 1:100, 1:200, 1:400, 1:800, and 1:1600.

*PRNT Assay.* Sera was heat-inactivated for 30 minutes at 56°C and serially diluted in X. SARS-CoV-2 was diluted to 800 PFU/mL and mixed with an equal volume of diluted sera (final dilutions of serum with virus 1:20, 1:40, 1:80, 1:160, 1:320, 1:640, 1:1280, 1:2560, 1:5120, 1:10240, 1:20480, 1:40960). Virus mixed with an equal volume of media alone was used as a control. After a 1 hour incubation at 37°C, 250 $\mu$L of virus/serum or virus/media mixes were used to inoculate Vero-E6 monolayers in 6-well plates. Inoculations proceeded for 1 hour at 37°C with occasional rocking, before monolayers were overlaid with 3 mL of 0.4% low-melting agarose in Minimal Essential Media supplemented with 2% fetal bovine serum and 1X penicillin/streptomycin. Plates were incubated at 37°C until plaques appeared, then fixed with 10% formaldehyde, and stained with 0.25% crystal violet. Plaques were counted and the number of plaques at each dilution was plotted and used to determine the PRNT$_{EC50}$ value for each sample by a nonlinear regression model. Plaque counts greater than 30 were too numerous to count and were considered as equivalent to the virus/media control. The PRNT50% titer was determined as the highest dilution (represented as the reciprocal) of serum that inhibited 50% of plaques relative to the virus/media control well.

*Flow cytometry.* Vero-DSP1 cells were dislodged using Versene, counted, and transferred to microcentrifuge tubes ($5\times10^5$ cells/tube was used for ACE2 staining and $1.5\times10^6$ cells/tube was used for TMPRSS2 staining). For ACE2 staining, cells were pelleted and resuspended in 100 $\mu$L FACS buffer (2% FBS in DPBS) containing 0.2 $\mu$g goat-anti-human ACE2 (R&D Systems #AF933). After 30 minutes on ice, cells were rinsed with 1 mL FACS buffer and resuspended in 100 $\mu$L FACS buffer containing 5 $\mu$E donkey-anti-goat IgG-PE secondary antibody. After 30 minutes on ice, cells were rinsed with 1 mL FACS buffer and fixed with 1% paraformaldehyde for 15 minutes on ice. Cells were washed twice with FACS buffer, resuspended in 500 $\mu$L FACS buffer and analyzed on a CYTOFLEX flow cytometer (Beckman Coulter). For TMPRSS2 staining cells were resuspended in 1 mL ice-cold 70% ethanol in DPBS and incubated on ice for 10 minutes. Cells were centrifuged, washed once with 1 mL FACS buffer, and resuspended in 100 $\mu$L of a 0.5% saponin solution containing 4 $\mu$g rabbit anti-TMPRSS2 (Invitrogen #PA5-14264). After 30 minutes on ice, samples were washed twice with 1 mL FACS buffer and resuspended in 100 $\mu$L of a 0.5% saponin solution containing 2 $\mu$L goat anti-rabbit IgG-AF647 secondary antibody. After 30 minutes on ice, cells were washed twice with FACS buffer and fixed with 1% paraformaldehyde for 15 minutes on ice. Cells were washed twice with FACS buffer, resuspended in 500 $\mu$L FACS buffer and analyzed on a CYTOFLEX flow cytometer (Beckman Coulter). For both ACE2 and TMPRSS2 staining, secondary antibody only controls were used for isotype.

*Immunoblot.* Total cells and culture supernatants of Vero cells infected with VSV-GFP or VSV-SARS-CoV-2-S-Δ19CT or mock-infected were harvested 48 hours after infection. Virus was released from the cell membranes by freeze-thaw and cell debris was removed by centrifugation. Samples were diluted in duplicate in LDS sample buffer (Invitrogen #B0007) and reducing agent (Invitrogen #B0009) according to the manufacturer's directions, incubated at 70°C for 10 minutes, and 40 $\mu$L of each sample was ran on duplicate 4-12% Bis-Tris 15-well gels (Invitrogen #NW04125Box) along with precision plus protein dual color standard (Bio-Rad #161-0374). Proteins were transferred to nitrocellulose membranes using a Power Blotter XL. Membranes were blocked in 5% non-fat dry milk in TBST, washed three times with TBST, and incubated for 1 hour at room temperature with primary antibody mouse anti-SARS-CoV-2 spike (1:1000, GeneTex #GTX632604) or rabbit polyclonal anti-VSV (1:5000, Imanis Life Sciences #REA005). Membranes were washed three times with TBST and incubated for 1 hour at room temperature with secondary antibody goat anti-mouse IgG-HRP (Prometheus #20-304) or goat anti-rabbit IgG-HRP (Prometheus #20-303) at 1:20,000. Membranes were washed three times with TBST, and protein bands were developed for 2 minutes at room temperature using ProSignal® Dura ECL Reagent (Prometheus #20-301). Protein bands were imaged using a BioRad ChemiDoc Imaging System.

*Determination of percent response and virus neutralization units.* Raw relative light unit (RLU) values for each well were blank corrected using the mean RLU value from background wells receiving pooled SARS-CoV-2 seronegative serum at 1:100 but not virus. The percent response was determined for each sample well as the corrected RLU value divided by the mean corrected RLU value from negative control wells receiving virus and pooled SARS-CoV-2 seronegative serum at 1:100 multiplied by 100%. Samples with a percent response < 50% for both sample replicates were classified as positive. Samples with a percent response $\geq$ 50% for both sample replicates were classified as negative. When one replicate exhibited a positive response and the other a negative response, the sample was classified as indeterminant and assay was repeated. Virus neutralizing titers (VNUs) were determined for positive samples based on a calibration curve. The calibration curve was run on each plate and consisted of mAb10914 spiked into pooled SARS-CoV-2 seronegative sera at 3, 1, 0.33, 0.11, and 0.037 $\mu$g/mL. From the calibration curve, the equivalent concentration of neutralizing antibody for a given percent response was determined. To convert to VNU, the antibody equivalent concentration was multiplied by 100.

*Statistical Analyses.* Descriptive statistics, comparisons, and regression analyses were performed in Graph Pad Prism, v8.4.0 (San Diego, CA). Tests for normality of variance were conducted, and whenever possible parametric comparisons were used. For non-normal datasets, non-parametric approaches were used. A four-parameter non-linear regression was used for the calibration curve of the virus neutralizing units within the assay. For correlation analyses, Spearman's rank-order correlation analysis was conducted.

**Table 1: Assay specificity and sensitivity**

| | Analyst A | | Analyst B | |
|---|---|---|---|---|
| | **Diluted Matrix** | **CPC spike*** | **Diluted Matrix** | **CPC spike** |
| **Total Tested** | **30** | **30** | **30** | **30** |
| Number Positive | 0 | 30 | 0 | 30 |
| Number Negative | 29 | 0 | 30 | 0 |
| Number Indeterminant | 1† | 30 | 0 | 0 |

* CPC spike: mAb10914 spiked into diluted matrix sample at 2 μg/mL.
t Sample identified as indeterminant due to one replicate with signal <50%.

**Table 2: Assay specificity near the limit of detection**

| | Sample Type* | | |
|---|---|---|---|
| | **Negative** | **Borderline** | **Positive** |
| **Number of samples** | **30** | **19** | **11** |
| Result positive | 0 | 13 | 10 |
| Result negative | 26 | 4 | 1† |
| Result indeterminant$ | 4 | 2 | 0 |

* Sample Type: Negative is diluted seronegative (matrix) sample; borderline is diluted matrix spiked with mAb10914 at 0.35 μg/mL; positive is diluted matrix spiked with mAb10914 at 2 μg/mL.
$ Indeterminant: one replicate with signal >50%, other replicate with signal <50%.
† Technical error during sample preparation is suspected.

**Table 3: Clinical correlation - blinded studies**

| | Total tested | Number Negative Results | Number Positive Results |
|---|---|---|---|
| **Negatives** | **125** | **125** | **0** |
| Presumptive (not tested) | 83 | 83 | 0 |
| Negative by ELISA* | 42 | 42 | 0 |
| **Positives** | **72** | 3t | 69 |
| Positive by PCR | 34 | 3t | 31 |
| Positive by ELISA | 38 | 0 | 38 |
| **Contact Positives$** | **33** | **20** | **13** |

$ Donors were not tested but had contact with someone confirmed positive by PCR.
t One sample received only 8 days after positive PCR result.
* Samples also tested by EPITOPE IgG ELISA (clinically validated

**Table 4: Correlation between IMMUNO-COV v. 1 and SARS-CoV2 Plaque Reduction Neutralization Test**

| Random ID | IMMUNO-COV Result | IMMUNO-COV VNU* | PRNT$_{EC50}$† | Clinical Correlation |
|---|---|---|---|---|
| RID-165 | Negative | N/A | < 20$ | Presumed Negative |
| RID-152 | Negative | N/A | < 20 | Presumed Negative |
| RID-121 | Negative | N/A | < 20 | Contact positive |
| RID-195 | Negative | N/A | < 20 | Contact positive |
| RID-239 | Negative | N/A | < 20 | PCR positive 8-days before testing |
| RID-142 | Positive | 31.8 | 340.0 | PCR Positive |

(continued)

| Random ID | IMMUNO-COV Result | IMMUNO-COV VNU* | PRNT$_{EC50}$† | Clinical Correlation |
|---|---|---|---|---|
| RID-168 | Positive | 69.9 | 277.7 | Contact tested positive |
| RID-272 | Positive | 95.2 | 319.8 | PCR Positive |
| RID-288 | Positive | 125.5 | 530.7 | Contact tested positive |
| RID-144 | Positive | 134.2 | 665.8 | PCR Positive |
| RID-138 | Positive | 281.7 | 2061 | Contact tested positive |
| RID-193 | Positive | 345.4 | 1545 | PCR Positive |
| RID-183 | Positive | 402.6 | 1449 | PCR Positive |
| RID-167 | Positive | 430.7 | 845.6 | Contact tested positive |
| RID-287 | Positive | 447.8 | 2130 | PCR Positive |
| RID-290 | Positive | 491.0 | 2999 | Contact tested positive |
| RID-192 | Positive | 775.7 | 4362 | Contact tested positive |
| RID-194 | Positive | 1504.9 | 1502 | Contact tested positive |
| RID-235 | Positive | 2046.6 | 7311 | PCR Positive |
| RID-236 | Positive | 2702.5 | 5203 | PCR Positive |

\* VNU: Virus Neutralization Unit. Arbitrary quantitative value assigned based on calibration curve; higher VNUs represent higher neutralizing antibody levels.

\$ The limit of detection for this assay is 20.

† PRNT50: Plaque Reduction Neutralization Titer 50%. Highest sample dilution able to inhibit at least 50% of plaque formation; expressed as the recipricol value.

*Table 5:* **Correlation between IMMUNO-COV v. 1 and SARS-CoV2 Plaque Reduction Neutralization Test**

| VNU | PRNT | Result |
|---|---|---|
| < 10 | < 1:40 | Negative |
| 10 to 30 | 1:40 to 1:80 | Borderline |
| 30 to 75 | 1:160 | Positive |
| 75 to 150 | 1:320 | Positive |
| 150 to 300 | 1:640 | Positive |
| 300 to 600 | 1:1280 | Positive |
| 600 to 1500 | 1:2560 | Positive |
| 1500 to 3000 | 1:5120 | Positive |
| > 3000 | ≥ 10240 | Positive |

***Core Validation***

Core specificity is tested as follows. At least 30 sera samples from assumed SARS-CoV-2 seronegative individuals are analyzed on the assay in duplicate at their MRD. Additionally, for each serum sample analyzed, a corresponding CPCH sample is prepared by spiking into each sample at the concentration determined in the above section on optimized sample dilution, and then analyzed in duplicate. This assessment should be completed on at least two assay runs by two separate analysts. The mean response and %CV for each replicate dilution is reported.

A false positive rate is determined by the percentage of control matrix samples that generate a response below 50%. Acceptance criteria for false positive rate is below 5%.

A false negative rate is determined by the percentage of CPCH samples that generate a response above 50%. The false negative rate is reported in the final assay validation summary but does not have acceptance criteria.

Clinical agreement is determined as follows. At least three sera samples from convalescent patients who previously tested positive for SARS-CoV-2 by a molecular diagnostic test or another serological method, or were declared presumed positive by a medical professional due to onset of symptoms is tested on the assay. Information from presumed-positive patients is self-reported. This assessment is completed by plating the diluted samples in duplicate on at least one assay run, performed by one analyst. If possible, due to available sample volumes, this analysis is completed with three assay runs by at least two analysts. Submission of samples is conducted single-blinded to the analyst(s) with a mix a presumed positive and presumed negative samples. Presumed positive samples is analyzed by another serological method to allow for an accuracy assessment.

The mean response and %CV for each replicate dilution is reported. Clinical agreement is reported if response is observed below 50% at the MRD for all presumed positive samples tested. Following the initial screen, the samples are analyzed in a dilution series starting at the MRD, and determining the dilution titer result may be performed. Obtaining a dilution titer result is optional, and is not required for the assay validation. There should be 90% agreement for positive versus negative determination for samples compared to results obtained by the alternative serological method.

Intra-assay precision is determined as follows. The intra-operator and intra-assay precision is tested by preparing CPCH, CPCL, and untreated serum matrix pool for use as quality control (QC) samples. Twenty replicates of each QC sample are tested by one operator within a single assay run. An assay run may consist of sample plates due to sample capacity. All twenty replicates for each QC sample may be analyzed on the same assay plate, but this is not required.

The mean response, SD, and %CV are reported. The acceptance for each QC sample is based on the %CV, with an acceptance level of 20%. For the CPCH sample, acceptance criteria of 30% CV or within a reference range of Mean Response $\pm$ 3SD is accepted. This measure is chosen due to the high %CV expected in low response outputs. An error rate of up to 10% is accepted.

Inter-assay precision and inter-analyst precision are determined as follows. CPCH, CPCL and the untreated serum matrix pool is prepared and analyzed in duplicate for at least three assay runs by at least two analysts across multiple days.

The mean response, SD, and %CV is reported. The acceptance for each QC sample is based on the %CV, with an acceptance level of 20%. For the CPCH sample, acceptance criteria of 30% CV or within a reference range of Mean Response $\pm$ 3SD is accepted. This measure was chosen due to the high %CV expected in low response outputs. An error rate of up to 10% is accepted.

Inter-laboratory precision is determined as follows. CPCH, CPCL and untreated serum matrix pool is prepared at the testing facility. Sample levels is single-blinded and sent with reagents to complete the assay at an alternate test site. At least one assay run should be completed with the samples analyzed in duplicate. The alternate test site and responsible personnel for the alternate test site is identified in the final assay validation report.

Mean response, SD, and %CV are reported. Acceptance for each QC sample is based on the %CV, with an acceptance level of 520%. For the CPCH sample, acceptance criteria of 530% CV or within a reference range of Mean Response $\pm$ 3SD is accepted. This measure was chosen due to the high %CV expected in low response outputs. An error rate of up to 10% is accepted.

The robustness of stability of a first cell line is determined as follows. Analysis of ACE-2 expression on Vero-DSP1 and Vero-DSP2 cell lines is determined by flow cytometry. Based on the anticipated use of the cells, each cell line is tested at multiple intervals to determine consistent receptor expression throughout the anticipated use of the cells. Cells initially are tested within 48 hours after plating from frozen stocks at an initial passage after thawing. Repeat analyses take place at multiple passages out to a minimum of 6 passages or to a minimum time length of 3 weeks from thaw. The specific intervals tested and determined interval for acceptable stability is indicated in the final assay validation report. A single analysis run consisting of an anti-ACE-2 stained sample and an appropriate flow cytometry negative control sample is obtained at each interval tested.

The median fluorescence intensity (MFI) and Half-Peak %CV is reported for both the ACE-2 stained cells, and for the negative staining control. The staining index is calculated at each interval and reported in the final assay validation report. Acceptance criteria for the flow cytometry assessment is described as follows.

The median fluorescence intensity (MFI) and Half-Peak %CV is reported for both the ACE-2 stained cells, and for the negative staining control. The acceptance range is calculated from data collected at the first cell passage, within 48-hours of the cells being thawed. The acceptable MFI range is calculated as follows:

$$Range = MFI_{1st\ Passage} \pm \left( \frac{\%CV_{hp}}{100\%} \times MFI_{1st\ Passage} \right)$$

Where %CVhp is defined as the half-peak %CV from the flow cytometer instrument. All analyses conducted at other intervals should fall within MFI range established from this calculation for both the ACE-2 stained cells, and for the negative staining control.

**EP 4 136 457 B1**

The robustness of stability of a second cell line is determined as follows. Analysis of functional DSP on Vero-DSP1 and Vero-DSP2 cell lines is determined by level of luminescence generated in presence of CPCH, CPCL, and CM. An assay run is conducted on cells thawed from a cryovial aliquot, allowed to expand for two days, and then passaged for use in the assay. The time from thawing of cells to reading of the assay plate would be 4 total days. The assay run should include CPCH, CPCL and untreated matrix pool prepared and analyzed in duplicate. For each successive split of the cells, assay plates are prepared, and an assay run is completed including CPCH, CPCL and untreated matrix pool prepared and analyzed in duplicate. The specific number of passages and assay runs is not known, but cells are carried for a period of at least three weeks from when the cells initially thawed, and at each split, an assay plate is plated to conduct an assay run, setup on the next day, and read two days after plating.

Mean response, SD, and %CV are reported. Performance at each level is compared to the mean response obtained from the first assay run on the cells (read 4 days cell thaw). Percent Relative Error (%RE) from the first assay run on the cell is calculated. Acceptance criteria are defined as $\pm$30%RE obtained from the CPCL sample. The CPCL sample was chosen to evaluate stability when it is suspected the CPCL response falls within the linear range of the assay.

The robustness of viral stock stability is determined as follows. A virus stock tube is thawed for use in the assay, and then stored on wet ice or refrigerated conditions (2 to 8°C) for up to 24-hours. At least three assay runs are prepared from this single tube at multiple intervals. Each assay run includes CPCH, CPCL and untreated matrix pool plated in duplicate. The specific timed intervals tested includes an assay plate prepared within 1 hour of thawing ("fresh virus"), an assay plate prepared between 22 and 24 hours after thawing, and at least one intermediate time interval (i.e. within 12 hours of thawing).

Mean response, SD, and %CV are reported. Performance at each level is compared to the mean response obtained from the less than one hour interval. The percent relative error (%RE) from the less than one hour interval is calculated. Acceptance criteria are defined as $\pm$30%RE obtained from the CPCL sample. The CPCL sample was chosen to evaluate stability as it is suspected the CPCL response falls within the linear range of the assay.

The robustness of sample matrix stability is determined as follows. From the minimum three samples obtained above, analysis is conducted as quickly as possible following collection. Duration of time from collection to analysis is reported. During the analysis procedure, additional matrix aliquots is prepared to be used for stability assessment. Stability is conducted for samples stored in a freezer set to maintain a temperature -20°C, as well as the other conditions described.

Time/Temperature: Due to variability in sample volume, the specific number of aliquots able be prepared is unknown but is reported in the validation assay report. Minimum stability assessments are prepared at the following intervals as shown in Table 6, below.

**Table 6**

|  | Refrigerator | Freezer | | Benchtop |
|---|---|---|---|---|
| Time | 1-week | 1-week | 1-month | hours |
| Temperature | 2 to 8°C | 5-20°C | 5-20°C | 19 to 28°C |

A freeze-thaw (FT) cycle is defined as allowing frozen sample to thaw completely (>_15 minutes removal from freezer, and visual determination of sample appearance as completely liquid state), and then returning sample to freezer 6 hours prior to thawing again to conduct analysis. Three FT cycles are performed.

Samples are analyzed in duplicate at each of the described intervals listed above. The mean response, SD, and %CV are reported. Performance at each level is compared to the mean response obtained from the initial analysis result. Percent Relative Error (%RE) from the initial analysis is calculated. Acceptance criteria are defined as $\pm$30%RE.

### Statistical Analysis

A negative threshold determination is performed as follows. Biological outliers are identified using a Tukey Boxplot1. The first quartile, the third quartile, interquartile range or IQR (third quartile - first quartile), upper fence (third quartile + k*IQR), and lower fence (first quartile - k*IQR) are calculated. The constant k are defined as equal to 3. Biological outliers are excluded from further calculation of the negative threshold determination.

Non-excluded data from all runs are pooled. Variance in normality is analyzed using an appropriate statistical test such as the Shapiro-Wilk test or the Anderson-Darling test. See, John W. Tukey, Journal of Computational and Graphical Statistics, 1993, 2:1-33 and Shapiro, S.S. and Wilk, M.B., Biometrika, 1965, 52(3-4): 591-611. See, Anderson, T.W. and Darling, D.A., A Test of Goodness of Fit. Journal of the American Statistical Association, 1954, 49(268):765-769.

If the data distribution is normal, use a parametric approach with $\alpha$ = 0.05:

$$Negative\ Threshold\ Determination = mean\ normalized\ response + 1.646\ x\ SD$$

If the data distribution is non-normal, use a non-parametric approach:
*Negative Threshold Determination = 95th percentile of the data*

The flow cytometry cell analysis acceptance criteria is as follows. The median fluorescence intensity (MFI) and Half-Peak %CV is reported for both the ACE-2 stained cells, and for the negative staining control. The acceptance range is calculated from data collected at the first cell passage, within 48-hours of the cells being thawed. The acceptable MFI range is calculated as follows:

$$Range = MFI_{1st\ Passage} \pm \left( \frac{\%CV_{hp}}{100\%} \times MFI_{1st\ Passage} \right)$$

The %CVhp is defined as the half-peak %CV from the flow cytometer instrument. All analyses conducted at other intervals should fall within MFI range established from this calculation for both the ACE-2 stained cells, and for the negative staining control.

**Example 3: The use of IMMUNO-COV v. 1 assay to detect SRS-CoV-2-neturalizing antibodies in plasma**

The present Example demonstrates compatibility of plasma with the IMMUNO-COV v. 1 assay of the invention.

Figures 11A-C show luciferase activity in plasma and serum of three subjects at different dilutions. Plasma and serum from three COVID-19 convalescing individuals were heat inactivated for 30 min at 56°C. Serial dilutions were then prepared and incubated with VSV-SARS-CoV-2-S-Δ19CT for 30 minutes at room temperature before being overlaid onto Vero-DSP1/DPS2 monolayers. Luciferase activity was measured after approximately 24 hours. The figures show that virus neutralizing titers are similar between plasma and sera.

Pooled SARS-CoV-2 seronegative plasma (not heat-inactivated) containing stepped concentrations of anti-SARS-CoV-2 spike antibody mAb10914 was mixed with VSV-SARS-CoV2 v1.0 (VSV-SARS-CoV-2-S-Δ19CT). After a 30-minute incubation at room temperature, the plasma/virus mixes were overlaid onto Vero-DSP1/DSP2 monolayers. Luciferase activity was measured after approximately 24 hours. As shown in Figure 11D, the addition of stepped concentrations of anti-SARS-CoV-2 spike antibody mAb10914 to pooled SARS-CoV-2 seronegative plasma showed a dose-dependent capacity to block the VSV-SARS-CoV2 v.1.0 virus (VSV-SARS-CoV-2-S-Δ19CT) used in the assay.

In another experiment, dilutions of pooled SARS-CoV-2 seronegative plasma or plasma from a COVID-19 convalescing individual (NL1) were mixed with VSV-SARS-CoV2 v.1.0 virus (VSV-SARS-CoV-2-S-Δ19CT). After a 1-hour incubation at 37°C, the plasma/virus mixes were overlaid onto Vero-DSP1/DSP2 cell monolayers. Luciferase activity was measured approximately 24 hours later. As shown in Figure 11E, COVID-19 convalescing plasma was able to block the virus and luciferase activity in the assay. Both heat-inactivated (56°C, 30 min) and non-heat-inactivated plasma samples were tested and demonstrated assay compatibility, though non-heat-inactivated samples demonstrated higher background.

The effect of heat inactivation on plasma samples was also investigated. One aliquot was stored on ice while the other was incubated for 30 minutes at 56°C. Due to plasma thermal coagulation, samples were clarified by centrifugation before they were used in the assay. Each aliquot was then diluted in OptiMEM and mixed with VSV-SARS-CoV-2-S-Δ19CT to a final dilution of 1:80, 1:160, 1:320, 1:640, 1:1280, and 1:2560. Following a 30-minute incubation at room temperature, the virus/plasma mixes were overlaid onto Vero-DSP1/Vero-DSP2 monolayers. Luciferase activity was measured after approximately 24 hours. The results are shown in Figure 11F. It appears that heat inactivation has less of an effect on the neutralizing capacity of plasma samples as compared to serum samples. In some plasma samples, heat inactivation lead to reduced sensitivity for borderline samples.

**Example 4: The use of IMMUNO-COV v. 1 assay to detect SRS-CoV-2-neturalizing antibodies in saliva**

Compatibility of saliva with the IMMUNO-COV v. 1 assay was demonstrated by spiking pooled human saliva with anti-SARS-CoV-2 spike antibody mAb10914. Specifically, pooled saliva prepared from individuals prior to the COVID pandemic spiked with mAb10914 at stepped concentrations (QC-High, Mid, and Low). In order to prevent bacterial contamination associated with the use of saliva, all saliva samples were spiked with an Antibiotic/Antimycotic (Ab/Am) mix, diluted to a final dilution of 1:6.25 in OptiMEM, and filtered through a 0.22 μm filter pre-wet with OptiMEM containing Ab/Am mix. The final concentrations of the Ab/Am components in the saliva were as follows: 0.2 mg/mL Ampicillin, 0.1 mg/mL Gentamicin sulfate, 4 μg/mL Amphotericin B. Contrived positive control samples with various concentrations of mAb 10914 were prepared by adding the mAb 10914 to the saliva before the addition of Ab/Am or filtration, to ensure that the filtration did not remove inhibitory antibody from the saliva samples.

The filtered samples were further diluted serially in OptiMEM before being mixed with VSV-SARS-CoV2 v.1.0 virus (VSV-SARS-CoV-2-S-Δ19CT). After a 30-minute incubation at room temperature the saliva/virus mixes were overlaid onto Vero-DSP1/Vero-DSP2 monolayers. Luciferase activity was measured approximately 24 hours later. The concentration of mAb10914 in the samples after mix with virus at the 1:100 dilution shown were: 2 μg/mL (QC-High), 0.6 μg/mL (QC-Mid), and 0.4 μg/mL (QC-Low). At higher saliva concentrations sample interference was observed.

As demonstrated in Figure 12, the IMMUNO-COV v. 1 assay works well with saliva samples.

## Example 5: Development of IMMUNO-COV assay v. 2

As depicted in Figure 13A, version 2 (v. 2) of the IMMUNO-COV assay uses a recombinant VSV encoding the SARS-CoV-2 spike glycoprotein (e.g., VSV-SARS-CoV-2-S-Δ19CT or VSV-SARS-CoV-2-S) in place of the VSV-G glycoprotein in addition to a reporter gene (e.g., firefly luciferase (Luc2/Fluc) gene or *Gaussia* luciferase (gLuc) gene). Because the virus itself encodes the reporter, Vero-DSP cells are no longer used for the assay. The assay principle is as follows: the recombinant virus (VSV-SARS-CoV-2-S-Δ19CT-luc or VSV-SARS-CoV-2-S-luc) infects Vero cells, delivering a luciferase gene, which causes the cells to make luciferase protein. The presence of luciferase can be detected using a substrate such as, e.g., d-luciferin for Flue (available at Gold Biotechnology www.goldbio.com) or coelenterazine for gLuc (Cat. #3031, NanoLight Technology; nanolight.com/product/coelenterazine-in-vivo/). *Gaussia* luciferase is a secreted form of luciferase that uses coelenterazine as a substrate in an ATP independent reaction.

In IMMUNO-COV assay v. 2, serum or plasma samples are incubated with the virus (e.g., VSV-SARS-CoV-2-S-Δ19CT-luc or VSV-SARS-CoV-2-S-luc), and the mixtures are then combined with or overlaid onto Vero cells (or other susceptible cells). In the absence of SARS-CoV-2-neutralizing antibodies, the virus infects cells and reporter (e.g., luciferase) activity is detected. If the test samples contain SARS-CoV-2-neutralizing antibodies, virus infection is inhibited and reporter (e.g., luciferase) activity is reduced. Controls such as pooled SARS-CoV-2 seronegative serum/plasma or serum/plasma alone or serum/plasma mixed with stepped concentrations of anti-SARS-CoV-2 spike monoclonal antibody mAb10914 are used to quantitate the level of the neutralizing response. The IMMUNO-COV assay v. 2 workflow is shown in Figure 13B.

In the experiment shown in Figure 14A, VSV-SARS-CoV-2-S-Δ19CT-Fluc encoding a mutant Luc2 variant of firefly luciferase (GenBank Accession No. AY738222) was incubated with pooled negative sera (at 1:80 dilution), COVID-19 convalescing sera (at 1:80 dilution), or pooled negative sera (at 1:80 dilution) mixed with 10, 2, or 0.2 μg/mL mAb10914. After a 45-minute incubation at room temperature, the virus/sera mixes were overlaid onto Vero cell monolayers (an alternative incubation time can be 30 minutes). After an additional 18 hours (can be 24 hours or even longer for a stronger signal), d-luciferin substrate was added to wells and luciferase activity (RLUs) were measured using a luminometer. As shown in Figure 14A, VSV-SARS-CoV-2-S-Δ19CT-Fluc virus induces luciferase expression and activity in Vero cells that can be blocked by COVID-19 convalescing serum as well as mAb 10914 in a dose dependent manner.

In the experiment shown in Figure 14B, pooled SARS-CoV-2 seronegative sera (at 1:80 dilution) was incubated with media only (no virus) or VSV-SARS-CoV-2-S-Δ19CT-gLuc (1:48 dilution of virus stock) alone or spiked with 0.2 or 2 μg/mL mAb10914. After 30 minutes at room temperature, the virus/sera mixes were overlaid onto Vero-Ace-2 monolayers (see below for the generation of Vero-Ace-2 cells) seeded the day before in black, clear-bottomed 96-well plates at 1e4 cells/well. After an additional 24 hours, luciferase activity in the wells was measured. Coelenterazine (20 μL of a 5 μM stock) was added to each well using an injector in the TECAN (luminescence plate reader) that added the substrate and proceeded to immediately measure luminescence. The results shown in Figure 14B indicate that luciferase activity can be detected in Vero-Ace-2 cells that are infected (24 hour previously) with VSV-SARS-CoV-2-S-Δ19CT-gLuc in the presence of pooled SARS-CoV-2 seronegative sera. The luciferase activity can be reduced by the addition of mAb10914 to the pooled sera. While mAb10914 was able to inhibit luciferase activity, the level of inhibition was slightly lower than that observed when using VSV-SARS-CoV-2-S-Δ19CT-Fluc. However, the main advantage of VSV-SARS-CoV-2-S-Δ19CT-gLuc is that the gLuc gene is significantly smaller than the Flue gene, which may result in increased stability and manufacturability of the gLuc-containing virus.

Additional experiments were directed to optimizing IMMUNO-COV assay v. 2 conditions, including determining whether Vero-αHis, Vero-E6, or engineered Vero cells that overexpress Ace-2 and/or TRMPSS2 work best in the assay.

For generation of Vero-Ace-2 and Vero-TRMPSS2 cells, plasmids with Ace-2 (GenBank Accession No. BC039902) and/or TMPRSS2 (GenBank Accession No. BC051839) were purchased from ABM (Cat. # 1116701; abmgood.com/ACE2-ORF-Vector-1116701.html#Ace2 and Cat. # 471480110000; abmgood.com/TMPRSS2-ORF-Vector-4714801.html#471480110000). The Ace-2 and TMPRSS2 were subcloned into a lentiviral vector construct. Vero cells were then transduced with the lentiviral vector(s) and selected with appropriate selection antibiotic.

Figures 15A-B demonstrate that Vero-Ace-2 cells are particularly effective in IMMUNO-COV assay v. 2. In the experiment shown in Figure 15A, VSV-SARS-CoV-2-S-Δ19CT-Fluc or media alone (no virus control) was incubated with

**EP 4 136 457 B1**

pooled SARS-CoV-2 seronegative sera (at a dilution of 1:80) mixed with 2 or 0.2 $\mu$g/mL of mAb10914. After a 30-minute room temperature incubation, the virus/sera mixes were overlaid onto monolayers of cells (Vero-$\alpha$Hs, Vero-E6 or Vero-Ace-2). After an additional 26 hours, d-luciferin substrate was added to the wells and luciferase activity (RLUs) was measured using a luminometer. As shown in Figure 15A, both Vero-Ace-2 and Vero-$\alpha$His worked well in the assay, with Vero-Ace-2 generating a higher luciferase signal in the absence of SARS-CoV-2-neutralizing antibodies.

In the experiment shown in Figure 15B, pooled SARS-CoV-2 seronegative sera (at 1:80 dilution) was incubated with VSV-SARS-CoV-2-S-$\Delta$19CT-gLuc (4800 TCID50/well) alone or spiked with 0.2 ug/mL mAb10914. After 30 min at room temperature, the virus/sera mixes were overlaid onto Vero-$\alpha$His, Vero-Ace-2, or Vero-Ace-2/TMPRSS2 cell monolayers seeded the day before in black, clear-bottomed 96-well plates at 1e4 cells/well. After an additional 24 hours, luciferase activity in the wells was measured after the addition of d-luciferin. Although both Vero-Ace-2 and Vero-Ace2/TMPRSS2 cells gave similarly high levels of luciferase expression in the presence of pooled negative sera, luciferase signal and therefore virus was only inhibited by mAb10914 effectively in the Vero-Ace2 cell line.

As shown in Figures 16A-B, luciferase signal is substantially lower when cells are co-plated with virus (as compared to when cells are pre-plated), but significantly recovers when given a 4 hour recovery time. In Figure 16A, virus was incubated with pooled SARS-CoV-2 seronegative sera (1:80) for 30 minutes at room temperature then overlaid onto Vero cell monolayers or mixed with Vero cells at a similar density. At the indicated times after cell overlay, luciferase activity was measured. In Figure 16B, virus was incubated with pooled SARS-CoV-2 seronegative sera (1:80) for 30 minutes at room temperature then overlaid onto Vero cell monolayers plated either 4 or 24 hours prior. After an additional 24 hours luciferase activity was measured.

The experiments shown in Figures 17A-B tested the effects of cell density and virus TCID50 on luciferase activity. Virus was diluted in OptiMEM to the indicated TCID50 per well and overlaid onto Vero cell monolayers plated at the indicated cells/well the day before. After an additional 16, 20, and 24 hours luciferase activity was measured. Data in Figure 17A are from 24 hours. Figure 17A shows that luciferase activity increases with increased cell density. Figure 17B shows that luciferase activity increases with increased virus quantity/well.

As shown in Figure 18, IMMUNO-COV assay v. 2 is highly sensitive allowing to detect weak neutralizing antibody responses and shows dose-dependent inhibition of VSV-SARS-CoV-2-S-$\Delta$19CT-Fluc. Virus was incubated with pooled negative serum containing stepped concentrations of mAb10914 for 30 minutes at room temperature. The virus/serum mixes were then overlaid onto Vero cell monolayers. Luciferase activity was measured after an additional 24 hours.

**Table 7:** Comparison of percent responses in IMMUNO-COV Assay versions 1 and 2

| Concentration of mAb10914 | % Luciferase Signal | |
|---|---|---|
| | Version 1 | Version 2 (Flue) |
| 0.6 $\mu$g/mL | 32.5% | 1.2% |
| 0.4 $\mu$g/mL | 47.5% | 2.6% |

$$\text{Percent Response} = (\text{luciferase signal} \div \text{luciferase signal from negative control}) \times 100\%$$

Lower percent response indicates a greater reduction in luciferase and virus inhibition Values represent the average (mean) from 24 wells run on 6 different plates for each sample.

Figure 19 shows kinetic curve of Fluc activity in VSV-SARS-CoV-2-S-$\Delta$19CT-Fluc-infected Vero-Ace-2 cells following d-luciferin addition. Because Flue exhibits flash kinetics, the expected peak luminescence signal is expected to be 0.5 sec after the addition of d-luciferin to wells. This was confirmed to be true for VSV-SARS-CoV-2-S-$\Delta$19CT-Fluc-infected Vero-Ace-2 cells as shown in Figure 19. For this experiment, Vero-Ace-2 cell monolayers (seeded at 1e4 cells/well the day before infection) were infected with 4800 TCID50 units of VSV-SARS-CoV-2-S-$\Delta$19CT-Fluc. After 24 hours the plate was loaded in the TECAN instrument (luminescence plate reader) that was fitted with an injector. The injector was programmed to inject the desired quantity of substrate (in this experiment 20 $\mu$L of a 3.75 mg/mL solution of d-luciferin) into each well, and then after 0.5 sec read luminescence (1000 ms integration time). Additional luciferase reads were performed every 5 seconds for 3 min to generate a kinetic curve of luciferase activity (two reads done, d-luciferin added (final concentration 0.44 mg/mL in well) at 9 sec (arrow), additional reads done through 3 min). As expected, activity peaked immediately (0.5 sec) after the addition of d-luciferin.

47

**EP 4 136 457 B1**

**References**

Casadevall A, Pirofski LA. The convalescent sera option for containing COVID-19.
379 J Clin Invest 2020 Mar 13. pii: 138003. doi: 10.1172/JCI138003
Corman et al., 2016. Viral shedding and antibody response in 37 patients with Middle East respiratory syndrome coronavirus infection. Clin Infect Dis 62:477-483.
Gui, M et al. (2017) Cryo-electron microscopy structure of the SARS-CoV spike flycoprotein reveal a prerequisite conformational state for receptor binding. Cell Res. 27:119-129.
Ho M.S., Chen W.J., Chen H.Y. Neutralizing antibody response and SARS severity. Emerg Infect Dis. 2005;11(11):1730-1737.
Hoffmann, M., Kleine-Weber, H., Schroeder, S., Kruger, N., Herrler, T., Erichsen, S.,
Schiergens, T.S., Herrler, G., Wu, N.H., Nitsche, A., et al. (2020). SARS-CoV-2 Cell Entry Depends on ACE2 and TMPRSS2 and Is Blocked by a Clinically Proven Protease Inhibitor.
Cell.181: 271-280
Jaume M., Yip M.S., Cheung C.Y. Anti-severe acute respiratory syndrome coronavirus spike antibodies trigger infection of human immune cells via a pH- and cysteine protease-independent FcgammaR pathway. J Virol. 2011;85(20):10582-10597.
Koff WC, Burton DR, Johnson PR, et al. Accelerating next-generation vaccine development for global disease prevention. Science. 2013;340(6136).
Liu, W., Fontanet, A., Zhang, P.H., Zhan, L., Xin, Z.T., Baril, L., Tang, F., Lv, H., and Cao, W.C. (2006). Two-year prospective study of the humoral immune response of patients with severe acute respiratory syndrome. J. Infect. Dis. 193, 792-795.
Liu, L et al. (2019) Anti-spike IgG causes severe acute lung injury by skewing macrophage responses during acute ARS-CoV infection. JCI Insight. 4 (4): e123158.
Nie Y, Wang G, Shi X, Zhang H, Qiu Y, He Z, et al. Neutralizing antibodies in patients with severe acute respiratory syndrome-associated coronavirus infection. J Infect Dis. 2004; 190: 1119-26.
Peiris J.S., Chu C.M., Cheng V.C. Clinical progression and viral load in a community outbreak of coronavirus-associated SARS pneumonia: a prospective study. Lancet. 2003;361(9371):1767-1772.
Walls, A.C., Park, Y.J., Tortorici, M.A., Wall, A., McGuire, A.T., and Veesler, D. (2020).
Structure, Function, and Antigenicity of the SARS-CoV-2 Spike Glycoprotein. Cell.181:281-292.
Wan, Y., Shang, J., Graham, R., Baric, R.S., and Li, F. (2020). Receptor recognition by novel coronavirus from Wuhan: An analysis based on decade-long structural studies of SARS. Journal of Virology, JVI.00127-00120.
Wang S.F., Tseng S.P., Yen C.H. Antibody-dependent SARS coronavirus infection is mediated by antibodies against spike proteins. Biochem Biophys Res Commun. 2014;451(2):208-214.
Yip M.S., Leung H.L., Li P.H. Antibody-dependent enhancement of SARS coronavirus infection and its role in the pathogenesis of SARS. Hong Kong Med J. 2016;22(3 Suppl 4):25-31.
Yuan, Y. et al. (2017) Cryo-EM structure of MERS-CoV and SARS-CoV spike flyocpoteins reveal the dynamic receptor binding domains. Natu. Commun. 8:15092.
Zhao J et al Recovery from the Middle East respiratory syndrome is associated with antibody and T-cell responses (2017). Sci Immunol 2:5393.

**Example 6: Generation of Variants of SARS-CoV-2**

The SARS-CoV-2 spike glycoprotein mutants were human codon optimized and synthesized with a deletion in the nucleotides encoding the C-terminal 19 amino acids (S-Δ19CT). The variants of SARS-CoV-2 were cloned into a plasmid encoding the VSV genome using the restriction sites MluI and NheI. The plasmid was sequence verified and used for infectious virus rescue on BHK-21 cells. VSV-G was co-transfected into the BHK-21 cells to facilitate rescue but was not present in subsequent passages of the virus.

**Table 8: Non-Limiting Examples of Amino Acid Residue Positions for Insertion, Deletion, and/or Substitution to Generate Recombinant VSV Particle Variants**

| Mutations | Location | Phenotypes | References |
|---|---|---|---|
| **COVID VARIANT: B.1.1.7 lineage, (20I/501Y.V1 or VOC 202012/01)** <br> **Origin: UK** <br> **hCoV-19/England/SHEF-10C8326/2021** | | | |
| N501Y | RBD | One of the six key amino acids interacting with ACE-2 receptor. Associated with increased transmissibility (more efficient/rapid transmissibility). | Horby P, Huntley C, Davies N, Edmunds J, Ferguson N, Medley G, et al. NERVTAG paper on COVID-19 variant of concern B.1.1.7 (2021) [www.gov.uk/govemment/publications/nervtag-paper-on-covid-19-variant-ofconcem-b117] Accession number: SAMN17373206 |
| 69-70 deletion | | Potential conformational change in spike protein. Reduced sensitivity to neutralizing antibodies. Associated with increased transmissibility (more efficient/rapid transmissibility). | Wu K, Werner AP, Moliva JI, et al. mRNA-1273 vaccine induces neutralizing antibodies against spike mutants from global SARS-CoV-2 variants. [Preprint Posted January 25, 2021] GenBank: MW487270.1 |
| P681H | Near S1/S2 furin cleavage site | Associated with increased transmissibility (more efficient/rapid transmissibility). | Xie X, Zou J, Fontes-Garfias CR, et al. Neutralization of N501Y mutant SARS-CoV-2 by BNT162b2 vaccine-elicited sera. [Preprint Posted January 7, 2021] Greaney AJ, Loes AN, Crawford KHD, et al. Comprehensive mapping of mutations to the SARS-CoV-2 receptor-binding domain that affect recognition by polyclonal human serum antibodies. [Preprint Posted January 4, 2021] Severe acute respiratory syndrome coronavirus 2 isolate SARS-CoV-2/human/USA/NYI.B 1-7.01-21/2021, complete genome |
| Y144 del | | | Weisblum Y, Schmidt F, Zhang F, et al. Escape from neutralizing antibodies by SARS-CoV-2 spike protein variants [eLife 2020;9:e61312] |
| A570D | | | |
| T716I | | | |
| S982A | | | |
| D1118H | | | |
| **COVID VARIANT: B.1.351 (20H/501Y.V2)** <br> **Origin: South Africa** | | | |
| K417N | RBD | Resistant to neutralizing antibodies. | Weisblum Y, Schmidt F, Zhang F, et al. Escape from neutralizing antibodies by SARS-CoV-2 spike protein variants [eLife 2020;9:e61312] hCoV-19/Belgium/AZDelta05413-2105R/2021 |
| E484K | RBD | Resistant to neutralizing antibodies. E484K may affect neutralization by some polyclonal and mAb, potentially by disrupting the immunodominant B cell epitope, and is thought to be the mutation that drives immune escape. | Resende PC, Bezerra JF, de Vasconcelos RHT, at al. Spike E484K mutation in the first SARS-CoV-2 reinfection case confirmed in Brazil, 2020extemal icon. [Posted on www.virological.orgexternal icon on January 10, 2021] |

(continued)

| COVID VARIANT: B.1.351 (20H/501Y.V2) | | | |
|---|---|---|---|
| Origin: South Africa | | | |
| N501Y | RBD | Resistant to neutralizing antibodies, increased transmissibility. | |
| D614G | | | |
| A701V | | | |
| L18F | NTD | | |
| D80A | NTD | | |
| D215G | NTD | | |
| L242-244 del | NTD | | |
| R246I | NTD | Disrupts N5-loop (large, solvent exposed loop in NTD) and displaces the **loop** | |
| COVID VARIANT: P.1 lineage (B1.1.28.1 or 20J/501.V3, 484K.V2) | | | |
| Origin: Brazil | | | |
| K417T | RBD | altered transmissibility and antigenic profile, which may affect ability of Ab generated through previous natural infection or vaccination to recognize and neutralize virus | Resende PC, Bezerra JF, de Vasconcelos RHT, at al. Spike E484K mutation in the first SARS-CoV-2 reinfection case confirmed in Brazil, 2020extemal icon. [Posted on www.virological.orgexternal icon on January 10, 2021] hCoV-19/Brazil/RR-1087/2021 |
| E484K | RBD | | |
| N501Y | RBD | | |
| L18F | NTD | | |
| T20N | NTD | | |
| P26S | | | |
| D138Y | | | |
| R190S | | | |
| D614G | | | |
| H655Y | | | |
| T1027I | | | |
| COVID VARIANT: B.1.429 (CAL.20C, CA VUI) | | | |
| Origin: California | | | |
| S131 | | | |
| W152C | | | |
| L452R | | | |
| D614G | | | |
| COVID VARIANT: B.1.2 lineage 20C-US | | | |
| Q677H | Adjacent to furin cleavage site | | Adrian A. Pater et al., Emergence and Evolution of a Prevalent New SARS-CoV-2 Variant in the United States [doi.org/10.1101/2021.01.11.426287] |
| Other mutations in ORFs | | | |

(continued)

| COVID VARIANT: B1.1.17 | | | |
|---|---|---|---|
| | | | Weisblum Y, Schmidt F, Zhang F, et al. Escape from neutralizing antibodies by SARS-CoV-2 spike protein variants [eLife 2020;9:e61312] |
| **COVID VARIANT: 20E (EU1)** | | | |
| A22V | | | |
| D614G | | | |
| **COVID VARIANT: 20A.EU2** | | | |
| S477N | | | |
| D614G | | | |
| **COVID VARIANT: N439K-D614G** | | | |
| N439K | | | |
| D614G | | | |
| **COVID VARIANT: Mink Cluster 5 variant** | | | |
| H69 del | | | |
| V70 del | | | |
| Y453F | RBD | Increased binding affinity for mink Ace2. | |
| D614G | | | |
| I692V | | | |
| M1229I | | | |

**Table 9: Non-Limiting Examples of Recombinant VSV Particle Variants**

| Variant Name | Mutations |
|---|---|
| VSV-SARS-CoV2-S_E484K | E484K |
| VSV-SARS-CoV2-S B.1.351 NTD | L18F, D80A, D215G, 242-244 del, R246I, A701V |
| VSV-SARS-CoV2-S B.1.351 RBD | K417N, E484K, N501Y, D614G |
| VSV-SARS-CoV2-S_B.1.351 | K417N, E484K, N501Y, D614G, L18F, D80A, D215G, 242-244 del, R246I, A701V |
| VSV-SARS-CoV2-S_B.1.1.7_RBD | N501Y, 69-70 del, P681H |
| VSV-SARS-CoV2-S_B. 1.1.7 | N501Y, 69-70 del, P681H, Y144 del, A570D, T716I, S982A, D1118H |
| VSV-SARS-CoV2-S B.1.1.28 RBD | K417T, E484K, N501Y |

**Example 7: Sample Collection Kits for Dried Bloodspot (DBS) Sampling**

A non-limiting example of a standard Dried Bloodspot (DBS) collection procedure was performed as described below. Sample collection kits for DBS sampling contained all of the necessary components to perform the sample collection.

For the DBS collection procedure, the following steps were performed:

1. A bloodspot collection card was prepared for collection:

a. The bloodspot collection card contained Whatman 903™ filter paper, which was protected by a paper cover.
b. The cover was folded back and creased to keep the filter paper portion of the card exposed.
c. If self-performing the collection, the card was taped to the edge of a table with the exposed filter paper overhanging the table.

2. The sample collection site was wiped with an alcohol prep pad, and then the skin was allowed to dry for about 30 seconds.

    a. The Recommended collection site was on the finger pad of a preferred finger, near the fingertip.

3. A contact-activated lancet (BD Microtainer®, Ref#: 366594) was provided as the recommended lancet.

    a. Blue-color contact-activated lancet was considered "high flow" and comprised either a 14-guage needle or 1.5mm wide blade with a 2.0mM incision depth.
    b. There was a blue twist-off cover that was removed for the lancet to be activated.
    c. With the cover removed, the activation area of the lancet was firmly pushed against collection site, which resulted in a minor incision.

4. A gauze square (included in the kit) was used to wipe the first drop of blood away.
5. A droplet of blood on fingertip was then allowed to fall onto the filter paper, which was printed with 5 circles. A description of the DBS collection card comprising the 5 printed circles is provided in Figure 20.
6. When possible all 5 circles were completely filled with sample. The finger was not directly touched to the card, but if a large blood droplet was not falling on its own onto the card, the droplet was touched to the paper (but touching the paper with finger was avoided).
7. After circles on card were filled, pressure was applied with a gauze pad at the collection site to slow/stop bleeding, and then an included band aid apply was applied.
8. The collection card was left open and allowed to dry. Once dry, samples were then shipped to analysis laboratory.

    a. CDC recommendations for dried bloodspot collections are to allow samples to dry for 3 hours prior to shipment.
    b. Collection card may be stored at room temperature ($\leq$75°F, $\leq$24°C) or refrigerated condition once dry (34-46°F, 2-8°C). Avoid extreme heat conditions or storing in direct sunlight.
    c. Sample was shipped within 3 days of collection, and received within 2 weeks from collection date.

### Example 8: IMMUNO-COV V2 Bloodspot Assay Sample Analysis Protocol

The present example provides a IMMUNO-COV V2 Bloodspot Assay Setup and Bloodspot Assay Maps, which describe the Dried Bloodspot Assay Sample Analysis protocol. Specifically, the procedure describes assaying of 6 bloodspot samples using 6 dilutions beginning at a 1:4 and diluting serial 2-fold out to a 1:128 dilution. Exemplar Bloodspot Assay Maps showing various bloodspot sample dilutions are described for a U-well (or U-bottom) Plate (Figure 23) and an Assay Plate (Figure 24).

The following is a list of reagents was needed to perform the procedure:

VSV-SARS2-Fluc
OptiMEM
mAb10914
mAb10922
Pooled negative serum
D-luciferin

For the IMMUNO-COV V2 Bloodspot Assay Setup, the following steps were performed:
Cells were seeded according to IMMUNO-COV V2 Cell Preparation procedures of the present disclosure. Cells were seeded 16 to 24 hours before being used for assay.
1. Dried Bloodspot (DBS) sample processing was started approximately 1.5 hours before assay plating began.
2. For each DBS sample, a ¼ inch (6mm) hole punch was used to punch a single punch/spot into an appropriately labeled 1.7mL microcentrifuge tube. Bloodspot collection cards were viewed from the back when punching to ensure the punchout location was being taken from a location where capillary whole blood had saturated through the entire area of the filter paper being sampled.
3. Once all sample punches were added to tubes, 400$\mu$L OptiMEM was added to each sample tube. For DBS sampling clarification, extractions were conducted using 200$\mu$L, the assay recommended minimum volume to use per each 6mm spot. Because 400$\mu$L was used in the present extraction, this was considered a 1:2 dilution, which after mixing with the virus resulted in a 1:4 starting dilution. This provided adequate volume to transfer 240$\mu$L of eluent to a U-bottom plate at later steps.

4. The tubes were incubated at room temperature for 1 hour on a plate shaker, shaking at 450rpm setting.

5. Following 1 hour incubation, a micropipette was used to transfer supernatant to a new tube. The filter paper punch was pushed to the side with the pipette tip, and the extracted whole blood eluent was easily collected.

6. A brief centrifugation step (1000 × g for approximately 30 seconds) was performed before sampling bloodspot eluent to reduce the frequency of small filter paper debris from transferring to the U-bottom plate during assay setup.

7. 120$\mu$L OptiMEM was placed in wells of U-bottom suspension 96-well culture plates indicated by grey shading according to plate layout maps, as shown in Figure 23.

8. A working stock of mAb10914 antibody was prepared at 0.$\mu$g/$\mu$L by diluting 10$\mu$L mAb10914 stock in 98$\mu$L OptiMEM. The working stock was mixed by pipetting.

9. ST1 (standard) was prepare at 1.6/$\mu$g/mL mAb10914 (0.8$\mu$g/mL after virus addition) as follows:

For 2 plates: 9.6$\mu$L working stock mAb10914 was combined with 590.4$\mu$L OptiMEM.

10. Working stock of mAb 10922 antibody was prepared at 0.01 $\mu$g/$\mu$L by diluting 5$\mu$L mAb 10922 stock in 245$\mu$L OptiMEM. The working stock was mixed by pipetting.

11. Assay controls (NC, negative control; PC, positive control) were prepared as follows, mixed by pipetting:

| Mix | Pooled negative serum | Working stock mAb10922 | OptiMEM |
|-----|-----------------------|------------------------|---------|
| NC | 8$\mu$L | None | 312$\mu$L |
| PC | 12.5$\mu$L | 3.1$\mu$L | 484.4$\mu$L |

12. 120 $\mu$L of controls were added to U-bottom plates according to the plate layout map (Figure 23).

13. 240 $\mu$L of ST1 was added to wells labeled ST1 in the plate layout map (Figure 23).

14. 240 $\mu$L of bloodspot sample eluent was added to sample wells A1, B1, C1 and A7, B7, C7 according to the plate layout map (Figure 23).

15. Six-point 2-fold serial dilutions were performed as follows:

120 $\mu$L were transferred from Column 1 wells to Column 2 wells and mixed by pipetting.
120 $\mu$L were transferred from Column 2 wells to Column 3 wells and mixed by pipetting.
120 $\mu$L were transferred from Column 3 wells to Column 4 wells and mixed by pipetting.
120 $\mu$L were transferred from Column 4 wells to Column 5 wells and mixed by pipetting.
120 $\mu$L were transferred from Column 5 wells to Column 6 wells and mixed by pipetting.
120 $\mu$L from Column 6 wells was discarded.

The entire process was repeated for Samples S4, S5, S6, on right side of the plate (Column 7 through 12; NC, POS, or Media controls were not diluted).

16. The virus was thawed. Once completely thawed, the virus was stored on ice.

17. The virus was diluted to 6000 pfu/mL. Actual volume of OptiMEM needed for dilution can be calculated based on virus lot and titer using the following table:

| Virus Lot | Virus Titer | Vol. Virus ($8.4 \times 10^4 \div$ titer) | Vol. OptiMEM (14 - vol. virus) |
|-----------|-------------|-------------------------------------------|--------------------------------|
| | | mL | mL |

18. 120$\mu$L of prepared virus was added to all wells of U-well plates and mixed by pipetting.

19. Plates were incubated at room temperature for 30 to 45 min.

20. 100 $\mu$L of samples were transferred from U-bottom plates to a plate of cells in duplicate according to Assay Plate layout maps (Figure 24). Cells were placed in 37°C/5% $CO_2$ incubator.

21. Between 24 and 28 hours after the sample was overlayed onto the cells, the luciferase activity was read as follows:

a. 15 mg/mL d-luciferin was diluted 1: 10 (to 1.5 mg/mL) in DPBS. As a non-limiting example, 1 mL of 15 mg/mL d-luciferin was combined with 9 mL DPBS. Mixing was performed by inversion.

It was acceptable to prepare a larger volume of diluted (1.5mg/mL) d-luciferin if reading luciferase from multiple plates/assays at the same time.

b. A 12-channel electronic or manual pipette was used to add 50$\mu$L of 1.5mg/mL d-luciferin to each well of assay plates.

D-luciferin was added to rows in order (*i.e.,* A, B, C, D, E, F, G, H).
The timing of d-luciferin addition to rows was 4 to 8 seconds between each row.

c. One at a time, plates were placed in TECAN, and read using an appropriate program.

Initiation of the plate read occurred within 1 min of the addition of d-luciferin to the last row of the cell plate.

The assay was reviewed and reviewer details and date were recorded.

As a non-limiting example, extracting of the blood spots was performed with either OptiMEM or PBST. For OptiMEM conditions, the OptiMEM started at 1:2 dilutions in the assay (neat elution, 1:2 when diluted with virus) and PBST started at a 1:10 dilution (1:10 after virus addition). Figure 21 shows a graphical summary of exemplar percent relative luciferase response data generated by extracting bloodspots using OptiMEM across various sample dilutions in accordance with above-described methods.

A proof-of-concept experiment demonstrated that the bloodspot matrix was completely compatible at a 1:20 dilution of matrix (Figures 22A-B). No major issues were seen regarding either cell health or virus infectivity in the bloodspot samples that were tested.

**Sequences**

Amino Acid Sequence of SARS-CoV-2 full-length S glycoprotein (SEQ ID NO: 1; variant 1 and variant 4; NCBI Reference Sequence: YP_009724390.1; S1 subunit is underlined; RBD site is shown in bold)

```
MFVFLVLLPLVSSQCVNLTTRTQLPPAYTNSFTRGVYYPDKVFRSSVLHSTQDLFLPFFSNV
TWFHAIHVSGTNGTKRFDNPVLPFNDGVYFASTEKSNIIRGWIFGTTLDSKTQSLLIVNNAT
NVVIKVCEFQFCNDPFLGVYYHKNNKSWMESEFRVYSSANNCTFEYVSQPFLMDLEGKQGNF
KNLREFVFKNIDGYFKIYSKHTPINLVRDLPQGFSALEPLVDLPIGINITRFQTLLALHRSY
LTPGDSSSGWTAGAAAYYVGYLQPRTFLLKYNENGTITDAVDCALDPLSETKCTLKSFTVEK
GIYQTSNFRVQPTESIVRFPNITNLCPFGEVFNATRFASVYAWNRKRISNCVADYSVLYNSA
SFSTFKCYGVSPTKLNDLCFTNVYADSFVIRGDEVRQIAPGQTGKIADYNYKLPDDFTGCVI
AWNSNNLDSKVGGNYNYLYRLFRKSNLKPFERDISTEIYQAGSTPCNGVEGFNCYFPLQSYG
FQPTNGVGYQPYRVVVLSFELLHAPATVCGPKKSTNLVKNKCVNFNFNGLTGTGVLTESNKK
FLPFQQFGRDIADTTDAVRDPQTLEILDITPCSFGGVSVITPGTNTSNQVAVLYQDVNCTEV
PVAIHADQLTPTWRVYSTGSNVFQTRAGCLIGAEHVNNSYECDIPIGAGICASYQTQTNSPR
RARSVASQSIIAYTMSLGAENSVAYSNNSIAIPTNFTISVTTEILPVSMTKTSVDCTMYICG
DSTECSNLLLQYGSFCTQLNRALTGIAVEQDKNTQEVFAQVKQIYKTPPIKDFGGFNFSQIL
PDPSKPSKRSFIEDLLFNKVTLADAGFIKQYGDCLGDIAARDLICAQKFNGLTVLPPLLTDE
MIAQYTSALLAGTITSGWTFGAGAALQIPFAMQMAYRFNGIGVTQNVLYENQKLIANQFNSA
IGKIQDSLSSTASALGKLQDVVNQNAQALNTLVKQLSSNFGAISSVLNDILSRLDKVEAEVQ
IDRLITGRLQSLQTYVTQQLIRAAEIRASANLAATKMSECVLGQSKRVDFCGKGYHLMSFPQ
SAPHGVVFLHVTYVPAQEKNFTTAPAICHDGKAHFPREGVFVSNGTHWFVTQRNFYEPQIIT
TDNTFVSGNCDVVIGIVNNTVYDPLQPELDSFKEELDKYFKNHTSPDVDLGDISGINASVVN
IQKEIDRLNEVAKNLNESLIDLQELGKYEQYIKWPWYIWLGFIAGLIAIVMVTIMLCCMTSC
CSCLKGCCSCGSCCKFDEDDSEPVLKGVKLHYT
```

Codon Optimized Nucleotide Sequence of SARS-CoV-2 full-length S glycoprotein (SEQ ID NO: 2; variant 1 and variant 4)

```
atgttcgtcttcctggtcctgctgcctctggtctcctcacagtgcgtcaatctgacaactcg
gactcagctgccacctgcttatactaatagcttcaccagaggcgtgtactatcctgacaagg
tgtttagaagctccgtgctgcactctacacaggatctgtttctgccattctttagcaacgtg
acctggttccacgccatccacgtgagcggcaccaatggcacaaagcggttcgacaatcccgt
gctgccttttaacgatggcgtgtacttcgcctctaccgagaagagcaacatcatcagaggct
ggatctttggcaccacactggactccaagacacagtctctgctgatcgtgaacaatgccacc
aacgtggtcatcaaggtgtgcgagttccagttttgtaatgatcccttcctgggcgtgtacta
tcacaagaacaataagagctggatggagtccgagtttagagtgtattctagcgccaacaact
gcacatttgagtacgtgagccagcctttcctgatggacctggagggcaagcagggcaatttc
aagaacctgagggagttcgtgtttaagaatatcgacggctacttcaaaatctactctaagca
cacccccatcaacctggtgcgcgacctgcctcagggcttcagcgccctggagcccctggtgg
atctgcctatcggcatcaacatcacccggtttcagacactgctggccctgcacagaagctac
ctgacacccggcgactcctagcggatggaccgccggcgctgccgcctactatgtgggcta
```

```
cctccagccccggaccttcctgctgaagtacaacgagaatggcaccatcacagacgcagtgg
attgcgccctggaccccctgagcgagacaaagtgtacactgaagtcctttaccgtggagaag
ggcatctatcagacatccaatttcagggtgcagccaaccgagtctatcgtgcgctttcctaa
tatcacaaacctgtgcccatttggcgaggtgttcaacgcaacccgcttcgccagcgtgtacg
cctggaataggaagcggatcagcaactgcgtggccgactatagcgtgctgtacaactccgcc
tctttcagcacctttaagtgctatggcgtgtccccacaaagctgaatgacctgtgctttac
caacgtctacgccgattctttcgtgatcaggggcgacgaggtgcgccagatcgcccccggcc
agacaggcaagatcgcagactacaattataagctgccagacgatttcaccggctgcgtgatc
gcctggaacagcaacaatctggattccaaagtgggcggcaactacaattatctgtaccggct
gtttagaaagagcaatctgaagcccttcgagagggacatctctacagaaatctaccaggccg
gcagcacccccttgcaatggcgtggagggctttaactgttatttcccactccagtcctacggc
ttccagcccacaaacggcgtgggctatcagccttaccgcgtggtggtgctgagctttgagct
gctgcacgccccagcaacagtgtgcggccccaagaagtccaccaatctggtgaagaacaagt
gcgtgaacttcaacttcaacggcctgaccggcacaggcgtgctgaccgagtccaacaagaag
ttcctgccatttcagcagttcggcagggacatcgcagataccacagacgccgtgcgcgaccc
acagaccctggagatcctggacatcacaccctgctctttcggcggcgtgagcgtgatcacac
ccggcaccaatacaagcaaccaggtggccgtgctgtatcaggacgtgaattgtaccgaggtg
cccgtggctatccacgccgatcagctgaccccaacatggcgggtgtacagcaccggctccaa
cgtcttccagacaagagccggatgcctgatcggagcagagcacgtgaacaattcctatgagt
gcgacatcccaatcggcgccggcatctgtgcctcttaccagacccagacaaactctcccaga
agagcccggagcgtggcctcccagtctatcatcgcctataccatgtccctgggcgccgagaa
cagcgtggcctactctaacaatagcatcgccatcccaaccaacttcacaatctctgtgacca
cagagatcctgcccgtgtccatgaccaagacatctgtggactgcacaatgtatatctgtggc
gattctaccgagtgcagcaacctgctgctccagtacggcagcttttgtacccagctgaatag
agccctgacaggcatcgccgtggagcaggataagaacacacaggaggtgttcgcccaggtga
agcaaatctacaagaccccccctatcaaggactttggcggcttcaatttttcccagatcctg
cctgatccatccaagccttctaagcggagctttatcgaggacctgctgttcaacaaggtgac
cctggccgatgccggcttcatcaagcagtatggcgattgcctgggcgacatcgcagccaggg
acctgatctgcgcccagaagtttaatggcctgaccgtgctgccacccctgctgacagatgag
atgatcgcacagtacacaagcgccctgctggccggcaccatcacatccggatggaccttcgg
cgcaggagccgcccttccagatccccttgccatgcagatggcctataggttcaacggcatcg
gcgtgacccagaatgtgctgtacgagaaccagaagctgatcgccaatcagtttaactccgcc
atcggcaagatccaggacagcctgtcctctacagccagcgccctgggcaagctccaggatgt
ggtgaatcagaacgcccaggccctgaataccctggtgaagcagctgagcagcaacttcggcg
ccatctctagcgtgctgaatgacatcctgagccggctggacaaggtggaggcagaggtgcag
atcgaccggctgatcaccggccggctccagagcctccagacctatgtgacacagcagctgat
cagggccgccgagatcagggccagcgccaatctggcagcaaccaagatgtccgagtgcgtgc
tgggccagtctaagagagtggacttttgtggcaagggctatcacctgatgtccttccctcag
tctgccccacacggcgtggtgtttctgcacgtgacctacgtgcccgcccaggagaagaactt
caccacagcccctgccatctgccacgatggcaaggcccactttccaagggagggcgtgttcg
tgtccaacggcacccactggtttgtgacacagcgcaatttctacgagccccagatcatcacc
acagacaacaccttcgtgagcggcaactgtgacgtggtcatcggcatcgtgaacaataccgt
gtatgatccactccagcccgagctggacagctttaaggaggagctggataagtatttcaaga
atcacacctcccctgacgtggatctgggcgacatcagcggcatcaatgcctccgtggtgaac
atccagaaggagatcgaccgcctgaacgaggtggctaagaatctgaacgagagcctgatcga
cctccaggagctgggcaagtatgagcagtacatcaagtggccctggtacatctggctgggct
tcatcgccggcctgatcgccatcgtgatggtgaccatcatgctgtgctgtatgacatcctgc
tgttcttgcctgaagggctgctgtagctgtggctcctgctgtaagtttgacgaggatgactc
tgaacctgtgctgaagggcgtgaagctgcattacacctaa
```

Amino Acid Sequence of SARS-CoV-2 Δ19CT S glycoprotein (SEQ ID NO: 3; variant 2; S1 subunit is underlined; RBD site is shown in bold)

```
MFVFLVLLPLVSSQCVNLTTRTQLPPAYTNSFTRGVYYPDKVFRSSVLHSTQDLFLPFFSNV
TWFHAIHVSGTNGTKRFDNPVLPFNDGVYFASTEKSNIIRGWIFGTTLDSKTQSLLIVNNAT
NVVIKVCEFQFCNDPFLGVYYHKNNKSWMESEFRVYSSANNCTFEYVSQPFLMDLEGKQGNF
KNLREFVFKNIDGYFKIYSKHTPINLVRDLPQGFSALEPLVDLPIGINITRFQTLLALHRSY
LTPGDSSSGWTAGAAAYYVGYLQPRTFLLKYNENGTITDAVDCALDPLSETKCTLKSFTVEK
GIYQTSNFRVQPTESIVRFPNITNLCPFGEVFNATRFASVYAWNRKRISNCVADYSVLYNSA
SFSTFKCYGVSPTKLNDLCFTNVYADSFVIRGDEVRQIAPGQTGKIADYNYKLPDDFTGCVI
AWNSNNLDSKVGGNYNYLYRLFRKSNLKPFERDISTEIYQAGSTPCNGVEGFNCYFPLQSYG
FQPTNGVGYQPYRVVVLSFELLHAPATVCGPKKSTNLVKNKCVNFNFNGLTGTGVLTESNKK
FLPFQQFGRDIADTTDAVRDPQTLEILDITPCSFGGVSVITPGTNTSNQVAVLYQDVNCTEV
PVAIHADQLTPTWRVYSTGSNVFQTRAGCLIGAEHVNNSYECDIPIGAGICASYQTQTNSPR
RARSVASQSIIAYTMSLGAENSVAYSNNSIAIPTNFTISVTTEILPVSMTKTSVDCTMYICG
DSTECSNLLLQYGSFCTQLNRALTGIAVEQDKNTQEVFAQVKQIYKTPPIKDFGGFNFSQIL
PDPSKPSKRSFIEDLLFNKVTLADAGFIKQYGDCLGDIAARDLICAQKFNGLTVLPPLLTDE
MIAQYTSALLAGTITSGWTFGAGAALQIPFAMQMAYRFNGIGVTQNVLYENQKLIANQFNSA
IGKIQDSLSSTASALGKLQDVVNQNAQALNTLVKQLSSNFGAISSVLNDILSRLDKVEAEVQ
IDRLITGRLQSLQTYVTQQLIRAAEIRASANLAATKMSECVLGQSKRVDFCGKGYHLMSFPQ
SAPHGVVFLHVTYVPAQEKNFTTAPAICHDGKAHFPREGVFVSNGTHWFVTQRNFYEPQIIT
TDNTFVSGNCDVVIGIVNNTVYDPLQPELDSFKEELDKYFKNHTSPDVLGDISGINASVVN
IQKEIDRLNEVAKNLNESLIDLQELGKYEQYIKWPWYIWLGFIAGLIAIVMVTIMLCCMTSC
CSCLKGCCSCGSCC
```

Codon Optimized Nucleotide Sequence of SARS-CoV-2 Δ19CT S glycoprotein (SEQ ID NO: 4; variant 2)

```
atgttcgtcttcctggtcctgctgcctctggtctcctcacagtgcgtcaatctgacaactcg
gactcagctgccacctgcttatactaatagcttcaccagaggcgtgtactatcctgacaagg
tgtttagaagctccgtgctgcactctacacaggatctgtttctgccattctttagcaacgtg
acctggttccacgccatccacgtgagcggcaccaatggcacaaagcggttcgacaatcccgt
gctgccttttaacgatggcgtgtacttcgcctctaccgagaagagcaacatcatcagaggct
ggatctttggcaccacactggactccaagacacagtctctgctgatcgtgaacaatgccacc
aacgtggtcatcaaggtgtgcgagttccagttttgtaatgatcccttcctgggcgtgtacta
tcacaagaacaataagagctggatggagtccgagtttagagtgtattctagcgccaacaact
gcacatttgagtacgtgagccagcctttcctgatggacctggagggcaagcagggcaatttc
aagaacctgagggagttcgtgtttaagaatatcgacggctacttcaaaatctactctaagca
cacccccatcaacctggtgcgcgacctgcctcagggcttcagcgccctggagcccctggtgg
atctgcctatcggcatcaacatcacccggtttcagacactgctggccctgcacagaagctac
ctgacacccggcgactcctctagcggatggaccgccggcgctgccgcctactatgtgggcta
cctccagccccggaccttcctgctgaagtacaacgagaatggcaccatcacagacgcagtgg
attgcgccctggaccccctgagcgagacaaagtgtacactgaagtcctttaccgtggagaag
ggcatctatcagacatccaatttcagggtgcagccaaccgagtctatcgtgcgctttcctaa
tatcacaaacctgtgcccatttggcgaggtgttcaacgcaacccgcttcgccagcgtgtacg
cctggaataggaagcggatcagcaactgcgtggccgactatagcgtgctgtacaactccgcc
tctttcagcacctttaagtgctatggcgtgtcccccacaaagctgaatgacctgtgctttac
caacgtctacgccgattctttcgtgatcaggggcgacgaggtgcgccagatcgcccccggcc
agacaggcaagatcgcagactacaattataagctgccagacgatttcaccggctgcgtgatc
gcctggaacagcaacaatctggattccaaagtgggcggcaactacaattatctgtaccggct
gtttagaaagagcaatctgaagcccttcgagagggacatctctacagaaatctaccaggccg
```

gcagcacccccttgcaatggcgtggagggctttaactgttatttcccactccagtcctacggc
ttccagcccacaaacggcgtgggctatcagccttaccgcgtggtggtgctgagctttgagct
gctgcacgccccagcaacagtgtgcggccccaagaagtccaccaatctggtgaagaacaagt
gcgtgaacttcaacttcaacggcctgaccggcacaggcgtgctgaccgagtccaacaagaag
ttcctgccatttcagcagttcggcagggacatcgcagataccacagacgccgtgcgcgaccc
acagaccctggagatcctggacatcacaccctgctctttcggcggcgtgagcgtgatcacac
ccggcaccaatacaagcaaccaggtggccgtgctgtatcaggacgtgaattgtaccgaggtg
cccgtggctatccacgccgatcagctgaccccaacatggcgggtgtacagcaccggctccaa
cgtcttccagacaagagccggatgcctgatcggagcagagcacgtgaacaattcctatgagt
gcgacatcccaatcggcgccggcatctgtgcctcttaccagacccagacaaactctcccaga
agagcccggagcgtggcctcccagtctatcatcgcctataccatgtccctgggcgccgagaa
cagcgtggcctactctaacaatagcatcgccatcccaaccaacttcacaatctctgtgacca
cagagatcctgcccgtgtccatgaccaagacatctgtggactgcacaatgtatatctgtggc
gattctaccgagtgcagcaacctgctgctccagtacggcagcttttgtacccagctgaatag
agccctgacaggcatcgccgtggagcaggataagaacacacaggaggtgttcgcccaggtga
agcaaatctacaagacccccccctatcaaggactttggcggcttcaattttttcccagatcctg
cctgatccatccaagccttctaagcggagctttatcgaggacctgctgttcaacaaggtgac
cctggccgatgccggcttcatcaagcagtatggcgattgcctgggcgacatcgcagccaggg
acctgatctgcgcccagaagtttaatggcctgaccgtgctgccacccctgctgacagatgag
atgatcgcacagtacacaagcgccctgctggccggcaccatcacatccggatggaccttcgg
cgcaggagccgccctccagatccccttgccatgcagatggcctataggttcaacggcatcg
gcgtgacccagaatgtgctgtacgagaaccagaagctgatcgccaatcagtttaactccgcc
atcggcaagatccaggacagcctgtcctctacagccagcgccctgggcaagctccaggatgt
ggtgaatcagaacgcccaggccctgaatatacctggtgaagcagctgagcagcaacttcggcg
ccatctctagcgtgctgaatgacatcctgagccggctggacaaggtggaggcagaggtgcag
atcgaccggctgatcaccggccggctccagagcctccagacctatgtgacacagcagctgat
cagggccgccgagatcagggccagcgccaatctggcagcaaccaagatgtccgagtgcgtgc
tgggccagtctaagagagtggactttgtggcaaggctatcacctgatgtccttccctcag
tctgccccacacggcgtggtgtttctgcacgtgacctacgtgcccgcccaggagaagaactt
caccacagcccctgccatctgccacgatggcaaggcccactttccaagggagggcgtgttcg
tgtccaacggcacccactggtttgtgacacagcgcaatttctacgagccccagatcatcacc
acagacaacaccttcgtgagcggcaactgtgacgtggtcatcggcatcgtgaacaataccgt
gtatgatccactccagcccgagctggacagctttaaggaggagctggataagtatttcaaga
atcacacctcccctgacgtggatctgggcgacatcagcggcatcaatgcctccgtggtgaac
atccagaaggagatcgaccgcctgaacgaggtggctaagaatctgaacgagagcctgatcga
cctccaggagctgggcaagtatgagcagtacatcaagtggccctggtacatctggctgggct
tcatcgccggcctgatcgccatcgtgatggtgaccatcatgctgtgctgtatgacatcctgc
tgttcttgcctgaagggctgctgtagctgtggctcctgctgttaa

Amino Acid Sequence of SARS-CoV-2 VSV G CT glycoprotein (SEQ ID NO: 5; variant 3; the sequence of VSV G cytoplasmic tail is shown in bold, underline)

MFVFLVLLPLVSSQCVNLTTRTQLPPAYTNSFTRGVYYPDKVFRSSVLHSTQDLFLPFFS
NVTWFHAIHVSGTNGTKRFDNPVLPFNDGVYFASTEKSNIIRGWIFGTTLDSKTQSLLIV
NNATNVVIKVCEFQFCNDPFLGVYYHKNNKSWMESEFRVYSSANNCTFEYVSQPFLMDLE
GKQGNFKNLREFVFKNIDGYFKIYSKHTPINLVRDLPQGFSALEPLVDLPIGINITRFQT
LLALHRSYLTPGDSSSGWTAGAAAYYVGYLQPRTFLLKYNENGTITDAVDCALDPLSETK
CTLKSFTVEKGIYQTSNFRVQPTESIVRFPNITNLCPFGEVFNATRFASVYAWNRKRISN
CVADYSVLYNSASFSTFKCYGVSPTKLNDLCFTNVYADSFVIRGDEVRQIAPGQTGKIAD
YNYKLPDDFTGCVIAWNSNNLDSKVGGNYNYLYRLFRKSNLKPFERDISTEIYQAGSTPC

```
NGVEGFNCYFPLQSYGFQPTNGVGYQPYRVVVLSFELLHAPATVCGPKKSTNLVKNKCVN
FNFNGLTGTGVLTESNKKFLPFQQFGRDIADTTDAVRDPQTLEILDITPCSFGGVSVITP
GTNTSNQVAVLYQDVNCTEVPVAIHADQLTPTWRVYSTGSNVFQTRAGCLIGAEHVNNSY
ECDIPIGAGICASYQTQTNSPRRARSVASQSIIAYTMSLGAENSVAYSNNSIAIPTNFTI
SVTTEILPVSMTKTSVDCTMYICGDSTECSNLLLQYGSFCTQLNRALTGIAVEQDKNTQE
VFAQVKQIYKTPPIKDFGGFNFSQILPDPSKPSKRSFIEDLLFNKVTLADAGFIKQYGDC
LGDIAARDLICAQKFNGLTVLPPLLTDEMIAQYTSALLAGTITSGWTFGAGAALQIPFAM
QMAYRFNGIGVTQNVLYENQKLIANQFNSAIGKIQDSLSSTASALGKLQDVVNQNAQALN
TLVKQLSSNFGAISSVLNDILSRLDKVEAEVQIDRLITGRLQSLQTYVTQQLIRAAEIRA
SANLAATKMSECVLGQSKRVDFCGKGYHLMSFPQSAPHGVVFLHVTYVPAQEKNFTTAPA
ICHDGKAHFPREGVFVSNGTHWFVTQRNFYEPQIITTDNTFVSGNCDVVIGIVNNTVYDP
LQPELDSFKEELDKYFKNHTSPDVDLGDISGINASVVNIQKEIDRLNEVAKNLNESLIDL
QELGKYEQYIKWPWYIWLGFIAGLIAIVMVTIMLCCM**KLKHTKKRQIYTDIEMNRLGK**
```

Codon Optimized Nucleotide Sequence of SARS-CoV-2 VSV G CT glycoprotein (SEQ ID NO: 6; variant 3; the sequence encoding VSV G cytoplasmic tail is shown in CAPs)

```
atgttcgtcttcctggtcctgctgcctctggtctcctcacagtgcgtcaatctgacaactcg
gactcagctgccacctgcttatactaatagcttcaccagaggcgtgtactatcctgacaagg
tgtttagaagctccgtgctgcactctacacaggatctgtttctgccattctttagcaacgtg
acctggttccacgccatccacgtgagcggcaccaatggcacaaagcggttcgacaatcccgt
gctgccttttaacgatggcgtgtacttcgcctctaccgagaagagcaacatcatcagaggct
ggatctttggcaccacactggactccaagacacagtctctgctgatcgtgaacaatgccacc
aacgtggtcatcaaggtgtgcgagttccagttttgtaatgatcccttcctgggcgtgtacta
tcacaagaacaataagagctggatggagtccgagtttagagtgtattctagcgccaacaact
gcacatttgagtacgtgagccagcctttcctgatggacctggagggcaagcagggcaatttc
aagaacctgagggagttcgtgtttaagaatatcgacggctacttcaaaatctactctaagca
cacccccatcaacctggtgcgcgacctgcctcagggcttcagcgccctggagcccctggtgg
atctgcctatcggcatcaacatcacccggtttcagacactgctggccctgcacagaagctac
ctgacacccggcgactcctctagcggatggaccgccggcgctgccgcctactatgtgggcta
cctccagccccggaccttcctgctgaagtacaacgagaatggcaccatcacagacgcagtgg
attgcgccctggaccccctgagcgagacaaagtgtacactgaagtcctttaccgtggagaag
ggcatctatcagacatccaatttcagggtgcagccaaccgagtctatcgtgcgctttcctaa
tatcacaaacctgtgcccatttggcgaggtgttcaacgcaacccgcttcgccagcgtgtacg
cctggaataggaagcggatcagcaactgcgtggccgactatagcgtgctgtacaactccgcc
tctttcagcacctttaagtgctatggcgtgtccccccacaaagctgaatgacctgtgctttac
caacgtctacgccgattctttcgtgatcaggggcgacgaggtgcgccagatcgcccccggcc
agacaggcaagatcgcagactacaattataagctgccagacgatttcaccggctgcgtgatc
gcctggaacagcaacaatctggattccaaagtgggcggcaactacaattatctgtaccggct
gtttagaaagagcaatctgaagcccttcgagagggacatctctacagaaatctaccaggccg
gcagcacccccttgcaatggcgtggagggctttaactgttatttcccactccagtcctacggc
ttccagcccacaaacggcgtgggctatcagccttaccgcgtggtggtgctgagctttgagct
gctgcacgccccagcaacagtgtgcggccccaagaagtccaccaatctggtgaagaacaagt
gcgtgaacttcaacttcaacggcctgaccggcacaggcgtgctgaccgagtccaacaagaag
ttcctgccatttcagcagttcggcagggacatcgcagataccacagacgccgtgcgcgaccc
acagaccctggagatcctggacatcacaccctgctctttcggcggcgtgagcgtgatcacac
ccggcaccaatacaagcaaccaggtggccgtgctgtatcaggacgtgaattgtaccgaggtg
cccgtggctatccacgccgatcagctgaccccaacatggcgggtgtacagcaccggctccaa
cgtcttccagacaagagccggatgcctgatcggagcagagcacgtgaacaattcctatgagt
gcgacatcccaatcggcgccggcatctgtgcctcttaccagacccagacaaactctcccaga
agagcccggagcgtggcctcccagtctatcatcgcctaccatgtccctgggcgccgagaa
```

```
cagcgtggcctactctaacaatagcatcgccatcccaaccaacttcacaatctctgtgacca
cagagatcctgcccgtgtccatgaccaagacatctgtggactgcacaatgtatatctgtggc
gattctaccgagtgcagcaacctgctgctccagtacggcagcttttgtacccagctgaatag
agccctgacaggcatcgccgtggagcaggataagaacacacaggaggtgttcgcccaggtga
agcaaatctacaagacccccctatcaaggactttggcggcttcatttttcccagatcctg
cctgatccatccaagccttctaagcggagctttatcgaggacctgctgttcaacaaggtgac
cctggccgatgccggcttcatcaagcagtatggcgattgcctgggcgacatcgcagccaggg
acctgatctgcgcccagaagtttaatggcctgaccgtgctgccacccctgctgacagatgag
atgatcgcacagtacacaagcgccctgctggccggcaccatcacatccggatggaccttcgg
cgcaggagccgccctccagatcccctttgccatgcagatggcctataggttcaacggcatcg
gcgtgacccagaatgtgctgtacgagaaccagaagctgatcgccaatcagtttaactccgcc
atcggcaagatccaggacagcctgtcctctacagccagcgccctgggcaagctccaggatgt
ggtgaatcagaacgcccaggccctgaatacctggtgaagcagctgagcagcaacttcggcg
ccatctctagcgtgctgaatgacatcctgagccggctggacaaggtggaggcagaggtgcag
atcgaccggctgatcaccggccggctccagagcctccagacctatgtgacacagcagctgat
cagggccgccgagatcagggccagcgccatctggcagcaaccaagatgtccgagtgcgtgc
tgggccagtctaagagagtggacttttgtggcaaggctatcacctgatgtccttccctcag
tctgccccacacggcgtggtgtttctgcacgtgacctacgtgcccgcccaggagaagaactt
caccacagcccctgccatctgccacgatggcaaggcccactttccaagggagggcgtgttcg
tgtccaacggcacccactggtttgtgacacagcgcaatttctacgagccccagatcatcacc
acagacaacaccttcgtgagcggcaactgtgacgtggtcatcggcatcgtgaacaataccgt
gtatgatccactccagcccgagctggacagctttaaggaggagctggataagtatttcaaga
atcacacctcccctgacgtggatctgggcgacatcagcggcatcaatgcctccgtggtgaac
atccagaaggagatcgaccgcctgaacgaggtggctaagaatctgaacgagagcctgatcga
cctccaggagctgggcaagtatgagcagtacatcaagtggccctggtacatctggctgggct
tcatcgccggcctgatcgccatcgtgatggtgaccatcatgctgtgctgtatgAAATTAAAG
CACACCAAGAAAAGACAGATTTATACAGACATAGAGATGAACCGACTTGGAAAGTAA
```

Amino Acid Sequence of Mutant VSV Matrix (M) Protein (M51R) (SEQ ID NO: 7)

```
MSSLKKILGLKGKGKKSKKLGIAPPPYEEDTSMEYAPSAPIDKSYFGVDERDTHDPNQLRYE
KSFFTVKMTVRSNRPFRTYSDVAAAVSHWDHMYIGMAGKRPFYKILAFLGSSNLKATPAVLA
DQGQPEYHAHCEGRAYLPHRMGKTPPMLNVPEHFRRPFNIGLYKGTIELTMTIYDDESLEAA
PMIWDHFNSSKFSDFREKALMFGLIVEEEASGAWVLDSVRHSKWASLASSF
```

Nucleotide Sequence of Mutant VSV Matrix (M) Protein (M51R) (SEQ ID NO: 8)

```
ATGAGTTCCTTAAAGAAGATTCTCGGTCTGAAGGGGAAAGGTAAGAAATCTAAGAAATTAGG
GATCGCACCACCCCCTTATGAAGAGGACACTAGCATGGAGTATGCTCCGAGCGCTCCAATTG
ACAAATCCTATTTTGGAGTTGACGAGCGAGACACCTATGATCCGAATCAATTAAGATATGAG
AAATTCTTCTTTACAGTGAAAATGACGGTTAGATCTAATCGTCCGTTCAGAACATACTCAGA
TGTGGCAGCCGCTGTATCCATTGGGATCACATGTACATCGGAATGGCAGGGAAACGTCCCT
TCTACAAAATCTTGGCTTTTTTGGGTTCTTCTAATCTAAAGGCCACTCCAGCGGTATTGGCA
GATCAAGGTCAACCAGAGTATCACGCTCACTGCGAAGGCAGGGCTTATTTGCCACATAGGAT
GGGGAAGACCCCTCCCATGCTCAATGTACCAGAGCACTTCAGAAGACCATTCAATATAGGTC
TTTACAAGGGAACGATTGAGCTCACAATGACCATCTACGATGATGAGTCACTGGAAGCAGCT
CCTATGATCTGGGATCATTTCAATTCTTCCAAATTTTCTGATTTCAGAGAGAAGGCCTTAAT
GTTTGGCCTGATTGTCGAGAAAAAGGCATCTGGAGCGTGGGTCCTGGACTCTATCGGCCACT
TCAAATGA
```

Amino Acid Sequence of the Wild-Type VSV Matrix (M) Protein (SEQ ID NO: 9)

MSSLKKILGLKGKGKKSKKLGIAPPPYEEDTSMEYAPSAPIDKSYFGVDEMDTHDPNQLRYE
KSFFTVKMTVRSNRPFRTYSDVAAAVSHWDHMYIGMAGKRPFYKILAFLGSSNLKATPAVLA
DQGQPEYHAHCEGRAYLPHRMGKTPPMLNVPEHFRRPFNIGLYKGTIELTMTIYDDESLEAA
PMIWDHFNSSKFSDFREKALMFGLIVEEEASGAWVLDSVRHSKWASLASSF

Nucleotide Sequence of the Wild-Type VSV Matrix (M) Protein (SEQ ID NO: 10)

ATGAGTTCCTTAAAGAAGATTCTCGGTCTGAAGGGGAAAGGTAAGAAATCTAAGAAATTAGG
GATCGCACCACCCCCTTATGAAGAGGACACTAGCATGGAGTATGCTCCGAGCGCTCCAATTG
ACAAATCCTATTTTGGAGTTGACGAGATGGACACCTATGATCCGAATCAATTAAGATATGAG
AAATTCTTCTTTACAGTGAAAATGACGGTTAGATCTAATCGTCCGTTCAGAACATACTCAGA
TGTGGCAGCCGCTGTATCCCATTGGGATCACATGTACATCGGAATGGCAGGGAAACGTCCCT
TCTACAAATCTTGGCTTTTTTGGGTTCTTCTAATCTAAAGGCCACTCCAGCGGTATTGGCA
GATCAAGGTCAACCAGAGTATCACGCTCACTGCGAAGGCAGGGCTTATTTGCCACATAGGAT
GGGGAAGACCCCTCCCATGCTCAATGTACCAGAGCACTTCAGAAGACCATTCAATATAGGTC
TTTACAAGGGAACGATTGAGCTCACAATGACCATCTACGATGATGAGTCACTGGAAGCAGCT
CCTATGATCTGGGATCATTTCAATTCTTCCAAATTTTCTGATTTCAGAGAGAAGGCCTTAAT
GTTTGGCCTGATTGTCGAGAAAAAGGCATCTGGAGCGTGGGTCCTGGACTCTATCGGCCACT
TCAAATGA

wild-type VSV Kozak sequence (SEQ ID NO: 11)
CACTATG
optimized Kozak sequence (SEQ ID NO: 12)
CACCATG
Amino Acid Sequence of SARS-CoV-1 Spike (S) protein (SEQ ID NO: 13;
UniProtKB/Swiss-Prot: P59594.1)

MFIFLLFLTLTSGSDLDRCTTFDDVQAPNYTQHTSSMRGVYYPDEIFRSDTLYLTQDLFLPF
YSNVTGFHTINHTFGNPVIPFKDGIYFAATEKSNVVRGWVFGSTMNNKSQSVIIINNSTNVV
IRACNFELCDNPFFAVSKPMGTQTHTMIFDNAFNCTFEYISDAFSLDVSEKSGNFKHLREFV
FKNKDGFLYVYKGYQPIDVVRDLPSGFNTLKPIFKLPLGINITNFRAILTAFSPAQDIWGTS
AAAYFVGYLKPTTFMLKYDENGTITDAVDCSQNPLAELKCSVKSFEIDKGIYQTSNFRVVPS
GDVVRFPNITNLCPFGEVFNATKFPSVYAWERKKISNCVADYSVLYNSTFFSTFKCYGVSAT
KLNDLCFSNVYADSFVVKGDDVRQIAPGQTGVIADYNYKLPDDFMGCVLAWNTRNIDATSTG
NYNYKYRYLRHGKLRPFERDISNVPFSPDGKPCTPPALNCYWPLNDYGFYTTTGIGYQPYRV
VVLSFELLNAPATVCGPKLSTDLIKNQCVNFNFNGLTGTGVLTPSSKRFQPFQQFGRDVSDF
TDSVRDPKTSEILDISPCSFGGVSVITPGTNASSEVAVLYQDVNCTDVSTAIHADQLTPAWR
IYSTGNNVNFSISITTEVMPVSMAKTSVDCNMYICGDSTECANLLLQYGSFCTQLNRALSGI
AAEQDRNTREVFAQVKQMYKTPTLKYFGGFNFSQILPDPLKPTKRSFIEDLLFNKVTLADAG
FMKQYGECLGDINARDLICAQKFNGLTVLPPLLTDDMIAAYTAALVSGTATAGWTFGAGAAL
QIPFAMQMAYRFNGIGVTQNVLYENQKQIANQFNKAISQIQESLTTTSTALGKLQDVVNQNA
QALNTLVKQLSSNFGAISSVLNDILSRLDKVEAEVQIDRLITGRLQSLQTYVTQQLIRAAEI
RASANLAATKMSECVLGQSKRVDFCGKGYHLMSFPQAAPHGVVFLHVTYVPSQERNFTTAPA
ICHEGKAYFPREGVFVFNGTSWFITQRNFFSPQIITTDNTFVSGNCDVVIGIINNTVYDPLQ
PELDSFKEELDKYFKNHTSPDVDLGDISGINASVVNIQKEIDRLNEVAKNLNESLIDLQELG
KYEQYIKWPWYVWLGFIAGLIAIVMVTILLCCMTSCCSCLKGACSCGSCCKFDEDDSEPVLK
GVKLHYT

| SARS-CoV-1 subunits/domains | Residues SARS-CoV-1 (SEQ ID NO: 13) | Residues SARS-CoV-2 (SEQ ID NO: 1) | % identity |
|---|---|---|---|
| Full protein | 1-1255 | | 75.9 |

(continued)

| SARS-CoV-1 subunits/domains | Residues SARS-CoV-1 (SEQ ID NO: 13) | Residues SARS-CoV-2 (SEQ ID NO: 1) | % identity |
|---|---|---|---|
| Signal peptide | 1-13 | | 53.9 |
| Extracellular | 14-1195 | | |
| Transmembrane | 1196-1216 | | |
| Cytoplasmic | 1217-1255 | | 97.4 |
| S1 | 14-667 | 14-684 | 63.6 |
| S2 | 668-1255 | | 90 |
| S2' | 798-1255 | | 93 |
| Cleavage site | 667-668 | | 100 |
| Cleavage site | 797-798 | | 100 |
| Receptor-binding domain (RBD) | 306-527 | 319-541 | 73.1 |
| Fusion peptide | 770-788 | | 83.3 |

CLUSTAL 0(1.2.4) multiple sequence alignment of spike (S) glycoprotein sequences from SARS-CoV-1 (SEQ ID NO: 13) and SARS-CoV-2 (SEQ ID NO: 1)

```
SARS1    MFIFLLFLTLTSGSDLDRCTTFDDVQAPNYTQHTSSMRGVYYPDEIFRSDTLYLTQDLFL 60
SARS2    MFVFLVLLPLVSSQCVNLTT--RTQLPPAY--TNSFTRGVYYPDKVFRSSVLHSTQDLFL 56
         **:**::* *.*.. :: *       * *   .* *******::***..*: ******
SARS1    PFYSNVTGFHTIN-------HTFGNPVIPFKDGIYFAATEKSNVVRGWVFGSTMNNKSQS 113
SARS2    PFFSNVTWFHAIHVSGTNGTKRFDNPVLPFNDGVYFASTEKSNIIRGWIFGTTLDSKTQS 116
         **:**** **:*:        : *.***:**:**:***:*****::***:**:*::.*:**
SARS1    VIIINNSTNVVIRACNFELCDNPFFAVSKPMGT----QTHTMIFDNAFNCTFEYISDAFS 169
SARS2    LLIVNNATNVVIKVCEFQFCNDPFLGVYYHKNNKSWMESEFRVYSSANNCTFEYVSQPFL 176
         ::*:**:*****:.*:*::*::**:.*     ..    ::.  ::..* ******:*: *
SARS1    LDVSEKSGNFKHLREFVFKNKDGFLYVYKGYQPIDVVRDLPSGFNTLKPIFKLPLGINIT 229
SARS2    MDLEGKQGNFKNLREFVFKNIDGYFKIYSKHTPINLVRDLPQGFSALEPLVDLPIGINIT 236
         :*:. *.****:*******  **:: :*. : **::****.**.:*:*:...**:*****
SARS1    NFRAILTAFS------PAQDIWGTSAAAYFVGYLKPTTFMLKYDENGTITDAVDCSQNPL 283
SARS2    RFQTLLALHRSYLTPGDSSSGWTAGAAAYYVGYLQPRTFLLKYNENGTITDAVDCALDPL 296
         .*:::*: .        :.. * :.****:****:* **:***:*********** :**
SARS1    AELKCSVKSFEIDKGIYQTSNFRVVPSGDVVRFPNITNLCPFGEVFNATKFPSVYAWERK 343
SARS2    SETKCTLKSFTVEKGIYQTSNFRVQPTESIVRFPNITNLCPFGEVFNATRFASVYAWNRK 356
         :* **::*** ::.************ *:. :********************:* *****:**
SARS1    KISNCVADYSVLYNSTFFSTFKCYGVSATKLNDLCFSNVYADSFVVKGDDVRQIAPGQTG 403
SARS2    RISNCVADYSVLYNSASFSTFKCYGVSPTKLNDLCFTNVYADSFVIRGDEVRQIAPGQTG 416
         :************** :* ********* ******** :******:**:*:**********
SARS1    VIADYNYKLPDDFMGCVLAWNTRNIDATSTGNYNYKYRYLRHGKLRPFERDISNVPFSPD 463
SARS2    KIADYNYKLPDDFTGCVIAWNSNNLDSKVGGNYNYLYRLFRKSNLKPFERDISTEIYQAG 476
          *********** ***:***:.*:*:.  ***** *** **:.*:********.  :. .
SARS1    GKPCTP-PALNCYWPLNDYGFYTTTGIGYQPYRVVVLSFELLNAPATVCGPKLSTDLIKN 522
SARS2    STPCNGVEGFNCYFPLQSYGFQPTNGVGYQPYRVVVLSFELLHAPATVCGPKKSTNLVKN 536
         ..**.    .:***:**:*** * *:*****************:********* **:*:**
SARS1    QCVNFNFNGLTGTGVLTPSSKRFQPFQQFGRDVSDFTDSVRDPKTSEILDISPCSFGGVS 582
SARS2    KCVNFNFNGLTGTGVLTESNKKFLPFQQFGRDIADTTDAVRDPQTLEILDITPCSFGGVS 596
         :************** *.*:* *********:* **:**** **:*******:********
SARS1    VITPGTNASSEVAVLYQDVNCTDVSTAIHADQLTPAWRIYSTGNNV-------------- 628
SARS2    VITPGTNTSNQVAVLYQDVNCTEVPVAIHADQLTPTWRVYSTGSNVFQTRAGCLIGAEHV 656
```

```
                  *******:*.:***********:* .********:**:****.**
SARS1   ------------------------------------------------------------ 628
SARS2   NNSYECDIPIGAGICASYQTQTNSPRRARSVASQSIIAYTMSLGAENSVAYSNNSIAIPT 716

SARS1   NFSISITTEVMPVSMAKTSVDCNMYICGDSTECANLLLQYGSFCTQLNRALSGIAAEQDR 688
SARS2   NFTISVTTEILPVSMTKTSVDCTMYICGDSTECSNLLLQYGSFCTQLNRALTGIAVEQDK 776
                  **:**:***.:****.****** .**********:****************:***.***:
SARS1   NTREVFAQVKQMYKTPTLKYFGGFNFSQILPDPLKPTKRSFIEDLLFNKVTLADAGFMKQ 748
SARS2   NTQEVFAQVKQIYKTPPIKDFGGFNFSQILPDPSKPSKRSFIEDLLFNKVTLADAGFIKQ 836
                  **:********:**** .:* .************ **:***************** *:**
SARS1   YGECLGDINARDLICAQFNGLTVLPPLLTDDMIAAYTAALVSGTATAGWTFGAGAALQI 808
SARS2   YGDCLGDIAARDLICAQFNGLTVLPPLLTDEMIAQYTSALLAGTITSGWTFGAGAALQI 896
                  **:***** *******************:*** **:**.:** *:***********
SARS1   PFAMQMAYRFNGIGVTQNVLYENQKQIANQFNKAISQIQESLTTTSTALGKLQDVVNQNA 868
SARS2   PFAMQMAYRFNGIGVTQNVLYENQKLIANQFNSAIGKIQDSLSSTASALGKLQDVVNQNA 956
                  ************************ ******.**.:**:**::*::************
SARS1   QALNTLVKQLSSNFGAISSVLNDILSRLDKVEAEVQIDRLITGRLQSLQTYVTQQLIRAA 928
SARS2   QALNTLVKQLSSNFGAISSVLNDILSRLDKVEAEVQIDRLITGRLQSLQTYVTQQLIRAA 1016
                  ************************************************************
SARS1   EIRASANLAATKMSECVLGQSKRVDFCGKGYHLMSFPQAAPHGVVFLHVTYVPSQERNFT 988
SARS2   EIRASANLAATKMSECVLGQSKRVDFCGKGYHLMSFPQSAPHGVVFLHVTYVPAQEKNFT 1076
                  **************************************:***************:**:***
SARS1   TAPAICHEGKAYFPREGVFVFNGTSWFITQRNFFSPQIITTDNTFVSGNCDVVIGIINNT 1048
SARS2   TAPAICHDGKAHFPREGVFVSNGTHWFVTQRNFYEPQIITTDNTFVSGNCDVVIGIVNNT 1136
                  *******:***:******** *** **:*****:.****************:***
SARS1   VYDPLQPELDSFKEELDKYFKNHTSPDVDLGDISGINASVVNIQKEIDRLNEVAKNLNES 1108
SARS2   VYDPLQPELDSFKEELDKYFKNHTSPDVDLGDISGINASVVNIQKEIDRLNEVAKNLNES 1196
                  ************************************************************
SARS1   LIDLQELGKYEQYIKWPWYVWLGFIAGLIAIVMVTILLCCMTSCCSCLKGACSCGSCCKF 1168
SARS2   LIDLQELGKYEQYIKWPWYIWLGFIAGLIAIVMVTIMLCCMTSCCSCLKGCCSCGSCCKF 1256
                  *******************:****************:*************.********
SARS1   DEDDSEPVLKGVKLHYT 1185
SARS2   DEDDSEPVLKGVKLHYT 1273
                  *****************
```

<u>Rluc8 155-156DSP1-7 luciferase-GFP fusion protein (SEQ ID NO: 14; Renilla luciferase fragment aa 1-155 is underlined;
linker is not highlighted; fragment aa 1-156 of engineered GFP is shown in bold)</u>

<u>MASKVYDPEQRKRMITGPQWWARCKQMNVLDSFINYYDSEKHAENAVIFLHGNATSSYLWRH
VVPHIEPVARCIIPDLIGMGKSGKSGNGSYRLLDHYKYLTAWFELLNLPKKIIFVGHDWGAA
LAFHYAYEHQDRIKAIVHMESVVDVIESWDESG</u>GGG**MSKGEELFTGVVPILVELDGDVNGHK
FSVRGEGEGDATIGKLTLKFICTTGKLPVPWPTLVTTLTYGVQCFSRYPDHMKRHDFFKSAM
PEGYVQERTISFKDDGKYKTRAVVKFEGDTLVNRIELKGTDFKEDGNILGHKLEYNFNSHNV
YITADK**

<u>Nucleotide Sequence of Rluc8155-156DSP1-7 construct (SEQ ID NO: 15; coding ORF nt 1068-2018)</u>

```
agatcttcaatattggccattagccatattattcattggttatatagcataaatcaatat
tggctattggccattgcatacgttgtatctatatcataatatgtacatttatattggctc
atgtccaatatgaccgccatgttggcattgattattgactagttattaatagtaatcaat
tacggggtcattagttcatagcccatatggagttccgcgttacataacttacggtaaa
tggcccgcctggctgaccgcccaacgaccccgcccattgacgtcaataatgacgtatgt
tcccatagtaacgccaatagggactttccattgacgtcaatgggtggagtatttacggta
aactgcccacttggcagtacatcaagtgtatcatatgccaagtccgccccctattgacgt
caatgacggtaaatggcccgcctggcattatgcccagtacatgaccttacgggactttcc
tacttggcagtacatctacgtattagtcatcgctattaccatggtgatgcggttttggca
gtacaccaatgggcgtggatagcggtttgactcacggggatttccaagtctcacccccat
tgacgtcaatgggagtttgttttggcaccaaaatcaacgggactttccaaaatgtcgtaa
taaccccgccccgttgacgcaaatgggcggtaggcgtgtacggtgggaggtctatataag
cagagctcgtttagtgaaccgtcagatcactagaagctttattgcggtagtttatcacag
ttaaattgctaacgcagtcagtgcttctgacacaacagtctcgaacttaagctgcagaag
ttggtcgtgaggcactgggcaggtaagtatcaaggttacaagacaggtttaaggagacca
atagaaactgggcttgtcgagacagagaagactcttgcgtttctgataggcacctattgg
tcttactgacatccactttgcctttctctccacaggtgtccactcccagttcaattacag
ctcttaaggctagagtacttaatacgactcactataggctagccaccatggcttccaagg
tgtacgaccccgagcaacgcaaacgcatgatcactgggcctcagtggtgggctcgctgca
agcaaatgaacgtgctggactccttcatcaactactatgattccgagaagcacgccgaga
acgccgtgatttttctgcatggtaacgctacctccagctacctgtggaggcacgtcgtgc
ctcacatcgagcccgtggctagatgcatcatccctgatctgatcggaatgggtaagtccg
gcaagagcgggaatggctcatatcgcctcctggatcactacaagtacctcaccgcttggt
tcgagctgctgaaccttccaaagaaaatcatctttgtgggccacgactggggggctgctc
tggcctttcactacgcctacgagcaccaagacaggatcaaggccatcgtccatatggaga
gtgtcgtggacgtgatcgagtcctgggacgagtccggaggaggcggaatgagcaagggcg
aggagctgttcaccggcgtggtgcccatcctggtggagctggacggcgacgtgaacggcc
acaagttcagcgtgagaggcgagggcgagggcgacgccaccatcggcaagctgaccctga
agttcatctgcaccaccggcaagctgcccgtgccctggcccaccctggtgaccaccctga
cctacggcgtgcagtgcttcagcagataccccgaccacatgaagagacacgacttcttca
agagcgccatgcccgagggctacgtgcaggagagaaccatcagcttcaaggacgacggca
agtacaagaccagagccgtggtgaagttcgagggcgacaccctggtgaacagaatcgagc
tgaagggcaccgacttcaaggaggacggcaacatcctgggccacaagctggagtacaact
tcaacagccacaacgtgtacatcaccgccgacaagtgatctagagcggccgcttcgagca
gacatgataagatacattgatgagtttggacaaaccacaactagaatgcagtgaaaaaaa
tgctttatttgtgaaatttgtgatgctattgctttatttgtaaccattataagctgcaat
aaacaagttaacaacaacaattgcattcattttatgtttcaggttcaggggggaggtgtgg
gaggttttttaaagcaagtaaaacctctacaaatgtggtaaaatcgataaggatccaggt
ggcacttttcggggaaatgtgcgcggaacccctatttgtttattttctaaatacattca
aatatgtatccgctcatgagacaataaccctgataaatgcttcaataatattgaaaaagg
aagagtatgagtattcaacatttccgtgtcgcccttattcccttttttgcggcatttttgc
cttcctgttttgctcacccagaaacgctggtgaaagtaaaagatgctgaagatcagttg
ggtgcacgagtgggttacatcgaactggatctcaacagcggtaagatccttgagagtttt
cgccccgaagaacgttttccaatgatgagcacttttaaagttctgctatgtggcgcggta
ttatcccgtattgacgccgggcaagagcaactcggtcgccgcatacactattctcagaat
gacttggttgagtactcaccagtcacagaaaagcatcttacggatggcatgacagtaaga
gaattatgcagtgctgccataaccatgagtgataacactgcggccaacttacttctgaca
acgatcggaggaccgaaggagctaaccgcttttttgcacaacatgggggatcatgtaact
cgccttgatcgttgggaaccggagctgaatgaagccataccaaacgacgagcgtgacacc
acgatgcctgtagcaatggcaacaacgttgcgcaaactattaactggcgaactacttact
ctagcttcccggcaacaattaatagactggatggaggcggataaagttgcaggaccactt
```

```
ctgcgctcggcccttccggctggctggtttattgctgataaatctggagccggtgagcgt
gggtctcgcggtatcattgcagcactggggccagatggtaagccctcccgtatcgtagtt
atctacacgacggggagtcaggcaactatggatgaacgaaatagacagatcgctgagata
ggtgcctcactgattaagcattggtaactgtcagaccaagtttactcatatatactttag
attgatttaaaacttcattttttaatttaaaaggatctaggtgaagatcctttttgataat
ctcatgaccaaaatcccttaacgtgagttttcgttccactgagcgtcagaccccgtagaa
aagatcaaaggatcttcttgagatcctttttttctgcgcgtaatctgctgcttgcaaaca
aaaaaaccaccgctaccagcggtggtttgtttgccggatcaagagctaccaactcttttt
ccgaaggtaactggcttcagcagagcgcagataccaaatactgttcttctagtgtagccg
tagttaggccaccacttcaagaactctgtagcaccgcctacatacctcgctctgctaatc
ctgttaccagtggctgctgccagtggcgataagtcgtgtcttaccgggttggactcaaga
cgatagttaccggataaggcgcagcggtcgggctgaacggggggttcgtgcacacagccc
agcttggagcgaacgacctacaccgaactgagatacctacagcgtgagctatgagaaagc
gccacgcttcccgaagggagaaaggcggacaggtatccggtaagcggcagggtcggaaca
ggagagcgcacgagggagcttccaggggggaaacgcctggtatctttatagtcctgtcggg
tttcgccacctctgacttgagcgtcgatttttgtgatgctcgtcaggggggcggagccta
tggaaaaacgccagcaacgcggcctttttacggttcctggccttttgctggccttttgct
cacatggctcgac
```

Rluc8 155-156DSP8-11 luciferase-GFP fusion protein (SEQ ID NO: 16; Renilla luciferase fragment aa 156-311 is underlined; linker is not highlighted, fragment aa 157-231 of engineered GFP is shown in bold)

**MQKNGIKANFTVRHNVEDGSVQLADHYQQNTPIGDGPVLLPDNHYLSTQTVLSKDPNEKRDH MVLHEYVNAAGIT**GGGGS<u>WPDIEEDIALIKSEEGEKMVLENNFFVETVLPSKIMRKLEPEEF AAYLEPFKEKGEVRRPTLSWPREIPLVKGGKPDVVQIVRNYNAYLRASDDLPKLFIESDPGF FSNAIVEGAKKFPNTEFVKVKGLHFLQEDAPDEMGKYIKSFVERVLKNEQ</u>

Nucleotide Sequence of Rluc8155-156DSP8-11 construct (SEQ ID NO: 17; coding ORF nt 1068-1778)

```
agatcttcaatattggccattagccatattattcattggttatatagcataaatcaatat
tggctattggccattgcatacgttgtatctatatcataatatgtacatttatattggctc
atgtccaatatgaccgccatgttggcattgattattgactagttattaatagtaatcaat
tacggggtcattagttcatagcccatatatggagttccgcgttacataacttacggtaaa
tggcccgcctggctgaccgcccaacgacccccgcccattgacgtcaataatgacgtatgt
tcccatagtaacgccaatagggactttccattgacgtcaatgggtggagtatttacggta
aactgcccacttggcagtacatcaagtgtatcatatgccaagtccgccccctattgacgt
caatgacggtaaatggcccgcctggcattatgcccagtacatgaccttacgggactttcc
tacttggcagtacatctacgtattagtcatcgctattaccatggtgatgcggttttggca
gtacaccaatgggcgtggatagcggtttgactcacggggatttccaagtctccaccccat
tgacgtcaatgggagtttgttttggcaccaaaatcaacgggactttccaaaatgtcgtaa
taaccccgccccgttgacgcaaatgggcggtaggcgtgtacggtgggaggtctataagg
cagagctcgtttagtgaaccgtcagatcactagaagctttattgcggtagtttatcacag
ttaaattgctaacgcagtcagtgcttctgacacaacagtctcgaacttaagctgcagaag
ttggtcgtgaggcactgggcaggtaagtatcaaggttacaagacaggtttaaggagacca
atagaaactgggcttgtcgagacagagaagactcttgcgtttctgataggcacctattgg
tcttactgacatccactttgcctttctctccacaggtgtccactcccagttcaattacag
ctcttaaggctagagtacttaatacgactcactataggctagccaccatgcagaagaacg
gcatcaaggccaacttcaccgtgagacacaacgtggaggacggcagcgtgcagctggccg
```

```
accactaccagcagaacaccccatcggcgacggccccgtgctgctgcccgacaaccact
acctgagcacccagaccgtgctgagcaaggaccccaacgagaagagagaccacatggtgc
tgcacgagtacgtgaacgccgccggcatcaccggcggtggcggatcctggcctgacatcg
aggaggatatcgccctgatcaagagcgaagagggcgagaaatggtgcttgagaataact
tcttcgtcgagaccgtgctcccaagcaagatcatgcggaaactggagcctgaggagttcg
ctgcctacctggagccattcaaggagaagggcgaggttagacggcctaccctctcctggc
ctcgcgagatccctctcgttaagggaggcaagcccgacgtcgtccagattgtccgcaact
acaacgcctaccttcgggccagcgacgatctgcctaagctgttcatcgagtccgaccctg
ggttcttttccaacgctattgtcgagggagctaagaagttccctaacaccgagttcgtga
aggtgaagggcctccacttcctccaggaggacgctccagatgaaatgggtaagtacatca
agagcttcgtggagcgcgtgctgaagaacgagcagtaattctagagcggccgcttcgagc
agacatgataagatacattgatgagtttggacaaaccacaactagaatgcagtgaaaaaa
atgctttatttgtgaaatttgtgatgctattgctttatttgtaaccattataagctgcaa
taaacaagttaacaacaacaattgcattcattttatgtttcaggttcaggggggaggtgtg
ggaggttttttaaagcaagtaaaacctctacaaatgtggtaaaatcgataaggatccagg
tggcacttttcggggaaatgtgcgcggaacccctatttgtttattttttctaaatacattc
aaatatgtatccgctcatgagacaataaccctgataaatgcttcaataatattgaaaaag
gaagagtatgagtattcaacatttccgtgtcgcccttattcccttttttgcggcattttg
ccttcctgtttttgctcacccagaaacgctggtgaaagtaaaagatgctgaagatcagtt
gggtgcacgagtgggttacatcgaactggatctcaacagcggtaagatccttgagagtttt
cgccccgaagaacgttttccaatgatgagcacttttaaagttctgctatgtggcgcggt
attatcccgtattgacgccgggcaagagcaactcggtcgccgcatacactattctcagaa
tgacttggttgagtactcaccagtcacagaaaagcatcttacggatggcatgacagtaag
agaattatgcagtgctgccataaccatgagtgataacactgcggccaacttacttctgac
aacgatcggaggaccgaaggagctaaccgcttttttgcacaacatgggggatcatgtaac
tcgccttgatcgttgggaaccggagctgaatgaagccataccaaacgacgagcgtgacac
cacgatgcctgtagcaatggcaacaacgttgcgcaaactattaactggcgaactacttac
tctagcttcccggcaacaattaatagactggatggaggcggataaagttgcaggaccact
tctgcgctcggcccttccggctggctggtttattgctgataaatctggagccggtgagcg
tgggtctcgcggtatcattgcagcactggggccagatggtaagccctcccgtatcgtagt
tatctacacgacggggagtcaggcaactatggatgaacgaaatagacagatcgctgagat
aggtgcctcactgattaagcattggtaactgtcagaccaagtttactcatatatacttta
gattgatttaaaacttcatttttaatttaaaaggatctaggtgaagatcctttttgataa
tctcatgaccaaaatcccttaacgtgagttttcgttccactgagcgtcagaccccgtaga
aaagatcaaaggatcttcttgagatcctttttttctgcgcgtaatctgctgcttgcaaac
aaaaaaaccaccgctaccagcggtggtttgtttgccggatcaagagctaccaactctttt
tccgaaggtaactggcttcagcagagcgcagataccaaatactgttcttctagtgtagcc
gtagttaggccaccacttcaagaactctgtagcaccgcctacatacctcgctctgctaat
cctgttaccagtggctgctgccagtggcgataagtcgtgtcttaccgggttggactcaag
acgatagttaccggataaggcgcagcggtcgggctgaacggggggttcgtgcacacagcc
cagcttggagcgaacgacctacaccgaactgagatacctacagcgtgagctatgagaaag
cgccacgcttcccgaagggagaaaggcggacaggtatccggtaagcggcagggtcggaac
aggagagcgcacgagggagcttccaggggaaacgcctggtatctttatagtcctgtcgg
gtttcgccacctctgacttgagcgtcgatttttgtgatgctcgtcaggggggcggagcct
atggaaaaacgccagcaacgcggcctttttacggttcctggcctttgctggcctttttgc
tcacatggctcgac
```

Engineered GFP (SEQ ID NO: 18)

MSKGEELFTGVVPILVELDGDVNGHKFSVRGEGEGDATIGKLTLKFICTTGKLPVPWPTLVT
TLTYGVQCFSRYPDHMKRHDFFKSAMPEGYVQERTISFKDDGKYKTRAVVKFEGDTLVNRIE

LKGTDFKEDGNILGHKLEYNFNSHNVYITADKMQKNGIKANFTVRHNVEDGSVQLADHYQQN
TPIGDGPVLLPDNHYLSTQTVLSKDPNEKRDHMVLHEYVNAAGIT

RLuc8 (SEQ ID NO: 19)

MASKVYDPEQRKRMITGPQWWARCKQMNVLDSFINYYDSEKHAENAVIFLHGNATSSYLWRH
VVPHIEPVARCIIPDLIGMGKSGKSGNGSYRLLDHYKYLTAWFELLNLPKKIIFVGHDWGAA
LAFHYAYEHQDRIKAIVHMESVVDVIESWDEWPDIEEDIALIKSEEGEKMVLENNFFVETVL
PSKIMRKLEPEEFAAYLEPFKEKGEVRRPTLSWPREIPLVKGGKPDVVQIVRNYNAYLRASD
DLPKLFIESDPGFFSNAIVEGAKKFPNTEFVKVKGLHFLQEDAPDEMGKYIKSFVERVLKNE
Q

19 amino acids of SARS-CoV-2 S glycoprotein cytoplasmic tail deleted in variant 2 (SEQ ID NO: 20)
KFDEDDSEPVLKGVKLHYT
VSV G glycoprotein cytoplasmic tail sequence used in variant 3 (SEQ ID NO: 21)
KLKHTKKRQIYTDIEMNRLGK
Sequences of mAb10914 antibody:

| Component Part | Sequence | SEQ ID NO |
|---|---|---|
| Amino Acid Sequences | | |
| HCVR | EVQLVESGGGLVQPGGSLRLSCAASGLIVSRNYMIWVRQAPGKGLEWVSV IYSGGSTFYADSVKGRFTISRHNSKNTLYLQMNSLRAEDTAVYYCARDLG TGGMDVWGQGTTVTVSS | 22 |
| HCDR1 | GLIVSRNY | 24 |
| HCDR2 | IYSGGST | 26 |
| HCDR3 | ARDLGTGGMDV | 28 |
| LCVR | DIQLTQSPSFLSASVGDRVTITCWASQGISSYLAWYQQKPGKAPKLLIYA ASTLQSGVPSRFSGSGSGTEFTLTISSLQPEDFATYYCQQLNSYPLTFGG GTKVEIK | 30 |
| LCDR1 | QGISSY | 32 |
| LCDR2 | AAS | 34 |
| LCDR3 | QQLNSYPLT | 36 |
| HC | EVQLVESGGGLVQPGGSLRLSCAASGLIVSRNYMIWVRQAPGKGLEWVSV IYSGGSTFYADSVKGRFTISRHNSKNTLYLQMNSLRAEDTAVYYCARDLG TGGMDVWGQGTTVTVSSASTKGPSVFPLAPSSKSTSGGTAALGCLVKDYF PEPVTVSWNSGALTSGVHTFPAVLQSSGLYSLSSVVTVPSSSLGTQTYIC NVNHKPSNTKVDKKVEPKSCDKTHTCPPCPAPELLGGPSVFLFPPKPKDT LMISRTPEVTCVVVDVSHEDPEVKFNWYVDGVEVHNAKTKPREEQYNSTY RVVSVLTVLHQDWLNGKEYKCKVSNKALPAPIEKTISKAKGQPREPQVYT LPPSRDELTKNQVSLTCLVKGFYPSDIAVEWESNGQPENNYKTTPPVLDS DGSFFLYSKLTVDKSRWQQGNVFSCSVMHEALHNHYTQKSLSLSPGK | 38 |
| LC | DIQLTQSPSFLSASVGDRVTITCWASQGISSYLAWYQQKPGKAPKLLIYA ASTLQSGVPSRFSGSGSGTEFTLTISSLQPEDFATYYCQQLNSYPLTFGG GTKVEIKRTVAAPSVFIFPPSDEQLKSGTASVVCLLNNFYPREAKVQWKV | 40 |

(continued)

| Component Part | Sequence | SEQ ID NO |
|---|---|---|
| Amino Acid Sequences | | |
| | DNALQSGNSQESVTEQDSKDSTYSLSSTLTLSKADYEKHKVYACEVTHQG LSSPVTKSFNRGEC | |
| Nucleotide Sequences | | |
| HCVR | gaggtgcagctggtggagtctggaggaggcttggtccagcctggggggtc cctgagactctcctgtgcagcctctgggttaatagtcagtcgcaactaca tgatctgggtccgccaggctccagggaaggggctggagtgggtctcagtt atttatagcggtggtagcacattctacgcagactccgtgaagggccgatt caccatctccagacacaattccaagaacacgctgtatcttcaaatgaaca gcctgagggctgaggacacggccgtatattactgtgcgagagatctgggt acaggaggtatggacgtctggggccaagggaccacggtcaccgtctcctc a | 23 |
| HCDR1 | gggttaatagtcagtcgcaactac | 25 |
| HCDR2 | atttatagcggtggtagcaca | 27 |
| HCDR3 | gcgagagatctgggtacaggaggtatggacgtc | 29 |
| LCVR | gacatccagttgacccagtctccatccttcctgtctgcatctgtaggaga cagagtcaccatcacttgctgggccagtcagggcattagcagttatttag cctggtatcagcaaaaaccagggaaagcccctaagctcctgatctatgct gcatccactttgcaaagtggggtcccatcaaggttcagcggcagtggatc tgggacagaattcactctcacaatcagcagcctgcagcctgaagattttg caacttattactgtcaacagcttaatagttaccctctcactttcggcgga gggaccaaggtggagatcaaa | 31 |
| LCDR1 | cagggcattagcagttat | 33 |
| LCDR2 | gctgcatcc | 35 |
| LCDR3 | caacagcttaatagttaccctctcact | 37 |

(continued)

| Nucleotide Sequences | | |
|---|---|---|
| HC | gaggtgcagctggtggagtctggaggaggcttggtccagcctggggggtc<br>cctgagactctcctgtgcagcctctgggttaatagtcagtcgcaactaca<br>tgatctgggtccgccaggctccagggaaggggctggagtgggtctcagtt<br>atttatagcggtggtagcacattctacgcagactccgtgaagggccgatt<br>caccatctccagacacaattccaagaacacgctgtatcttcaaatgaaca<br>gcctgagggctgaggacacggccgtatattactgtgcgagagatctgggt<br>acaggaggtatggacgtctggggccaagggaccacggtcaccgtctcctc<br>agcctccaccaagggcccatcggtcttccccctggcaccctcctccaaga<br>gcacctctgggggcacagcggccctgggctgcctggtcaaggactacttc<br>cccgaaccggtgacggtgtcgtggaactcaggcgccctgaccagcggcgt<br>gcacaccttcccggctgtcctacagtcctcaggactctactccctcagca<br>gcgtggtgaccgtgccctccagcagcttgggcacccagacctacatctgc<br>aacgtgaatcacaagcccagcaacaccaaggtggacaagaaagttgagcc<br>caaatcttgtgacaaaactcacacatgcccaccgtgcccagcacctgaac<br>tcctggggggaccgtcagtcttcctcttccccccaaaacccaaggacacc<br>ctcatgatctcccggacccctgaggtcacatgcgtggtggtggacgtgag<br>ccacgaagaccctgaggtcaagttcaactggtacgtggacggcgtggagg<br>tgcataatgccaagacaaagccgcgggaggagcagtacaacagcacgtac<br>cgtgtggtcagcgtcctcaccgtcctgcaccaggactggctgaatggcaa<br>ggagtacaagtgcaaggtctccaacaaagccctcccagcccccatcgaga<br>aaaccatctccaaagccaaagggcagccccgagaaccacaggtgtacacc<br>ctgcccccatcccgggatgagctgaccaagaaccaggtcagcctgacctg<br>cctggtcaaaggcttctatcccagcgacatcgccgtggagtgggagagca<br><br>atgggcagccggagaacaactacaagaccacgcctcccgtgctggactcc<br>gacggctccttcttcctctacagcaagctcaccgtggacaagagcaggtg<br>gcagcaggggaacgtcttctcatgctccgtgatgcatgaggctctgcaca<br>accactacacgcagaagtccctctccctgtctccgggtaaatga | 39 |
| LC | gacatccagttgacccagtctccatccttcctgtctgcatctgtaggaga<br>cagagtcaccatcacttgctgggccagtcagggcattagcagttatttag<br>cctggtatcagcaaaaaccagggaaagcccctaagctcctgatctatgct<br>gcatccactttgcaaagtggggtcccatcaaggttcagcggcagtggatc<br>tgggacagaattcactctcacaatcagcagcctgcagcctgaagattttg<br>caacttattactgtcaacagcttaatagttaccctctcactttcggcgga<br>gggaccaaggtggagatcaaacgaactgtggctgcaccatctgtcttcat<br>cttcccgccatctgatgagcagttgaaatctggaactgcctctgttgtgt<br>gcctgctgaataacttctatcccagagaggccaaagtacagtggaaggtg<br>gataacgccctccaatcgggtaactcccaggagagtgtcacagagcagga<br>cagcaaggacagcacctacagcctcagcagcaccctgacgctgagcaaag<br>cagactacgagaaacacaaagtctacgcctgcgaagtcacccatcagggc<br>ctgagctcgcccgtcacaaagagcttcaacaggggagagtgttag | 41 |

Sequences of mAb 10922 antibody:

| Amino Acid Sequences | | |
|---|---|---|
| HCVR | EVQLVESGGGLVQPGGSLRLSCAASGFTFSIYEMNWVRQAPGKGLEWVSY<br>ITSSGTTIYYADSVKGRFTISRDNAKNSLYLQMNSLRAEDTAVYYCASSS<br>SSGYYFDYWGQGTLVTVSS | 45 |
| HCDR1 | GFTFSIYE | 46 |

(continued)

| Amino Acid Sequences | | |
|---|---|---|
| HCDR2 | ITSSGTTI | 47 |
| HCDR3 | ASSSSSGYYFDY | 48 |
| LCVR | DIVMTQSPDSLAVSLGERATINCKSSQSVLYSSNNKNYLAWYQQKPGQPP NLLIYWASTRESGVPDRFSGSGSGTDFTLTISSLQAEDVAVYYCQQYYST PPTFGQGTKLEIK | 49 |
| LCDR1 | QSVLYSSNNKNY | 50 |
| LCDR2 | WAS | 51 |
| LCDR3 | QQYYSTPPT | 52 |
| HC | EVQLVESGGGLVQPGGSLRLSCAASGFTFSIYEMNWVRQAPGKGLEWVSY ITSSGTTIYYADSVKGRFTISRDNAKNSLYLQMNSLRAEDTAVYYCASSS SSGYYFDYWGQGTLVTVSSASTKGPSVFPLAPSSKSTSGGTAALGCLVKD YFPEPVTVSWNSGALTSGVHTFPAVLQSSGLYSLSSVVTVPSSSLGTQTY ICNVNHKPSNTKVDKKVEPKSCDKTHTCPPCPAPELLGGPSVFLFPPKPK DTLMISRTPEVTCVVVDVSHEDPEVKFNWYVDGVEVHNAKTKPREEQYNS TYRVVSVLTVLHQDWLNGKEYKCKVSNKALPAPIEKTISKAKGQPREPQV YTLPPSRDELTKNQVSLTCLVKGFYPSDIAVEWESNGQPENNYKTTPPVL DSDGSFFLYSKLTVDKSRWQQGNVFSCSVMHEALHNHYTQKSLSLSPGK | 53 |
| LC | DIVMTQSPDSLAVSLGERATINCKSSQSVLYSSNNKNYLAWYQQKPGQPP NLLIYWASTRESGVPDRFSGSGSGTDFTLTISSLQAEDVAVYYCQQYYST PPTFGQGTKLEIKRTVAAPSVFIFPPSDEQLKSGTASVVCLLNNFYPREA KVQWKVDNALQSGNSQESVTEQDSKDSTYSLSSTLTLSKADYEKHKVYAC EVTHQGLSSPVTKSFNRGEC | 54 |
| Nucleotide Sequences | | |
| HCVR | gaggtgcagctggtggagtctggggggaggcttggtacagcctggagggtc cctgagactctcctgtgcagcctctggattcaccttcagtatttatgaaa tgaactgggtccgccaggctccagggaaggggctggagtgggtttcatac attactagtagtggtactaccatatactacgcagactctgtgaagggccg attcaccatctccagagacaacgccaagaactcactgtatctgcaaatga acagcctgagagccgaggacacggctgtttattactgtgcgagtagcagc tcgtccgggtactactttgactactggggccagggaaccctggtcaccgt ctcctca | 55 |
| HCDR1 | ggattcaccttcagtatttatgaa | 56 |
| HCDR2 | attactagtagtggtactaccata | 57 |
| HCDR3 | gcgagtagcagctcgtccgggtactactttgactac | 58 |
| LCVR | gacatcgtgatgacccagtctccagactccctggctgtgtctctgggcga gagggccaccatcaactgcaagtccagccagagtgttttatacagctcca acaataagaactacttagcttggtaccagcagaaaccaggacagcctcct aatctgctcatttactgggcatctacccgggaatccggggtccctgaccg attcagtggcagcgggtctgggacagatttcactctcaccatcagcagcc tgcaggctgaagatgtggcagtttattactgtcagcaatattatagtact cctcccacttttggccaggggaccaagctggagatcaaa | 59 |
| LCDR1 | cagagtgttttatacagctccaacaataagaactac | 60 |
| LCDR2 | tgggcatct | 61 |
| LCDR3 | cagcaatattatagtactcctcccact | 62 |

| Nucleotide Sequences | | |
|---|---|---|
| HC | gaggtgcagctggtggagtctggggggaggcttggtacagcctggagggtc<br>cctgagactctcctgtgcagcctctggattcaccttcagtatttatgaaa<br>tgaactgggtccgccaggctccagggaaggggctggagtgggtttcatac<br>attactagtagtggtactaccatatactacgcagactctgtgaagggccg<br>attcaccatctccagagacaacgccaagaactcactgtatctgcaaatga<br>acagcctgagagccgaggacacggctgtttattactgtgcgagtagcagc<br>tcgtccgggtactactttgactactggggccagggaaccctggtcaccgt<br>ctcctcagcctccaccaagggcccatcggtcttccccctggcaccctcct<br>ccaagagcacctctgggggcacagcggccctgggctgcctggtcaaggac<br>tacttccccgaaccggtgacggtgtcgtggaactcaggcgccctgaccag<br>cggcgtgcacaccttcccggctgtcctacagtcctcaggactctactccc<br>tcagcagcgtggtgaccgtgccctccagcagcttgggcacccagacctac<br>atctgcaacgtgaatcacaagcccagcaacaccaaggtggacaagaaagt<br>tgagcccaaatcttgtgacaaaactcacacatgcccaccgtgcccagcac<br>ctgaactcctggggggaccgtcagtcttcctcttccccccaaaacccaag<br>gacaccctcatgatctcccggacccctgaggtcacatgcgtggtggtgga<br>cgtgagccacgaagaccctgaggtcaagttcaactggtacgtggacggcg<br>tggaggtgcataatgccaagacaaagccgcgggaggagcagtacaacagc<br>acgtaccgtgtggtcagcgtcctcaccgtcctgcaccaggactggctgaa<br>tggcaaggagtacaagtgcaaggtctccaacaaagccctcccagccccca<br><br>tcgagaaaaccatctccaaagccaaagggcagccccgagaaccacaggtg<br>tacaccctgcccccatcccgggatgagctgaccaagaaccaggtcagcct<br>gacctgcctggtcaaaggcttctatcccagcgacatcgccgtggagtggg<br>agagcaatgggcagccggagaacaactacaagaccacgcctcccgtgctg<br>gactccgacggctccttcttcctctacagcaagctcaccgtggacaagag<br>caggtggcagcaggggaacgtcttctcatgctccgtgatgcatgaggctc<br>tgcacaaccactacacgcagaagtccctctccctgtctccgggtaaatga | 63 |
| LC | gacatcgtgatgacccagtctccagactccctggctgtgtctctgggcga<br>gagggccaccatcaactgcaagtccagccagagtgtttttatacagctcca<br>acaataagaactacttagcttggtaccagcagaaaccaggacagcctcct<br>aatctgctcatttactgggcatctacccgggaatccggggtccctgaccg<br>attcagtggcagcgggtctgggacagatttcactctcaccatcagcagcc<br>tgcaggctgaagatgtggcagtttattactgtcagcaatattatagtact<br>cctcccacttttggccaggggaccaagctggagatcaaacgaactgtggc<br>tgcaccatctgtcttcatcttcccgccatctgatgagcagttgaaatctg<br>gaactgcctctgttgtgtgcctgctgaataacttctatcccagagaggcc<br>aaagtacagtggaaggtggataacgcctccaatcgggtaactcccagga<br>gagtgtcacagagcaggacagcaaggacagcacctacagcctcagcagca<br>ccctgacgctgagcaaagcagactacgagaaacacaaagtctacgcctgc<br>gaagtcacccatcagggcctgagctcgcccgtcacaaagagcttcaacag<br>gggagagtgttag | 64 |

Amino Acid Sequence of SARS-CoV-2 Δ19CT CPE Lytic Variant (SEQ ID NO: 42; CPE Variant)

MFVFLVLLPLVSSQCVNLTTRTQLPPAYTNSFTRGVYYPDKVFRSSVLHSTQDLFLPFFSNV
TWFHAIHVSGTNGTKRFDNPVLPFNDGVYFASTEKSNIIRGWIFGTTLDSKTQSLLIVNNAT
NVVIKVCEFQFCNDPFLGVYYHKNNKSWMESEFRVYSSANNCTFEYVSQPFLMDLEGKQGNF
KNLREFVFKNIDGYFKIYSKHTPINLVRDLPQGFSALEPLVDLPIGINITRFQTLLALHRRY
LTPGDSSSGWTAGAAAYYVGYLQPRTFLLKYNENGTITDAVDCALDPLSETKCTLKSFTVEK
GIYQTSNFRVQPTESIVRFPNITNLCPFGEVFNATRFASVYAWNRKRISNCVADYSVLYNSA
SFSTFKCYGVSPTKLNDLCFTNVYADSFVIRGDEVRQIAPGQTGKIADYNYKLPDDFTGCVI
AWNSNNLDSKVGGNYNYLYRLFRKSNLKPFERDISTEIYQAGSTPCNGVEGFNCYFPLQSYG
FQPTNGVGYQPYRVVVLSFELLHAPATVCGPKKSTNLVKNKCVNFNFNGLTGTGVLTESNKK
FLPFQQFGRDIADTTDAVRDPQTLEILDITPCSFGGVSVITPGTNTSNQVAVLYQNVNCTEV
PVAIHADQLTPTWRVYSTGSNVFQTRAGCLIGAEHVNNSYECDIPIGAGICASYQTQTNSPR
RAQSVASQSIIAYTMSLGAENSVAYSNNSIAIPTNFTISVTTEILPVSMTKTSVDCTMYICG
DSTECSNLLLQYGSFCTQLNRALTGIAVEQDKNTQEVFAQVKQIYKTPPIKDFGGFNFSQIL
PDPSKPSKRSFIEDLLFNKVTLADAGFIKQYGDCLGDIAARDLICAQKFNGLTVLPPLLTDE
MIAQYTSALLAGTITSGWTFGAGAALQIPFAMQMAYRFNGIGVTQNVLYENQKLIANQFNSA
IGKIQDSLSSTASALGKLQDVVNQNAQALNTLVKQLSSNFGAISSVLNDILSRLDKVEAEVQ
IDRLITGRLQSLQTYVTQQLIRAAEIRASANLAATKMSECVLGQSKRVDFCGKGYHLMSFPQ
SAPHGVVFLHVTYVPAQEKNFTTAPAICHDGKAHFPREGVFVSNGTHWFVTQRNFYEPQIIT
TDNTFVSGNCDVVIGIVNNTVYDPLQPELDSFKEELDKYFKNHTSPDVDLGDISGINASVVN
IQKEIDRLNEVAKNLNESLIDLQELGKYEQYIKWPWYIWLGFIAGLIAIVMVTIMLCCMTSC
CSCLKGCCSCGSCC

Codon Optimized Polynucleotide Sequence of SARS-CoV-2 Δ19CT CPE Lytic Variant (SEQ ID NO: 43; CPE Variant)

```
atgttcgtcttcctggtcctgctgcctctggtctcctcacagtgcgtcaatctgacaactcg
gactcagctgccacctgcttatactaatagcttcaccagaggcgtgtactatcctgacaagg
tgtttagaagctccgtgctgcactctacacaggatctgtttctgccattctttagcaacgtg
acctggttccacgccatccacgtgagcggcaccaatggcacaaagcggttcgacaatcccgt
gctgccttttaacgatggcgtgtacttcgcctctaccgagaagagcaacattatcagaggct
ggatctttggcaccacactggactccaagacacagtctctgctgatcgtgaacaatgccacc
aacgtggtcatcaaggtgtgcgagttccagttttgtaatgatcccttcctgggcgtgtacta
tcacaagaacaataagagctggatggagtccgagtttagagtgtattctagcgccaacaact
gcacatttgagtacgtgagccagccttttcctgatggacctggagggcaagcagggcaatttc
aagaacctgagggagttcgtgtttaagaatatcgacggctacttcaaaatctactctaagca
cacccccatcaacctggtgcgcgacctgcctcagggcttcagcgccctggagcccctggtgg
atctgcctatcggcatcaacatcacccggtttcagacactgctggccctgcacagaagatac
ctgacacccggcgactcctctagcggatggaccgccggcgctgccgcctactatgtgggcta
cctccagcccggaccttcctgctgaagtacaacgagaatggcaccatcacagacgcagtgg
attgcgccctggaccccctgagcgagacaaagtgtacactgaagtcctttaccgtggagaag
ggcatctatcagacatccaatttcaggtgcagccaaccgagtctatcgtgcgctttcctaa
tatcacaaacctgtgcccatttggcgaggtgttcaacgcaacccgcttcgccagcgtgtacg
cctggaataggaagcggatcagcaactgcgtggccgactatagcgtgctgtacaactccgcc
tctttcagcacctttaagtgctatggcgtgtccccccacaaagctgaatgacctgtgctttttac
caacgtctacgccgattctttcgtgatcaggggcgacgaggtgcgccagatcgcccccggcc
agacaggcaagatcgcagactacaattataagctgccagacgatttcaccggctgcgtgatc
gcctggaacagcaacaatctggattccaaagtgggcggcaactacaattatctgtaccggct
gtttagaaagagcaatctgaagcccttcgagagggacatctctacagaaatctaccaggccg
gcagcacccccttgcaatggcgtggagggctttaactgttatttcccactccagtcctacggc
ttccagcccacaaacggcgtgggctatcagccttaccgcgtggtggtgctgagctttgagct
gctgcacgccccagcaacagtgtgcggccccaagaagtccaccaatctggtgaagaacaagt
gcgtgaacttcaacttcaacggcctgaccggcacaggcgtgctgaccgagtccaacaagaag
ttcctgccatttcagcagttcggcagggacatcgcagataccacagacgccgtgcgcgaccc
acagaccctggagatcctggacatcacaccctgctctttcggcggcgtgagcgtgatcacac
ccggcaccaatacaagcaaccaggtggccgtgctgtatcagaacgtgaattgtaccgaggtg
cccgtggctatccacgccgatcagctgaccccaacatggcgggtgtacagcaccggctccaa
cgtcttccagacaagagccggatgcctgatcggagcagagcacgtgaacaattcctatgagt
gcgacatcccaatcggcgccggcatctgtgcctcttaccagacccagacaaactctcccaga
agagcccagagcgtggcctcccagtctatcatcgcctataccatgtccctgggcgccgagaa
cagcgtggcctactctaacaatagcatcgccatcccaaccaacttcacaatctctgtgacca
cagagatcctgcccgtgtccatgaccaagacatctgtggactgcacaatgtatatctgtggc
gattctaccgagtgcagcaacctgctgctccagtacggcagcttttgtacccagctgaatag
agccctgacaggcatcgccgtggagcaggataagaacacacaggaggtgttcgcccaggtga
agcaaatctacaagaccccccctatcaaggactttggcggcttcaattttttcccagatcctg
cctgatccatccaagccttctaagcggagctttatcgaggacctgctgttcaacaaggtgac
cctggccgatgccggcttcatcaagcagtatggcgattgcctgggcgacatcgcagccaggg
acctgatctgcgcccagaagtttaatggcctgaccgtgctgccacccctgctgacagatgag
atgatcgcacagtacacaagcgccctgctggccggcaccatcacatccggatggaccttcgg
cgcaggagccgccctccagatcccctttgccatgcagatggcctataggttcaacggcatcg
gcgtgacccagaatgtgctgtacgagaaccagaagctgatcgccaatcagtttaactccgcc
atcggcaagatccaggacagcctgtcctctacagccagcgccctgggcaagctccaggatgt
ggtgaatcagaacgcccaggccctgaataccctggtgaagcagctgagcagcaacttcggcg
ccatctctagcgtgctgaatgacatcctgagccggctggacaaggtggaggcagaggtgcag
atcgaccggctgatcaccggccggtccagagcctccagacctatgtgacacagcagctgat
```

```
cagggccgccgagatcagggccagcgccaatctggcagcaaccaagatgtccgagtgcgtgc
tgggccagtctaagagagtggacttttgtggcaagggctatacctgatgtccttccctcag
tctgccccacacggcgtggtgtttctcacgtgacctacgtgcccgcccaggagaagaactt
caccacagcccctgccatctgccacgatggcaaggcccactttccaagggagggcgtgttcg
tgtccaacggcacccactggtttgtgacacagcgcaatttctacgagccccagatcatcacc
acagaacaccttcgtgagcggcaactgtgacgtggtcatcggcatcgtgaacaataccgt
gtatgatccactccagcccgagctggacagctttaaggaggagctggataagtatttcaaga
atcacacctccctgacgtggatctgggcgacatcagcggcatcaatgcctccgtggtgaac
atccagaaggagatcgaccgcctgaacgaggtggctaagaatctgaacgagagcctgatcga
cctccaggagctgggcaagtatgagcagtacatcaagtggccctggtacatctggctgggct
tcatcgccggcctgatcgccatcgtgatggtgaccatcatgctgtgctgtatgacatcctgc
tgttcttgcctgaagggctgctgtagctgtggctcctgctgttaa
```

Amino Acid Sequence of SARS-CoV-2 Δ19CT Variant (SEQ ID NO: 44; Variant)

```
MFVFLVLLPLVSSQCVNLTTRTQLPPAYTNSFTRGVYYPDKVFRSSVLHSTQDLFLPFFSNV
TWFHAISGTNGTKRFDNPVLPFNDGVYFASTEKSNIIRGWIFGTTLDSKTQSLLIVNNATNV
VIKVCEFQFCNDPFLGVYYHKNNKSWMESEFRVYSSANNCTFEYVSQPFLMDLEGKQGNFKN
LREFVFKNIDGYFKIYSKHTPINLVRDLPQGFSALEPLVDLPIGINITRFQTLLALHRSYLT
PGDSSSGWTAGAAAYYVGYLQPRTFLLKYNENGTITDAVDCALDPLSETKCTLKSFTVEKGI
YQTSNFRVQPTESIVRFPNITNLCPFGEVFNATRFASVYAWNRKRISNCVADYSVLYNSASF
STFKCYGVSPTKLNDLCFTNVYADSFVIRGDEVRQIAPGQTGKIADYNYKLPDDFTGCVIAW
NSNNLDSKVGGNYNYLYRLFRKSNLKPFERDISTEIYQAGSTPCNGVKGFNCYFPLQSYGFQ
PTYGVGYQPYRVVVLSFELLHAPATVCGPKKSTNLVKNKCVNFNFNGLTGTGVLTESNKKFL
PFQQFGRDIADTTDAVRDPQTLEILDITPCSFGGVSVITPGTNTSNQVAVLYQGVNCTEVPV
AIHADQLTPTWRVYSTGSNVFQTRAGCLIGAEHVNNSYECDIPIGAGICASYQTQTNSPRRA
RSVASQSIIAYTMSLGAENSVAYSNNSIAIPTNFTISVTTEILPVSMTKTSVDCTMYICGDS
TECSNLLLQYGSFCTQLNRALTGIAVEQDKNTQEVFAQVKQIYKTPPIKDFGGFNFSQILPD
PSKPSKRSFIEDLLFNKVTLADAGFIKQYGDCLGDIAARDLICAQKFNGLTVLPPLLTDEMI
AQYTSALLAGTITSGWTFGAGAALQIPFAMQMAYRFNGIGVTQNVLYENQKLIANQFNSAIG
KIQDSLSSTASALGKLQDVVNQNAQALNTLVKQLSSNFGAISSVLNDILSRLDKVEAEVQID
RLITGRLQSLQTYVTQQLIRAAEIRASANLAATKMSECVLGQSKRVDFCGKGYHLMSFPQSA
PHGVVFLHVTYVPAQEKNFTTAPAICHDGKAHFPREGVFVSNGTHWFVTQRNFYEPQIITTD
NTFVSGNCDVVIGIVNNTVYDPLQPELDSFKEELDKYFKNHTSPDVDLGDISGINASVVNIQ
KEIDRLNEVAKNLNESLIDLQELGKYEQYIKWPWYIWLGFIAGLIAIVMVTIMLCCMTSCCS
CLKGCCSCGSCC
```

## Claims

1. A replication competent recombinant rhabdovirus particle, wherein (i) the rhabdovirus glycoprotein (G) is replaced by a Severe Acute Respiratory Syndrome coronavirus 2 (SARS-CoV-2) spike (S) glycoprotein or a fragment of the SARS-CoV-2 S glycoprotein comprising the receptor binding domain thereof, wherein said SARS-CoV-2 S glycoprotein or fragment is capable of mediating infection of a target cell, and wherein (ii) the rhabdovirus particle comprises a nucleic acid molecule comprising a nucleic acid sequence encoding a reporter protein, wherein the nucleic acid sequence encoding the reporter protein is inserted between a nucleic acid sequence encoding the SARS-CoV-2 S glycoprotein or fragment thereof, and a nucleic acid sequence encoding rhabdovirus large (L) protein.

2. The replication competent recombinant rhabdovirus particle of claim 1, wherein the reporter protein is a luciferase or a fluorescent protein.

3. The replication competent recombinant rhabdovirus particle of claim 1 or 2, wherein the recombinant rhabdovirus particle is a recombinant vesiculovirus particle, optionally a recombinant vesicular stomatitis virus (VSV) particle.

4. The replication competent recombinant rhabdovirus particle of any one of claims 1-3, wherein:

    (i) the SARS-CoV-2 S glycoprotein is a full-length SARS-CoV-2 S glycoprotein; or
    (ii) the fragment is a SARS-CoV-2 S glycoprotein lacking the 19 C-terminal amino acids as specified in relation to the amino acid sequence of the SARS-CoV-2 full-length S glycoprotein of SEQ ID NO: 1; or
    (iii) the SARS-CoV-2 S glycoprotein comprises one or more amino acid insertions, deletions, and/or substitutions listed in Tables 8 and 9, wherein the positions of said insertions, deletions, and/or substitutions are specified in relation to the amino acid sequence of the SARS-CoV-2 full-length S glycoprotein of SEQ ID NO: 1.

5. The replication competent recombinant rhabdovirus particle of any one of the preceding claims, wherein the recombinant rhabdovirus particle comprises a mutant rhabdovirus matrix (M) protein, optionally wherein the recombinant rhabdovirus particle is a recombinant VSV particle comprising the mutant VSV M protein which comprises a mutation at methionine 51, optionally from methionine (M) to arginine (R).

6. A method for determining the presence of a SARS-CoV-2 neutralizing antibody in a sample, the method comprising:

    a) contacting the sample with a replication competent recombinant rhabdovirus particle according to any one of claims 1-5;
    b) after step (a), contacting the replication competent recombinant rhabdovirus particle with the target cell;
    c) measuring a reporter signal in the cell after step (b), and
    d) comparing the reporter signal measured in step (c) with a control.

7. The method of claim 6, wherein the target cell is a Vero cell, or Vero-E6 cell.

8. The method of claim 6 or 7, further comprising adding a reporter protein substrate for obtaining the reporter signal, optionally wherein the reporter protein comprises a luciferase and the reporter protein substrate comprises Luciferin or EnduRen luciferase substrate.

9. The method of any one of claims 6-8, wherein the sample is:

    (i) serum or plasma; and/or
    (ii) is heat-inactivated.

10. The method of any one of claims 6-9, wherein the method further comprises diluting the sample by a factor of 1:10 to 1:320.

11. The method of any one of claims 6-10, wherein the control is the reporter signal obtained with a control sample not comprising coronavirus neutralizing antibodies, and the method comprises concluding that the tested sample comprises the SARS-CoV-2 neutralizing antibodies when the reporter signal obtained in step (c) is reduced as compared to the control, optionally wherein the method comprises determining the concentration of SARS-CoV-2 neutralizing antibodies in the tested sample by comparing the reporter signal to a calibration curve determined from a serial dilution of the control sample comprising a SARS-CoV-2 neutralizing antibody, or a molecule that blocks interaction of the SARS-CoV-2 S glycoprotein with a protein with which the coronavirus S glycoprotein interacts on the target cell, or any combination thereof.

12. The method of any one of claims 6-11, wherein:

    (i) the reporter signal is measured between 24 to 30 hours after step (b); and/or
    (ii) in step (a) the sample is contacted with the recombinant rhabdovirus particle for 30 minutes at room temperature.

13. The method of any one of claims 6-12, wherein the method is conducted in a high throughput format.

14. A polynucleotide encoding the replication competent recombinant rhabdovirus particle according to any one of claims 1-5, wherein the polynucleotide comprises a nucleic acid sequence encoding a rhabdovirus nucleoprotein (N), a nucleic acid sequence encoding a rhabdovirus phosphoprotein (P), the nucleic acid sequence encoding the rhabdovirus L protein, the nucleic acid sequence encoding the SARS-CoV-2 S glycoprotein or fragment thereof, and the nucleic acid sequence encoding the reporter protein inserted as specified in claim 1.

**15.** A kit for determining the presence of a SARS-CoV-2 neutralizing antibody in a sample comprising:

a) a replication competent recombinant rhabdovirus particle according to claims 1-5, or the polynucleotide of claim 14;
b) a target cell;
c) optionally, a control sample not comprising coronavirus neutralizing antibodies;
d) optionally, a control sample comprising a SARS-CoV-2 neutralizing antibody, or a molecule that blocks interaction of the SARS-CoV-2 S glycoprotein with a protein with which it interacts on the target cell, or any combination thereof;
e) optionally, a substrate for the reporter protein, and
f) optionally, instructions for use.

**Patentansprüche**

**1.** Replikationskompetentes, rekombinantes Rhabdovirus-Partikel, wobei (i) das Rhabdovirus-Glykoprotein (G) durch ein Spike-(S-)Glykoprotein des schweren akuten Atemwegssyndrom-Coronavirus 2 (SARS-CoV-2) oder ein Fragment des SARS-CoV-2-S-Glykoproteins, das die Rezeptorbindungsdomäne davon umfasst, ersetzt ist, wobei das SARS-CoV-2-S-Glykoprotein oder Fragment in der Lage ist, eine Infektion einer Zielzelle zu vermitteln, und wobei (ii) das Rhabdovirus-Partikel ein Nukleinsäuremolekül umfasst, das eine Nukleinsäuresequenz umfasst, die für ein Reporterprotein kodiert, wobei die Nukleinsäuresequenz, die für das Reporterprotein kodiert, zwischen einer Nukleinsäuresequenz, die für das SARS-CoV-2-S-Glykoprotein oder ein Fragment davon kodiert, und einer Nukleinsäuresequenz, die für das große (L) Rhabdovirus-Protein kodiert, eingefügt ist.

**2.** Replikationskompetentes, rekombinantes Rhabdovirus-Partikel nach Anspruch 1, wobei das Reporterprotein eine Luciferase oder ein fluoreszierendes Protein ist.

**3.** Replikationskompetentes, rekombinantes Rhabdovirus-Partikel nach Anspruch 1 oder 2, wobei das rekombinante Rhabdovirus-Partikel ein rekombinantes Vesiculovirus-Partikel, optional ein rekombinantes vesikuläres Stomatitisvirus-(VSV-)Partikel ist.

**4.** Replikationskompetentes, rekombinantes Rhabdovirus-Partikel nach einem der Ansprüche 1-3, wobei:

(i) das SARS-CoV-2-S-Glykoprotein ein SARS-CoV-2-S-Glykoprotein voller Länge ist; oder
(ii) das Fragment ein SARS-CoV-2-S-Glykoprotein ist, dem die 19 C-terminalen Aminosäuren fehlen, wie in Bezug auf die Aminosäuresequenz des SARS-CoV-2-S-Glykoproteins voller Länge mit der SEQ ID NO: 1 spezifiziert; oder
(iii) das SARS-CoV-2-S-Glykoprotein eine oder mehrere Aminosäureinsertionen, - deletionen und/oder -substitutionen umfasst, die in Tabelle 8 und 9 aufgeführt sind, wobei die Positionen der Insertionen, Deletionen und/oder Substitutionen in Bezug auf die Aminosäuresequenz des SARS-CoV-2-S-Glykoproteins voller Länge mit der SEQ ID NO: 1 spezifiziert sind.

**5.** Replikationskompetentes, rekombinantes Rhabdovirus-Partikel nach einem der vorhergehenden Ansprüche, wobei das rekombinante Rhabdovirus-Partikel ein mutiertes Rhabdovirusmatrix-(M-)Protein umfasst, wobei das rekombinante Rhabdovirus-Partikel optional ein rekombinantes VSV-Partikel ist, welches das mutierte VSV-M-Protein umfasst, das eine Mutation bei Methionin 51 umfasst, optional von Methionin (M) zu Arginin (R).

**6.** Verfahren zum Bestimmen des Vorhandenseins eines SARS-CoV-2-neutralisierenden Antikörpers in einer Probe, das Verfahren umfassend:

a) Inkontaktbringen der Probe mit einem replikationskompetenten, rekombinanten Rhabdovirus-Partikel gemäß einem der Ansprüche 1-5;
b) nach Schritt (a), Inkontaktbringen des replikationskompetenten, rekombinanten Rhabdovirus-Partikels mit der Zielzelle;
c) Messen eines Reportersignals in der Zelle nach Schritt (b), und
d) Vergleichen des in Schritt (c) gemessenen Reportersignals mit einer Kontrolle.

**7.** Verfahren nach Anspruch 6, wobei die Zielzelle eine Vero-Zelle oder Vero-E6-Zelle ist.

**8.** Verfahren nach Anspruch 6 oder 7, ferner umfassend das Hinzufügen eines Reporterproteinsubstrats zum Erhalten des Reportersignals, wobei das Reporterprotein optional eine Luciferase umfasst und das Reporterproteinsubstrat ein Luciferin- oder EnduRen-Luciferasesubstrat umfasst.

**9.** Verfahren nach einem der Ansprüche 6-8, wobei die Probe Folgendes ist:

(i) Serum oder Plasma; und/oder
(ii) hitzeinaktiviert ist.

**10.** Verfahren nach einem der Ansprüche 6-9, wobei das Verfahren ferner das Verdünnen der Probe um einen Faktor von 1:10 bis 1:320 umfasst.

**11.** Verfahren nach einem der Ansprüche 6-10, wobei die Kontrolle das Reportersignal ist, das bei einer Kontrollprobe erhalten wurde, die keine Coronavirus-neutralisierenden Antikörper umfasst, und das Verfahren das Schlussfolgern umfasst, dass die getestete Probe die SARS-CoV-2-neutralisierenden Antikörper umfasst, wenn das in Schritt (c) erhaltene Reportersignal im Vergleich zur Kontrolle reduziert ist, wobei das Verfahren optional das Bestimmen der Konzentration von SARS-CoV-2-neutralisierenden Antikörpern in der getesteten Probe durch Vergleichen des Reportersignals mit einer Kalibrierkurve, die aus einer seriellen Verdünnung der Kontrollprobe bestimmt wurde, die einen SARS-CoV-2-neutralisierenden Antikörper umfasst, oder ein Molekül, das eine Interaktion des SARS-CoV-2-S-Glykoproteins mit einem Protein blockiert, mit dem das Coronavirus-S-Glykoprotein auf der Zielzelle interagiert, oder eine beliebige Kombination davon umfasst.

**12.** Verfahren nach einem der Ansprüche 6-11, wobei:

(i) das Reportersignal 24 bis 30 Stunden nach Schritt (b) gemessen wird; und/oder
(ii) die Probe in Schritt (a) 30 Minuten lang bei Raumtemperatur mit dem rekombinanten Rhabdovirus-Partikel in Kontakt gebracht wird.

**13.** Verfahren nach einem der Ansprüche 6-12, wobei das Verfahren in einem Hochdurchsatzformat durchgeführt wird.

**14.** Polynukleotid, das für das replikationskompetente, rekombinante Rhabdovirus-Partikel gemäß einem der Ansprüche 1-5 kodiert, wobei das Polynukleotid eine Nukleinsäuresequenz, die für ein Rhabdovirus-Nukleoprotein (N) kodiert, eine Nukleinsäuresequenz, die für ein Rhabdovirus-Phosphoprotein (P) kodiert, die Nukleinsäuresequenz, die für das Rhabdovirus-L-Protein kodiert, die Nukleinsäuresequenz, die für das SARS-CoV-2-S-Glykoprotein oder ein Fragment davon kodiert, und die Nukleinsäuresequenz umfasst, die für das Reporterprotein kodiert, das wie in Anspruch 1 spezifiziert eingefügt ist.

**15.** Kit zum Bestimmen des Vorhandenseins eines SARS-CoV-2-neutralisierenden Antikörpers in einer Probe, umfassend:

a) ein replikationskompetentes, rekombinantes Rhabdovirus-Partikel gemäß den Ansprüchen 1-5 oder das Polynukleotid nach Anspruch 14;
b) eine Zielzelle;
c) optional eine Kontrollprobe, die keine Coronavirus-neutralisierenden Antikörper umfasst;
d) optional eine Kontrollprobe, die einen SARS-CoV-2-neutralisierenden Antikörper oder ein Molekül, das eine Interaktion des SARS-CoV-2-S-Glykoproteins mit einem Protein blockiert, mit dem es auf der Zielzelle interagiert, oder eine beliebige Kombination davon umfasst;
e) optional ein Substrat für das Reporterprotein und
f) optional Gebrauchsanweisungen.

## Revendications

**1.** Particule de rhabdovirus recombinante compétente pour la réplication, dans laquelle (i) la glycoprotéine (G) de rhabdovirus est remplacée par une glycoprotéine de pointe (S) du coronavirus 2 du syndrome respiratoire aigu sévère (SARS-CoV-2) ou un fragment de la glycoprotéine S du SARS-CoV-2 comprenant le domaine de liaison au récepteur de celle-ci, dans laquelle ladite glycoprotéine S du SARS-CoV-2 ou ledit fragment de celle-ci est capable de médier l'infection d'une cellule cible, et dans laquelle (ii) la particule de rhabdovirus comprend une molécule d'acide nucléique comprenant une séquence d'acide nucléique codant pour une protéine rapporteuse, dans laquelle la séquence d'acide nucléique codant pour la protéine rapporteuse est insérée entre une séquence d'acide nucléique codant pour la glycoprotéine S du SARS-CoV-2 ou un fragment de celle-ci, et une séquence d'acide nucléique codant pour une protéine large (L) de rhabdovirus.

2. Particule de rhabdovirus recombinante compétente pour la réplication de la revendication 1, dans laquelle la protéine rapporteuse est une luciférase ou une protéine fluorescente.

3. Particule de rhabdovirus recombinante compétente pour la réplication de la revendication 1 ou 2, dans laquelle la particule de rhabdovirus recombinante est une particule de vésiculovirus recombinante, éventuellement une particule de virus de la stomatite vésiculaire (VSV) recombinante.

4. Particule de rhabdovirus recombinante compétente pour la réplication de l'une quelconque des revendications 1 à 3, dans laquelle :

   (i) la glycoprotéine S du SARS-CoV-2 est une glycoprotéine S pleine longueur du SARS-CoV-2 ; ou
   (ii) le fragment est une glycoprotéine S du SARS-CoV-2 dépourvue des 19 acides aminés C-terminaux tels que spécifiés par rapport à la séquence d'acides aminés de la glycoprotéine S pleine longueur du SARS-CoV-2 de SEQ ID N° : 1 ; ou
   (iii) la glycoprotéine S du SARS-CoV-2 comprend une ou plusieurs insertions, délétions et/ou substitutions d'acides aminés répertoriées dans les tableaux 8 et 9, dans laquelle les positions desdites insertions, délétions et/ou substitutions sont spécifiées par rapport à la séquence d'acides aminés de la glycoprotéine S pleine longueur du SARS-CoV-2 de SEQ ID N° : 1.

5. Particule de rhabdovirus recombinante compétente pour la réplication de l'une quelconque des revendications précédentes, dans laquelle la particule de rhabdovirus recombinante comprend une protéine de matrice (M) de rhabdovirus mutante, éventuellement dans laquelle la particule de rhabdovirus recombinante est une particule de VSV recombinante comprenant la protéine M VSV mutante qui comprend une mutation au niveau de la méthionine 51, éventuellement de la méthionine (M) à l'arginine (R).

6. Procédé permettant la détermination de la présence d'un anticorps neutralisant le SARS-CoV-2 dans un échantillon, le procédé comprenant :

   a) la mise en contact de l'échantillon avec une particule de rhabdovirus recombinante compétente pour la réplication selon l'une quelconque des revendications 1 à 5 ;
   b) après l'étape (a), la mise en contact de la particule de rhabdovirus recombinante compétente pour la réplication avec la cellule cible ;
   c) la mesure d'un signal rapporteur dans la cellule après l'étape (b), et
   d) la comparaison du signal rapporteur mesuré à l'étape (c) à un témoin.

7. Procédé de la revendication 6, dans lequel la cellule cible est une cellule Vero ou une cellule Vero-E6.

8. Procédé de la revendication 6 ou 7, comprenant en outre l'ajout d'un substrat de protéine rapporteuse pour obtenir le signal rapporteur, éventuellement dans lequel la protéine rapporteuse comprend une luciférase et le substrat de protéine rapporteuse comprend un substrat de luciférase de luciférine ou d'EnduRen.

9. Procédé de l'une quelconque des revendications 6 à 8, dans lequel l'échantillon est :

   (i) du sérum ou du plasma ; et/ou
   (ii) est inactivé par la chaleur.

10. Procédé de l'une quelconque des revendications 6 à 9, dans lequel le procédé comprend en outre la dilution de l'échantillon par un facteur de 1:10 à 1:320.

11. Procédé de l'une quelconque des revendications 6 à 10, dans lequel le témoin est le signal rapporteur obtenu avec un échantillon témoin ne comprenant pas d'anticorps neutralisant le coronavirus, et le procédé comprend la conclusion que l'échantillon testé comprend les anticorps neutralisant le SARS-CoV-2 lorsque le signal rapporteur obtenu à l'étape (c) est réduit par rapport au témoin, éventuellement dans lequel le procédé comprend la détermination de la concentration d'anticorps neutralisant le SARS-CoV-2 dans l'échantillon testé en comparant le signal rapporteur à une courbe d'étalonnage déterminée à partir d'une dilution en série de l'échantillon témoin comprenant un anticorps neutralisant le SARS-CoV-2, ou une molécule qui bloque l'interaction de la glycoprotéine S du SARS-CoV-2 avec une protéine avec laquelle la glycoprotéine S du coronavirus interagit sur la cellule cible, ou toute combinaison de ceux-ci.

12. Procédé de l'une quelconque des revendications 6 à 11, dans lequel :

   (i) le signal rapporteur est mesuré entre 24 et 30 heures après l'étape (b) ; et/ou
   (ii) à l'étape (a), l'échantillon est mis en contact avec la particule de rhabdovirus recombinante pendant 30 minutes à température ambiante.

13. Procédé de l'une quelconque des revendications 6 à 12, dans lequel le procédé est réalisé dans un format à haut débit.

14. Polynucléotide codant pour la particule de rhabdovirus recombinante compétente pour la réplication selon l'une quelconque des revendications 1 à 5, dans lequel le polynucléotide comprend une séquence d'acide nucléique codant pour une nucléoprotéine (N) de rhabdovirus, une séquence d'acide nucléique codant pour une phosphoprotéine (P) de rhabdovirus, la séquence d'acide nucléique codant pour la protéine L de rhabdovirus, la séquence d'acide nucléique codant pour la glycoprotéine S du SARS-CoV-2 ou un fragment de celle-ci, et la séquence d'acide nucléique codant pour la protéine rapporteuse insérée comme spécifié dans la revendication 1.

15. Kit permettant la détermination de la présence d'un anticorps neutralisant le SARS-CoV-2 dans un échantillon comprenant :

   a) une particule de rhabdovirus recombinante compétente pour la réplication selon les revendications 1 à 5, ou le polynucléotide de la revendication 14 ;
   b) une cellule cible ;
   c) éventuellement, un échantillon témoin ne comprenant pas d'anticorps neutralisant le coronavirus ;
   d) éventuellement, un échantillon témoin comprenant un anticorps neutralisant le SARS-CoV-2, ou une molécule qui bloque l'interaction de la glycoprotéine S du SARS-CoV-2 avec une protéine avec laquelle elle interagit sur la cellule cible, ou toute combinaison de ceux-ci ;
   e) éventuellement, un substrat pour la protéine rapporteuse, et
   f) éventuellement, des instructions d'utilisation.

FIG. 1

EP 4 136 457 B1

FIG. 2

82

FIG. 3

FIG. 4

EP 4 136 457 B1

**FIG. 5**

**FIG. 6**

FIG. 7

**FIG. 8**

C

Representative distribution of VNUs: PCR-positive cohort

D

| VNU | ARDS | Pneumonia | Fever | Feverish | Chills | Myalgia | Rhinorrhea | Sore Throat | Cough | Dyspnea | Nausea/ | Headache | Abdominal pain | Diarrhea |
|---|---|---|---|---|---|---|---|---|---|---|---|---|---|---|
| 30-100 | 0% | 0% | 38% | 25% | 50% | 50% | 13% | 13% | 50% | 25% | 13% | 50% | 0% | 38% |
| 100-250 | 25% | 0% | 75% | 38% | 63% | 63% | 50% | 38% | 88% | 63% | 13% | 75% | 38% | 25% |
| 250-500 | 14% | 14% | 71% | 14% | 57% | 86% | 29% | 43% | 100% | 86% | 43% | 71% | 43% | 43% |
| 500+ | 25% | 13% | 75% | 38% | 88% | 88% | 38% | 38% | 75% | 63% | 50% | 88% | 25% | 50% |

FIG. 8 (continued)

FIG. 9

| Assay | 1. Thaw and add virus to wells (2,000-10,000 TCID50 units/well)<br>2. Add dilutions of serum (1:100, 1:1000, etc.) – Incubate 30 min at RT<br>3. Add split DSP Vero luciferase Cells ($4 \times 10^4$ cells/well) - Incubate 24-30 hours at 37°C |

1. Add luciferase substrate (EnduRen™)
2. Quantify luciferase signal
3. Calculate antibody titer
4. Report

| Critical Reagents | 1. Qualified viruses (VSV-SARS-CoV2-S-Δ19CT or VSV-SARS-CoV2-S)<br>2. Qualified cells (Vero DSP luciferase)<br>3. Control |

**FIG. 10**

A

B

C

FIG. 11

**D**

**E**

**F**

**FIG. 11 (continued)**

**FIG. 12**

A

VSV-SARS-CoV2-Fluc

| ——— | N | P | M51R | SARS-2-Δ19S | Luc2 | L | ——— |

VSV-SARS-CoV2-gLuc

| ——— | N | P | M51R | SARS-2-Δ19S | gLuc | L | ——— |

B

| 1. Plate cells | → | 2. Incubation | → | 3. Virus neutralization | → | 4. Incubation |

| 5. Infection | → | 6. Incubation | → | 7. Substrate Addition/Read plate | → | 8. Interpret/report |

FIG. 13

FIG. 14

FIG. 15

FIG. 16

FIG. 17

**FIG. 18**

**FIG. 19**

**FIG. 20**

**FIG. 21**

FIG. 22A

FIG. 22B

# Assay: IMMUNO-COV DBS

Plate: ____ of ____

U-well plate

|   | 1 | 2 | 3 | 4 | 5 | 6 | 7 | 8 | 9 | 10 | 11 | 12 |
|---|---|---|---|---|---|---|---|---|---|----|----|----|
| A | S1 1:4 | S1 1:8 | S1 1:16 | S1 1:32 | S1 1:64 | S1 1:128 | S4 1:4 | S4 1:8 | S4 1:16 | S4 1:32 | S4 1:64 | S4 1:128 |
| B | S2 1:4 | S2 1:8 | S2 1:16 | S2 1:32 | S2 1:64 | S2 1:128 | S5 1:4 | S5 1:8 | S5 1:16 | S5 1:32 | S5 1:64 | S5 1:128 |
| C | S3 1:4 | S3 1:8 | S3 1:16 | S3 1:32 | S3 1:64 | S3 1:128 | S6 1:4 | S6 1:8 | S6 1:16 | S6 1:32 | S6 1:64 | S6 1:128 |
| D | ST1 | ST2 | ST3 | ST4 | ST5 | ST6 | NC | PC | Media | | | |
| E | | | | | | | | | | | | |
| F | | | | | | | | | | | | |
| G | | | | | | | | | | | | |
| H | | | | | | | | | | | | |

| | |
|---|---|
| Tech: | |
| Date: | |
| Time virus added: | |
| Virus lot: | |

**FIG. 23**

**Assay Plate**

| | 1 | 2 | 3 | 4 | 5 | 6 | 7 | 8 | 9 | 10 | 11 | 12 |
|---|---|---|---|---|---|---|---|---|---|---|---|---|
| A | S1 1:4 | S1 1:8 | S1 1:16 | S1 1:32 | S1 1:64 | S1 1:128 | S4 1:4 | S4 1:8 | S4 1:16 | S4 1:32 | S4 1:64 | S4 1:128 |
| B | S1 1:4 | S1 1:8 | S1 1:16 | S1 1:32 | S1 1:64 | S1 1:128 | S4 1:4 | S4 1:8 | S4 1:16 | S4 1:32 | S4 1:64 | S4 1:128 |
| C | S2 1:4 | S2 1:8 | S2 1:16 | S2 1:32 | S2 1:64 | S2 1:128 | S5 1:4 | S5 1:8 | S5 1:16 | S5 1:32 | S5 1:64 | S5 1:128 |
| D | S2 1:4 | S2 1:8 | S2 1:16 | S2 1:32 | S2 1:64 | S2 1:128 | S5 1:4 | S5 1:8 | S5 1:16 | S5 1:32 | S5 1:64 | S5 1:128 |
| E | S3 1:4 | S3 1:8 | S3 1:16 | S3 1:32 | S3 1:64 | S3 1:128 | S6 1:4 | S6 1:8 | S6 1:16 | S6 1:32 | S6 1:64 | S6 1:128 |
| F | S3 1:4 | S3 1:8 | S3 1:16 | S3 1:32 | S3 1:64 | S3 1:128 | S6 1:4 | S6 1:8 | S6 1:16 | S6 1:32 | S6 1:64 | S6 1:128 |
| G | ST1 | ST2 | ST3 | ST4 | ST5 | ST6 | NC | PC | Media | | | |
| H | ST1 | ST2 | ST3 | ST4 | ST5 | ST6 | NC | PC | Media | | | |

| | |
|---|---|
| Tech: | |
| Date: | |
| Time start (T=0h): | |
| Plate label: | |
| Read by: | |
| Date read: | |
| Time read: | |
| Read program: | |
| File    name: | |

**FIG. 24**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5071743 A
- US 5789166 A
- US 5932419 A
- US 5702931 A
- US 5780270 A
- US 6242222 B
- US 7153510 B
- US 9861668 B
- US 8012489 B
- US 9630996 B
- US 8287878 B
- US 9248178 B
- US 20140271564 A
- US 20120121650 A
- US 4683202 A
- US 4683195 A
- US 4889818 A
- WO 9806737 A
- WO 9711094 A
- WO 9623810 A
- WO 2019147831 A

### Non-patent literature cited in the description

- **ZHOU et al.** *Nature*, 03 February 2020
- **ZHU et al.** *New Engl J Med*, 2020, vol. 382, 727-733
- **WÖLFEL et al.** *Nature*, 01 April 2020
- **NIE et al.** *Emerg Microbes Infect.*, 2020, vol. 9 (1), 680-686
- **BECERRA-FLORES** ; **CARDOZO**. SARS-CoV-2 viral spike G614 mutation exhibits higher case fatality rate. *The International Journal of Clinical Practice*, 06 May 2020
- **EAASWARKHANTH et al.** Could the D614G substitution in the SARS-CoV-2 spike (S) protein be associated with higher COVID-19 mortality?. *International Journal of Infectious Diseases*, July 2020, vol. 96, 459-460
- **TANG et al.** The SARS-CoV-2 Spike Protein D614G Mutation Shows Increasing Dominance and May Confer a Structural Advantage to the Furin Cleavage Domain. *Preprints*, 2020
- **HANSEN**. Studies in humanized mice and convalescent humans yield a SARS-CoV-2 antibody cocktail. *Science*, 15 June 2020
- **LOKMAN et al.** Exploring the genomic and proteomic variations of SARS-CoV-2 spike glycoprotein: A computational biology approach. *Infection, Genetics and Evolution : Journal of Molecular Epidemiology and Evolutionary Genetics in Infectious Diseases*, June 2020, vol. 84, 104389
- **SCHORPP et al.** *Nucl. Acids Res.*, 1996, vol. 24 (9), 1787-1788
- **BYUN et al.** *Biochem. Biophys. Res. Comm*, 2005, vol. 332 (2), 518-523
- **ADDISON et al.** *J. Gen. Virol.*, 1997, vol. 78 (7), 1653-1661
- **HUNNINGHAKE et al.** *J. Virol.*, 1989, vol. 63 (7), 3026-3033
- **STINSKI** ; **ROEHR**. *J. Virol.*, 1985, vol. 55 (2), 431-441
- **CUI et al.** *Nature Reviews Microbiology*, 2019, vol. 17, 181-192
- **FUNG et al.** *Annu. Rev. Microbiol.*, 2019, vol. 73, 529-557
- **WALLS et al.** *Cell*, 09 March 2020
- **HOFFMANN et al.** *Cell*, 2020, vol. 181, 1-10
- **ANDERSEN et al.** *Nature Medicine*, 2020
- **SUN et al.** *Future Virol.*, 2010, vol. 5 (1), 85-96
- **AURÉLIE et al.** *Viruses*, 2012, vol. 4, 117-139
- **SAMBROOK** ; **FRITSCH** ; **MANIATIS**. Molecular Cloning: A Laboratory Manual. Cold Spring Harbor Laboratory Press, 1989
- DNA Cloning: A Practical Approach. 1985, vol. 1, 2
- Oligonucleotide Synthesis. 1984
- Nucleic Acid Hybridization. 1985
- Transcription And Translation. 1984
- Animal Cell Culture. 1986
- Immobilized Cells And Enzymes. IRL Press, 1986
- **B. PERBAL**. *A Practical Guide To Molecular Cloning*, 1984
- Current Protocols in Molecular Biology. John Wiley & Sons, Inc., 1994
- **KUNKEL**. *Proc. Natl. Acad. Sci. USA*, 1985, vol. 82, 488-492
- **FUKUOKA et al.** *Biochem. Biophys. Res. Commun.*, 1999, vol. 263, 357-360

- **KIM** ; **MAAS**. *BioTech.*, 2000, vol. 28, 196-198
- **PARIKH** ; **GUENGERICH**. *BioTech*, 1998, vol. 24 (4), 28-431
- **RAY** ; **NICKOLOFF**. *BioTech.*, 1992, vol. 13, 342-346
- **WANG et al.** *BioTech.*, 1995, vol. 19, 556-559
- **WANG** ; **MALCOLM**. *BioTech.*, 1999, vol. 26, 680-682
- **XU** ; **GONG**. *BioTech.*, 1999, vol. 26, 639-641
- **HOGREFE**. *Strategies*, 2001, vol. l4 (3), 74-75
- **ANGAG** ; **SCHUTZ**. *Biotech*, 2001, vol. 30, 486-488
- **WANG** ; **WILKINSON**. *Biotech*, 2000, vol. 29, 976-978
- **KANG et al.** *Biotech*, 1996, vol. 20, 44-46
- **OGEL** ; **MCPHERSON**. *Protein Engineer.*, 1992, vol. 5, 467-468
- **KIRSCH** ; **JOLY**. *Nucl. Acids. Res.*, 1998, vol. 26, 1848-1850
- **RHEM** ; **HANCOCK**. *J. Bacteriol.*, 1996, vol. 178, 3346-3349
- **BOLES** ; **MIOGSA**. *Curr. Genet.*, 1995, vol. 28, 197-198
- **BARRENTTINO et al.** *Nuc. Acids. Res.*, 1993, vol. 22, 541-542
- **TESSIER** ; **THOMAS**. *Meths. Molec. Biol.*, vol. 57, 229-237
- **PONS et al.** *Meth. Molec. Biol.*, vol. 67, 209-218
- **FUKISHI et al.** *J. Gen. Virol.*, 2005, vol. 86, 2269-2274
- **BELOT, L. et al.** Structural and cellular biology of rhabdovirus entry. *Adv. Virus Res.*, 2019, vol. 104, 147-183
- **ALBERTINI, A.A.V. et al.** Molecular and Cellular Aspects of Rhabdovirus Entry. *Viruses*, 2012, vol. 4, 117-139
- **SUN, X. et al.** Internalization and fusion mechanism of vesicular stomatitis virus and related rhabdoviruses. *Future Virol.*, 2010, vol. 5 (1), 85-96
- **IGONET, S. et al.** SnapShot: Viral and Eukaryotic Protein Fusogens. *Cell*, 2012, vol. 151, 1634e1
- **KONDO, N. et al.** Conformational changes of the HIV-1 envelope protein during membrane fusion are inhibited by the replacement of its membrane-spanning domain. *Journ. Biol. Chem.*, 2010, vol. 285 (19), 14681-88
- **ROSE** ; **SCHUBERT**. The Viruses: The Rhabdoviruses. Plenum Press, 1987, 129-166
- **ROSE** ; **GALLIONE**. *J. Virol.*, 1981, vol. 39 (2), 519-528
- **MULLIS** ; **FALOONA**. *Meth. Enzymol.*, 1987, vol. 155, 335-350
- **BERNOIST** ; **CHAMBON**. *Nature*, 1981, vol. 290, 304-310
- **YAMAMOTO et al.** *Cell*, 1980, vol. 22, 787-797
- **WAGNER et al.** *Proc. Natl. Acad. Sci. U.S.A.*, 1981, vol. 78, 1441-1445
- **BRINSTER et al.** *Nature*, 1982, vol. 296, 39-42
- **SWIFT et al.** *Cell*, 1984, vol. 38, 639-646
- **ORNITZ et al.** *Cold Spring Harbor Symp. Quant. Biol.*, 1986, vol. 50, 399-409
- **MACDONALD**. *Hepatology*, 1987, vol. 7, 425-515
- **HANAHAN**. *Nature*, 1985, vol. 315, 115-122
- **GROSSCHEDL et al.** *Cell*, 1984, vol. 38, 647-658
- **ADAMES et al.** *Nature*, 1985, vol. 318, 533-538
- **ALEXANDER et al.** *Mol. Cell. Biol.*, 1987, vol. 7, 1436-1444
- **LEDER et al.** *Cell*, 1986, vol. 45, 485-495
- **PINKERT et al.** *Genes and Devel.*, 1987, vol. 1, 268-276
- **KRUMLAUF et al.** *Mol. Cell. Biol.*, 1985, vol. 5, 1639-1648
- **HAMMER et al.** *Science*, 1987, vol. 235, 53-58
- **KELSEY et al.** *Genes and Devel.*, 1987, vol. 1, 161-171
- **MOGRAM et al.** *Nature*, 1985, vol. 315, 338-340
- **KOLLIAS et al.** *Cell*, 1986, vol. 46, 89-94
- **READHEAD et al.** *Cell*, 1987, vol. 48, 703-712
- **SANI**. *Nature*, 1985, vol. 314, 283-286
- **PERROTTA** ; **BEEN**. *Nature*, 1991, vol. 350, 434-436
- **PATTNAIK et al.** *Cell*, 1992, vol. 69, 1011-1020
- **ANDERSON et al.** *Nature Medicine*, 2020
- **GYLLENSTEIN et al.** *Proc. Natl. Acad. Sci. USA*, 1988, vol. 85, 7652-7656
- **OCHMAN et al.** *Genetics*, 1988, vol. 120, 621-623
- **LOH et al.** *Science*, 1989, vol. 243, 217-220
- **MULLIS** ; **FALOONA**. *Methods in Enzymology*, 1987, vol. 155, 335-350
- **FUERST et al.** *Proc. Natl. Acad. Sci. U.S.A.*, 1986, vol. 83, 8122-8126
- **DANIEL et al.** *Int. J. Cancer*, 1988, vol. 41, 601-608
- **ARTHUR et al.** *J. Cell. Biochem.*, 1986 (10A), 226
- **MATZ, M. V. et al.** *Nature Biotechnology*, vol. 17, 969-973
- **HEIM et al.** *Proc. Natl. Acad. Sci.*, 1994, vol. 91 (26), 12501-12504
- **HEIM, R.** ; **TSIEN, R. Y.** *Curr. Biol.*, 1996, vol. 6, 178-182
- **OKBA, N.M.A. et al.** SARS-CoV-2 specific antibody responses in COVID-19 patients. *Emerg. Infect. Dis.*, 2020, vol. 26 (7)

- Evaluating neutralizing antibodies against HIV, SIV, and SHIV in luciferase reporter gene assays. **MONTEFIORI, D.C.** Curr. Protoc. Immunol.. 2005
- **FUKUSKI et al.** *Journal of General Virology*, 2005, vol. 86, 2269-74
- **SAW, W.T. et al.** *Methods*, 2015, vol. 90, 68-75
- **MONTEFIORI, D.C.** Current Protocols in Immunology. 2004
- **WANG et al.** *Nat Commun*, 2020, vol. 11, 2251
- **OKBA et al.** *Emerg Infect Dis.*, July 2020
- **RANAWAKA et al.** *Euro Surveill.*, 2020, vol. 25 (16)
- **JOHN W. TUKEY**. *Journal of Computational and Graphical Statistics*, 1993, vol. 2, 1-33
- **SHAPIRO, S.S.** ; **WILK, M.B.** *Biometrika*, 1965, vol. 52 (3-4), 591-611
- **ANDERSON, T.W.** ; **DARLING, D.A.** A Test of Goodness of Fit. *Journal of the American Statistical Association*, 1954, vol. 49 (268), 765-769
- **CASADEVALL A, PIROFSKI LA**. The convalescent sera option for containing COVID-19. *J Clin Invest*, 13 March 2020, vol. 379
- **CORMAN et al.** Viral shedding and antibody response in 37 patients with Middle East respiratory syndrome coronavirus infection. *Clin Infect Dis*, 2016, vol. 62, 477-483
- **GUI, M et al.** Cryo-electron microscopy structure of the SARS-CoV spike flycoprotein reveal a prerequisite conformational state for receptor binding. *Cell Res.*, 2017, vol. 27, 119-129
- **HO M.S.** ; **CHEN W.J.** ; **CHEN H.Y**. Neutralizing antibody response and SARS severity. *Emerg Infect Dis.*, 2005, vol. 11 (11), 1730-1737
- **HOFFMANN, M., KLEINE-WEBER, H., SCHROEDER, S., KRUGER, N., HERRLER, T., ERICHSEN, S.,SCHIERGENS, T.S., HERRLER, G., WU, N.H., NITSCHE, A.** SARS-CoV-2 Cell Entry Depends on ACE2 and TMPRSS2 and Is Blocked by a Clinically Proven Protease Inhibitor. *Cell*, 2020, vol. 181, 271-280
- **JAUME M.** ; **YIP M.S.** ; **CHEUNG C.Y**. Anti-severe acute respiratory syndrome coronavirus spike antibodies trigger infection of human immune cells via a pH- and cysteine protease-independent FcgammaR pathway. *J Virol.*, 2011, vol. 85 (20), 10582-10597
- **KOFF WC** ; **BURTON DR** ; **JOHNSON PR et al.** Accelerating next-generation vaccine development for global disease prevention. *Science*, 2013, vol. 340 (6136)
- **LIU, W.** ; **FONTANET, A.** ; **ZHANG, P.H.** ; **ZHAN, L.** ; **XIN, Z.T.** ; **BARIL, L.** ; **TANG, F.** ; **LV, H.** ; **CAO, W.C.** Two-year prospective study of the humoral immune response of patients with severe acute respiratory syndrome. *J. Infect. Dis.*, 2006, vol. 193, 792-795
- **LIU, L et al.** Anti-spike IgG causes severe acute lung injury by skewing macrophage responses during acute ARS-CoV infection. *JCI Insight*, 2019, vol. 4 (4), e123158
- **NIE Y** ; **WANG G** ; **SHI X** ; **ZHANG H** ; **QIU Y** ; **HE Z et al.** Neutralizing antibodies in patients with severe acute respiratory syndrome-associated coronavirus infection. *J Infect Dis.*, 2004, vol. 190, 1119-26
- **PEIRIS J.S.** ; **CHU C.M.** ; **CHENG V.C**. Clinical progression and viral load in a community outbreak of coronavirus-associated SARS pneumonia: a prospective study. *Lancet*, 2003, vol. 361 (9371), 1767-1772
- **WALLS, A.C., PARK, Y.J., TORTORICI, M.A., WALL, A., MCGUIRE, A.T., AND VEESLER, D.** Structure, Function, and Antigenicity of the SARS-CoV-2 Spike Glycoprotein. *Cell*, 2020, vol. 181, 281-292
- **WAN, Y.** ; **SHANG, J.** ; **GRAHAM, R.** ; **BARIC, R.S.** ; **LI, F.** Receptor recognition by novel coronavirus from Wuhan: An analysis based on decade-long structural studies of SARS. *Journal of Virology*, 2020
- **WANG S.F.** ; **TSENG S.P.** ; **YEN C.H**. Antibody-dependent SARS coronavirus infection is mediated by antibodies against spike proteins. *Biochem Biophys Res Commun.*, 2014, vol. 451 (2), 208-214
- **YIP M.S.** ; **LEUNG H.L.** ; **LI P.H**. Antibody-dependent enhancement of SARS coronavirus infection and its role in the pathogenesis of SARS. *Hong Kong Med J.*, 2016, vol. 22 (3), 25-31
- **YUAN, Y. et al.** Cryo-EM structure of MERS-CoV and SARS-CoV spike flyocpoteins reveal the dynamic receptor binding domains. *Natu. Commun.*, 2017, vol. 8, 15092
- **ZHAO J et al.** Recovery from the Middle East respiratory syndrome is associated with antibody and T-cell responses. *Sci Immunol*, 2017, vol. 2, 5393
- **WEISBLUM Y** ; **SCHMIDT F** ; **ZHANG F et al.** Escape from neutralizing antibodies by SARS-CoV-2 spike protein variants. *eLife*, 2020, vol. 9, e61312
- **RESENDE PC** ; **BEZERRA JF** ; **DE VASCONCELOS RHT**. *Spike E484K mutation in the first SARS-CoV-2 reinfection case confirmed in Brazil*, 2020, www.virological.orgexternal icon
- **ADRIAN A. PATER et al.** *Emergence and Evolution of a Prevalent New SARS-CoV-2 Variant in the United States*